# EUROPEAN PATENT APPLICATION

(11) **EP 3 827 837 A1**
(43) Date of publication of application: **02.06.2021**
(21) Application number: 19841098.7
(22) Date of filing: 24.07.2019
(51) Int. Cl.: A61K 38/17, C07K 14/705, C12Q 1/6886, A61P 35/00, A61P 29/00, A61P 11/06, A61P 37/06

(54) **COMPOSITION FOR PREVENTING OR TREATING IMMUNE-RELATED DISEASES**

(30) Priority: 24.07.2018 KR 20180085908
(71) Applicant: Good T Cells, Inc., Seoul 03722 (KR)
(72) Inventor: KIM, Jung Ho, Seoul 04136 (KR); KIM, Beom Seok, Seoul 04029 (KR)
(74) Representative: Schnappauf, Georg
(86) International application number: PCT/KR2019/009202
(87) International publication number: WO 2020/022782

(57) **Abstract**

The present invention relates to a composition for preventing, ameliorating, or treating an immune-related disease, comprising, as an active ingredient, a fusion protein that includes an extracellular domain of leucine-rich and immunoglobulin-like domains-1 (Lrig-1) protein and an immunoglobulin Fc region. The fusion protein provided in the present invention interacts with a ligand for the Lrig-1 protein, which is present in activated T cells, to give a co-inhibitory signal to the activated T cells, so that death or suppressed activity of the activated T cells is induced, which makes it possible to effectively prevent, ameliorate, or treat an immune-related disease.

## Description

### Technical Field

The present invention relates to a composition capable of effectively preventing or treating immune-related diseases.

### Background Art

Immunoglobulin comprises four polypeptide chains, that is, two heavy chains and two light chains which are associated with each other via interchain disulfide bonds. Each light chain has two domains, that is, a variable light domain (VL) and a constant light domain (CL); and each heavy chain has two regions, that is, a variable heavy region (VH) and a constant heavy region (CH). The constant heavy region (CH) consists of constant heavy regions (for example, CH1, CH2, CH3, and the like) designated by the number (see, for example, US 6,086,875 (Blumberg R. S. etal.), US 5,624,821 (Winter G. P. etal.), and US 5,116,964 (Capon D. J. and Lasky L. A.)). Immunoglobulins are classified into different isotypes (that is, IgG, IgM, IgA, IgD, and IgE) based on their biological properties, location within an organism, and ability to process different antigens. Depending on the immunoglobulin isotype, the constant heavy region (CH) may have 3 or 4 CH domains. In addition, in some isotypes (IgA, IgD, and IgG), the heavy chain contains a hinge region that adds flexibility to the molecule (Janeway et al. 2001, Immunobiology, Garland Publishing, N.Y, N.Y).

In humans, there are four IgG subclasses (IgG1, 2, 3, and 4), and these IgGs are named in the order of their abundance in serum (that is, IgG1 is most abundant). The IgG isotype consists of two light chains and two heavy chains, in which each heavy chain includes three constant heavy domains (CH1, CH2, and CH3). The two heavy chains of IgG are linked to each other by disulfide bonds (-S-S-) and each heavy chain is linked to a light chain by disulfide bonds. The antigen binding site of IgG is located in the fragment antigen binding region (Fab region) that includes the variable domains of light chain (VL) and heavy chain (VH) as well as the constant domains of light chain (CL) and heavy chain (CH1). The fragment crystallizable region (Fc region) of IgG is a portion of the heavy chain containing the CH2 and CH3 domains which bind to Fc receptors found on the surface of certain cells, including a neonatal Fc receptor (FcRn). The heavy chain of IgG also has a hinge region between CH1 and CH2 which separates the Fab region from the Fc region and participates in linking the two heavy chains together via disulfide bonds. The structure of the hinge region contributes to unique biological properties of each of the four IgG subclasses.

IgG is secreted as a monomer that is small in size, allowing it to easily perfuse tissues. It is the only isotype that has a receptor (neonatal Fc receptor (FcRn)) to facilitate passage through the human placenta, thereby providing protection to the fetus in utero. IgG absorbed through the placenta provides the neonate with humoral immunity before its own immune system develops.

The IgG neonatal Fc receptor binding site is located in the Fc region of the antibody. FcRn is normally expressed in human placenta and epithelial cells and participates in an endocytic salvage pathway that prevents degradation of IgG. This salvage pathway is mediated by the highly pH-dependent binding affinity of IgG to FcRn at acidic pH. The high affinity of IgG to FcRn at acidic pH is believed to result in binding of internalized IgG to FcRn following its uptake into acidic endosomes (Goebl N A et al., 2008; Junghans R P et al., 1996). Most soluble proteins are directed to lysosomes after internalization; however, internalized FcRn-bound IgG returns to the plasma membrane and is effectively rescued from the underlying degradation pathway. Upon exposure to neutral pH in the extracellular space, IgG can dissociate from FcRn and return to the circulation. Thus, the extended serum half-life property of the antibody is retained in the Fc fragment.

The salvage pathway provides one mechanism for developing next-generation protein drugs with an extended half-life in blood circulation as compared with unmodified protein drugs. In particular, unmodified protein drugs have a short circulating half-life, and thus require frequent administration over a long-term treatment period that is needed. Extensive efforts have been made to extend the half-life of the protein drugs using a large number of approaches including PEGylation and fusion-protein technology (U.S. Food and Drug Administration; Osborn BL et al., 2002); however, these efforts have not produced ideal results.

The immune disease is a disease in which components of the mammalian immune system cause, mediate, or otherwise contribute to pathological conditions in a mammal. In particular, an inflammatory disorder is one of the most important health problems in the world. Inflammation is usually a localized protective response of body tissues against host invasion by foreign substances or harmful stimuli. Causes of inflammation include infectious agents such as bacteria, viruses, and parasites; physical factors such as burns or irradiation; chemicals such as toxins, drugs, or industrial agents; immunological responses such as allergies and autoimmune responses; or conditions associated with oxidative stress.

Inflammation is characterized by pain, redness phenomenon, swelling, heat, and eventual loss of function in an affected area. These symptoms are results of a series of complex interactions between cells in the immune system. Cellular responses result in a network of interactions between several groups of inflammatory mediators: proteins (for example, cytokine, enzyme (for example, protease, peroxidase)), major basic proteins, adhesion molecules (ICAM, VCAM), lipid mediators (for example, eicosanoid, prostaglandin, leukotriene, platelet activating factor (PAF)), reactive oxygen species (for example, hydroperoxide, superoxide anion O2-, nitric oxide (NO), and the like). However, most of these inflammatory mediators are also regulators of normal cellular activity. Thus, lack of inflammatory responses causes a host to be in an uncontrolled state so that the host is damaged (that is, infected). As a result, chronic inflammation partially leads to overproduction of several of the aforementioned mediators, by which an inflammatory disease is caused.

In addition, autoimmune disease, which is one of immune diseases, is characterized by spontaneous responses caused by the immune system attacking its own organs. These responses are due to recognition of auto-antigens by T lymphocytes, which causes a humoral immune response (production of autoantigens) and a cellular immune response (increased cytotoxic activity of lymphocytes and macrophages). Examples of the autoimmune disease may include: rheumatic diseases, psoriasis, systemic dermatomyositis, multiple sclerosis, lupus erythematosus, or deterioration of immune responses by antigens, that is, asthma, drug or food allergies, and the like. All of these diseases are restrictive and chronic diseases, which are fatal in some cases. To date, there is no effective treatment method that can treat the aforementioned diseases. Therefore, drugs, medicines, or media capable of reducing or alleviating these diseases during progression thereof will be an important solution for a patient's health.

### Technical Problem

Many researchers have been making intensive efforts to find suitable drugs and methods by searching for treatment methods for autoimmune diseases. Today, treatment of autoimmune diseases is mainly based on use of immunosuppressive drugs such as glucocorticoids, calcineurin inhibitors, and antiproliferatives-antimetabolites. However, such pharmacological therapy acts on a variety of targets, and thus may result in decreased immune function as a whole. Otherwise, in a case where this pharmacological therapy is used for a long time, various cytotoxic actions cause problems and suppress the immune system in a nonspecific manner, so that a patient may be at risk for infectious diseases or immune-related diseases. Calcineurin and glucocorticoids may pose other problems due to their nephrotoxicity and diabetogenic properties, and thus their use is restricted in a case where there are some clinical symptoms (for example, renal insufficiency, diabetes, and the like).

Therefore, as a substance capable of treating immune-related diseases such as autoimmune diseases, inflammatory diseases, and transplant rejection, there is a need to develop a new therapeutic agent that exhibits an excellent therapeutic effect with no adverse effects.

### Solution to Problem

An object of the present invention is to provide a composition for preventing, ameliorating, or treating an immune-related disease, comprising, as an active ingredient, a fusion protein that includes an extracellular domain of leucine-rich and immunoglobulin-like domains-1 (Lrig-1) protein and an immunoglobulin Fc region.

Another object of the present invention is to provide a method for preventing, ameliorating, or treating the immune-related disease.

However, the technical problem to be achieved by the present invention is not limited to the above-mentioned problems, and other problems that are not mentioned will be clearly understood by those of ordinary skill in the art from the following description.

### 1. Composition for preventing, ameliorating, or treating immune-related disease

In an embodiment of the present invention, there is provided a composition for preventing, ameliorating, or treating an immune-related disease.

The composition of the present invention can be used as a pharmaceutical composition; a food composition; or a cosmetic composition.

The composition of the present invention comprises, as an active ingredient, a fusion protein that includes an extracellular domain of leucine-rich and immunoglobulin-like domains-1 (Lrig-1) protein and an immunoglobulin Fc region; a nucleic acid molecule encoding the same; an expression vector containing the nucleic acid molecule; or a host cell transformed with the expression vector.

Hereinafter, each configuration of the present invention will be described in detail.

### Active ingredient

### 1) Fusion protein

### Extracellular domain of Lrig-1 protein

In the present invention, the Lrig-1 protein is a transmembrane protein consisting of 1091 amino acids present on the surface of regulatory T immune cells, and is composed of leucine-rich repeats (LRRs) and three immunoglobulin-like domains on the extracellular or lumen side, a cell transmembrane sequence, and a cytoplasmic tail portion. The LRIG gene family includes LRIG1, LRIG2, and LRIG3, and the amino acids therebetween are highly conserved. The LRIG1 gene is highly expressed in normal skin and can be expressed in basal and hair follicle cells to regulate proliferation of epithelial stem cells.

In an example of the present invention, the extracellular domain of the Lrig-1 protein may be an extracellular domain of Lrig-1 protein derived from mammals, including primates such as humans and monkeys, rodents such as mice and rats, and the like. For the purposes of the present invention, the extracellular protein of the Lrig-1 protein may be, but is not limited to, an extracellular domain of Lrig-1 protein derived from humans or mice.

In an example of the present invention, the extracellular domain of the Lrig-1 protein may be represented by, but is not limited to, SEQ ID NO: 1 that corresponds to a sequence of amino acids 35 to 794 of the human-derived Lrig-1 protein and can be encoded by the nucleic acid sequence represented by SEQ ID NO: 2 (see Table 1).

**[Table 1]**

| SEQ ID NO | Sequence information |
|---|---|
| SEQ ID NO: 1 | AGPRAPCAAACTCAGDS LDCGGRGLAA LPGDLPSWTRS LNLSYNKLSE I DPAGFEDLPNLQEVYLN NNEL TAVPS LGAASSHVVS LFLQHNKI RSVEGSQLKAYLS LEVLDLSLNNI TEVRNTCFPHGPPI K ELNLAGNRI GTLELGAFDGLSRSLL TLRLSKNRI TQLPVRAFKLPRLTQLDLNRNRI RLI EGLTFQ GLNS LEVLKLQRNNI SKLTDGAFWGLSKMHVLHLEYNS LVEVNSGSLYGLTALHQLHLSNNSI ARI HRKGWSFCQKLHELVLSFNNLTRLDEESLAELSSLSVLRLSHNSI SHI AEGAFKGLRSLRVLDLDH NEI SGTI EDTSGAFSGLDSLSKLTLFGNKI KSVAKRAFSGLEGLEHLNLGGNAI RSVQFDAFVKMK NLKELHI SSDSFLCDCQLKWLPPWLI GRMLQAFVTATCAHPESLKGQSI FSVPPESFVCDDFLKPQ I I TQPETTMAMVGKDI RFTCSAASSSSSPMTFAWKKDNEVLTNADMENFVHVHAQDGEVMEYTTI L HLRQVTFGHEGRYQCVI TNHFGSTYSHKARLTVNVLPSFTKTPHDI TI RTTTVARLECAATGHPNP QI AWQKDGGTDFPAARERRMHVMPDDDVFFI TDVKI DDAGVYSCTAQNSAGSI SANATLTVLETPS LVVPLEDRVVSVGETVALQCKATGNPPPRI TWFKGDRPLSLTERHHLTPDNQLLVVQNVVAEDAGR YTCEMSNTLGTERAHSQLSVLPAAGCRKDGTTVGI F |
| SEQ ID NO:2 | GCTGGGCCTCGGGCTCCTTGTGCTGCCGCCTGCACATGTGCAGGCGATTCCCTGGACTGCGGCGGC AGAGGCCTGGCCGCCCTGCCTGGCGATCTGCCATCCTGGACCCGGAGCCTGAACCTGAGCTACAAC AAGCTGAGCGAGATCGATCCCGCCGGCTTTGAGGACCTGCCTAACCTGCAGGAGGTGTATCTGAAC AATAACGAGCTGACCGCGGTACCatccctgggcgctgcttcatcacatgtcgtctctctctttctg cagcacaacaagattcgcagcgtggaggggagccagctgaaggcctacctttccttagaagtgtta gatctgagtttgaacaacatcacggaagtgcggaacacctgctttccacacggaccgcctataaag gagctcaacctggcaggcaatcggattggcaccctggagttgggagcatttgatggtctgtcacgg tcgctgctaactcttcgcctgagcaaaaacaggatcacccagcttcctgtaagagcattcaagcta cccaggctgacacaactggacctcaatcggaacaggattcggctgatagagggcctcaccttccag gggctcaacagcttggaggtgctgaagcttcagcgaaacaacatcagcaaactgacagatggggcc ttctgggggactgtccaagatgcatgtgctgcacctggagtacaacagcctggtagaagtgaacagc ggctcgctctacggcctcacggccctgcatcagctccacctcagcaacaattccatcgctcgcatt caccgcaagggctggagcttctgccagaagctgcatgagttggtcctgtccttcaacaacctgaca cggctggacgaggagagcctggccgagctgagcagcctgagtgtcctgcgtctcagccacaattcc atcagccacattgcggagggtgccttcaagggactcaggagcctgcgagtcttggatctggaccat aacgagatttcgggcacaatagaggacacgagcggcgccttctcagggctcgacagcctcagcaag ctgactctgtttggaaacaagatcaagtctgtggctaagagagcattctcggggctggaaggcctg gagcacctgaaccttggagggaatgcgatcagatctgtccagtttgatgcctttgtgaagatgaag aatcttaaagagctccatatcagcagcgacagcttcctgtgtgactgccagctgaagtggctgccc ccgtggctaattggcaggatgctgcaggcctttgtgacagccacctgtgcccacccagaatcactg aagggtcagagcattttctctgtgccaccagagagtttcgtgtgcgatgacttcctgaagccacag atcatcacccagccagaaaccaccatggctatggtgggcaaggacatccggtttacatgctcagca gccagcagcagcagctccccatgacctttgcctggaagaaagacaatgaagtcctgaccaatgca gacatggagaactttgtccacgtccacgcgcaggacggggaagtgatggagtacaccaccatcctg cacctccgtcaggtcactttcgggcacgagggccgctaccaatgtgtcatcaccaaccactttggc tccacctattcacataaggccaggctcaccgtgaatgtgttgccatcattcaccaaaacgccccac gacataaccatccggaccaccaccgtggcccgcctcgaatgtgctgccacaggtcacccaaaccct cagattgcctggcagaaggatggaggcacggatttccccgctgcccgtgagcgacgcatgcatgtc atgccggatgacgacgtgtttttcatcactgatgtgaaaatagatgacgcaggggtttacagctgt actgctcagaactcagccggttctatttcagctaatgccaccctgactgtcctagagaccccatcc ttggtggtccccttggaagaccgtgtggtatctgtgggagaaacagtggccctccaatgcaaagcc acggggaaccctccgccccgcatcacctggttcaaggggggaccgcccgctgagcctcactgagcgg caccacctgacccctgacaaccagctcctggtggttcagaacgtggtggcagaggatgcgggccga tatacctgtgagatgtccaacaccctgggcacggagcgagctcacagccagctgagcgtcctgccc gcagcaggctgcaggaaggatgggaccacggtaggcatcttc |

In another example of the present invention, the extracellular domain of the Lrig-1 protein may be represented by, but is not limited to, SEQ ID NO: 3 that corresponds to a sequence of amino acids 35 to 794 of the mouse-derived Lrig-1 protein and can be encoded by the nucleic acid sequence represented by SEQ ID NO: 4 (see Table 2).

**[Table 2]**

| SEQ ID NO | Sequence information |
|---|---|
| SEQ ID NO: 3 | AQAGPRAPCA AACTCAGDSL DCSGRGLATL PRDLPSWTRS LNLSYNRLSE IDSAAFEDLT NLQEVYLNSN ELTAIPSLGA ASIGVVSLFL QHNKILSVDG SQLKSYLSLE VLDLSSNNIT EIRSSCFPNG LRIRELNLAS NRISILESGA FDGLSRSLLT LRLSKNRITQ LPVKAFKLPR LTQLDLNRNR IRLIEGLTFQ GLDSLEVLRL QRNNISRLTD GAFWGLSKMH VLHLEYNSLV EVNSGSLYGL TALHQLHLSN NSISRIQRDG WSFCQKLHEL ILSFNNLTRL DEESLAELSS LSILRLSHNA ISHIAEGAFK GLKSLRVLDL DHNEISGTIE DTSGAFTGLD NLSKLTLFGN KIKSVAKRAF SGLESLEHLN LGENAIRSVQ FDAFAKMKNL KELYISSESF LCDCQLKWLP PWLMGRMLQA FVTATCAHPE SLKGQSIFSV LPDSFVCDDF PKPQIITQPE TTMAVVGKDI RFTCSAASSS SSPMTFAWKK DNEVLANADM ENFAHVRAQD GEVMEYTTIL HLRHVTFGHE GRYQCIITNH FGSTYSHKAR LTVNVLPSFT KIPHDIAIRT GTTARLECAA TGHPNPQIAW QKDGGTDFPA ARERRMHVMP DDDVFFITDV KIDDMGVYSC TAQNSAGSVS ANATLTVLET PSLAVPLEDR VVTVGETVAF QCKATGSPTP RITWLKGGRP LSLTERHHFT PGNQLLVVQN VMIDDAGRYT CEMSNPLGTE RAHSQLSILP TPGCRKDGTT |
| SEQ ID NO: 4 | GCTCAGGCTGGACCTAGGCTCCTTGGCTGCCGCCTGCACCTGTGCAGGCGATTCTCTGGACTGC AGCGGCCGGGCCTGGCCACACTGCCCAGGGACCTGCCTTCCTGGACCAGATCTCTGAACCTGAGC TACAATCGGCTGTCCGAGATCGATTCTGCCGCCTTTGAGGACCTGACAAATCTGCAGGAGGTGTAT CTGAACAGCAATGAGCTGACCGCAATCCCCTCCCTGGGAGCAGCCTCTATCGGCGTGGTGAGCCTG TTCCTGCAGCACAACAAGATCCTGAGCGTGGATGGCTCCCAGCTGAAGAGCTACCTGTCTCTGGAG GTGCTGGACCTGAGCTCCAACAATATCACCGAGATCAGATCTAGCTGTTTTCCTAATGGCCTGCG ATCAGAGAGCTGAACCTGGCCTCTAATCGGATCAGCATCCTGGAGTCCGGCGCCTTCGATGGCCTG AGCAGATCCCTGCTGACACTGCGCCTGTCCAAGAACCGGATCACCCAGCTGCCCGTGAAGGCCTTT AAGCTGCCTAGGCTGACACAGCTGGACCTGAACCGGAATAGAATCAGGCTGATCGAGGGCCTGACC TTCCAGGGCCTGGATAGCCTGGAGGTGCTGCGCCTGCAGCGGAACAATATCTCCCGCCTGACAGAC GGAGCATTTTGGGGCCTGTCTAAGATGCACGTGCTGCACCTGGAGTACAATAGCCTGGTGGAGGTG AACTCTGGCAGCCTGTATGGCCTGACCGCCCTGCACCAGCTGCACCTGTCCAACAATAGCATCAGC AGAATCCAGAGGGATGGCTGGTCCTTCTGCCAGAAGCTGCACGAGCTGATCCTGTCTTTTAACAAT CTGACCAGGCTGGACGAGGAGAGCCTGGCAGAGCTGTCCTCTCTGTCCATCCTGCGCCTGTCTCAC AATGCCATCAGCCACATCGCCGAGGGCGCCTTTAAGGGCCTGAAGAGCCTGAGGGTGCTGGATCTG GACCACAACGAGATCTCTGGCACCATCGAGGATACAAGCGGCGCCTTCACAGGCCTGGACAATCTG TCCAAGCTGACCCTGTTTGGCAACAAGATCAAGTCTGTGGCCAAGCGGGCCTTCTCTGGCCTGGAG AGCCTGGAGCACCTGAACCTGGGCGAGAATGCCATCAGATCCGTGCAGTTCGATGCCTTTGCCAAG ATGAAGAATCTGAAGGAGCTGTACATCAGCTCCGAGAGCTTCCTGTGCGACTGTCAGCTGAAGTGG CTGCCACCTTGGCTGATGGGAAGGATGCTGCAGGCCTTTGTGACCGCCACATGCGCCCACCCAGAG AGCCTGAAGGGCCAGAGCATCTTCTCCGTGCTGCCCGATAGCTTCGTGTGCGACGATTTCCCTAAG CCACAGATCATCACCCAGCCAGAGACAACAATGGCCGTGGTGGGCAAGGACATCCGGTTTACATGT TCCGCCGCCTCTAGCTCCTCTAGCCCCATGACCTTCGCCTGGAAGAAGGATAACGAGGTGCTGGCC AATGCCGACATGGAGAACTTCGCCCACGTGAGAGCCCAGGATGGCGAAGTGATGGAGTATACCACA ATCCTGCACCTGCGGCACGTGACCTTTGGCCACGAGGGCAGATACCAGTGCATCATCACAAATCAC TTCGGCTCTACCTATAGCCACAAGGCCAGGCTGACAGTGAACGTGCTGCCTAGCTTTACCAAGATC CCACACGACATCGCCATCAGAACAGGCACCACAGCAAGGCTGGAGTGTGCAGCAACCGGACACCCA AACCCTCAGATCGCATGGCAGAAGGATGGAGGCACAGACTTCCCTGCAGCCCGGGAGAGGAGAATG CACGTGATGCCAGACGATGACGTGTTCTTTATCACAGATGTGAAGATCGATGACATGGGCGTGTAC TCCTGCACCGCACAGAACAGCGCCGGCAGCGTGTCCGCCAACGCCACCCTGACCGTGCTGGAGACA CCATCCCTGGCCGTGCCCCTGGAGGACAGGGTGGTGACCGTGGGCGAGACAGTGGCCTTTCAGTGT AAGGCCACCGGCTCTCCAACACCAAGGATCACCTGGCTGAAGGGCGGCAGGCCCCTGAGCCTGACA GAGCGCCACCACTTCACCCCTGGCAATCAGCTGCTGGTGGTGCAGAACGTGATGATCGATGACGCC GGCAGGTATACATGCGAGATGAGCAATCCTCTGGGCACCGAGAGGGCACACTCCCAGCTGTCTATC CTGCCTACCCCAGGCTGCCGGAAGGATGGCACCACA |

### Immunoglobulin Fc region

As used herein, the term "immunoglobulin Fc region" refers to a region of immunoglobulin which includes the heavy chain constant region 2 (CH2) and/or the heavy chain constant region 3 (CH3), excluding the heavy and light chain variable regions. The immunoglobulin Fc region may be a component that constitutes a moiety in the protein combination of the present invention.

In the present invention, the immunoglobulin Fc region may include a hinge portion in the heavy chain constant region so as to not only affect structural flexibility of the fusion protein to be finally prepared, but also further increase productivity and stability of the fusion protein. However, the present invention is not limited thereto.

In addition, the immunoglobulin Fc region of the present invention may be an extended Fc region of immunoglobulin which includes some or all of the heavy chain constant region 1 (CH1) and/or the light chain constant region 1 (CL1), excluding only the heavy and light chain variable regions, as long as the immunoglobulin Fc region has an effect that is substantially the same or improved as compared with its native type. In addition, the immunoglobulin Fc region may be a region obtained by removal of a significantly long partial amino acid sequence that corresponds to CH2 and/or CH3.

For example, the immunoglobulin Fc region of the present invention may be 1) CH1 domain, CH2 domain, CH3 domain, and CH4 domain; 2) CH1 domain and CH2 domain; 3) CH1 domain and CH3 domain; 4) CH2 domain and CH3 domain; 5) a combination of an immunoglobulin hinge region (or part of the hinge region) with one or two or more domains of CH1 domain, CH2 domain, CH3 domain, and CH4 domain and; and 6) a dimer formed of each domain of the heavy chain constant region and the light chain constant region. However, the present invention is not limited thereto.

In the present invention, the immunoglobulin Fc region of the present invention includes its native amino acid sequence as well as a sequence derivative thereof. An amino acid sequence derivative refers to an amino acid sequence that differs from its native amino acid sequence by deletion, insertion, or non-conservative or conservative substitution of at least one amino acid residue, or a combination thereof.

In the present invention, for example, for IgG Fc, the amino acid residues at positions 214 to 238, 297 to 299, 318 to 322, or 327 to 331, which are known to be important for binding, may be used as suitable sites for modification.

In the present invention, various types of derivatives are possible, including one obtained by deletion of a site capable of forming a disulfide bond, one obtained by deletion of some amino acid residues at the N-terminus of native Fc, one obtained by addition of a methionine residue at the N-terminus of native Fc, and the like. In addition, to remove an effector function therein, a complement-binding site, such as a C1q-binding site, may be deleted, or an antibody dependent cell mediated cytotoxicity (ADCC) site may be deleted. Techniques for preparing such sequence derivatives of the immunoglobulin Fc region are disclosed in International Patent Publication Nos. WO 97/34631 and WO 96/32478, and the like.

In the present invention, amino acid exchanges in proteins and peptides, which do not alter the molecular activity as a whole, are known in the art (H. Neurath, R. L. Hill, The Proteins, Academic Press, New York, 1979). The most common exchanges are exchanges occurring between the amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, or Asp/Gly. As the case may be, modification may be achieved by phosphorylation, sulfation, acrylation, glycosylation, methylation, farnesylation, acetylation, amidation, and the like.

In the present invention, the above-described Fc derivatives exhibit biological activity equivalent to that of the Fc region of the present invention, and may be those having increased structural stability against heat, pH, or the like.

In the present invention, the Fc region may be obtained from native types isolated in vivo from animals such as humans, cows, goats, pigs, mice, rabbits, hamsters, rats, or guinea pigs, or may be recombinants, which are obtained from transformed animal cells or microorganisms, or derivatives thereof. Here, the method of obtaining the Fc region from the native types may be a method in which the entire immunoglobulin is isolated from a living human or animal body, and then treated with protease to obtain the Fc region. In a case of being treated with papain, the entire immunoglobulin is cleaved into Fab and Fc; and in a case of being treated with pepsin, the entire immunoglobulin is cleaved into pF'c and F(ab)₂. Fc or pF'c may be isolated using size-exclusion chromatography or the like. In a more specific embodiment, the immunoglobulin Fc region is a recombinant immunoglobulin Fc region obtained from a microorganism using a human- or mouse-derived Fc region.

In the present invention, the immunoglobulin Fc region may have native-type glycan, or increased or decreased level of glycan as compared with the native-type, or may be in a deglycosylated form. Conventional methods such as chemical methods, enzymatic methods, and genetic engineering methods using microorganisms may be used for such increase or decrease of glycan or deglycosylation in the immunoglobulin Fc. Here, the immunoglobulin Fc region, in which glycan has been removed on Fc, exhibits remarkably decreased binding capacity with a complement (C1q) and has decreased or eliminated antibody-dependent cytotoxicity or complement-dependent cytotoxicity; and therefore does not cause any unnecessary immune responses in vivo. In this viewpoint, the deglycosylated or aglycosylated immunoglobulin Fc region would be a more suitable form for the original purpose as a drug carrier.

As used herein, the term "deglycosylation" refers to an Fc region from which sugar is enzymatically removed, and the term "aglycosylation" refers to an Fc region that is not glycosylated by being produced in a prokaryote that is E. coli in a more specific embodiment.

Meanwhile, the immunoglobulin Fc region may be derived from humans or other animals such as cows, goats, pigs, mice, rabbits, hamsters, rats, and guinea pigs. In a more specific embodiment, it is derived from humans or mouse.

In addition, the immunoglobulin Fc region of the present invention may be an IgG-, IgA-, IgD-, IgE-, or IgM-derived Fc region, heavy chain constant region 2 (CH2), heavy chain constant region 3 (CH3), hinge, a fragment thereof, or a combination thereof, or a hybrid Fc including the combination.

Meanwhile, as used herein, the term "combination" means that a polypeptide coding for single-chain immunoglobulin Fc region, heavy chain constant region 2 (CH2), or heavy chain constant region 3 (CH3), which is of the same origin, forms a bond with a single-chain polypeptide of a different origin when they form a dimer or multimer. That is, it is possible to prepare a dimer or a multimer from two or more fragments selected from IgG-, IgA-, IgM-, IgD-, or IgE-derived Fc region, heavy chain constant region 2 (CH2), or heavy chain constant region 3 (CH3).

In the present invention, the "hybrid Fc" may be derived from combinations of human IgG subclasses or combinations of human IgD and IgG. In an embodiment, the hybrid Fc may include, for example, an IgD hinge region, and a CH2 N-terminal region + IgG4 CH2 and CH3 regions. For example, a hybrid Fc form disclosed in Korean Patent No. 0897938, which is incorporated herein by reference, may be adopted and used in the same manner. In the present invention, in a case where the hybrid Fc binds to a biologically active molecule, a polypeptide, or the like, it has an effect of increasing the serum half-life of the biologically active molecule as well as an effect of increasing an expression level of the polypeptide when the polypeptide is expressed by a nucleotide sequence encoding an Fc-polypeptide fusion protein.

As an example in the present invention, the immunoglobulin Fc region may be an IgG-, IgA-, IgM-, IgD-, or IgE-derived Fc region, or may include IgG-, IgA-, IgM-, IgD-, or IgE-derived heavy chain constant region 2 (CH2) and heavy chain constant region 3 (CH3). However, the present invention is not limited thereto.

As an example in the present invention, the immunoglobulin Fc region may be an IgG- or IgM-derived Fc region which is most abundant in human blood, or may include IgG- or IgM-derived heavy chain constant region 2 (CH2) and heavy chain constant region 3 (CH3). As another example, the immunoglobulin Fc region may be an IgG-derived Fc region known to enhance the half-life of a ligand-binding protein, or may include IgG-derived heavy chain constant region 2 (CH2) and heavy chain constant region 3 (CH3). As yet another example, the immunoglobulin Fc region may be an IgG1-, IgG2-, IgG3-, or IgG4-derived Fc region, or may include the IgG1-, IgG2-, IgG3-, or IgG4-derived heavy chain constant region 2 (CH2) and heavy chain constant region 3 (CH3). As still yet another example, the immunoglobulin Fc region may include IgG1- or IgG2-derived heavy chain constant region 2 (CH2) and heavy chain constant region 3 (CH3).

As a preferred example in the present invention, the immunoglobulin Fc region may include, but is not limited to, human IgG1-derived heavy chain constant region 2 (CH2) and heavy chain constant region 3 (CH3), which are represented by SEQ ID NO: 5, or mouse IgG2-derived heavy chain constant region 2 (CH2) and heavy chain constant region 3 (CH3), which are represented by SEQ ID NO: 6 (see Table 3 below).

**[Table 3]**

| SEQ ID NO | Sequence information |
|---|---|
| SEQ ID NO: 5 | LFPPKPKDTLMISRTPEVTCVVVDVSHEDPEVKFNWYVDGVEVH NAKTKPREEQYNSTYRVVSVLTVLHQDWLNGKEYKCKVSNKAL PAPIEKTISKAKGQPREPQVYTLPPSRDELTKNQVSLTCLVKGFYPS DIAVEWESNGQPENNYKTTPPVLDSDGSFFLYSKLTVDKSRWQQ GNVFSCSVMHEALHNHYTQKSLSLSPGK |
| SEQ ID NO: 6 | IFPPKIKDVLMISLSPIVTCVVVDVSEDDPDVQISWFVNNVEVHTA QTQTHREDYNSTLRVVSALPIQHQDWMSGKEFKCKVNNKDLPAP IERTISKPKGSVRAPQVYVLPPPEEEMTKKQVTLTCMVTDFMPEDI YVEWTNNGKTELNYKNTEPVLDSDGSYFMYSKLRVEKKNWVER NSYSCSVVHEGLHNHHTTKSFSRTPGK |

As an example of the present invention, the immunoglobulin Fc region may include an IgG-, IgA-, IgM-, IgD-, IgE-, or Abatacept-derived hinge region; and as another example, the immunoglobulin Fc region may include an IgG-, IgD-, or Abatacept-derived hinge region, or may include an IgG1-, IgG2-, IgG3-, IgG4-, IgD-, or Abatacept-derived hinge region. However, the present invention is not limited thereto.

As a preferred example in the present invention, the immunoglobulin Fc region may include one or more selected from the group consisting of a human IgG1-derived hinge region represented by SEQ ID NO: 7; a mouse IgG2-derived hinge region represented by SEQ ID NO: 8; a human IgD-derived hinge region represented by SEQ ID NO: 9; and a hinge region of Abatacept represented by SEQ ID NO: 10 (see Table 4 below), thereby increasing structural flexibility of the fusion protein to be finally prepared and remarkably enhancing productivity and stability of the fusion protein. However, the present invention is not limited thereto.

**[Table 4]**

| SEQ ID NO | Sequence information |
|---|---|
| SEQ ID NO: 7 | EPKSSDKTHTSPPCPAPELLGGPSVF |
| SEQ ID NO: 8 | EPRGPTIKPCPPCKCPAPNLLGGPSVF |
| SEQ ID NO: 9 | RNTGRGGEEKKKEKEKEKEEQEERETKT PECPSHTQPLGVF |
| SEQ ID NO: 10 | EPKSSDKTHTSPPSPAPELLGGSSVF |

In the present invention, in a case where the extracellular domain of the Lrig-1 protein and the Fc region are connected via a linker, the linker may be linked to the N-terminus, C-terminus, or free radical of the Fc fragment and may be linked to the N-terminus, C-terminus, or free radical of the extracellular domain of the Lrig-1 protein. In a case where the linker is a peptide linker, linkage may occur at any site. For example, the linker may be linked to the C-terminus of the extracellular domain of the Lrig-1 protein and the N-terminus of the immunoglobulin Fc region, or may be linked to the C-terminus of the immunoglobulin Fc region and the N-terminus of the extracellular domain of the Lrig-1 protein.

### Linker

In the present invention, the "linker" can decrease interference between the extracellular domain of the Lrig-1 protein and the immunoglobulin Fc region in the fusion protein, thereby increasing the desired activity of the extracellular domain of the Lrig-1 protein in target cells. In addition, in the present invention, the linker may include a sequence that can be cleaved by an enzyme overexpressed in the tissue or cell with the target disease. In a case where the linker can be cleaved by the enzyme overexpressed as described above, it is possible to effectively prevent the activity of the polypeptide from being decreased due to the Fc portion.

As an example of the present invention, the linker preferably has 1 to 100 amino acids; however, the present invention is not limited thereto. Any peptide, which is capable of separating the extracellular domain of the Lrig-1 protein from the immunoglobulin Fc region, may be used. Although there is no particular limitation on the amino acid sequence that constitutes the linker, it is preferable to include glycine (G) and serine (S), or to include them in a repeated or random pattern. As such an example, the linker may include at least one of a peptide linker represented by SEQ ID NO: 11 and a peptide linker represented by SEQ ID NO: 12 (see Table 5 below), or may be at least one selected from the group consisting of peptide linkers, each of which consists of an amino acid sequence represented by any one of Formulas 1 to 4:

[Formula 1] (G)ₙ

[Formula 2] (GGGGS)ₘ

[Formula 3] (EAAAK)ₚ

[Formula 4] (XP)_{q}

In Formulas 1 to 4,
n, m, and p are each independently an integer of 1 or higher; and
q is an integer of 5 to 40;

X is any one amino acid selected from the group consisting of A (Ala), C (Cys), D (Asp), E (Glu), F (Phe), G (Gly), H (His), I (Ile), K (Lys), L (Leu), M (Met), N (Asn), O (Pyl), P (Pro), Q (Gln), R (Arg), S (Ser), T (Thr), U (Sec), V (Val), W (Trp), and Y (Tyr).

**[Table 5]**

| SEQ ID NO | Sequence information |
|---|---|
| SEQ ID NO: 11 | GS |
| SEQ ID NO: 12 | GGG |

In the present invention, n, m, and q in Formulas 1 to 3 may be, are not limited thereto, integers of 1 or higher, with integers of 1 to 20 being preferred.

In the present invention, X in Formula 4 may be A(Ala), but is not limited thereto.

In addition, as an example in the present invention, the linker may include at least one selected from, but not limited to, the group consisting of a peptide linker (A(EAAAK)₄ALEA(EAAAK)₄A) represented by SEQ ID NO: 96; a peptide linker (PAPAP) represented by SEQ ID NO: 97; a peptide linker (AEAAAKEAAAKA) represented by SEQ ID NO: 98; a peptide linker (IEGRMD) represented by SEQ ID NO: 99; and a peptide linker (DIEGRMD) represented by SEQ ID NO: 100.

In the present invention, in a case where the fusion protein further comprises the peptide linker, it is possible to increase stability of active substances in a cell while further enhancing productivity thereof.

In addition, as an example of the linker in the present invention, a peptide linker consisting of 33 amino acids located at positions 282 to 314, more preferably 13 amino acids located at positions 292 to 304, of human albumin, which is most abundant in the blood, may be mentioned. Such a portion is exposed in most parts to the outside as viewed in a three-dimensional structure, and thus has minimized possibility of inducing an immune response in the body. However, the present invention is not limited thereto.

In addition, in the present invention, in a case where the linker and the Fc region are separately expressed and then jointed to each other, the linker may be a crosslinking agent known in the art. The crosslinking agent may be, for example, 1,1-bis(diazoacetyl)-2-phenylethane, glutaraldehyde, N-hydroxysuccinimide esters such as 4-azidosalicylic acid, imidoestesr including disuccinimidyl esters such as 3,3'-dithiobis(succinimidylpropionate), and bifunctional maleimide such as bis-N-maleimido-1,8-octane. However, the crosslinking agent is not limited thereto.

### Fusion protein including Lrig-1 extracellular domain and immunoglobulin

As an example of the fusion protein provided in the present invention, a fusion protein, including the extracellular domain of the Lrig-1 protein represented by SEQ ID NO: 1; the hinge region represented by any one of SEQ ID NOs: 7 to 10; and the human IgG1-derived heavy chain constant region 2 (CH2) and heavy chain constant region 3 (CH3) which are represented by SEQ ID NO: 5, may be mentioned (see Table 6 below). The fusion proteins represented by SEQ ID NOs: 13 to 16 in Table 6 below may be encoded by the nucleic acid sequences represented by SEQ ID NOs: 17 to 20 in Table 7 below (see Table 7 below).

**[Table 6]**

| SEQ ID NO | Sequence information |
|---|---|
| SEQ ID NO: 13 | AGPRAPCAAACTCAGDSLDCGGRGLAALPGDLPSWTRSLNLSYNKLSEIDPAGFEDLPNLQEVYL NNNELTAVPSLGAASSHVVSLFLQHNKIRSVEGSQLKAYLSLEVLDLSLNNITEVRNTCFPHGPP IKELNLAGNRIGTLELGAFDGLSRSLLTLRLSKNRITQLPVRAFKLPRLTQLDLNRNRIRLIEGL TFQGLNSLEVLKLQRNNISKLTDGAFWGLSKMHVLHLEYNSLVEVNSGSLYGLTALHQLHLSNNS IARIHRKGWSFCQKLHELVLSFNNLTRLDEESLAELSSLSVLRLSHNSISHIAEGAFKGLRSLRV LDLDHNEISGTIEDTSGAFSGLDSLSKLTLFGNKIKSVAKRAFSGLEGLEHLNLGGNAIRSVQFD AFVKMKNLKELHISSDSFLCDCQLKWLPPWLIGRMLQAFVTATCAHPESLKGQSIFSVPPESFVC DDFLKPQIITQPETTMAMVGKDIRFTCSAASSSSSPMTFAWKKDNEVLTNADMENFVHVHAQDGE VMEYTTILHLRQVTFGHEGRYQCVITNHFGSTYSHKARLTVNVLPSFTKTPHDITIRTTTVARLE CAATGHPNPQIAWQKDGGTDFPAARERRMHVMPDDDVFFITDVKIDDAGVYSCTAQNSAGSISAN ATLTVLETPSLVVPLEDRVVSVGETVALQCKATGNPPPRITWFKGDRPLSLTERHHLTPDNQLLV VQNVVAEDAGRYTCEMSNTLGTERAHSQLSVLPAAGCRKDGTTVGIF EPKSSDKTHT SPPCPAPELL GGPSVF LFPPKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHY TQKSLSLSPGK |
| SEQ ID NO: 14 | AGPRAPCAAACTCAGDSLDCGGRGLAALPGDLPSWTRSLNLSYNKLSEIDPAGFEDLPNLQEVYL NNNELTAVPSLGAASSHVVSLFLQHNKIRSVEGSQLKAYLSLEVLDLSLNNITEVRNTCFPHGPP IKELNLAGNRIGTLELGAFDGLSRSLLTLRLSKNRITQLPVRAFKLPRLTQLDLNRNRIRLIEGL TFQGLNSLEVLKLQRNNISKLTDGAFWGLSKMHVLHLEYNSLVEVNSGSLYGLTALHQLHLSNNS IARIHRKGWSFCQKLHELVLSFNNLTRLDEESLAELSSLSVLRLSHNSISHIAEGAFKGLRSLRV LDLDHNEISGTIEDTSGAFSGLDSLSKLTLFGNKIKSVAKRAFSGLEGLEHLNLGGNAIRSVQFD AFVKMKNLKELHISSDSFLCDCQLKWLPPWLIGRMLQAFVTATCAHPESLKGQSIFSVPPESFVC DDFLKPQIITQPETTMAMVGKDIRFTCSAASSSSSPMTFAWKKDNEVLTNADMENFVHVHAQDGE VMEYTTILHLRQVTFGHEGRYQCVITNHFGSTYSHKARLTVNVLPSFTKTPHDITIRTTTVARLE CAATGHPNPQIAWQKDGGTDFPAARERRMHVMPDDDVFFITDVKIDDAGVYSCTAQNSAGSISAN ATLTVLETPSLVVPLEDRVVSVGETVALQCKATGNPPPRITWFKGDRPLSLTERHHLTPDNQLLV VQNVVAEDAGRYTCEMSNTLGTERAHSQLSVLPAAGCRKDGTTVGIF EPRGPTIKPCPPCKCPAPNLLGGPSVF LFPPKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHY TQKSLSLSPGK |
| SEQ ID NO: 15 | AGPRAPCAAACTCAGDSLDCGGRGLAALPGDLPSWTRSLNLSYNKLSEIDPAGFEDLPNLQEVYL NNNELTAVPSLGAASSHVVSLFLQHNKIRSVEGSQLKAYLSLEVLDLSLNNITEVRNTCFPHGPP IKELNLAGNRIGTLELGAFDGLSRSLLTLRLSKNRITQLPVRAFKLPRLTQLDLNRNRIRLIEGL TFQGLNSLEVLKLQRNNISKLTDGAFWGLSKMHVLHLEYNSLVEVNSGSLYGLTALHQLHLSNNS IARIHRKGWSFCQKLHELVLSFNNLTRLDEESLAELSSLSVLRLSHNSISHIAEGAFKGLRSLRV LDLDHNEISGTIEDTSGAFSGLDSLSKLTLFGNKIKSVAKRAFSGLEGLEHLNLGGNAIRSVQFD AFVKMKNLKELHISSDSFLCDCQLKWLPPWLIGRMLQAFVTATCAHPESLKGQSIFSVPPESFVC DDFLKPQIITQPETTMAMVGKDIRFTCSAASSSSSPMTFAWKKDNEVLTNADMENFVHVHAQDGE VMEYTTILHLRQVTFGHEGRYQCVITNHFGSTYSHKARLTVNVLPSFTKTPHDITIRTTTVARLE CAATGHPNPQIAWQKDGGTDFPAARERRMHVMPDDDVFFITDVKIDDAGVYSCTAQNSAGSISAN ATLTVLETPSLVVPLEDRVVSVGETVALQCKATGNPPPRITWFKGDRPLSLTERHHLTPDNQLLV VQNVVAEDAGRYTCEMSNTLGTERAHSQLSVLPAAGCRKDGTTVGIF RNTGRGGEEKKKEKEKEEQEERETKTPECPSHTQPLGVF LFPPKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHY |
| | TQKSLSLSPGK |
| 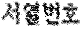 16 SEQ D NO:16 | AGPRAPCAAACTCAGDSLDCGGRGLAALPGDLPSWTRSLNLSYNKLSEIDPAGFEDLPNLQEVYL NNNELTAVPSLGAASSHVVSLFLQHNKIRSVEGSQLKAYLSLEVLDLSLNNITEVRNTCFPHGPP IKELNLAGNRIGTLELGAFDGLSRSLLTLRLSKNRITQLPVRAFKLPRLTQLDLNRNRIRLIEGL TFQGLNSLEVLKLQRNNISKLTDGAFWGLSKMHVLHLEYNSLVEVNSGSLYGLTALHQLHLSNNS IARIHRKGWSFCQKLHELVLSFNNLTRLDEESLAELSSLSVLRLSHNSISHIAEGAFKGLRSLRV LDLDHNEISGTIEDTSGAFSGLDSLSKLTLFGNKIKSVAKRAFSGLEGLEHLNLGGNAIRSVQFD AFVKMKNLKELHISSDSFLCDCQLKWLPPWLIGRMLQAFVTATCAHPESLKGQSIFSVPPESFVC DDFLKPQIITQPETTMAMVGKDIRFTCSAASSSSSPMTFAWKKDNEVLTNADMENFVHVHAQDGE VMEYTTILHLRQVTFGHEGRYQCVITNHFGSTYSHKARLTVNVLPSFTKTPHDITIRTTTVARLE CAATGHPNPQIAWQKDGGTDFPAARERRMHVMPDDDVFFITDVKIDDAGVYSCTAQNSAGSISAN ATLTVLETPSLVVPLEDRVVSVGETVALQCKATGNPPPRITWFKGDRPLSLTERHHLTPDNQLLV VQNVVAEDAGRYTCEMSNTLGTERAHSQLSVLPAAGCRKDGTTVGIF EPKSSDKTHTSPPSPAPELLGGSSVF LFPPKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHY TQKSLSLSPGK |

**[Table 7]**

| SEQ ID NO | Sequence information |
|---|---|
| SEQ ID NO: 17 | GCTGGCCCTCGGCTCCTTGTGCTGCCGCCTGCACATGTGCAGGCGATTCCCTGGACTGCGGCGG CAGAGGCCTGGCCGCCCTGCCTGGCGATCTGCCATCCTGGACCCGGAGCTGAACCTGAGCTACA ACAAGCTGAGCGAGATCGATCCGCCGGCTTTGAGGACCTGCCTAACCTGCAGGAGGTGTATCTG AACAATAACGAGCTGACCGCGGTACCatccctggcgctgcttcatcacatgtcgtctctcttt tctgcagcacaacagattcgcagcgtggagggagccagctgaaggcctacctttccttagaag tgttagatctgagtttgaacaacatcacggaagtgcggaacacctgctttccacacggaccgcct ataaaggagctcaacctggcaggcaatcggattggcaccctggagttgggagcatttgatggtct gtcacggtcgctgctaactcttcgcctgagcaaaaacaggatcacccagcttcctgtaagagcat tcaagctacccaggctgacacaactggacctcaatcggaacaggattcggctgatagagggcctc accttccagggggctcaacagcttggaggtgctgaagcttcagcggaaacaacatcagcaaactgac agatgggccttctgggactgtccaagatgcatgtgctgcacctggagtacaacagcctggtag aagtgaacagcggctcgctctacggcctcacggccctgcatcagctccacctcagcaacaattcc atcgctcgcattcaccgcaagggctggagcttctgccagaagctgcatgagttggtcctgtcctt caacaacctgacacggctggacgaggagagcctggccgagctgagcagcctgagtgtcctgcgtc tcagccacaattccatcagccacattgcggagggtgccttcaagggactcaggagcctgcgagtc ttggatctggaccataacgagatttcgggcacaatagaggacacgagcggcgccttctcagggct cgacagcctcagcaagctgactctgtttggaaacaagatcaagtctgtggctaagagagcattct cgggctggaaggcctggagcacctgaaccttggagggaatgcgatcagatctgtccagtttgat gcctttgtgaagatgaagaatcttaaagagctccatatcagcagcgacagcttcctgtgtgactg ccagctgaagtggctgccccgtggctaattggcaggatgctgcaggcctttgtgacagccacct gtgcccacccagaatcactgaagggtcagagcatttctctgtgcccaccagagagtttcgtgtgc gatgacttcctgaagccacagatcatcacccagccagaaaccaccatggctatggtgggcaagga catccggtttacatgctcagcagccagcagcagcagctcccccatgacctttgcctggaagaaag acaatgaagtcctgaccaatgcagacatggagaactttgtccacgtccacgcgcaggacgggga gtgatggagtacaccaccatcctgcacctccgtcaggtcactttcgggcacgagggccgctacca atgtgtcatcaccaaccactttggctccacctattcacataaggccaggctcaccgtgaatgtgt tgccatcattcaccaaaacgccccacgacataaccatccggaccaccaccgtggcccgcctcgaa tgtgctgccacaggtcacccaaaccctcagattgcctggcagaaggatggaggcacggatttccc cgctgcccgtgagcgacgcatgcatgtcatgccggatgacgacgtgtttttcatcactgatgtga aaatagatgacgcaggggtttacagctgtactgctcagaactcagccggttctatttcagctaat gccaccctgactgtcctagagaccccatccttggtggtccccttggaagaccgtgtggtatctgt gggagaaacagtggccctccaatgcaaagccacggggaacctccgccccgcatcacctggttca aggggggaccgcccgctgagcctcactgagcggcaccacctgacccctgacaaccagctcctggtg gttcagaacgtggtggcagaggatgcgggccgatatacctgtgagatgtccaacaccctgggcac ggagcgagctcacagccagctgagcgtcctgcccgcagcaggctgcaggaaggatgggaccacgg taggcatcttc GAGCCAAAGTCCTCTGATAAGACACACACCTCTCCACCATGCCCAGCACCAGAGCTGCTGGAGG ACCAAGCGTGTTC CTGTTCCTCCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCCTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAAGCACTGCACAATCACTAC ACCCAGAAATCACTGTCACTGAGCCCTGGCAAA |
| SEQ IDNO: 18 | GCTGGGCCTCGGCTCCTTGTGCTGCCGCCTGCACATGTGCAGGCGATTCCCTGGACTGCGGCGG CAGAGGCCTGGCCGCCCTGCCTGGCGATCTGCCATCCTGGACCCGGAGCCTGAACCTGAGCTACA ACAAGCTGAGCGAGATCGATCCGCCGGCTTTGAGGACCTGCCTAACCTGCAGGAGGTGTATCTG AACAATAACGAGCTGACCGCGGTACCatccctggcgctgcttcatcacatgtcgtctctctt tctgcagcacaacagattcgcagcgtggaggggagccagctgaaggcctacctttccttagaag tgttagatctgagtttgaacaacatcacggaagtgcggaacacctgctttccacacggaccgcct ataaaggagctcaacctggcaggcaatcggattggcaccctggagttggagcatttgatggtct gtcacggtcgctgctaactcttcgcctgagcaaaaacaggatcacccagcttcctgtaagagcat tcaagctacccaggctgacacaactggacctcaatcggaacaggattcggctgatagagggcctc accttccagggcctcaacagcttggaggtgctgaagcttcagcgaaacaacatcagcaaactgac agatgggccttctgggactgtccaagatgcatgtgctgcacctggagtacaacagcctggtag aagtgaacagcggctcgctctacggcctcacggccctgcatcagctccacctcagcaacaattcc atcgctcgcattcaccgcaagggctggagcttctgccagaagctgcatgagttggtcctgtcctt caacaacctgacacggctggacgaggagagcctggccgagctgagcagcctgagtgtcctgcgtc tcagccacaattccatcagccacattgcggagggtgccttcaagggactcaggagcctgcgagtc ttggatctggaccataacgagatttcgggcacaatagaggacacgagcggcgccttctcagggct cgacagcctcagcaagctgactctgtttggaaacaagatcaagtctgtggctaagagagcattct cgggctggaaggcctggagcacctgaaccttggagggaatgcgatcagatctgtccagtttgat gcctttgtgaagatgaagaatcttaaagagctccatatcagcagcgacagcttcctgtgtgactg ccagctgaagtggctgccccgtggctaattggcaggatgctgcagcctttgtgacagccacct gtgcccacccagaatcactgaagggtcagagcatttctctgtgccaccagagagtttcgtgtgc gatgacttcctgaagccacagatcatcacccagccagaaaccaccatggctatggtgggcaagga catccggtttacatgctcagcagccagcagcagcagctcccccatgacctttgcctggaagaaag acaatgaagtcctgaccaatgcagacatggagaactttgtccacgtccacgcgcaggacggggaa gtgatggagtacaccaccatcctgcacctccgtcaggtcactttcgggcacgagggccgctacca atgtgtcatcaccaaccactttggctccacctattcacataagccagctcaccgtgaatgtgt tgccatcattcaccaaacgccccacgacataaccatccggaccaccaccgtggcccgcctcgaa tgtgctgccacaggtcacccaaaccctcagattgcctggcagaaggatggaggcacggatttccc cgctgcccgtgagcgacgcatgcatgtcatgccggatgacgacgtgtttttcatcactgatgtga aaatagatgacgcaggggtttacagctgtactgctcagaactcagccggttctattcagctaat gccaccctgactgtcctagagaccccatccttggtggtccccttggaagaccgtgtggtatctgt gggagaaacagtggccctccaatgcaaagccacggggaaccctccgccccgcatcacctggttca aggggaccgcccgctgagcctcactgagcggcaccacctgaccctgacaaccagctcctggtg gttcagaacgtggtggcagaggatgcgggccgatatacctgtgagatgtccaacaccctgggcac ggagcgagctcacagccagctgagcgtcctgcccgcagcaggctgcaggaaggatggaccacgg taggcatcttc Gagcctcgggccctaccatcaagccctgccccccttgcaagtgccctgcccctaatctgctggg cggaccctccgtgttc CTGTTTCCTCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCCTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAAGCACTGCACAATCACTAC ACCCAGAAATCACTGTCACTGAGCCCTGGCAAA |
| SEQ ID NO: 19 | GCTGGGCCTCGGGCTCCTTGTGCTGCCGGCCTGCACATGTGCAGGCGATTCCCTGGACTGCGCGG CAGAGGCCTGGCCGCCCTGCCTGGCGATCTGCCATCCTGGACCCGGAGCCTGAACCTGAGCTACA ACAAGCTGAGCGAGATCGATCCCGCCGGCTTTGAGGACCTGCCTAACCTGCAGGAGGTGTATCTG |
| | AACAATAACGAGCTGACCGCGTACCatccctggcgctgcttcatcacatgtcgtctctctt tctgcagcacaacaggattcgcacgtggagggagccagctgaaggcctacctttccttagaag tgttagatctgagtttgaacaacatcacggaagtgcggaacacctgctttccacacggaccgcct ataaaggagctcaacctggcaggcaatcggattggcaccctggagttgggagcatttgatggtct gtcacggtcgctgctaactcttcgcctgagcaaaacaggatcacccagcttcctgtaagagcat tcaagctacccaggctgacacaactggacctcaatcggaacaggattcggctgatagagggcctc accttccagggctcaacagcttggaggtgctgaagcttcagcgaaacaacatcagcaaactgac agatgggccttctgggactgtccaagatgcatgtgctgcacctggagtacaacagcctggtag aagtgaacagcggctcgctctacggcctcacggccctgcatcagctccacctcagcaacaattcc atcgctcgcattcaccgcaagggctggagcttctgccagaagctgcatgagttggtcctgtcctt caacaacctgacacggctggacgaggagagcctggccgagctgagcagcctgagtgtcctgcgtc tcagccacaattccatcagccacattgcggagggtgccttcaagggactcaggagcctgcgagtc ttggatctggaccataacgagatttcgggcacaatagaggacacgagcggcgccttctcagggct cgacagcctcagcaagctgactctgtttggaaacaagatcaagtctgtggctaagagagcattct cggggctggaaggcctggagcacctgaaccttggagggaatgcgatcagatctgtccagtttgat gcctttgtgaagatgaagaatcttaaagagctccatatcagcagcgacagcttcctgtgtgactg ccagctgaagtggctgccccgtggctaattggcaggatgctgcaggcctttgtgacagccacct gtgcccacccagaatcactgaagggtcagagcatttctctgtgccaccagagagtttcgtgtgc gatgacttcctgaagccacagatcatcacccagccagaaaccaccatggctatggtgggcaagga catccggtttacatgctcagcagccagcagcagcagctcccccatgacctttgcctggaagaaag acaatgaagtcctgaccaatgcagacatggagaactttgtccacgtccacgcgcaggacggggaa gtgatggagtacaccaccatcctgcacctccgtcaggtcactttcgggcacgagggccgctacca atgtgtcatcaccaaccactttggctccacctattcacataaggccaggctcaccgtgaatgtgt tgccatcattcaccaaaacgccccacgacataaccatccggaccaccaccgtggcccgcctcgaa tgtgctgccacaggtcacccaaaccctcagattgcctggcagaaggatggaggcacggatttccc cgctgcccgtgagcgacgcatgcatgtcatgccggatgacgacgtgtttttcatcactgatgtga aaatagatgacgcaggggtttacagctgtactgctcagaactcagccggttctatttcagctaat gccaccctgactgtcctagagacccatccttggtggtccccttggaagaccgtgtggtatctgt gggagaaacagtggccctccaatgcaaagccacggggaaccctccgccccgcatcacctggttca aggggaccgcccgctgagcctcactgagcggcaccacctgaccctgacaaccagctcctggtg gttcagaacgtggtggcagaggatgcgggccgatatacctgtgagatgtccaacaccctgggcac ggagcgagctcacagccagctgagcgtcctgcccgcagcaggctgcaggaaggatggaccacgg taggcatcttc cgcaacaccggccgcggcggcgaggagaagaagaaggagaaggagaaggaggagcaggaggagcg cgagaccaagaccccgagtgcccagccacacccagcccctggcgtgttc CTGTTTCCTCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCTTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAGGCACTGCACAATCACTAC ACCCAGAAATCACTGTCACTGAGCCCTGGCAAA |
| SEQ ID NO: 20 | GCTGGGCCTCGGGCTCCTTGTGCTGCCGCCTGCACATGTGCAGGCGATTCCCTGGACTGCGGCGG CAGAGGCCTGGCCGCCCTGCCTGGCGATCTGCCATCCTGGACCCGGAGCCTGAACCTGAGCTACA ACAAGCTGAGCGAGATCGATCCGCCGGCTTTGAGGACCTGCCTAACCTGCAGGAGGTGTATCTG AACAATAACGAGCTGACCGCGGTACCatccctggcgctgcttcatcacatgtcgtctctctctt tctgcagcacaacaagattcgcagcgtggaggggagccagctgaaggcctacctttccttagaag tgttagatctgagtttgaacaacatcacggaagtgcggaacacctgctttccacacggaccgcct |
| | ataaaggagctcaacctggcaggcaatcggattggcaccctggagttgggagcatttgatggtct gtcacggtcgctgctaactcttcgcctgagcaaaacaggatcacccagcttcctgtaagagcat tcaagctacccaggctgacacaactggacctcaatcggaacaggattcggctgatagagggcctc accttccaggggctcaacagcttggaggtgctgaagcttcagcgaaacaacatcagcaaactgac agatggggccttctgggactgtccaagatgcatgtgctgcacctggagtacaacagcctggtag aagtgaacagcggctcgctctacggcctcacggccctgcatcagctccacctcagcaacaattcc atcgctcgcattcaccgcaagggctggagcttctgccagaagctgcatgagttggtcctgtcctt caacaacctgacacggctggacgaggagagcctggccgagctgagcagcctgagtgtcctgcgtc tcagccacaattccatcagccacattgcggagggtgccttcaagggactcaggagcctgcgagtc ttggatctggaccataacgagatttcgggcacaatagaggacacgagcggcgccttctcagggct cgacagcctcagcaagctgactctgtttggaaacaagatcaagtctgtggctaagagagcattct cggggctggaaggcctggagcacctgaaccttggagggaatgcgatcagatctgtccagtttgat gcctttgtgaagatgaagaatcttaaagagctccatatcagcagcgacagcttcctgtgtgactg ccagctgaagtggctgccccgtggctaattggcaggatgctgcaggcctttgtgacagccacct gtgcccacccagaatcactgaagggtcagagcatttctctgtgccaccagagagtttcgtgtgc gatgacttcctgaagccacagatcatcacccagccagaaaccaccatggctatggtgggcaagga catccggtttacatgctcagcagccagcagcagctcccccatgacctttgcctggaagaaag acaatgaagtcctgaccaatgcagacatggagaactttgtccacgtccacgcgcaggacgggaa gtgatggagtacaccaccatcctgcacctccgtcaggtcactttcgggcacgagggccgctacca atgtgtcatcaccaaccactttggctccacctattcacataaggccaggctcaccgtgaatgtgt tgccatcattcaccaaaacgccccacgacataaccatccggaccaccaccgtggcccgcctcgaa tgtgctgccacaggtcacccaaacctcagattgcctggcagaaggatggaggcacggattccc cgctgcccgtgagcgacgcatgcatgtcatgccggatgacgacgtgtttttcatcactgatgtga aaatagatgacgcagggtttacagctgtactgctcagaactcagccggttctatttcagctaat gccaccctgactgtcctagagacccatccttggtggtccccttggaagaccgtgtggtatctgt gggagaaacagtggccctccaatgcaaagccacgggaaccctccgccccgcatcacctggttca aggggaccgcccgctgagcctcactgagcggcaccacctgaccctgacaaccagctcctggtg gttcagaacgtggtggcagaggatgcgggccgatatacctgtgagatgtccaacaccctgggcac ggagcgagctcacagccagctgagcgtcctgcccgcagcaggctgcaggaaggatgggaccacgg taggcatcttc gaaccgaaatcttctgacaaaacccacacctctccgccgtctccggctccggaactgctgggtgg ttcttctgtttc CTGTTTCCTCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCCTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAAGCACTGCACAATCACTAC ACCCAGAAATCACTGTCACTGAGCCCTGGCAAA |

An example of the fusion protein provided by the present invention, a fusion protein, including the extracellular domain of the Lrig-1 protein represented by SEQ ID NO: 3; the hinge region represented by any one of SEQ ID NOs: 7 to 10; human IgG1-derived heavy chain constant region 2 (CH2) and heavy chain constant region 3 (CH3)which are represented by SEQ ID NO: 5, may be mentioned (see Table 8 below). The fusion proteins represented by SEQ ID NOs: 21 to 24 in Table 8 below may be encoded by nucleic acid sequences represented by SEQ ID NOs: 25 to 28 in Table 9 below (see Table 9 below).

**[Table 8]**

| SEQ ID NO | Sequence information |
|---|---|
| SEQ ID NO: 21 | AQAGPRAPCAAACTCAGDSLDCSGRGLATLPRDLPSWTRSLNLSYNRLSEIDSAAFEDLTNLQEV YLNSNELTAIPSLGAASIGVVSLFLQHNKILSVDGSQLKSYLSLEVLDLSSNNITEIRSSCFPNG LRIRELNLASNRISILESGAFDGLSRSLLTLRLSKNRITQLPVKAFKLPRLTQLDLNRNRIRLIE GLTFQGLDSLEVLRLQRNNISRLTDGAFWGLSKMHVLHLEYNSLVEVNSGSLYGLTALHQLHLSN NSISRIQRDGWSFCQKLHELILSFNNLTRLDEESLAELSSLSILRLSHNAISHIAEGAFKGLKSL RVLDLDHNEISGTIEDTSGAFTGLDNLSKLTLFGNKIKSVAKRAFSGLESLEHLNLGENAIRSVQ FDAFAKMKNLKELYISSESFLCDCQLKWLPPWLMGRMLQAFVTATCAHPESLKGQSIFSVLPDSF VCDDFPKPQIITQPETTMAVVGKDIRFTCSAASSSSSPMTFAWKKDNEVLANADMENFAHVRAQD GEVMEYTTILHLRHVTFGHEGRYQCIITNHFGSTYSHKARLTVNVLPSFTKIPHDIAIRTGTTAR LECAATGHPNPQIAWQKDGGTDFPAARERRMHVMPDDDVFFITDVKIDDMGVYSCTAQNSAGSVS ANATLTVLETPSLAVPLEDRVVTVGETVAFQCKATGSPTPRITWLKGGRPLSLTERHHFTPGNQL LVVQNVMIDDAGRYTCEMSNPLGTERAHSQLSILPTPGCRKDGTT EPKSSDKTHT SPPCPAPELL GGPSVF LFPPKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHY TQKSLSLSPGK |
| SEQ ID NO: 22 | AQAGPRAPCAAACTCAGDSLDCSGRGLATLPRDLPSWTRSLNLSYNRLSEI DSAAFEDLTNLQEV YLNSNELTAI PSLGAAS IGVVSLFLQHNKI LSVDGSQLKSYLSLEVLDLSSNNITEI RSSCFPNG LRI RELNLASNRI SI LESGAFDGLSRSLLTLRLSKNRI TQLPVKAFKLPRLTQLDLNRNRI RLI E GLTFQGLDSLEVLRLQRNNI SRLTDGAFWGLSKMHVLHLEYNSLVEVNSGSLYGLTALHQLHLSN NSI SRI QRDGWSFCQKLHELI LSFNNLTRLDEESLAELSSLSI LRLSHNAI SHI AEGAFKGLKSL RVLDLDHNEI SGTI EDTSGAFTGLDNLSKLTLFGNKI KSVAKRAFSGLESLEHLNLGENAI RSVQ FDAFAKMKNLKELYI SSESFLCDCQLKWLPPWLMGRMLQAFVTATCAHPESLKGQSI FSVLPDSF VCDDFPKPQI I TQPETTMAVVGKDI RFTCSAASSSSSPMTFAWKKDNEVLANADMENFAHVRAQD GEVMEYTTI LHLRHVTFGHEGRYQCI I TNHFGSTYSHKARLTVNVLPSFTKI PHDI AI RTGTTAR LECAATGHPNPQI AWQKDGGTDFPAARERRMHVMPDDDVFFI TDVKI DDMGVYSCTAQNSAGSVS ANATLTVLETPSLAVPLEDRVVTVGETVAFQCKATGSPTPRI TWLKGGRPLSLTERHHFTPGNQL LVVQNVMI DDAGRYTCEMSNPLGTERAHSQLSI LPTPGCRKDGTT EPRGPTI KPCPPCKCPAPNLLGGPSVF LFPPKPKDTLMI SRTPEVTCVVVDVSHEDPEVKFNWYVDGVEVHNAKTKPREEQYNSTYRVVSVL TVLHQDWLNGKEYKCKVSNKALPAPI EKTI SKAKGQPREPQVYTLPPSRDELTKNQVSLTCLVKG FYPSDI AVEWESNGQPENNYKTTPPVLDSDGSFFLYSKLTVDKSRWQQGNVFSCSVMHEALHNHY TQKSLSLSPGK |
| SEQ ID NO: 23 | AQAGPRAPCAAACTCAGDSLDCSGRGLATLPRDLPSWTRSLNLSYNRLSEIDSAAFEDLTNLQEV YLNSNELTAIPSLGAASIGVVSLFLQHNKILSVDGSQLKSYLSLEVLDLSSNNITEIRSSCFPNG LRIRELNLASNRISILESGAFDGLSRSLLTLRLSKNRITQLPVKAFKLPRLTQLDLNRNRIRLIE GLTFQGLDSLEVLRLQRNNISRLTDGAFWGLSKMHVLHLEYNSLVEVNSGSLYGLTALHQLHLSN NSISRIQRDGWSFCQKLHELILSFNNLTRLDEESLAELSSLSILRLSHNAISHIAEGAFKGLKSL RVLDLDHNEISGTIEDTSGAFTGLDNLSKLTLFGNKIKSVAKRAFSGLESLEHLNLGENAIRSVQ FDAFAKMKNLKELYISSESFLCDCQLKWLPPWLMGRMLQAFVTATCAHPESLKGQSIFSVLPDSF VCDDFPKPQIITQPETTMAVVGKDIRFTCSAASSSSSPMTFAWKKDNEVLANADMENFAHVRAQD GEVMEYTTILHLRHVTFGHEGRYQCIITNHFGSTYSHKARLTVNVLPSFTKIPHDIAIRTGTTAR LECAATGHPNPQIAWQKDGGTDFPAARERRMHVMPDDDVFFITDVKIDDMGVYSCTAQNSAGSVS ANATLTVLETPSLAVPLEDRVVTVGETVAFQCKATGSPTPRITWLKGGRPLSLTERHHFTPGNQL LVVQNVMIDDAGRYTCEMSNPLGTERAHSQLSILPTPGCRKDGTT RNTGRGGEEKKKEKEKEEQEERETKTPECPSHTQPLGVF LFPPKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHY |
| | TQKSLSLSPGK |
| SEQ ID NO: 24 | AQAGPRAPCAAACTCAGDSLDCSGRGLATLPRDLPSWTRSLNLSYNRLSEIDSAAFEDLTNLQEV YLNSNELTAIPSLGAASIGVVSLFLQHNKILSVDGSQLKSYLSLEVLDLSSNNITEIRSSCFPNG LRIRELNLASNRISILESGAFDGLSRSLLTLRLSKNRITQLPVKAFKLPRLTQLDLNRNRIRLIE GLTFQGLDSLEVLRLQRNNISRLTDGAFWGLSKMHVLHLEYNSLVEVNSGSLYGLTALHQLHLSN NSISRIQRDGWSFCQKLHELILSFNNLTRLDEESLAELSSLSILRLSHNAISHIAEGAFKGLKSL RVLDLDHNEISGTIEDTSGAFTGLDNLSKLTLFGNKIKSVAKRAFSGLESLEHLNLGENAIRSVQ FDAFAKMKNLKELYISSESFLCDCQLKWLPPWLMGRMLQAFVTATCAHPESLKGQSIFSVLPDSF VCDDFPKPQIITQPETTMAVVGKDIRFTCSAASSSSSPMTFAWKKDNEVLANADMENFAHVRAQD GEVMEYTTILHLRHVTFGHEGRYQCIITNHFGSTYSHKARLTVNVLPSFTKIPHDIAIRTGTTAR LECAATGHPNPQIAWQKDGGTDFPAARERRMHVMPDDDVFFITDVKIDDMGVYSCTAQNSAGSVS ANATLTVLETPSLAVPLEDRVVTVGETVAFQCKATGSPTPRITWLKGGRPLSLTERHHFTPGNQL LVVQNVMIDDAGRYTCEMSNPLGTERAHSQLSILPTPGCRKDGTT EPKSSDKTHTSPPSPAPELLGGSSVF LFPPKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHY TQKSLSLSPGK |

**[Table 9]**

| SEQ ID NO | Sequence information |
|---|---|
| SEQ ID NO: 25 | GCTCAGGCTGGACCTAGGGCTCCTTGGCTGCGCCTGCACCTGTGCAGGCGATTCTCTGGACTGCAGCG GCCGGGGCCTGGCCACACTGCCCAGGACCTGCCTTCCTGGACCAGATCTCTGAACCTGAGCTACAATCG GCTGTCCGAGATCGATTCTGCCGCCTTTGAGGACCTGACAAATCTGCAGGAGGTGTATCTGAACAGCAAT GAGCTGACCGCAATCCCCTCCCTGGGAGCAGCCTCTATCGGCGTGGTGAGCCTGTTCCTGCAGCACAACA AGATCCTGAGCGTGGATGGCTCCCAGCTGAAGAGCTACCTGTCTCTGGAGGTGCTGGACCTGAGCTCCAA CAATATCACCGAGATCAGATCTAGCTGTTTTCCTAATGGCCTGCGGATCAGAGAGCTGAACCTGGCCTCT AATCGGATCAGCATCCTGGAGTCCGGCGCCTTCGATGGCCTGAGCAGATCCCTGCTGACACTGCGCCTGT CCAAGAACCGGATCACCCAGCTGCCCGTGAAGGCCTTTAAGCTGCCTAGGCTGACACAGCTGGACCTGAA CCGGAATAGAATCAGGCTGATCGAGGGCCTGACCTTCCAGGGCCTGGATAGCCTGGAGGTGCTGCGCCTG CAGCGGAACAATATCTCCCGCCTGACAGACGGAGCATTTTGGGGCCTGTCTAAGATGCACGTGCTGCACC TGGAGTACAATAGCCTGGTGGAGGTGAACTCTGGCAGCCTGTATGGCCTGACCGCCCTGCACCAGCTGCA CCTGTCCAACAATAGCATCAGCAGAATCCAGAGGGATGGCTGGTCCTTCTGCCAGAAGCTGCACGAGCTG ATCCTGTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTGAGGGT GCTGGATCTGGACCACAACGAGATCTCTGGCACCATCGAGGATACAAGCGGCGCCTTCACAGGCCTGGAC AATCTGTCCAAGCTGACCCTGTTTGGCAACAAGATCAAGTCTGTGGCCAAGCGGGCCTTCTCTGGCCTGG AGAGCCTGGAGCACCTGAACCTGGGCGAGAATGCCATCAGATCCGTGCAGTTCGATGCCTTTGCCAAGAT GAAGAATCTGAAGGAGCTGTACATCAGCTCCGAGAGCTTCCTGTGCGACTGTCAGCTGAAGTGGCTGCCA CCTTGGCTGATGGGAAGGATGCTGCAGGCCTTTGTGACCGCCACATGCGCCCACCCAGAGAGCCTGAAGG GCCAGAGCATCTTCTCCGTGCTGCCCGATAGCTTCGTGTGCGACGATTTCCCTAAGCCACAGATCATCAC CCAGCCAGAGACAACAATGGCCGTGGTGGGCAAGGACATCCGGTTTACATGTTCCGCCGCCTCTAGCTCC TCTAGCCCCATGACCTTCGCCTGGAAGAAGGATAACGAGGTGCTGGCCAATGCCGACATGGAGAACTTCG CCCACGTGAGAGCCCAGGATGGCGAAGTGATGGAGTATACCACAATCCTGCACCTGCGGCACGTGACCTT TGGCCACGAGGGCAGATACCAGTGCATCATCACAAATCACTTCGGCTCTACCTATAGCCACAAGGCCAGG CTGACAGTGAACGTGCTGCCTAGCTTTACCAAGATCCCACACGACATCGCCATCAGAACAGGCACCACAG CAAGGCTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAGATGTG AAGATCGATGACATGGGCGTGTACTCCTGCACCGCACAGAACAGCGCCGGCAGCGTGTCCGCCAACGCCA CCCTGACCGTGCTGGAGACACCATCCCTGGCCGTGCCCCTGGAGGACAGGGTGGTGACCGTGGGCGAGAC AGTGGCCTTTCAGTGTAAGGCCACCGGCTCTCCAACACCAAGGATCACCTGGCTGAAGGGCGGCAGGCCC CTGAGCCTGACAGAGCGCCACCACTTCACCCCTGGCAATCAGCTGCTGGTGGTGCAGAACGTGATGATCG ATGACGCCGGCAGGTATACATGCGAGATGAGCAATCCTCTGGGCACCGAGAGGGCACACTCCCAGCTGTC TATCCTGCCTACCCCAGGCTGCCGGAAGGATGGCACCACA GAGCCAAAGTCCTCTGATAAGACACACACCTCTCCACCATGCCCAGCACCAGAGCTGCTGGAGGACCAA GCGTGTTC CTGTTTCCTCCAAAGCCCAAGGACACACTGATGATCTCCAGGACACCAGAGGTGACCTGCGTGGTGGTGG ACGTGAGCCACGAGGACCCCGAGGTGAAGTTCAACTGGTACGTGGATGGCGTGGAGGTGCACAATGCCAA GACCAAGCCCAGAGAGGAGCAGTACAACTCTACCTATAGGGTGGTGAGCGTGCTGACAGTGCTGCACCAG GACTGGCTGAACGGCAAGGAGTATAAGTGCAAGGTGAGCAATAAGGCCCTGCCTGCCCCAATCGAGAAGA CAATCTCCAAGGCCAAGGGCCAGCCAAGAGAGCCCCAGGTGTACACCCTGCCCCCTAGCAGGGATGAGCT GACAAAGAACCAGGTGTCCCTGACCTGTCTGGTGAAGGGCTTTTATCCCTCCGACATCGCCGTGGAGTGG GAGTCTAATGGCCAGCCTGAGAATAACTACAAGACAACCCCACCCGTGCTGGATTCTGACGGCAGCTTCT TCCTGTATTCTAAGCTGACCGTGGACAAGAGCAGGTGGCAGCAGGGCAACGTGTTCAGCTGCTCCGTGAT GCACGAAGCACTGCACAATCACTACACCCAGAAATCACTGTCACTGAGCCCTGGCAAA |
| SEQ ID NO: 26 | GCTCAGGCTGGACCTAGGGCTCCTTGGGCTGGCGCCTGCACCTGTGCAGGCGATTCTCTGGACTGCAGCG GCGGGGGCCTGGCCACACTGGCCAGGGACCTGCCTTCCTGGACCAGATCTCTGAACCTGAGCTACAATCG GCTGTCCGAGATCGATTCTGCCGCCTTTGAGGACCTGACAAATCTGCAGGAGGTGTATCTGAACAGCAAT GAGCTGACCGCAATCCCCTCCCTGGGAGCAGCCTCTATCGGCCTGGTGAGCCTGTTCCTGCAGCACAACA AGATCCTGAGCGTGGATGGCTCCCAGCTGAAGAGCTACCTGTCTCTGGAGGTGCTGGACCTGAGCTCCAA CAATATCACCGAGATCAGATCTAGCTGTTTTCCTAATGGCCTGCGGATCAGAGAGCTGAACCTGGCCTCT |
| | AATCGGATCAGCATCCTGGAGTCCGGGCCTTCGATGGCCTGAGCAGATCCCTGCTGACACTGGCCCTGT CCAAGAACCGGATCACCCAGCTGCCCGTGAAGGCCTTTAAGCTGCCTAGGCTGACACAGCTGGACCTGAA CCGGAATAGAATCAGGCTGATCGAGGGCCTGACCTTCCAGGGCCTGGATAGCCTGGAGGTGCTGCGCCTG CAGCGGAACAATATCTCCCGGCTGACAGACGGAGCATTTTGGGGCCTGTCTAAGATGCACGTGCTGCACC TGGAGTACAATAGCCTGGTGGAGGTGAACTCTGGCAGCCTGTATGGCCTGACCGCCCTGCACCAGCTGCA CCTGTCCAACAATAGCATCAGCAGAATCCAGAGGGATGGCTGGTCCTTCTGCCAGAAGCTGCACGAGCTG ATCCTGTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTGAGGGT GCTGGATCTGGACCACAACGAGATCTCTGGCACCATCGAGGATACAAGCGGCGCCTTCACAGGCCTGGAC AATCTGTCCAAGCTGACCCTGTTTGGCAACAAGATCAAGTCTGTGGCCAAGCGGGCCTTCTCTGGCCTGG AGAGCCTGGAGCACCTGAACCTGGGCGAGAATGCCATCAGATCCGTGCAGTTCGATGCCTTTGCCAAGAT GAAGAATCTGAAGGAGCTGTACATCAGCTCCGAGAGCTTCCTGTGCGACTGTCAGCTGAAGTGGCTGCCA CCTTGGCTGATGGGAAGGATGCTGCAGGCCTTTGTGACCGCCACATGCGCCCACCCAGAGAGCCTGAAGG GCCAGAGCATCTTCTCCGTGCTGCCCGATAGCTTCGTGTGCGACGATTTCCCTAAGCCACAGATCATCAC CCAGCCAGAGACAACAATGGCCGTGGTGGGCAAGGACATCCGGTTTACATGTTCCGCCGCTCTAGCTCC TCTAGCCCCATGACCTTCGCCTGGAAGAAGGATAACGAGGTGCTGGCCAATGCCGACATGGAGAACTTCG CCCACGTGAGAGCCCAGGATGGCGAAGTGATGGAGTATACCACAATCCTGCACCTGCGGCACGTGACCTT TGGCCACGAGGGCAGATACCAGTGCATCATCACAAATCACTTCGGCTCTACCTATAGCCACAAGGCCAGG CTGACAGTGAACGTGCTGCCTAGCTTTACCAAGATCCCACACGACATCGCCATCAGAACAGGCACCACAG CAAGGCTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAGATGTG AAGATCGATGACATGGGCGTGTACTCCTGCACCGCACAGAACAGCGCCGGCAGCGTGTCCGCCAACGCCA CCCTGACCGTGCTGGAGACACCATCCCTGGCGTGCCCCTGGAGGACAGGGTGGTGACCGTGGGCGAGAC AGTGGCCTTTCAGTGTAAGGCCACCGGCTCTCCAACACCAAGGATCACCTGGCTGAAGGGCGGCAGGCCC CTGAGCCTGACAGAGCGCCACCACTTCACCCCTGGCAATCAGCTGCTGGTGGTGCAGAACGTGATGATCG ATGACGCCGGCAGGTATACATGCGAGATGAGCAATCCTCTGGGCACCGAGAGGGCACACTCCCAGCTGTC TATCCTGCCTACCCCAGGCTGCCCGAAGGATGGCACCACA Gagcctcgggccctaccatcaagccctgcccccttgcaagtgccctgcccctaatctgctgggcggac cctccgtgttc CTGTTTCCTCCAAAGCCCAAGGACACACTGATGATCTCCAGGACACCAGAGGTGACCTGCGTGGTGGTGG ACGTGAGCCACGAGGACCCCGAGGTGAAGTTCAACTGGTACGTGGATGGCGTGGAGGTGCACAATGCCAA GACCAAGCCCAGAGAGGAGCAGTACAACTCTACCTATAGGGTGGTGAGCGTGCTGACAGTGCTGCACCAG GACTGGCTGAACGGCAAGGAGTATAAGTGCAAGGTGAGCAATAAGGCCCTGCCTGCCCCAATCGAGAAGA CAATCTCCAAGGCCAAGGGCCAGCCAAGAGAGCCCCAGGTGTACACCCTGCCCCCTAGCAGGGATGAGCT GACAAAGAACCAGGTGTCCCTGACCTGTCTGGTGAAGGGCTTTTATCCCTCCGACATCGCCGTGGAGTGG GAGTCTAATGGCCAGCCTGAGAATAACTACAAGACAACCCCACCCGTGCTGGATTCTGACGGCAGCTTCT TTCTGTATTCTAAGCTGACCGTGGACAAGAGCAGGTGGCAGCAGGGCAACGTGTTCAGCTGCTCCGTGAT GCACGAAGCACTGCACAATCACTACACCCAGAAATCACTGTCACTGAGCCCTGGCAAA |
| SEQ ID NO: 27 | GCTCAGGCTGGACCTAGGGCTCCTTGGGCTGGCGCCTGCACCTGTGCAGGCGATTCTCTGGACTGCAGCG GCCGGGGCCTGGCCACACTGCCCAGGGACCTGCCTTCCTGGACCAGATCTCTGAACCTGAGCTACAATCG GCTGTCCGAGATCGATTCTGCCGCTTTTGAGGACCTGACAAATCTGCAGGAGGTGTATCTGAACAGCAAT GAGCTGACCGCAATCCCCTCCCTGGGAGCAGCCTCTATCGGCGTGGTGAGCCTGTTCCTGCAGCACAACA AGATCCTGAGCGTGGATGGCTCCCAGCTGAAGAGCTACCTGTCTCTGGAGGTGCTGGACCTGAGCTCCAA CAATATCACCGAGATCAGATCTAGCTGTTTTCCTAATGGCCTGCGGATCAGAGAGCTGAACCTGGCCTCT AATCGGATCAGCATCCTGGAGTCCGGCGCCTTCGATGGCCTGAGCAGATCCCTGCTGACACTGCGCCTGT CCAAGAACCGGATCACCCAGCTGCCCGTGAAGGCCTTTAAGCTGCCTAGGCTGACACAGCTGGACCTGAA CCGGAATAGAATCAGGCTGATCGAGGGCCTGACCTTCCAGGGCCTGGATAGCCTGGAGGTGCTGCGCCTG CAGCGGAACAATATCTCCCGCCTGACAGACGGAGCATTTTGGGGCCTGTCTAAGATGCACGTGCTGCACC TGGAGTACAATAGCCTGGTGGAGGTGAACTCTGGCAGCCTGTATGGCCTGACCGCCCTGCACCAGCTGCA CCTGTCCAACAATAGCATCAGCAGAATCCAGAGGGATGGCTGGTCCTTCTGCCAGAAGCTGCACGAGCTG ATCCTGTCTTTTAACAATCTGACCAGGCTGGACGAGGAGAGCCTGGCAGAGCTGTCCTCTCTGTCCATCC |
| | TGGGCCTGTCTCACAATGCCATCAGCCACATCGCCGAGGGCGCCTTTAAGGGCCTGAAGAGCCTGAGGGT GCTGGATCTGGACCACAACGAGATCTCTGGCACCATCGAGGATACAAGCGGCGCTTTCACAGGCCTGGAC AATCTGTCCAAGCTGACCCTGTTTGGCAACAAGATCAAGTCTGTGGCCAAGCGGGCCTTCTCTGGCCTGG AGAGCCTGGAGCACCTGAACCTGGGCGAGAATGCCATCAGATCCGTGCAGTTCGATGCCTTTGCCAAGAT GAAGAATCTGAAGGAGCTGTACATCAGCTCCGAGAGCTTCCTGTGCGACTGTCAGCTGAAGTGGCTGCCA CCTTGGCTGATGGGAAGGATGCTGCAGGCCTTTGTGACCGCCACATGCGCCCACCCAGAGAGCCTGAAGG GCCAGAGCATCTTCTCCGTGCTGCCCGATAGCTTCGTGTGCGACGATTTTCCTAAGCCACAGATCATCAC CCAGCCAGAGACAACAATGGCCGTGGTGGGCAAGGACATCCGGTTTACATGTTCCGCCGCCTCTAGCTCC TCTAGCCCCATGACCTTCGCCTGGAAGAAGGATAACGAGGTGCTGGCCAATGCCGACATGGAGAACTTCG CCCACGTGAGAGCCCAGGATGGCGAAGTGATGGAGTATACCACAATCCTGCACCTGCGGCACGTGACCTT TGGCCACGAGGGCAGATACCAGTGCATCATCACAAATCACTTCGGCTCTACCTATAGCCACAAGGCCAGG CTGACAGTGAACGTGCTGCCTAGCTTTACCAAGATCCCACACGACATCGCCATCAGAACAGGCACCACAG CAAGGCTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAGATGTG AAGATCGATGACATGGGCGTGTACTCCTGCACCGCACAGAACAGCGCCGGCAGCGTGTCCGCCAACGCCA CCCTGACCGTGCTGGAGACACCATCCCTGGCCGTGCCCCTGGAGGACAGGGTGGTGACCGTGGGCGAGAC AGTGGCCTTTCAGTGTAAGGCCACCGGCTCTCCAACACCAAGGATCACCTGGCTGAAGGGCGGCAGGCCC CTGAGCCTGACAGAGCGCCACCACTTCACCCCTGGCAATCAGCTGCTGGTGGTGCAGAACGTGATGATCG ATGACGCCGGCAGGTATACATGCGAGATGAGCAATCCTCTGGGCACCGAGAGGGCACACTCCCAGCTGTC TATCCTGCCTACCCCAGGCTGCCCGAAGGATGGCACCACA cgcaacaccggcccacggcggcgaggagaagaagaaggagaaggagaaggaggagcaggaggagcgcgaga ccaagacccccgagtgcccagccacacccagcccctggcgtgttc CTGTTTCCTCCAAAGCCCAAGGACACACTGATGATCTCCAGGACACCAGAGGTGACCTGCGTGGTGGTGG ACGTGAGCCACGAGGACCCCGAGGTGAAGTTCAACTGGTACGTGGATGGCGTGGAGGTGCACAATGCCAA GACCAAGCCCAGAGAGGAGCAGTACAACTCTACCTATAGGGTGGTGAGCGTGCTGACAGTGCTGCACCAG GACTGGCTGAACGGCAAGGAGTATAAGTGCAAGGTGAGCAATAAGGCCCTGCCTGCCCCAATCGAGAAGA CAATCTCCAAGGCCAAGGGCCAGCCAAGAGAGCCCCAGGTGTACACCCTGCCCCCTAGCAGGGATGAGCT GACAAAGAACCAGGTGTCCCTGACCTGTCTGGTGAAGGGCTTTTATCCCTCCGACATCGCCGTGGAGTGG GAGTCTAATGGCCAGCCTGAGAATAACTACAAGACAACCCCACCCGTGCTGGATTCTGACGGCAGCTTCT TTCTGTATTCTAAGCTGACCGTGGACAAGAGCAGGTGGCAGCAGGGCAACGTGTTCAGCTGCTCCGTGAT GCACGAAGCACTGCACAATCACTACACCCAGAAATCACTGTCACTGAGCCCTGGCAAA |
| SEQ ID NO: 28 | GCTCAGGCTGGACCTAGGGCTCCTTGGGCTGCCGCCTGCACCTGTGCAGGCGATTCTCTGGACTGCAGCG GCCGGGGCCTGGCCACACTGCCCAGGGACCTGCCTTCCTGGACCAGATCTCTGAACCTGAGCTACAATCG GCTGTCCGAGATCGATTCTGCCGCCTTTGAGGACCTGACAAATCTGCAGGAGGTGTATCTGAACAGCAAT GAGCTGACCGCAATCCCCTCCCTGGGAGCAGCCTCTATCGGCGTGGTGAGCCTGTTCCTGCAGCACAACA AGATCCTGAGCGTGGATGGCTCCCAGCTGAAGAGCTACCTGTCTCTGGAGGTGCTGGACCTGAGCTCCAA CAATATCACCGAGATCAGATCTAGCTGTTTTCCTAATGGCCTGCGGATCAGAGAGCTGAACCTGGCCTCT AATCGGATCAGCATCCTGGAGTCCGGCGCCTTCGATGGCCTGAGCAGATCCCTGCTGACACTGCGCCTGT CCAAGAACCGGATCACCCAGCTGCCCGTGAAGGCCTTTAAGCTGCCTAGGCTGACACAGCTGGACCTGAA CCGGAATAGAATCAGGCTGATCGAGGGCCTGACCTTCCAGGGCCTGGATAGCCTGGAGGTGCTGCGCCTG CAGCGGAACAATATCTCCCGCCTGACAGACGGAGCATTTTGGGGCCTGTCTAAGATGCACGTGCTGCACC TGGAGTACAATAGCCTGGTGGAGGTGAACTCTGGCAGCCTGTATGGCCTGACCGCCCTGCACCAGCTGCA CCTGTCCAACAATAGCATCAGCAGAATCCAGAGGGATGGCTGGTCCTTCTGCCAGAAGCTGCACGAGCTG ATCCTGTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTGAGGGT GCTGGATCTGGACCACAACGAGATCTCTGGCACCATCGAGGATACAAGCGGCGCCTTCACAGGCCTGGAC AATCTGTCCAAGCTGACCCTGTTTGGCAACAAGATCAAGTCTGTGGCCAAGCGGGCCTTCTCTGGCCTGG AGAGCCTGGAGCACCTGAACCTGGGCGAGAATGCCATCAGATCCGTGCAGTTCGATGCCTTTGCCAAGAT GAAGAATCTGAAGGAGCTGTACATCAGCTCCGAGAGCTTCCTGTGCGACTGTCAGCTGAAGTGGCTGCCA CCTTGGCTGATGGGAAGGATGCTGCAGGCCTTTGTGACCGCCACATGCGCCCACCCAGAGAGCCTGAAGG GCCAGAGCATCTTCTCCGTGCTGCCCGATAGCTTCGTGTGCGACGATTTTCCTAAGCCACAGATCATCAC |
| | CCAGCCAGAGACAACAATGGCCGTGGTGGCAAGGACATCGGTTTACATGTTCGCCGCTCTAGCTCC TCTAGCCCCATGACCTTCGCCTGGAAGAAGGATAACGAGGTGCTGGCCAATGCCGACATGGAGAACTTCG CCCACGTGAGAGCCCAGGATGGCGAAGTGATGGAGTATACCACAATCCTGCACCTGCCGCACGTGACCTT TGGCCACGAGGGCAGATACCAGTGCATCATCACAAATCACTTCGGCTCTACCTATAGCCACAAGGCCAGG CTGACAGTGAACGTGCTGCCTAGCTTTACCAAGATCCCACACGACATCGCCATCAGAACAGGCACCACAG CAAGGCTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAGATGTG AAGATCGATGACATGGGCGTGTACTCCTGCACCGCACAGAACAGCGCCGGCAGCGTGTCCGCCAACGCCA CCCTGACCGTGCTGGAGACACCATCCCTGGCCGTGCCCCTGGAGGACAGGGTGGTGACCGTGGGCGAGAC AGTGGCCTTTCAGTGTAAGGCCACCGGCTCTCCAACACCAAGGATCACCTGGCTGAAGGGCGGCAGGCCC CTGAGCCTGACAGAGCGCCACCACTTCACCCCTGGCAATCAGCTGCTGGTGGTGCAGAACGTGATGATCG ATGACGCCGGCAGGTATACATGCGAGATGAGCAATCCTCTGGGCACCGAGAGGGCACACTCCCAGCTGTC TATCCTGCCTACCCCAGGCTGCCGGAAGGATGGCACCACA gaaccgaaatcttctgacaaaacccacacctctccgccatctccggctccggaactgctgggtggttctt ctgtttc CTGTTCCCTCCAAAGCCCAAGGACACACTGATGATCTCCAGGACACCAGAGGTGACCTGCGTGGTGGTGG ACGTGAGCCACGAGGACCCCGAGGTGAAGTTCAACTGGTACGTGGATGGCGTGGAGGTGCACAATGCCAA GACCAAGCCCAGAGAGGAGCAGTACAACTCTACCTATAGGGTGGTGAGCGTGCTGACAGTGCTGCACCAG GACTGGCTGAACGGCAAGGAGTATAAGTGCAAGGTGAGCAATAAGGCCCTGCCTGCCCCAATCGAGAAGA CAATCTCCAAGGCCAAGGGCCAGCCAAGAGAGCCCCAGGTGTACACCCTGCCCCCTAGCAGGGATGAGCT GACAAAGAACCAGGTGTCCCTGACCTGTCTGGTGAAGGGCTTTTATCCCTCCGACATCGCCGTGGAGTGG GAGTCTAATGGCCAGCCTGAGAATAACTACAAGACAACCCCACCCGTGCTGGATTCTGACGGCAGCTTCT TTCTGTATTCTAAGCTGACCGTGGACAAGAGCAGGTGGCAGCAGGGCAACGTGTTCAGCTGCTCCGTGAT GCACGAAGCACTGCACAATCACTACACCCAGAAATCACTGTCACTGAGCCCTGGCAAA |

As another example of the fusion protein provided in the present invention, a fusion protein, including the extracellular domain of the Lrig-1 protein represented by SEQ ID NO: 3; the hinge region represented by any one of SEQ ID NOs: 7 to 10; and mouse IgG2-derived heavy chain constant region 2 (CH2) and heavy chain constant region 3 (CH3) which are represented by SEQ ID NO: 6, may be mentioned (see Table 10 below). The fusion proteins represented by SEQ ID NOs: 29 to 32 in Table 10 below may be encoded by the nucleic acid sequences represented by SEQ ID NOs: 33 to 36 in Table 11 below (see Table 11 below).

**[Table 10]**

| | |
|---|---|
| SEQ ID NO | Sequence information |
| SEQ ID NO: 29 | AQAGPRAPCAAACTCAGDSLDCSGRGLATLPRDLPSWTRSLNLSYNRLSEIDSAAFEDLTNLQEV YLNSNELTAIPSLGAASIGVVSLFLQHNKILSVDGSQLKSYLSLEVLDLSSNNITEIRSSCFPNG LRIRELNLASNRISILESGAFDGLSRSLLTLRLSKNRITQLPVKAFKLPRLTQLDLNRNRIRLIE GLTFQGLDSLEVLRLQRNNISRLTDGAFWGLSKMHVLHLEYNSLVEVNSGSLYGLTALHQLHLSN NSISRIQRDGWSFCQKLHELILSFNNLTRLDEESLAELSSLSILRLSHNAISHIAEGAFKGLKSL RVLDLDHNEISGTIEDTSGAFTGLDNLSKLTLFGNKIKSVAKRAFSGLESLEHLNLGENAIRSVQ FDAFAKMKNLKELYISSESFLCDCQLKWLPPWLMGRMLQAFVTATCAHPESLKGQSIFSVLPDSF VCDDFPKPQIITQPETTMAVVGKDIRFTCSAASSSSSPMTFAWKKDNEVLANADMENFAHVRAQD GEVMEYTTILHLRHVTFGHEGRYQCIITNHFGSTYSHKARLTVNVLPSFTKIPHDIAIRTGTTAR LECAATGHPNPQIAWQKDGGTDFPAARERRMHVMPDDDVFFITDVKIDDMGVYSCTAQNSAGSVS ANATLTVLETPSLAVPLEDRVVTVGETVAFQCKATGSPTPRITWLKGGRPLSLTERHHFTPGNQL LVVQNVMIDDAGRYTCEMSNPLGTERAHSQLSILPTPGCRKDGTT EPKSSDKTHTSPPCPAPELLGGPSVF IFPPKIKDVLMISLSPIVTCVVVDVSEDDPDVQISWFVNNVEVHTAQTQTHREDYNSTLRVVSAL PIQHQDWMSGKEFKCKVNNKDLPAPIERTISKPKGSVRAPQVYVLPPPEEEMTKKQVTLTCMVTD FMPEDIYVEWTNNGKTELNYKNTEPVLDSDGSYFMYSKLRVEKKNWVERNSYSCSVVHEGLHNHH TTKSFSRTPGK |
| SEQ ID NO: 30 | AQAGPRAPCAAACTCAGDSLDCSGRGLATLPRDLPSWTRSLNLSYNRLSEIDSAAFEDLTNLQEV YLNSNELTAIPSLGAASIGVVSLFLQHNKILSVDGSQLKSYLSLEVLDLSSNNITEIRSSCFPNG LRIRELNLASNRISILESGAFDGLSRSLLTLRLSKNRITQLPVKAFKLPRLTQLDLNRNRIRLIE GLTFQGLDSLEVLRLQRNNISRLTDGAFWGLSKMHVLHLEYNSLVEVNSGSLYGLTALHQLHLSN NSISRIQRDGWSFCQKLHELILSFNNLTRLDEESLAELSSLSILRLSHNAISHIAEGAFKGLKSL RVLDLDHNEISGTIEDTSGAFTGLDNLSKLTLFGNKIKSVAKRAFSGLESLEHLNLGENAIRSVQ FDAFAKMKNLKELYISSESFLCDCQLKWLPPWLMGRMLQAFVTATCAHPESLKGQSIFSVLPDSF VCDDFPKPQIITQPETTMAVVGKDIRFTCSAASSSSSPMTFAWKKDNEVLANADMENFAHVRAQD GEVMEYTTILHLRHVTFGHEGRYQCIITNHFGSTYSHKARLTVNVLPSFTKIPHDIAIRTGTTAR LECAATGHPNPQIAWQKDGGTDFPAARERRMHVMPDDDVFFITDVKIDDMGVYSCTAQNSAGSVS ANATLTVLETPSLAVPLEDRVVTVGETVAFQCKATGSPTPRITWLKGGRPLSLTERHHFTPGNQL LVVQNVMIDDAGRYTCEMSNPLGTERAHSQLSILPTPGCRKDGTT EPRGPTIKPCPPCKCPAPNLLGGPSVF IFPPKIKDVLMISLSPIVTCVVVDVSEDDPDVQISWFVNNVEVHTAQTQTHREDYNSTLRVVSAL PIQHQDWMSGKEFKCKVNNKDLPAPIERTISKPKGSVRAPQVYVLPPPEEEMTKKQVTLTCMVTD FMPEDIYVEWTNNGKTELNYKNTEPVLDSDGSYFMYSKLRVEKKNWVERNSYSCSVVHEGLHNHH TTKSFSRTPGK |
| SEQ ID NO: 31 | AQAGPRAPCAAACTCAGISLDCSGRGLATLPRDLPSWTRSLNLSYNRLSEIDSAAFEDLTNLQEV YLNSNELTAIPSLGAASIGVVSLFLQHNKILSVDGSQLKSYLSLEVLDLSSNNITEIRSSCFPNG LRIRELNLASNRISILESGAFDGLSRSLLTLRLSKNRITQLPVKAFKLPRLTQLDLNRNRIRLIE GLTFQGLISLEVLRLQRNNISRLTDGAFWGLSKMHVLHLEYNSLVEVNSGSLYGLTALHQLHLSN NSISRIQRDGWSFCQKLHELILSFNNLTRLDEESLAELSSLSILRLSHNAISHIAEGAFKGLKSL RVLDLDHNEISGTIEDTSGAFTGLDNLSKLTLFGNKIKSVAKRAFSGLESLEHLNLGENAIRSVQ FDAFAKMKNLKELYISSESFLCDCQLKWLPPWLMGRMLQAFVTATCAHPESLKGQSIFSVLPDSF VCDDFPKPQIITQPETTMAVVGKDIRFTCSAASSSSSPMTFAWKKDNEVLANADMENFAHVRAQD GEVMEYTTILHLRHVTFGHEGRYQCIITNHFGSTYSHKARLTVNVLPSFTKIPHDIAIRTGTTAR LECAATGHPNPQIAWQKDGGTDFPAARERRMHVMPDDDVFFITDVKIDDMGVYSCTAQNSAGSVS ANATLTVLETPSLAVPLEDRVVTVGETVAFQCKATGSPTPRITWLKGGRPLSLTERHHFTPGNQL LVVQNVMIDDAGRYTCEMSNPLGTERAHSQLSILPTPGCRKDGTT RNTGRGGEEKKKEKEKEEQEERETKTPECPSHTQPLGVF IFPPKIKDVLMISLSPIVTCVVVDVSEDDPDVQISWFVNNVEVHTAQTQTHREDYNSTLRVVSAL PIQHQDWMSGKEFKCKVNNKDLPAPIERTISKPKGSVRAPQVYVLPPPEEEMTKKQVTLTCMVTD FMPEDIYVEWTNNGKTELNYKNTEPVLDSDGSYFMYSKLRVEKKNWVERNSYSCSVVHEGLHNHH |
| | TTKSFSRTPGK |
| SEQ ID NO: 32 | AQAGPRAPCAAACTCAGDSLDCSGRGLATLPRDLPSWTRSLNLSYNRLSEI DSAAFDDLTNLQEV YLNSNELTAI PSLGAASI GVVSLFLQHNKI LSVDGSQLKSYLSLEVLDLSSNNI TEI RSSCFPNG LRI RELNLASNRI SI LESGAFDGLSRSLLTLRLSKNRI TQLPVKAFKLPRLTQLDLNRNRI RLI E GLTFQGLDSLEVLRLQRNNI SRLTDGAFWGLSKMHVLHLEYNSLVEVNSGSLYGLTALHQLHLSN NSI SRI QRDGWSFCQKLHELI LSFNNLTRLDEESLAELSSLSI LRLSHNAI SHI AEGAFKGLKSL RVLDLDHNEI SGTI EDTSGAFSGLDSLSKLTLFGNKI KSVAKRAFSGLEGLEHLNLGGNAI RSVQ FDAFVKMKNLKELHI SSDSFLCDCQLKWLPPWLI GRMLQAFVTATCAHPESLKGQSI FSVPPESF VCDDFLKPQI I TQPETTMAMVGKDI RFTCSAASSSSSPMTFAWKKDNEVLANADMENFAHVRAQD GEVMEYTTI LHLRHVTFGHEGRYQCI I TNHFGSTYSHKARLTVNVLPSFTKTPHDI AI RTGTTAR LECAATGHPNPQI AWQKDGGTDFPAARERRMHVMPDDDVFFI TDVKI DDAGVYSCTAQNSAGSVS ANATLTVLETPSLVVPLEDRVVSVGETVALQCKATGNPPPRI TWFKGDRPLSLTERHHLTPDNQL LVVQNVVAEDAGRYTCEMSNTLGTERAHSQLSI LPTPGCRKDGTT EPKSSDKTHTSPPSPAPELLGGSSVF I FPPKI KDVLMI SLSPI VTCVVVDVSEDDPDVQI SWFVNNVEVHTAQTQTHREDYNSTLRVVSAL PI QHQDWMSGKEFKCKVNNKDLPAPI ERTI SKPKGSVRAPQVYVLPPPEEEMTKKQVTLTCMVTD FMPEDI YVEWTNNGKTELNYKNTEPVLDSDGSYFMYSKLRVEKKNWVERNSYSCSVVHEGLHNHH TTKSFSRTPGK |

**[Table 11]**

| SEQ ID NO | Sequence information |
|---|---|
| SEQ ID NO: 33 | GCTCAGGCTGGACCTAGGGCTCCTTGCGCTGCGCCTGCACCTGTGCAGCCGATTCTCTGGACTG CAGCGGCCGGGCCTGGCCACACTGGCCAGGGACCTGCCTTCCTGGACCAGATCTCTGAACCTGA GCTACAATGGGCTGTCCGAGATCGATTCTGCCGCCTTTGAGGACCTGACAAATCTGCAGGAGGTG TATCTGAACAGCAATGAGCTGACCGCCATCCCCTCCCTGGGAGCAGCCTCTATCGGCGTGGTGAG CCTGTTCCTGCAGCACAACAAGATCCTGAGCGTGGATGGCTCCCAGCTGAAGAGCTACCTGTCTC TGGAGGTGCTGGACCTGAGCTCCAACAATATCACCGAGATCAGATCTAGCTGTTTTCCTAATGGC CTGCGGATCAGAGAGCTGAACCTGGCCTCTAATCGGATCAGCATCCTGGAGTCCGGCGCCTTCGA TGGCCTGAGCAGATCCCTGCTGACACTGCGCCTGTCCAAGAACCGGATCACCCAGCTGCCCGTGA AGGCCTTTAAGCTGCCTAGGCTGACACAGCTGGACCTGAACCGGAATAGAATCAGGCTGATCGAG GGCCTGACCTTCCAGGGCCTGGATAGCCTGGAGGTGCTGCGCCTGCAGCGGAACAATATCTCCCG CCTGACAGACGGAGCATTTTGGGGCCTGTCTAAGATGCACGTGCTGCACCTGGAGTACAATAGCC TGGTGGAGGTGAACTCTGGCAGCCTGTATGGCCTGACCGCCCTGCACCAGCTGCACCTGTCCAAC AATAGCATCAGCAGAATCCAGAGGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCCTTCAC AGGCCTGGACAATCTGTCCAAGCTGACCCTGTTTGGCAACAAGATCAAGTCTGTGGCCAAGCGGG CCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCACCTTGGCTGATGGGAAGGATGCTGCAGGCCTTTGTGACCG CCACATGCGCCCACCCAGAGAGCCTGAAGGGCCAGAGCATCTTCTCCGTGCTGCCCGATAGCTTC GTGTGCGACGATTTCCCTAAGCCACAGATCATCACCCAGCCAGAGACAACAATGGCCGTGGTGGG CAAGGACATCCGGTTTACATGTTCCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCCTGCACCGCACAGAACAGCGCCGGCAGCGTGTCC GCCAACGCCACCCTGACCGTGCTGGAGACACCATCCCTGGCCGTGCCCCTGGAGGACAGGGTGGT GACCGTGGGCGAGACAGTGGCCTTTCAGTGTAAGGCCACCGGCTCTCCAACACCAAGGATCACCT GGCTGAAGGGCGGCAGGCCCCTGAGCCTGACAGAGCGGCACCACTTCACCCCTGGCAATCAGCTG CTGGTGGTGCAGAACGTGATGATCGATGACGCCGGCAGGTATACATGCGAGATGAGCAATCCTCT GGGCACCGAGAGGGCACACTCCCAGCTGTCTATCCTGCCTACCCCAGGCTGCCGGAAGGATGGCA CCACA GAGCCAAAGTCCTCTGATAAGACACACACCTCTCCACCATGCCCAGCACCAGACCTGCTGGGAGG ACCAAGCGTGTTC atcttcccacccaagatcaaggacgtgctgatgatctccctgtcccccatcgtgacctgcgtggt ggtggacgtgtccgaggacgaccccgacgtccagatcagttggttcgtgaacaacgtggaagtcc acaccgcccagacccagacccacagagaggactacaactccaccctgcgggtggtgtccgccctg cccatccagcaccaggactggatgtccggcaaagaattcaagtgcaaagtgaacaacaaggacct gcctgcccccatcgagcggaccatctccaagcccaagggctccgtgcgggctccccaggtgtacg tgctgcccccctccagaggaagagatgaccaagaaacaggtcacactgacctgcatggtcaccgac ttcatgcccgaggacatctacgtggaatggaccaacaacggcaagaccgagctgaactacaagaa caccgagcctgtgctggactccgacggctcctacttcatgtactccaagctgcgggtggaaaaga agaactgggtcgagcggaactcctactcctgctccgtggtgcacgagggcctgcacaaccaccac accaccaagtccttctcccggacccccggcaaa |
| SEQ ID NO: 34 | CCTCAGCACTGGACCCTAGCCCTCCCTTGCACTCCCCCCTGCACCTCTGCACCCCATTCTCTCTGCACTG CACCCCCCCCCCCTCCCCACACTGCCCAGCCACCCTCCCTTCCTCCACCCAGATCTCTGAGCCTCA |
| | GCTACAATGGCTGTCCGAGATGGATTCTGCCGCTTTGAGGACCTGACAAATCTGCAGGAGGTG TATCTGAACAGCAATGAGCTGACCGCAATCCCCTCCCTGGGAGCAGCCTCTATGGGCGTGGTGAG CCTGTTCCTGCAGCACAACAAGATCCTGAGCGTGGATGGCTCCCAGCTGAAGAGCTACCTGTCTC TGGAGGTGCTGGACCTGAGCTCCAACAATATCACCGAGATCAGATCTAGCTGTTTTCCTAATGGC CTGCGGATCAGAGAGCTGAACCTGGCCTCTAATCGGATCAGCATCCTGGAGTCCGGCGCCTTCGA TGGCCTGAGCAGATCCCTGCTGACACTGCGCCTGTCCAAGAACCGGATCACCCAGCTGCCCGTGA AGGCCTTTAAGCTGCCTAGGCTGACACAGCTGGACCTGAACCGGAATAGAATCAGGCTGATCGAG GGCCTGACCTTCCAGGGCCTGGATAGCCTGGAGGTGCTGCGCCTGCAGCGGAACAATATCTCCCG CCTGACAGACGGAGCATTTTGGGGCCTGTCTAAGATGCACGTGCTGCACCTGGAGTACAATAGCC TGGTGGAGGTGAACTCTGGCAGCCTGTATGGCCTGACCGCCCTGCACCAGCTGCACCTGTCCAAC AATAGCATCAGCAGAATCCAGAGGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCCTTCAC AGGCCTGGACAATCTGTCCAAGCTGACCCTGTTTGGCAACAAGATCAAGTCTGTGGCCAAGCGGG CCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCACCTTGGCTGATGGAAGGATGCTGCAGGCCTTTGTGACCG CCACATGCGCCCACCCAGAGAGCCTGAAGGGCCAGAGCATCTTCTCCGTGCTGCCCGATAGCTTC GTGTGCGACGATTTTCCTAAGCCACAGATCATCACCCAGCCAGAGACAACAATGGCCGTGGTGGG CAAGGACATCCGGTTTACATGTTCCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCCTGCACCGCACAGAACAGCGCCGGCAGCGTGTCC GCCAACGCCACCCTGACCGTGCTGGAGACACCATCCCTGGCCGTGCCCCTGGAGGACAGGGTGGT GACCGTGGGCGAGACAGTGGCCTTTCAGTGTAAGGCCACCGGCTCTCCAACACCAAGGATCACCT GGCTGAAGGGCGGCAGGCCCCTGAGCCTGACAGAGCGCCACCACTTCACCCCTGGCAATCAGCTG CTGGTGGTGCAGAACGTGATGATCGATGACGCCGGCAGGTATACATGCGAGATGAGCAATCCTCT GGCACCGAGAGGGCACACTCCCAGCTGTCTATCCTGCCTACCCCAGGCTGCCGGAAGGATGGCA CCACA Gagcctcgggccctaccatcaagccctgccccccttgcaagtgccctgccccctaatctgctggg cggaccctccgtgttc atcttcccacccaagatcaaggacgtgctgatgatctccctgtcccccatcgtgacctgcgtggt ggtggacgtgtccgaggacgacccgacgtgcagatcagttggttcgtgaacaacgtggaagtgc acaccgcccagacccagacccacagagaggactacaactccaccctgcgggtggtgtccgccctg cccatccagcaccaggactggatgtccggcaaagaattcaagtgcaaagtgaacaacaaggacct gcctgcccccatcgagcggaccatctccaagcccaagggctccgtgcgggctccccaggtgtacg tgctgcccccctccagaggaagagatgaccaagaagcaggtcacactgacctgcatggtcaccgac ttcatgcccgaggacatctacgtggaatggaccaacaatggcaagaccgagctgaactacaagaa caccgagcctgtgctggactccgacggctcctacttcatgtactccaagctgcgggtggaaaaga agaactgggtcgagcggaactcctactcctgctccgtggtgcacgagggcctgcacaaccaccac accaccaagtccttctcccggacccccgcaaa |
| SEQ ID NO: 35 | GCTCAGGCTGGACCTAGGGCTCCTTGCGCTGCCGCCTGCACCTGTGCAGGCGATTCTCTGGACTG CAGCGGCCGGGGCCTGGCCACACTGCCCAGGGACCTGCCTTCCTGGACCAGATCTCTGAACCTGA GCTACAATCGGCTGTCCGAGATCGATTCTGCCGCCTTTGAGGACCTGACAAATCTGCAGGAGGTG TATCTGAACAGCAATGAGCTGACCGCAATCCCCTCCCTGGGAGCAGCCTCTATCGGCGTGGTGAG CCTGTTCCTGCAGCACAACAAGATCCTGAGCGTGGATGGCTCCCAGCTGAAGAGCTACCTGTCTC |
| | TGGAGGTGCTGGACCTGAGCTCCAACAATATCACCGAGATCAGATCTAGCTGTTTTCCTAATGGC CTGCGGATCAGAGAGCTGAACCTGGCTCTAATGGATCAGCATCTGGAGTCGGCGCTTCGA TGGCCTGAGCAGATCCCTGCTGACACTGCGCCTGTCCAAGAACCGGATCACCCAGCTGCCCGTGA AGGCCTTTAAGCTGCCTAGGCTGACACAGCTGGACCTGAACCGGAATAGAATCAGGCTGATCGAG GGCCTGACCTTCCAGGGCCTGGATAGCCTGGAGGTGCTGCGCCTGCAGCGGAACAATATCTCCCG CCTGACAGACGGAGCATTTTGGGGCCTGTCTAAGATGCACGTGCTGCACCTGGAGTACAATAGCC TGGTGGAGGTGAACTCTGGCAGCCTGTATGGCCTGACCGCCCTGCACCAGCTGCACCTGTCCAAC AATAGCATCAGCAGAATCCAGAGGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCTTCAC AGGCCTGGACAATCTGTCCAAGCTGACCCTGTTTGGCAACAAGATCAAGTCTGTGGCCAAGCGG CCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCACCTTGGCTGATGGGAAGGATGCTGCAGGCCTTTGTGACCG CCACATGCGCCCACCCAGAGAGCCTGAAGGGCCAGAGCATCTTCTCCGTGCTGCCCGATAGCTTC GTGTGCGACGATTTCCCTAAGCCACAGATCATCACCCAGCCAGAGACAACAATGGCCGTGGTGGG CAAGGACATCCGGTTTACATGTTCCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCCTGCACCGCACAGAACAGCGCCGGCAGCGTGTCC GCCAACGCCACCCTGACCGTGCTGGAGACACCATCCCTGGCCGTGCCCCTGGAGGACAGGGTGGT GACCGTGGGCGAGACAGTGGCCTTTCAGTGTAAGGCCACCGGCTCTCCAACACCAAGGATCACCT GGCTGAAGGGCGGCAGGCCCCTGAGCCTGACAGAGCGCCACCACTTCACCCCTGGCAATCAGCTG CTGGTGGTGCAGAACGTGATGATCGATGACGCCGGCAGGTATACATGCGAGATGAGCAATCCTCT GGGCACCGAGAGGGCACACTCCCAGCTGTCTATCCTGCCTACCCCAGGCTGCCGGAAGGATGGCA CCACA cgcaacaccggccgcggcggcgaggagaagaagaaggagaaggagaaggaggaggcaggagagcg cgagaccaagacccccgagtgcccagccacacccagcccctggcgtgttc atcttcccacccaagatcaaggacgtgctgatgatctccctgtcccccatcgtgacctgcgtggt ggtggacgtgtccgaggacgacccggacgtgcagatcagttggttcgtgaacaacgtggaagtgc acaccgcccagacccagacccacagagaggactacaactccaccctgcgggtggtgtccgccctg cccatccagcaccaggactggatgtccggcaaagaattcaagtgcaaagtgaacaacaaggacct gcctgcccccatcgagcggaccatctccaagcccaagggctccgtgcgggctccccaggtgtacg tgctgcccccctccagaggaagagatgaccaagaagcaggtcacactgacctgcatggtcaccgac ttcatgcccgaggacatctacgtggaatggaccaacaatggcaagaccgagctgaactacaagaa caccgagcctgtgctggactccgacggctcctacttcatgtactccaagctgcgggtggaaaaga agaactgggtcgagcggaactcctactcctgctccgtggtgcacgagggcctgcacaaccaccac accaccaagtccttctcccggacccccggcaaa |
| SEQ ID NO: 36 | GCTCAGGCTGGACCTAGGGCTCCTTGGGCTGCCGCCTGCACCTGTGCAGGCGATTCTCTGGACTG CAGCGGCCGGGGCTGGCCACACTGGCCAGGGACCTGCCTTCCTGGACCAGATCTCTGAACCTGA GCTACAATCGGCTGTCCGAGATCGATTCTGCCGCCTTTGAGGACCTGACAAATCTGCAGGAGGTG TATCTGAACAGCAATGAGCTGACCGCAATCCCCTCCCTGGGAGCAGCCTCTATCGGCGTGGTGAG CCTGTTCCTGCAGCACAACAAGATCCTGAGCGTGGATGGCTCCCAGCTGAAGAGCTACCTGTCTC TGGAGGTGCTGGACCTGAGCTCCAACAATATCACCGAGATCAGATCTAGCTGTTTTCCTAATGGC CTGCGGATCAGAGAGCTGAACCTGGCCTCTAATCGGATCAGCATCCTGGAGTCCGGCGCCTTCGA TGGCCTGAGCAGATCCCTGCTGACACTGCGCCTGTCCAAGAACCGGATCACCCAGCTGCCCGTGA |
| | AGGCCTTTAAGCTGCCTAGGCTGACACAGCTGGACCTGAACGGAATAGAATCAGGCTGATGGAG GGCCTGACCTTCCAGGGCCTGGATAGCCTGGAGGTGCTGCGCCTGCAGCGGAACAATATCTCCG CCTGACAGACGGAGCATTTGGGCCTGTCTAAGATGCACGTGCTGCACCTGGAGTACAATAGCC TGGTGGAGGTGAACTCTGGCAGCCTGTATGGCTGACCGCCTGCACCAGCTGCACCTGTCCAAC AATAGCATCAGCAGAATCCAGAGGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCCTTCAC AGGCCTGGACAATCTGTCCAAGCTGACCCTGTTTGGCAACAAGATCAAGTCTGTGGCCAAGCGGG CCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCACCTTGGCTGATGGAAGGATGCTGCAGGCCTTTGTGACCG CCACATGCGCCCACCCAGAGAGCCTGAAGGGCCAGAGCATCTTCTCCGTGCTGCCCGATAGCTTC GTGTGCGACGATTTCCCTAAGCCACAGATCATCACCCAGCCAGAGACAACAATGGCCGTGGTGGG CAAGGACATCCGGTTTACATGTTCCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCCTGCACCGCACAGAACAGCGCCGGCAGCGTGTCC GCCAACGCCACCCTGACCGTGCTGGAGACACCATCCCTGGCCGTGCCCCTGGAGGACAGGCTGGT GACCGTGGGCGAGACAGTGGCCTTTCAGTGTAAGGCCACCGGCTCTCCAACACCAAGGATCACCT GGCTGAAGGGCGGCAGGCCCCTGAGCCTGACAGAGCGCCACCACTTCACCCCTGGCAATCAGCTG CTGGTGGTGCAGAACGTGATGATCGATGACGCCGGCAGGTATACATGCGAGATGAGCAATCCTCT GGGCACCGAGAGGGCACACTCCCAGCTGTCTATCCTGCCTACCCAGGCTGCCGGAAGGATGGCA CCACA gaaccgaaatcttctgacaaaacccacacctctccgccgtctccggctccggaactgctgggtgg ttcttctgttttc atcttcccacccaagatcaaggacgtgctgatgatctccctgtcccccatcgtgacctgcgtggt ggtggacgtgtccgaggacgaccccgacgtgcagatcagttggttcgtgaacaacgtggaagtgc acaccgcccagacccagacccacagagaggactacaactccaccctgcgggtggtgtccgccctg cccatccagcaccaggactggatgtccggcaaagaattcaagtgcaaagtgaacaacaaggacct gcctgcccccatcgagcggaccatctccaagcccaaggctccgtgcgggctcccaggtgtacg tgctgcccctccagaggaagagatgaccaagaagcaggtcacactgacctgcatggtcaccgac ttcatgcccgaggacatctacgtggaatggaccaacaatggcaagaccgagctgaactacaagaa caccgagcctgtgctggactccgacggctcctacttcatgtactccaagctgcgggtggaaaaga agaactgggtcgagcggaactcctactcctgctccgtggtgcacgagggcctgcacaaccaccac accaccaagtccttctcccggacccccggcaaa |

As yet another example of the fusion protein provided in the present invention, a fusion protein, including the extracellular domain of the Lrig-1 protein represented by SEQ ID NO: 1; the linker represented by SEQ ID NO: 11; the hinge region represented by any one of SEQ ID NOs: 7 to 10; and the human IgG1-derived heavy chain constant region 2 (CH2) and heavy chain constant region 3 (CH3) which are represented by SEQ ID NO: 5, may be mentioned (see Table 12 below). The fusion proteins represented by SEQ ID NOs: 37 to 40 in Table 12 below may be encoded by the nucleic acid sequences represented by SEQ ID NOs: 41 to 44 in Table 13 below (see Table 13 below).

**[Table 12]**

| SEQ ID NO | Sequence information |
|---|---|
| SEQ ID NO: 37 | AGPRAPCAAACTCAGISLDCGGRGLAALPGDLPSWTRSLNLSYNKLSEIDPAGFEDLPNLQEVYL NNNELTAVPSLGAASSHVVSLFLQHNKIRSVEGSQLKAYLSLEVLDLSLNNITEVRNTCFPHGPP IKELNLAGNRIGTLELGAFDGLSRSLLTLRLSKNRITQLPVRAFKLPRLTQLDLNRNRIRLIEGL TFQGLNSLEVLKLQRNNISKLTDGAFWGLSKMHVLHLEYNSLVEVNSGSLYGLTALHQLHLSNNS IARIHRKGWSFCQKLHELVLSFNNLTRLDEESLAELSSLSVLRLSHNSISHIAEGAFKGLRSLRV LDLDHNEISGTIEDTSGAFSGLDSLSKLTLFGNKIKSVAKRAFSGLEGLEHLNLGGNAIRSVQFD AFVKMKNLKELHISSDSFLCDCQLKWLPPWLIGRMLQAFVTATCAHPESLKGQSIFSVPPESFVC DDFLKPQIITQPETTMAMVGKDIRFTCSAASSSSSPMTFAWKKDNEVLTNADMENFVHVHAQDGE VMEYTTILHLRQVTFGHEGRYQCVITNHFGSTYSHKARLTVNVLPSFTKTPHDITIRTTTVARLE CAATGHPNPQIAWQKDGGTDFPAARERRMHVMPDDDVFFITDVKIDDAGVYSCTAQNSAGSISAN ATLTVLETPSLVVPLEDRVVSVGETVALQCKATGNPPPRITWFKGDRPLSLTERHHLTPDNQLLV VQNVVAEDAGRYTCEMSNTLGTERAHSQLSVLPAAGCRKDGTTVGIF GS EPKSSDKTHT SPPCPAPELL GGPSVF LFPPKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHY TQKSLSLSPGK |
| SEQ ID NO: 38 | AGPRAPCAAACTCAGDS LDCGGRGLAALPGDLPSWTRS LNLSYNKLSE I DPAGFEDLPNLQEVYL NNNELTAVPSLGAASSHVVSLFLQHNK I RSVEGSQLKAYLSLEVLDLSLNN I TEVRNTCFPHGPP I KELNLAGNR I GTLELGAFDGLSRSLLTLRLSKNR I TQLPVRAFKLPRLTQLDLNRNR I RL I EGL TFQGLNSLEVLKLQRNN I SKLTDGAFWGLSKMHVLHLEYNSLVEVNSGSLYGLTALHQLHLSNNS I AR I HRKGWSFCQKLHELVLSFNNLTRLDEESLAELSSLSVLRLSHNS I SH I AEGAFKGLRSLRV LDLDHNE I SGT I EDTSGAFSGLDSLSKLTLFGNK I KSVAKRAFSGLEGLEHLNLGGNA I RSVQFD AFVKMKNLKELH I SSDSFLCDCQLKWLPPWL I GRMLQAFVTATCAHPESLKGQS I FSVPPESFVC DDFLKPQ I I TQPETTMAMVGKD I RFTCSAASSSSSPMTFAWKKDNEVLTNADMENFVHVHAQDGE VMEYTT I LHLRQVTFGHEGRYQCV I TNHFGSTYSHKARLTVNVLPSFTKTPHD I T I RTTTVARLE CAATGHPNPQ I AWQKDGGTDFPAARERRMHVMPDDDVFF I TDVK I DDAGVYSCTAQNSAGS I SAN ATLTVLETPSLVVPLEDRVVSVGETVALQCKATGNPPPR I TWFKGDRPLSLTERHHLTPDNQLLV VQNVVAEDAGRYTCEMSNTLGTERAHSQLSVLPAAGCRKDGTTVG I F GS EPRGPT I KPCPPCKCPAPNLLGGPSVF LFPPKPKDTLM I SRTPEVTCVVVDVSHEDPEVKFNWYVDGVEVHNAKTKPREEQYNSTYRVVSVL TVLHQDWLNGKEYKCKVSNKALPAP I EKT I SKAKGQPREPQVYTLPPSRDELTKNQVSLTCLVKG FYPSD I AVEWESNGQPENNYKTTPPVLDSDGSFFLYSKLTVDKSRWQQGNVFSCSVMHEALHNHY TQKSLSLSPGK |
| SEQ ID NO: 39 | AGPRAPCAAACTCAGDS LDCGGRGLAALPGDLPSWTRS LNLSYNKLSE I DPAGFEDLPNLQEVYL NNNELTAVPSLGAASSHVVSLFLQHNK IRSVEGSQLKAYLSLEVLDLSLNN ITEVRNTCFPHGPP IKELNLAGNRIGTLELGAFDGLSRS LLTLRLSKNR ITQLPVRAFKLPRLTQLDLNRNR IRL IEGL TFQGLNS LEVLKLQRNN ISKLTDGAFWGLSKMHVLHLEYNSLVEVNSGSLYGLTALHQLHLSNNS IAR IHRKGWSFCQKLHELVLSFNNLTRLDEESLAELSSLSVLRLSHNS ISH IAEGAFKGLRSLRV LDLDHNE ISGT IEDTSGAFSGLDSLSKLTLFGNK IKSVAKRAFSGLEGLEHLNLGGNA IRSVQFD AFVKMKNLKELH ISSDSFLCDCQLKWLPPWL IGRMLQAFVTATCAHPESLKGQS IFSVPPESFVC DDFLKPQ I ITQPETTMAMVGKD IRFTCSAASSSSSPMTFAWKKDNEVLTNADMENFVHVHAQDGE VMEYTT ILHLRQVTFGHEGRYQCV ITNHFGSTYSHKARLTVNVLPSFTKTPHD IT IRTTTVARLE CAATGHPNPQ IAWQKDGGTDFPAARERRMHVMPDDDVFF ITDVK IDDAGVYSCTAQNSAGS ISAN ATLTVLETPSLVVPLEDRVVSVGETVALQCKATGNPPPR ITWFKGDRPLSLTERHHLTPDNQLLV VQNVVAEDAGRYTCEMSNTLGTERAHSQLSVLPAAGCRKDGTTVG IF GS RNTGRGGEEKKKEKEKEEQEERETKTPECPSHTQPLGVF |
| | LFPPKPKDTLMI SRTPEVTCVVVDVSHEDPEVKFNWYVDGVEVHNAKTKPREEQYNSTYRVVSVL TVLHQDWLNGKEYKCKVSNKALPAPI EKTI SKAKGQPREPQVYTLPPSRDELTKNQVSLTCLVKG FYPS DIAVEWESNGQPENNYKTTPPVLDSDGSFFLYSKLTVDKSRWQQGNVFSCSVMHEALHNHY TQKS LSLSPGK |
| SEQ ID NO: 40 | AGPRAPCAAACTCAGDSLDCGGRGLAALPGDLPSWTRSLNLSYNKLSEIDPAGFEDLPNLQEVYL NNNELTAVPSLGAASSHVVSLFLQHNKIRSVEGSQLKAYLSLEVLDLSLNNITEVRNTCFPHGPP IKELNLAGNRIGTLELGAFDGLSRSLLTLRLSKNRITQLPVRAFKLPRLTQLDLNRNRIRLIEGL TFQGLNSLEVLKLQRNNISKLTDGAFWGLSKMHVLHLEYNSLVEVNSGSLYGLTALHQLHLSNNS IARIHRKGWSFCQKLHELVLSFNNLTRLDEESLAELSSLSVLRLSHNSISHIAEGAFKGLRSLRV LDLDHNEISGTIEDTSGAFSGLDSLSKLTLFGNKIKSVAKRAFSGLEGLEHLNLGGNAIRSVQFD AFVKMKNLKELHISSDSFLCDCQLKWLPPWLIGRMLQAFVTATCAHPESLKGQSIFSVPPESFVC DDFLKPQIITQPETTMAMVGKDIRFTCSAASSSSSPMTFAWKKDNEVLTNADMENFVHVHAQDGE VMEYTTILHLRQVTFGHEGRYQCVITNHFGSTYSHKARLTVNVLPSFTKTPHDITIRTTTVARLE CAATGHPNPQIAWQKDGGTDFPAARERRMHVMPDDDVFFITDVKIDDAGVYSCTAQNSAGSISAN ATLTVLETPSLVVPLEDRVVSVGETVALQCKATGNPPPRITWFKGDRPLSLTERHHLTPDNQLLV VQNVVAEDAGRYTCEMSNTLGTERAHSQLSVLPAAGCRKDGTTVGIF GS EPKSSDKTHTSPPSPAPELLGGSSVF LFPPKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHY TQKSLSLSPGK |

**[Table 13]**

| SEQ ID NO | Sequence information |
|---|---|
| SEQ ID NO: 41 | GCTGGGCCTGGGCTCCTTGTGCTGCCGCCTGCACATGTGCAGGCGATTCCCTGGACTGCGGCGG CAGAGGCCTGGCCGCCCTGCCTGGCGATCTGCCATCCTGGACCGGAGCCTGAACCTGAGCTACA ACAAGCTGAGCGAGATCGATCCGGCCGGCTTTGAGGACCTGCCTAACCTGCAGGAGGTGTATCTG AACAATAACGAGCTGACCGCGGTACCatccctggcgctgcttcatcacatgtcgtctctcttt tctgcagcacaacaagattcgcagcgtggaggggagccagctgaaggcctacctttccttagaag tgttagatctgagtttgaacaacatcacggaagtgcggaacacctgctttccacacggaccgcct ataaaggagctcaacctggcaggcaatcggattggcaccctggagttgggagcatttgatggtct gtcacggtcgctgctaactcttcgcctgagcaaaacaggatcacccagcttcctgtaagagcat tcaagctacccaggctgacacaactggacctcaatcggaacaggattcggctgatagagggcctc accttccaggggctcaacagcttggaggtgctgaagcttcagcgaaacaacatcagcaaactgac agatggggccttctgggactgtccaagatgcatgtgctgcacctggagtacaacagcctggtag aagtgaacagcggctcgctctacggcctcacggccctgcatcagctccacctcagcaacaattcc atcgctcgcattcaccgcaagggctggagcttctgccagaagctgcatgagttggtcctgtcctt caacaacctgacacggctggacgaggagagcctggccgagctgagcagcctgagtgtcctgcgtc tcagccacaattccatcagccacattgcggagggtgccttcaagggactcaggagcctgcgagtc ttggatctggaccataacgagatttcgggcacaatagaggacacgagcggcgccttctcagggct cgacagcctcagcaagctgactctgtttggaaacaagatcaagtctgtggctaagagagcattct cggggctggaaggcctggagcacctgaaccttggagggaatgcgatcagatctgtccagtttgat gcctttgtgaagatgaagaatcttaaagagctccatatcagcagcgacagcttcctgtgtgactg ccagctgaagtggctgccccgtggctaattggcaggatgctgcaggcctttgtgacagccacct gtgcccacccagaatcactgaagggtcagagcatttctctgtgccaccagagagttcgtgtgc gatgacttcctgaagccacagatcatcacccagccagaaccaccatggctatggtgggcaagga catccggtttacatgctcagcagccagcagcagcagctccccatgacctttgcctggaagaaag acaatgaagtcctgaccaatgcagacatggagaactttgtccacgtccacgcgcaggacgggga gtgatggagtacaccaccatcctgcacctccgtcaggtcactttcgggcacgagggccgctacca atgtgtcatcaccaaccactttggctccacctattcacataaggccaggctcaccgtgaatgtgt tgccatcattcaccaaaacgccccacgacataaccatccggaccaccaccgtggcccgcctcgaa tgtgctgccacaggtcacccaaaccctcagattgcctggcagaaggatggaggcacggatttccc cgctgcccgtgagcgacgcatgcatgtcatgccggatgacgacgtgtttttcatcactgatgtga aaatagatgacgcaggggtttacagctgtactgctcagaactcagccggttctatttcagctaat gccaccctgactgtcctagagaccccatccttggtggtccccttggaagaccgtgtggtatctgt gggagaaacagtggccctccaatgcaaagccacggggaaccctccgccccgcatcacctggttca agggggaccgcccgctgagcctcactgagcggcaccacctgacccctgacaaccagctcctggtg gttcagaacgtggtggcagaggatgcgggccgatatacctgtgagatgtccaacaccctgggcac ggagcgagctcacagccagctgagcgtcctgcccgcagcaggctgcaggaaggatgggaccacgg taggcatcttc GGATCC GAGCCAAAGTCCTCTGATAAGACCACACCTCTCCACCATGCCCAGCACCAGAGCTGCTGGAGG ACCAAGCGTGTTC CTGTTTCCTCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCCTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAAGCACTGCACAATCACTAC ACCCAGAAATCACTGTCACTGAGCCTGGCAAA |
| SEQ ID NO: 42 | GCTGGGCCTCGGGCTGCTTGTGCTGCCGGCCTGCACATGTGCAGGCGATTCCCTGGACTGCGGCGG |
| | CAGAGGCCTGGCCGCCCTGCCTGGCGATCTGCCATCCTGGACCGGAGCCTGAACCTGAGCTACA ACAAGCTGAGCGAGATCGATCCGGCGGCTTTGAGGACCTGCCTAACCTGCAGGAGGTGTATCTG AACAATAACGAGCTGACCGCGGTACCatccctggcgctgcttcatcacatgtcgtctctctctt tctgcagcacaacagattcgcagcgtggagggagccagctgaaggcctacctttccttagaag tgttagatctgagtttgaacaacatcacggaagtgcggaacacctgctttccacacggaccgcct ataaaggagctcaacctggcaggcaatcggattggcaccctggagttgggagcatttgatggtct gtcacggtcgctgctaactcttcgcctgagcaaaacaggatcacccagcttcctgtaagagcat tcaagctacccaggctgacacaactggacctcaatcggaacaggattcggctgatagagggcctc accttccagggcctcaacagcttggaggtgctgaagcttcagcgaaacaacatcagcaaactgac agatgggggccttctgggggactgtccaagatgcatgtgctgcacctggagtacaacagcctggtag aagtgaacagcggctcgctctacggcctcacggccctgcatcagctccacctcagcaacaattcc atcgctcgcattcaccgcaagggctggagcttctgccagaagctgcatgagttggtcctgtcctt caacaacctgacacggctggacgaggagagcctggccgagctgagcagcctgagtgtcctgcgtc tcagccacaattccatcagccacattgcggagggtgccttcaagggactcaggagcctgcgagtc ttggatctggaccataacgagatttcgggcacaatagaggacacgagcggcgccttctcagggct cgacagcctcagcaagctgactctgtttggaaacaagatcaagtctgtggctaagagagcattct cggggctggaaggcctggagcacctgaaccttggagggaatgcgatcagatctgtccagtttgat gcctttgtgaagatgaagaatcttaaagagctccatatcagcagcgacagcttcctgtgtgactg ccagctgaagtggctgccccgtggctaattggcaggatgctgcaggcctttgtgacagccacct gtgcccacccagaatcactgaagggtcagagcatttctctgtgccaccagagagttcgtgtgc gatgacttcctgaagccacagatcatcacccagccagaaaccaccatggctatggtgggcaagga catccggtttacatgctcagcagccagcagcagcagctcccccatgacctttgcctggaagaaag acaatgaagtcctgaccaatgcagacatggagaactttgtccacgtccacgcgcaggacggggaa gtgatggagtacaccaccatcctgcacctccgtcaggtcactttcgggcacgagggccgctacca atgtgtcatcaccaaccactttggctccacctattcacataaggccaggctcaccgtgaatgtgt tgccatcattcaccaaaacgccccacgacataaccatccggaccaccaccgtggcccgcctcgaa tgtgctgccacaggtcacccaaaccctcagattgcctggcagaaggatggaggcacggatttccc cgctgcccgtgagcgacgcatgcatgtcatgccggatgacgacgtgtttttcatcactgatgtga aaatagatgacgcagggtttacagctgtactgctcagaactcagccggttctattcagctaat gccaccctgactgtcctagagacccatccttggtggtccccttggaagaccgtggtatctgt gggagaaacagtggccctccaatgcaaagccacgggaaccctccgcccgcatcacctggttca agggaccgcccgctgagcctcactgagcggcaccacctgaccctgacaaccagctcctggtg gttcagaacgtggtggcagaggatgcgggccgatatacctgtgagatgtccaacaccctgggcac ggagcgagctcacagccagctgagcgtcctgcccgcagcaggctgcaggaaggatgggaccacgg taggcatcttc GGATCC Gagcctcgggccctaccatcaagccctgccccccttgcaagtgccctgcccctaatctgctggg cggaccctccgtgttc CTGTTCCTCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCCTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAAGCACTGCACAATCACTAC ACCCAGAAATCACTGTCACTGAGCCCTGGCAAA |
| SEQ ID NO: 43 | GCTGGGCCTCGGGCTCCTTGTGCTGCCGCCTGCACATGTGCAGGCGATTCCCTGGACTGCGCGG CAGAGCCCTGGCCGCCCTGCCTGGCGATCTGCCATCCTGGACCCGAGCCTGAACCTGAGCTACA ACAAGCTGAGCGAGATCGATCCGCCGGCTTTGAGGACCTGCCTAACCTGCAGGAGGTGTATCTG |
| | AACAATAAGGAGCTGACCCGGTACCatccctgggcgctgcttcatcacatgtcgtctctcttt tctgcagcacaacaagattcgcagcgtggagggagccagctgaaggcctacctttccttagaag tgttagatctgagtttgaacaacatcacggaagtgcggaacacctgctttccacacggaccgcct ataaaggagctcaacctggcaggcaatcggattggcaccctggagttgggagcatttgatggtct gtcacggtcgctgctaactcttcgcctgagcaaaaacaggatcacccagcttcctgtaagagcat tcaagctacccaggctgacacaactggacctcaatcggaacaggattcggctgatagagggcctc accttccagggggctcaacagcttggaggtgctgaagcttcagcgaaacaacatcagcaaactgac agatggggccttctgggggactgtccaagatgcatgtgctgcacctggagtacaacagcctggtag aagtgaacagcggctcgctctacggcctcacggccctgcatcagctccacctcagcaacaattcc atcgctcgcattcaccgcaagggctggagcttctgccagaagctgcatgagttggtcctgtcctt caacaacctgacacggctggacgaggagagcctggccgagctgagcagcctgagtgtcctgcgtc tcagccacaattccatcagccacattgcggagggtgccttcaagggactcaggagcctgcgagtc ttggatctggaccataacgagatttcgggcacaatagaggacacgagcggcgccttctcagggct cgacagcctcagcaagctgactctgtttggaaacaagatcaagtctgtggctaagagagcattct cggggctggaaggcctggagcacctgaaccttggagggaatgcgatcagatctgtccagtttgat gcctttgtgaagatgaagaatcttaaagagctccatatcagcagcgacagcttcctgtgtgactg ccagctgaagtggctgcccccgtggctaattggcaggatgctgcaggcctttgtgacagccacct gtgcccacccagaatcactgaagggtcagagcatttctctgtgccaccagagagtttcgtgtgc gatgacttcctgaagccacagatcatcacccagccagaaaccaccatggctatggtgggcaagga catccggtttacatgctcagcagccagcagcagcagctccccatgacctttgcctggaagaaag acaatgaagtcctgaccaatgcagacatggagaactttgtccacgtccacgcgcaggacggggaa gtgatggagtacaccaccatcctgcacctccgtcaggtcactttcgggcacgagggccgctacca atgtgtcatcaccaaccactttggctccacctattcacataaggccaggctcaccgtgaatgtgt tgccatcattcaccaaaacgccccacgacataaccatccggaccaccaccgtggcccgcctcgaa tgtgctgccacaggtcacccaaaccctcagattgcctggcagaaggatggaggcacggatttccc cgctgcccgtgagcgacgcatgcatgtcatgccggatgacgacgtgtttttcatcactgatgtga aaatagatgacgcaggggtttacagctgtactgctcagaactcagccggttctatttcagctaat gccaccctgactgtcctagagaccccatccttggtggtccccttggaagaccgtgtggtatctgt gggagaaacagtggccctccaatgcaaagccacggggaaccctccgccccgcatcacctggttca aggggaccgcccgctgagcctcactgagcggcaccacctgaccctgacaaccagctcctggtg gttcagaacgtggtggcagaggatgcggccgatatacctgtgagatgtccaacaccctgggcac ggagcgagctcacagccagctgagcgtcctgcccgcagcaggctgcaggaaggatggaccacgg taggcatcttc GGATCC cgcaacaccggcccggcggcgaggagaagaagaaggagaaggagaaggaggacaggaggagcg cgagaccaagacccccgagtgcccagccacacccagcccctgggcgtgttc CTGTTCCTCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCCTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAAGCACTGCACAATCACTAC ACCCAGAAATCACTGTCACTGAGCCCTGGCAAA |
| SEQ ID NO: 44 | GCTGGGCCTCGGGCTCCTGTGCTGCCGGCCTGCACATGTGCAGGCGATTCCCTGGACTGCGGCGG CAGAGGCCTGGCCGCCCTGCCTGGCGATCTGCCATCCTGGACCCGGAGCCTGAACCTGAGCTACA ACAAGCTGAGCGAGATCGATCCGGCCGGCTTTGAGGACCTGCCTAACCTGCAGGAGGTGTATCTG AACAATAACGAGCTGACCGCGGTACCatccctgggcgctgcttcatcacatgtcgtctctctctt tctgcagcacaacaagattcgcagcgtggaggggagccagctgaaggcctacctttccttagaag |
| | tgttagatctgagtttgaacaacatcacggaagtgcggaacacctgctttccacacggaccgcct ataaaggagctcaacctggcaggcaatcggattggcaccctggagttggagcatttgatggtct gtcacggtcgctgctaactcttcgcctgagcaaaacaggatcacccagcttcctgtaagagcat tcaagctacccaggctgacacaactggacctcaatcggaacaggattcggctgatagagggcctc accttccagggggctcaacagcttggaggtgctgaagcttcagcgaaacaacatcagcaaactgac agatggggccttctgggggactgtccaagatgcatgtgctgcacctggagtacaacagcctggtag aagtgaacagcggctcgctctacggcctcacggccctgcatcagctccacctcagcaacaattcc atcgctcgcattcaccgcaagggctggagcttctgccagaagctgcatgagttggtcctgtcctt caacaacctgacacggctggacgaggagagcctggccgagctgagcagcctgagtgtcctgcgtc tcagccacaattccatcagccacattgcggagggtgccttcaagggactcaggagcctgcgagtc ttggatctggaccataacgagatttcgggcacaatagaggacacgagcggcgccttctcagggct cgacagcctcagcaagctgactctgtttggaaacaagatcaagtctgtggctaagagagcattct cggggctggaaggcctggagcacctgaaccttggagggaatgcgatcagatctgtccagtttgat gcctttgtgaagatgaagaatcttaaagagctccatatcagcagcgacagcttcctgtgtgactg ccagctgaagtggctgccccgtggctaattggcaggatgctgcaggcctttgtgacagccacct gtgcccacccagaatcactgaaggtcagagcatttctctgtgccaccagagagtttcgtgtgc gatgacttcctgaagccacagatcatcacccagccagaaaccaccatggctatggtgggcaagga catccggtttacatgctcagcagccagcagcagcagctcccccatgacctttgcctggaagaaag acaatgaagtcctgaccaatgcagacatggagaactttgtccacgtccacgcgcaggacggggaa gtgatggagtacaccaccatcctgcacctccgtcaggtcactttcgggcacgagggccgctacca atgtgtcatcaccaaccactttggctccacctattcacataaggccaggctcaccgtgaatgtgt tgccatcattcaccaaaacgccccacgacataaccatccggaccaccaccgtggcccgcctcgaa tgtgctgccacaggtcacccaaaccctcagattgcctggcagaaggatggaggcacggatttccc cgctgcccgtgagcgacgcatgcatgtcatgccggatgacgacgtgtttttcatcactgatgtga aaatagatgacgcagggtttacagctgtactgctcagaactcagccggttctatttcagctaat gccaccctgactgtcctagagaccccatccttggtggtccccttggaagaccgtgtggtatctgt gggagaaacagtggccctccaatgcaaagccacggggaaccctccgcccgcatcacctggttca aggggggaccgcccgctgagcctcactgagcggcaccacctgaccctgacaaccagctcctggtg gttcagaacgtggtggcagaggatgcgggccgatatacctgtgagatgtccaacaccctgggcac ggagcgagctcacagccagctgagcgtcctgcccgcagcaggctgcaggaaggatgggaccacgg taggcatcttc GGATCC gaaccgaaatcttctgacaaaacccacacctctccgccgtctccggctccggaactgctgggtgg ttcttctgtttc CTGTTTCCTCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCCTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAGGCACTGCACAATCACTAC ACCCAGAAATCACTGTCACTGAGCCCTGGCAAA |

As still yet another example of the fusion protein provided in the present invention, a fusion protein, including the extracellular domain of the Lrig-1 protein represented by SEQ ID NO: 3; the linker represented by SEQ ID NO: 11; the hinge region represented by any one of SEQ ID NOs: 7 to 10; and the human IgG1-derived heavy chain constant region 2 (CH2) and heavy chain constant region 3 (CH3) which are represented by SEQ ID NO: 5, may be mentioned (see Table 14 below). The fusion proteins represented by SEQ ID NOs: 45 to 48 in Table 14 below may be encoded by the nucleic acid sequences represented by SEQ ID NOs: 49 to 52 in Table 15 below (see Table 15 below).

**[Table 14]**

| SEQ ID NO | Sequence information |
|---|---|
| SEQ ID NO: 45 | AQAGPRAPCAAACTCAGDSLDCSGRGLATLPRDLPSWTRSLNLSYNRLSEIDSAAFEDLTNLQEV YLNSNELTAIPSLGAASIGVVSLFLQHNKILSVDGSQLKSYLSLEVLDLSSNNITEIRSSCFPNG LRIRELNLASNRISILESGAFDGLSRSLLTLRLSKNRITQLPVKAFKLPRLTQLDLNRNRIRLIE GLTFQGLDSLEVLRLQRNNISRLTDGAFWGLSKMHVLHLEYNSLVEVNSGSLYGLTALHQLHLSN NSISRIQRDGWSFCQKLHELILSFNNLTRLDEESLAELSSLSILRLSHNAISHIAEGAFKGLKSL RVLDLDHNEISGTIEDTSGAFTGLDNLSKLTLFGNKIKSVAKRAFSGLESLEHLNLGENAIRSVQ FDAFAKMKNLKELYISSESFLCDCQLKWLPPWLMGRMLQAFVTATCAHPESLKGQSIFSVLPDSF VCDDFPKPQIITQPETTMAVVGKDIRFTCSAASSSSSPMTFAWKKDNEVLANADMENFAHVRAQD GEVMEYTTILHLRHVTFGHEGRYQCIITNHFGSTYSHKARLTVNVLPSFTKIPHDIAIRTGTTAR LECAATGHPNPQIAWQKDGGTDFPAARERRMHVMPDDDVFFITDVKIDDMGVYSCTAQNSAGSVS ANATLTVLETPSLAVPLEDRVVTVGETVAFQCKATGSPTPRITWLKGGRPLSLTERHHFTPGNQL LVVQNVMIDDAGRYTCEMSNPLGTERAHSQLSILPTPGCRKDGTT GS EPKSSDKTHT SPPCPAPELL GGPSVF LFPPKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHY TQKSLSLSPGK |
| **SEQ** ID NO: 46 | AQAGPRAPCAAACTCAGDSLDCSGRGLATLPRDLPSWTRSLNLSYNRLSEI DSAAFEDLTNLQEV YLNSNELTA I PSLGAAS I GVVSLFLQHNK I LSVDGSQLKSYLSLEVLDLSSNN I TE I RSSCFPNG LR I RELNLASNR I S I LESGAFDGLSRSLLTLRLSKNR I TQLPVKAFKLPRLTQLDLNRNR I RL I E GLTFQGLDSLEVLRLQRNN I SRLTDGAFWGLSKMHVLHLEYNSLVEVNSGSLYGLTALHQLHLSN NS I SR I QRDGWSFCQKLHEL I LSFNNLTRLDEESLAELSSLS I LRLSHNA I SH I AEGAFKGLKSL RVLDLDHNE I SGT I EDTSGAFTGLDNLSKLTLFGNK I KSVAKRAFSGLESLEHLNLGENA I RSVQ FDAFAKMKNLKELY I SSESFLCDCQLKWLPPWLMGRMLQAFVTATCAHPESLKGQS I FSVLPDSF VCDDFPKPQ I I TQPETTMAVVGKD I RFTCSAASSSSSPMTFAWKKDNEVLANADMENFAHVRAQD GEVMEYTT I LHLRHVTFGHEGRYQC I I TNHFGSTYSHKARLTVNVLPSFTK I PHD I A I RTGTTAR LECAATGHPNPQ I AWQKDGTDFPAARERRMHVMPDDDVFF I TDVK I DDMGVYSCTAQNSAGSVS ANATLTVLETPSLAVPLEDRVVTVGETVAFQCKATGSPTPR I TWLKGGRPLSLTERHHFTPGNQL LVVQNVM I DDAGRYTCEMSNPLGTERAHSQLS I LPTPGCRKDGTT GS EPRGPT I KPCPPCKCPAPNLLGGPSVF LFPPKPKDTLM I SRTPEVTCVVVDVSHEDPEVKFNWYVDGVEVHNAKTKPREEQYNSTYRVVSVL TVLHQDWLNGKEYKCKVSNKALPAP I EKT I SKAKGQPREPQVYTLPPSRDELTKNQVSLTCLVKG FYPSD I AVEWESNGQPENNYKTTPPVLDSDGSFFLYSKLTVDKSRWQQGNVFSCSVMHEALHNHY TQKSLSLSPGK |
| SEQ ID NO: 47 | AQAGPRAPCAAACTCAGDSLDCSGRGLATLPRDLPSWTRSLNLSYNRLSEI DSAAFEDLTNLQEV YLNSNELTAI PSLGAAS IGVVSLFLQHNKI LSVDGSQLKSYLSLEVLDLSSNNITEI RSSCFPNG LRI RELNLASNRI SI LESGAFDGLSRSLLTLRLSKNRI TQLPVKAFKLPRLTQLDLNRNRI RLI E GLTFQGLDSLEVLRLQRNNI SRLTDGAFWGLSKMHVLHLEYNSLVEVNSGSLYGLTALHQLHLSN NS I SRI QRDGWSFCQKLHEL I LSFNNLTRLDEESLAELSSLSI LRLSHNAI SHI AEGAFKGLKSL RVLDLDHNE I SGT I EDTSGAFTGLDNLSKLTLFGNKI KSVAKRAFSGLESLEHLNLGENAI RSVQ FDAFAKMKNLKELYI SSESFLCDCQLKWLPPWLMGRMLQAFVTATCAHPESLKGQS I FSVLPDSF VCDDFPKPQI I TQPETTMAVVGKDI RFTCSAASSSSSPMTFAWKKDNEVLANADMENFAHVRAQD GEVMEYTT I LHLRHVTFGHEGRYQC I I TNHFGSTYSHKARLTVNVLPSFTKI PHDI AI RTGTTAR LECAATGHPNPQI AWQKDGGTDFPAARERRMHVMPDDDVFFI TDVKI DDMGVYSCTAQNSAGSVS ANATLTVLETPSLAVPLEDRVVTVGETVAFQCKATGSPTPRI TWLKGGRPLSLTERHHFTPGNQL LVVQNVMI DDAGRYTCEMSNPLGTERAHSQLSI LPTPGCRKDGTT GS RNTGRGGEEKKKEKEKEEGQEERETKTPECPSHTQPLGVF |
| | LFPPKPKDTLMI SRTPEVTCVVVDVSHEDPEVKFNWYV DGVEVHNAKTKPREEQYNSTYRVVSVL TVLHQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHY TQKSLSLSPGK |
| SEQ ID NO: 48 | AQAGPRAPCAAACTCAGDSLDCSGRGLATLPRDLPSWTRSLNLSYNRLSEIDSAAFEDLTNLQEV YLNSNELTAIPSLGAASIGVVSLFLQHNKILSVDGSQLKSYLSLEVLDLSSNNITEIRSSCFPNG LRIRELNLASNRISILESGAFDGLSRSLLTLRLSKNRITQLPVKAFKLPRLTQLDLNRNRIRLIE GLTFQGLDSLEVLRLQRNNISRLTDGAFWGLSKMHVLHLEYNSLVEVNSGSLYGLTALHQLHLSN NSISRIQRDGWSFCQKLHELILSFNNLTRLDEESLAELSSLSILRLSHNAISHIAEGAFKGLKSL RVLDLDHNEISGTIEDTSGAFTGLDNLSKLTLFGNKIKSVAKRAFSGLESLEHLNLGENAIRSVQ FDAFAKMKNLKELYISSESFLCDCQLKWLPPWLMGRMLQAFVTATCAHPESLKGQSIFSVLPDSF VCDDFPKPQIITQPETTMAVVGKDIRFTCSAASSSSSPMTFAWKKDNEVLANADMENFAHVRAQD GEVMEYTTILHLRHVTFGHEGRYQCIITNHFGSTYSHKARLTVNVLPSFTKIPHDIAIRTGTTAR LECAATGHPNPQIAWQKDGGTDFPAARERRMHVMPDDDVFFITDVKIDDMGVYSCTAQNSAGSVS ANATLTVLETPSLAVPLEDRVVTVGETVAFQCKATGSPTPRITWLKGGRPLSLTERHHFTPGNQL LVVQNVMIDDAGRYTCEMSNPLGTERAHSQLSILPTPGCRKDGTT GS EPKSSDKTHTSPPSPAPELLGGSSVF LFPPKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHY TQKSLSLSPGK |

**[Table 15]**

| SEQ ID NO | Sequence information |
|---|---|
| SEQ ID NO: 49 | GCTCAGGCTGGACCTAGGCTCCTTGCGCTGGCGCCTGCACCTGTGCAGGCGATTCTCTGGACTG CAGCGGCGGGGCCTGGCCACACTGCCCAGGGACCTGCCTTCCTGGACCAGATCTCTGAACCTGA GCTACAATCGGCTGTCCGAGATCGATTCTGCGGCCTTTGAGGACCTGACAAATCTGCAGGAGGTG TATCTGAACAGCAATGAGCTGACCGCAATCCCCTCCCTGGGAGCAGCCTCTATCGGCGTGGTGAG CCTGTTCCTGCAGCACAACAAGATCCTGAGCGTGGATGGCTCCCAGCTGAAGAGCTACCTGTCTC TGGAGGTGCTGGACCTGAGCTCCAACAATATCACCGAGATCAGATCTAGCTGTTTTCCTAATGGC CTGCGGATCAGAGAGCTGAACCTGGCCTCTAATCGGATCAGCATCCTGGAGTCCGGCGCCTTCGA TGGCCTGAGCAGATCCCTGCTGACACTGCGCCTGTCCAAGAACCGGATCACCCAGCTGCCCGTGA AGGCCTTTAAGCTGCCTAGGCTGACACAGCTGGACCTGAACCGGAATAGAATCAGGCTGATCGAG GGCCTGACCTTCCAGGGCCTGGATAGCCTGGAGGTGCTGCGCCTGCAGCGGAACAATATCTCCCG CCTGACAGACGGAGCATTTTGGGGCCTGTCTAAGATGCACGTGCTGCACCTGGAGTACAATAGCC TGGTGGAGGTGAACTCTGGCAGCCTGTATGGCCTGACCGCCCTGCACCAGCTGCACCTGTCCAAC AATAGCATCAGCAGAATCCAGAGGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCCTTCAC AGGCCTGGACAATCTGTCCAAGCTGACCCTGTTTGGCAACAAGATCAAGTCTGTGGCCAAGCGGG CCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCACCTTGGCTGATGGGAAGGATGCTGCAGGCCTTTGTGACCG CCACATGCGCCCACCCAGAGAGCCTGAAGGGCCAGAGCATCTTCTCCGTGCTGCCCGATAGCTTC GTGTGCGACGATTTTCCTAAGCCACAGATCATCACCCAGCCAGAGACAACAATGGCCGTGGTGGG CAAGGACATCCGGTTTACATGTTCCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCCTGCACCGCACAGAACAGCGCCGGCAGCGTGTCC GCCAACGCCACCCTGACCGTGCTGGAGACACCATCCCTGGCCGTGCCCCTGGAGGACAGGGTGGT GACCGTGGGCGAGACAGTGGCCTTTCAGTGTAAGGCCACCGGCTCTCCAACACCAAGGATCACCT GGCTGAAGGGCGGCAGGCCCCTGAGCCTGACAGAGCGCCACCACTTCACCCCTGGCAATCAGCTG CTGGTGGTGCAGAACGTGATGATCGATGACGCCGGCAGGTATACATGCGAGATGAGCAATCCTCT GGGCACCGAGAGGGCACACTCCCAGCTGTCTATCCTGCCTACCCCAGGCTGCCGGAAGGATGGCA CCACA GGATCC GAGCCAAAGTCCTCTGATAAGACACACACCTCTCCACCATGCCCAGCACCAGAGCTGCTGGGAGG ACCAAGCGTGTTC CTGTTTCCTCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCCTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAAGCACTGCACAATCACTAC ACCCAGAAATCACTGTCACTGAGCCCTGGCAAA |
| SEQ ID NO: 50 | GCTCAGGCTGGACCTAGGGCTCCTGCCCTGCCCGCCTGCACCTGTGCCAGGCGATTCTCTGGACTG |
| | CAGCGGCCGGGGCCTGGCCACACTGCCCAGGGACCTGCCTTCCTGGACCAGATCTCTGAACCTGA GCTACAATCGGCTGTCCGAGATCGATTCTGCCGCCTTTGAGGACCTGACAAATCTGCAGGAGGTG TATCTGAACAGCAATGAGCTGACCGCAATCCCCTCCCTGGGAGCAGCCTCTATCGGCGTGGTGAG CCTGTTCCTGCAGCACAACAAGATCCTGAGCGTGGATGGCTCCCAGCTGAAGAGCTACCTGTCTC TGGAGGTGCTGGACCTGAGCTCCAACAATATCACCGAGATCAGATCTAGCTGTTTTCCTAATGGC CTGCGGATCAGAGAGCTGAACCTGGCCTCTAATCGGATCAGCATCCTGGAGTCCGGCGCCTTCGA TGGCCTGAGCAGATCCCTGCTGACACTGCGCCTGTCCAAGAACCGGATCACCCAGCTGCCCGTGA AGGCCTTTAAGCTGCCTAGGCTGACACAGCTGGACCTGAACCGGAATAGAATCAGGCTGATCGAG GGCCTGACCTTCCAGGGCCTGGATAGCCTGGAGGTGCTGCGCCTGCAGCGGAACAATATCTCCCG CCTGACAGACGGAGCATTTTGGGGCCTGTCTAAGATGCACGTGCTGCACCTGGAGTACAATAGCC TGGTGGAGGTGAACTCTGGCAGCCTGTATGGCCTGACCGCCCTGCACCAGCTGCACCTGTCCAAC AATAGCATCAGCAGAATCCAGAGGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCCTTCAC AGGCCTGGACAATCTGTCCAAGCTGACCCTGTTTGGCAACAAGATCAAGTCTGTGGCCAAGCGGG CCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCACCTTGGCTGATGGAAGGATGCTGCAGGCCTTTGTGACCG CCACATGCGCCCACCCAGAGAGCCTGAAGGGCCAGAGCATCTTCTCCGTGCTGCCCGATAGCTTC GTGTGCGACGATTTTCCTAAGCCACAGATCATCACCCAGCCAGAGACAACAATGGCCGTGGTGGG CAAGGACATCCGGTTTACATGTTCCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCCTGCACCGCACAGAACAGCGCCGGCAGCGTGTCC GCCAACGCCACCCTGACCGTGCTGGAGACACCATCCCTGGCCGTGCCCCTGGAGGACAGGGTGGT GACCGTGGGCGAGACAGTGGCCTTTCAGTGTAAGGCCACCGGCTCTCCAACACCAAGGATCACCT GGCTGAAGGGCGGCAGGCCCCTGAGCCTGACAGAGCGCCACCACTTCACCCCTGGCAATCAGCTG CTGGTGGTGCAGAACGTGATGATCGATGACGCCGGCAGGTATACATGCGAGATGAGCAATCCTCT GGGCACCGAGAGGGCACACTCCCAGCTGTCTATCCTGCCTACCCCAGGCTGCCGGAAGGATGGCA CCACA GGATCC Gagcctcggggccctaccatcaagccctgccccccttgcaagtgccctgcccctaatctgctggg cggaccctccgtgttc CTGTTTCCTCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCCTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAAGCACTGCACAATCACTAC ACCCAGAAATCACTGTCACTGAGCCCTGGCAAA |
| SEQ ID NO: 51 | GCTCAGGCTGGACCTAGGGCTCCTTGGGCTGCCGCCTGCACCTGTGCAGGCGATTCTCTGGACTG CAGCGGCCGGGGCCTGGCCACACTGCCCAGGGACCTGCCCTTCCTGGACCAGATCTCTGAACCTGA GCTACAATCGGCTGTCCGAGATCGATTCTGCCGGCTTTGAGGACCTGACAAATCTGCAGGAGGTG |
| | TATCTGAACAGCAATGAGCTGACCGCAATCCCCTCCCTGGGAGCAGCCTCTATCGGCGTGGTGAG CCTGTTCCTGCAGCACAACAAGATCCTGAGCGTGGATGGCTCCCAGCTGAAGAGCTACCTGTCTC TGGAGGTGCTGGACCTGAGCTCCAACAATATCACCGAGATCAGATCTAGCTGTTTTCCTAATGGC CTGCGGATCAGAGAGCTGAACCTGGCCTCTAATCGGATCAGCATCCTGGAGTCCGGCGCCTTCGA TGGCCTGAGCAGATCCCTGCTGACACTGCGCCTGTCCAAGAACCGGATCACCCAGCTGCCCGTGA AGGCCTTTAAGCTGCCTAGGCTGACACAGCTGGACCTGAACCGGAATAGAATCAGGCTGATCGAG GGCCTGACCTTCCAGGGCCTGGATAGCCTGGAGGTGCTGCGCCTGCAGCGGAACAATATCTCCCG CCTGACAGACGGAGCATTTTGGGGCCTGTCTAAGATGCACGTGCTGCACCTGGAGTACAATAGCC TGGTGGAGGTGAACTCTGGCAGCCTGTATGGCCTGACCGCCCTGCACCAGCTGCACCTGTCCAAC AATAGCATCAGCAGAATCCAGAGGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCCTTCAC AGGCCTGGACAATCTGTCCAAGCTGACCCTGTTTGGCAACAAGATCAAGTCTGTGGCCAAGCGGG CCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCACCTTGGCTGATGGGAAGGATGCTGCAGGCCTTTGTGACCG CCACATGCGCCCACCCAGAGAGCCTGAAGGGCCAGAGCATCTTCTCCGTGCTGCCCGATAGCTTC GTGTGCGACGATTTTCCTAAGCCACAGATCATCACCCAGCCAGAGACAACAATGGCCGTGGTGGG CAAGGACATCCGGTTTACATGTTCCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCCTGCACCGCACAGAACAGCGCCGGCAGCGTGTCC GCCAACGCCACCCTGACCGTGCTGGAGACACCATCCCTGGCCGTGCCCCTGGAGGACAGGGTGGT GACCGTGGGCGAGACAGTGGCCTTTCAGTGTAAGGCCACCGGCTCTCCAACACCAAGGATCACCT GGCTGAAGGGCGGCAGGCCCCTGAGCCTGACAGAGCGCCACCACTTCACCCCTGGCAATCAGCTG CTGGTGGTGCAGAACGTGATGATCGATGACGCCGGCAGGTATACATGCGAGATGAGCAATCCTCT GGGCACCGAGAGGGCACACTCCCAGCTGTCTATCCTGCCTACCCCAGGCTGCCGGAAGGATGGCA CCACA GGATCC cgcaacaccggccgcggcggcgaggagaagaagaaggagaaggagaaggaggagcaggaggagcg cgagaccaagacccccgagtgcccagccacacccagccccctggcgtgttc CTGTTCCTCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCCTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAAGCACTGCACAATCACTAC ACCCAGAAATCACTGTCACTGAGCCCTGGCAAA |
| SEQ ID NO: 52 | GCTCAGGCTGGACCTAGGGCTCCTTGGGCTGCCGCCTGCACCTGTGCAGGCGATTCTCTGGACTG CAGCGGCCGGGGCCTGGCCACACTGGCCAGGGACCTGCCTTCCTGGACCAGATCTCTGAACCTGA GCTACAATCGGCTGTCCGAGATCGATTCTGCCGCCTTTGAGGACCTGACAAATCTGCAGGAGGTG TATCTGAACAGCAATGAGCTGACCGCAATCCCCTCCCTGGGAGCAGCCTCTATCGGCGTGGTGAG CCTGTTCCTGCAGCACAACAAGATCCTGAGCGTGGATGGCTCCCAGCTGAAGAGCTACCTGTCTC |
| | TGGAGGTGCTGGACCTGAGCTCCAACAATATCACCGAGATCAGATCTAGCTGTTTTCCTAATGGC CTGCGGATCAGAGAGCTGAACCTGGCCTCTAATCGGATCAGCATCCTGGAGTCCGGCGCTTCGA TGGCCTGAGCAGATCCCTGCTGACACTGCGCCTGTCCAAGAACCGGATCACCCAGCTGCCCGTGA AGGCCTTTAAGCTGCCTAGGCTGACACAGCTGGACCTGAACCGGAATAGAATCAGGCTGATCGAG GGCCTGACCTTCCAGGGCCTGGATAGCCTGGAGGTGCTGCGCCTGCAGCGGAACAATATCTCCCG CCTGACAGACGGAGCATTTTGGGGCCTGTCTAAGATGCACGTGCTGCACCTGGAGTACAATAGCC TGGTGGAGGTGAACTCTGGCAGCCTGTATGGCCTGACCGCCCTGCACCAGCTGCACCTGTCCAAC AATAGCATCAGCAGAATCCAGAGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCCTTCAC AGGCCTGGACAATCTGTCCAAGCTGACCCTGTTTGGCAACAAGATCAAGTCTGTGGCCAAGCGGG CCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCACCTTGGCTGATGGGAAGGATGCTGCAGGCCTTTGTGACCG CCACATGCGCCCACCCAGAGAGCCTGAAGGGCCAGAGCATCTTCTCCGTGCTGCCCGATAGCTTC GTGTGCGACGATTTTCCTAAGCCACAGATCATCACCCAGCCAGAGACAACAATGGCCGTGGTGGG CAAGGACATCCGGTTTACATGTTCCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCCTGCACCGCACAGAACAGCGCCGGCAGCGTGTCC GCCAACGCCACCCTGACCGTGCTGGAGACACCATCCCTGGCCGTGCCCCTGGAGGACAGGGTGGT GACCGTGGGCGAGACAGTGGCCTTTCAGTGTAAGGCCACCGGCTCTCCAACACCAAGGATCACCT GGCTGAAGGGCGGCAGGCCCCTGAGCCTGACAGAGCGCCACCACTTCACCCCTGGCAATCAGCTG CTGGTGGTGCAGAACGTGATGATCGATGACGCCGGCAGGTATACATGCGAGATGAGCAATCCTCT GGGCACCGAGAGGGCACACTCCCAGCTGTCTATCCTGCCTACCCAGGCTGCCGGAAGGATGGCA CCACA GGATCC gaaccgaaatcttctgacaaaacccacacctctccgccatctccggctccggaactgctgggtgg ttcttctgtttc CTGTTTCCTCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCCTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAAGCACTGCACAATCACTAC ACCCAGAAATCACTGTCACTGAGCCCTGGCAAA |

As still yet another example of the fusion protein provided in the present invention, a fusion protein, including the extracellular domain of the Lrig-1 protein represented by SEQ ID NO: 3; the linker represented by SEQ ID NO: 11; the hinge region represented by any one of SEQ ID NOs: 7 to 10; and the mouse IgG2-derived heavy chain constant region 2 (CH2) and heavy chain constant region 3 (CH3) which are represented by SEQ ID NO: 6, may be mentioned (see Table 16 below). The fusion proteins represented by SEQ ID NOs: 53 to 56 in Table 16 below may be encoded by the nucleic acid sequences represented by SEQ ID NOs: 57 to 60 in Table 17 below (see Table 17 below).

**[Table 16]**

| SEQ ID NO | Sequence information |
|---|---|
| SEQ ID NO: 53 | AQAGPRAPCAAACTCAGDSLDCSGRGLATLPRDLPSWTRSLNLSYNRLSEIDSAAFEDLTNLQEV YLNSNELTAIPSLGAASIGVVSLFLQHNKILSVDGSQLKSYLSLEVLDLSSNNITEIRSSCFPNG LRIRELNLASNRISILESGAFDGLSRSLLTLRLSKNRITQLPVKAFKLPRLTQLDLNRNRIRLIE GLTFQGLDSLEVLRLQRNNISRLTDGAFWGLSKMHVLHLEYNSLVEVNSGSLYGLTALHQLHLSN NSISRIQRDGWSFCQKLHELILSFNNLTRLDEESLAELSSLSILRLSHNAISHIAEGAFKGLKSL RVLDLDHNEISGTIEDTSGAFTGLDNLSKLTLFGNKIKSVAKRAFSGLESLEHLNLGENAIRSVQ FDAFAKMKNLKELYISSESFLCDCQLKWLPPWLMGRMLQAFVTATCAHPESLKGQSIFSVLPDSF VCDDFPKPQIITQPETTMAVVGKDIRFTCSAASSSSSPMTFAWKKDNEVLANADMENFAHVRAQD GEVMEYTTILHLRHVTFGHEGRYQCIITNHFGSTYSHKARLTVNVLPSFTKIPHDIAIRTGTTAR LECAATGHPNPQIAWQKDGGTDFPAARERRMHVMPDDDVFFITDVKIDDMGVYSCTAQNSAGSVS ANATLTVLETPSLAVPLEDRVVTVGETVAFQCKATGSPTPRITWLKGGRPLSLTERHHFTPGNQL LVVQNVMIDDAGRYTCEMSNPLGTERAHSQLSILPTPGCRKDGTT GS EPKSSDKTHTSPPCPAPELLGGPSVF IFPPKIKDVLMISLSPIVTCVVVDVSEDDPDVQISWFVNNVEVHTAQTQTHREDYNSTLRVVSAL PIQHQDWMSGKEFKCKVNNKDLPAPIERTISKPKGSVRAPQVYVLPPPEEEMTKKQVTLTCMVTD FMPEDIYVEWTNNGKTELNYKNTEPVLDSDGSYFMYSKLRVEKKNWVERNSYSCSVVHEGLHNHH TTKSFSRTPGK |
| SEQ ID NO: 54 | AQAGPRAPCAAACTCAGDSLDCSGRGLATLPRDLPSWTRSLNLSYNRLSEIDSAAFEDLTNLQEV YLNSNELTAIPSLGAASIGVVSLFLQHNKILSVDGSQLKSYLSLEVLDLSSNNITEIRSSCFPNG LRIRELNLASNRISILESGAFDGLSRSLLTLRLSKNRITQLPVKAFKLPRLTQLDLNRNRIRLIE GLTFQGLDSLEVLRLQRNNISRLTDGAFWGLSKMHVLHLEYNSLVEVNSGSLYGLTALHQLHLSN NSISRIQRDGWSFCQKLHELILSFNNLTRLDEESLAELSSLSILRLSHNAISHIAEGAFKGLKSL RVLDLDHNEISGTIEDTSGAFTGLDNLSKLTLFGNKIKSVAKRAFSGLESLEHLNLGENAIRSVQ FDAFAKMKNLKELYISSESFLCDCQLKWLPPWLMGRMLQAFVTATCAHPESLKGQSIFSVLPDSF VCDDFPKPQIITQPETTMAVVGKDIRFTCSAASSSSSPMTFAWKKDNEVLANADMENFAHVRAQD GEVMEYTTILHLRHVTFGHEGRYQCIITNHFGSTYSHKARLTVNVLPSFTKIPHDIAIRTGTTAR LECAATGHPNPQIAWQKDGGTDFPAARERRMHVMPDDDVFFITDVKIDDMGVYSCTAQNSAGSVS ANATLTVLETPSLAVPLEDRVVTVGETVAFQCKATGSPTPRITWLKGGRPLSLTERHHFTPGNQL LVVQNVMIDDAGRYTCEMSNPLGTERAHSQLSILPTPGCRKDGTT GS EPRGPTIKPCPPCKCPAPNLLGGPSVF IFPPKIKDVLMISLSPIVTCVVVDVSEDDPDVQISWFVNNVEVHTAQTQTHREDYNSTLRVVSAL PIQHQDWMSGKEFKCKVNNKDLPAPIERTISKPKGSVRAPQVYVLPPPEEEMTKKQVTLTCMVTD FMPEDIYVEWTNNGKTELNYKNTEPVLDSDGSYFMYSKLRVEKKNWVERNSYSCSVVHEGLHNHH TTKSFSRTPGK |
| SEQ ID NO: 55 | AQAGPRAPCAAACTCAGDSLDCSGRGLATLPRDLPSWTRSLNLSYNRLSEIDSAAFEDLTNLQEV YLNSNELTAIPSLGAASIGVVSLFLQHNKILSVDGSQLKSYLSLEVLDLSSNNITEIRSSCFPNG LRIRELNLASNRISILESGAFDGLSRSLLTLRLSKNRITQLPVKAFKLPRLTQLDLNRNRIRLIE GLTFQGLDSLEVLRLQRNNISRLTDGAFWGLSKMHVLHLEYNSLVEVNSGSLYGLTALHQLHLSN NSISRIQRDGWSFCQKLHELILSFNNLTRLDEESLAELSSLSILRLSHNAISHIAEGAFKGLKSL RVLDLDHNEISGTIEDTSGAFTGLDNLSKLTLFGNKIKSVAKRAFSGLESLEHLNLGENAIRSVQ FDAFAKMKNLKELYISSESFLCDCQLKWLPPWLMGRMLQAFVTATCAHPESLKGQSIFSVLPDSF VCDDFPKPQIITQPETTMAVVGKDIRFTCSAASSSSSPMTFAWKKDNEVLANADMENFAHVRAQD GEVMEYTTILHLRHVTFGHEGRYQCIITNHFGSTYSHKARLTVNVLPSFTKIPHDIAIRTGTTAR LECAATGHPNPQIAWQKDGGTDFPAARERRMHVMPDDDVFFITDVKIDDMGVYSCTAQNSAGSVS ANATLTVLETPSLAVPLEDRVVTVGETVAFQCKATGSPTPRITWLKGGRPLSLTERHHFTPGNQL LVVQNVMIDDAGRYTCEMSNPLGTERAHSQLSILPTPGCRKDGTT GS RNTGRGGEEKKKEKEKEEGQEERETKTPECPSHTQPLGVF |
| | IFPPKIKDVLMI SLSPIVTCVVVDVSEDDPDVQISWFVNNVEVHTAQTQTHREDYNSTLRVVSAL PI QHQDWMSGKEFKCKVNNKDLPAPI ERTI SKPKGSVRAPQVYVLPPPEEEMTKKQVTLTCMVTD FMPEDI YVEWTNNGKTELNYKNTEPVLDSDGSYFMYSKLRVEKKNWVERNSYSCSVVHEGLHNHH TTKSFSRTPGK |
| SEQ ID NO: 56 | AQAGPRAFCAAACTCAGISLDCSGRGLATLPRDLPSWTRSLNLSYNRLSEIDSAAFEDLTNLQEV YLNSNELTAIPSLGAASIGVVSLFLQHNKILSVDGSQLKSYLSLEVLDLSSNNITEIRSSCFPNG LRIRELNLASNRISILESGAFDGLSRSLLTLRLSKNRITQLPVKAFKLPRLTQLDLNRNRIRLIE GLTFQGLDSLEVLRLQRNNISRLTDGAFWGLSKMHVLHLEYNSLVEVNSGSLYGLTALHQLHLSN NSISRIQRDGWSFCQKLHELILSFNNLTRLDEESLAELSSLSILRLSHNAISHIAEGAFKGLKSL RVLDLDHNEISGTIEDTSGAFTGLDNLSKLTLFGNKIKSVAKRAFSGLESLEHLNLGENAIRSVQ FDAFAKMKNLKELYISSESFLCDCQLKWLPPWLMGRMLQAFVTATCAHPESLKGQSIFSVLPDSF VCDDFPKPQIITQPETTMAVVGKDIRFTCSAASSSSSPMTFAWKKDNEVLANADMENFAHVRAQD GEVMEYTTILHLRHVTFGHEGRYQCIITNHFGSTYSHKARLTVNVLPSFTKIPHDIAIRTGTTAR LECAATGHPNPQIAWQKDGGTDFPAARERRMHVMPDDDVFFITDVKIDDMGVYSCTAQNSAGSVS ANATLTVLETPSLAVPLEDRVVTVGETVAFQCKATGSPTPRITWLKGGRPLSLTERHHFTPGNQL LVVQNVMIDDAGRYTCEMSNPLGTERAHSQLSILPTPGCRKDGTT GS EPKSSDKTHTSPPSPAPELLGGSSVF IFPPKIKDVLMISLSPIVTCVVVDVSEDDPDVQISWFVNNVEVHTAQTQTHREDYNSTLRVVSAL PIQHQDWMSGKEFKCKVNNKDLPAPIERTISKPKGSVRAPQVYVLPPPEEEMTKKQVTLTCMVTD FMPEDIYVEWTNNGKTELNYKNTEPVLDSDGSYFMYSKLRVEKKNWVERNSYSCSVVHEGLHNHH TTKSFSRTPGK |

**[Table 17]**

| SEQ ID NO | Sequence information |
|---|---|
| SEQ ID NO: 57 | GCTCAGGCTGGACCTAGGGCTCCTTGGCTGGCGCTGCACCTGTGCAGGCGATTCTCTGGACTG CAGCGGCGGGGCTGGCCACACTGGCCAGGGACCTGCCTTCCTGGACCAGATCTCTGAACCTGA GCTACAATGGCTGTCCGAGATGGATTCTGCGGCCTTTGAGGACCTGACAAATCTGCAGGAGGTG TATCTGAACAGCAATGAGCTGACCGCAATCCCCTCCCTGGGAGCAGCCTCTATCGGCGTGGTGAG CCTGTTCCTGCAGCACAACAAGATCCTGAGCGTGGATGGCTCCCAGCTGAAGAGCTACCTGTCTC TGGAGGTGCTGGACCTGAGCTCCAACAATATCACCGAGATCAGATCTAGCTGTTTTCCTAATGGC CTGCGGATCAGAGAGCTGAACCTGGCCTCTAATCGGATCAGCATCCTGGAGTCCGGCGCTTCGA TGGCCTGAGCAGATCCCTGCTGACACTGCGCCTGTCCAAGAACCGGATCACCCAGCTGCCCGTGA AGGCCTTTAAGCTGCCTAGGCTGACACAGCTGGACCTGAACCGGAATAGAATCAGGCTGATCGAG GGCCTGACCTTCCAGGGCCTGGATAGCCTGGAGGTGCTGCGCCTGCAGCGGAACAATATCTCCG CCTGACAGACGGAGCATTTTGGGGCCTGTCTAAGATGCACGTGCTGCACCTGGAGTACAATAGCC TGGTGGAGGTGAACTCTGGCAGCCTGTATGGCCTGACCGCCCTGCACCAGCTGCACCTGTCCAAC AATAGCATCAGCAGAATCCAGAGGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCTTCAC AGGCCTGGACAATCTGTCCAAGCTGACCCTGTTTGGCAACAAGATCAAGTCTGTGGCCAAGCGG CCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCACCTTGGCTGATGGGAAGGATGCTGCAGGCCTTTGTGACCG CCACATGCGCCCAGCCAGAGAGCCTGAAGGGCCAGAGCATCTTCTCCGTGCTGCCCGATAGCTTC GTGTGCGACGATTTTCCTAAGCCACAGATCATCACCCAGCCAGAGACAACAATGGCCGTGGTGGG CAAGGACATCCGGTTTACATGTTCCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCCTGCACCGCACAGAACAGCGCCGGCAGCGTGTCC GCCAACGCCACCCTGACCGTGCTGGAGACACCATCCCTGGCCGTGCCCCTGGAGGACAGGGTGGT GACCGTGGGCGAGACAGTGGCCTTTCAGTGTAAGGCCACCGGCTCTCCAACACCAAGGATCACCT GGCTGAAGGGCGGCAGGCCCCTGAGCCTGACAGAGCGCCACCACTTCACCCCTGGCAATCAGCTG CTGGTGGTGCAGAACGTGATGATGATGACGCCGGCAGGTATACATGCGAGATGAGCAATCCTCT GGGCACCGAGAGGGCACACTCCCAGCTGTCTATCCTGCCTACCCCAGGCTGCCGGAAGGATGGCA CCACA GGATCC GAGCCAAAGTCCTCTGATAAGACACACACCTCTCCACCATGCCCAGCACCAGAGCTGCTGGGAG GACCAAGCGTGTTC atcttcccacccaagatcaaggacgtgctgatgatctccctgtcccccatcgtgacctgcgtggt ggtggacgtgtccgaggacgaccccgacgtgcagatcagttggttcgtgaacaacgtggaagtgc acaccgcccagacccagacccacagagaggactacaactccaccctgcgggtggtgtccgccctg cccatccagcaccaggactggatgtccggcaaagaattcaagtgcaaagtgaacaacaaggacct gcctgcccccatcgagcggaccatctccaagcccaagggctccgtgcgggctcccaggtgtacg tgctgcccctccagaggaagagatgaccaagaagcaggtcacactgacctgcatggtcaccgac ttcatgcccgaggacatctacgtggaatggaccaacaatggcaagaccgagctgaactacaagaa caccgagcctgtgctggactccgacggctcctacttcatgtactccaagctgcgggtggaaaaga agaactgggtcgagcggaactcctactcctgctccgtggtgcacgagggcctgcacaaccaccac accaccaagtccttctcccggacccccggcaaa |
| SEQ ID NO: 58 | GCTCAGGCTGGACCTAGGGCTCCTTGGGCTGGCGCCTGCACCTGTGCAGGGGATTCTCTGGACTG |
| | CAGCGGCGGGGCCTGGCCACACTGCCCAGGGACCTGCCTTCCTGGACCAGATCTCTGAACCTGA GCTACAATGGCTGTCCGAGATCGATTCTGCCGCTTTGAGGACCTGACAAATCTGCAGGAGGTG TATCTGAACAGCAATGAGCTGACCGCAATCCCCTCCCTGGGAGCAGCCTCTATCGGCGTGGTGAG CCTGTTCCTGCAGCACAACAAGATCCTGAGCGTGGATGGCTCCCAGCTGAAGAGCTACCTGTCTC TGGAGGTGCTGGACCTGAGCTCCAACAATATCACCGAGATCAGATCTAGCTGTTTTCCTAATGGC CTGCGGATCAGAGAGCTGAACCTGGCCTCTAATCGGATCAGCATCCTGGAGTCCGGCGCCTTCGA TGGCCTGAGCAGATCCCTGCTGACACTGCGCCTGTCCAAGAACCGGATCACCCAGCTGCCCGTGA AGGCCTTTAAGCTGCCTAGGCTGACACAGCTGGACCTGAACCGGAATAGAATCAGGCTGATCGAG GGCCTGACCTTCCAGGGCCTGGATAGCCTGGAGGTGCTGCGCCTGCAGCGGAACAATATCTCCCG CCTGACAGACGGAGCATTTTGGGGCCTGTCTAAGATGCACGTGCTGCACCTGGAGTACAATAGCC TGGTGGAGGTGAACTCTGGCAGCCTGTATGGCCTGACCGCCCTGCACCAGCTGCACCTGTCCAAC AATAGCATCAGCAGAATCCAGAGGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCCTTCAC AGGCCTGGACAATCTGTCCAAGCTGACCCTGTTTGGCAACAAGATCAAGTCTGTGGCCAAGCGGG CCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCCACCTTGGCTGATGGGAAGGATGCTGCAGGCCTTTGTGACCG CCACATGGCGCCACCCAGAGAGCCTGAAGGGCCAGAGCATCTTCTCCGTGCTGCCCGATAGCTTC GTGTGCGACGATTTTCCTAAGCCACAGATCATCACCCAGCCAGAGACAACAATGGCCGTGGTGG CAAGGACATCGGTTTACATGTTCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCCTGCACCGCACAGAACAGCGCCGGCAGCGTGTCC GCCAACGCCACCCTGACCGTGCTGGAGACACCATCCCTGGCCGTGCCCCTGGAGGACAGGGTGGT GACCGTGGGCGAGACAGTGGCCTTTCAGTGTAAGGCCACCGGCTCTCCAACACCAAGGATCACCT GGCTGAAGGGCGGCAGGCCCCTGAGCCTGACAGAGCGCCACCACTTCACCCCTGGCAATCAGCTG CTGGTGGTGCAGAACGTGATGATCGATGACGCCGGCAGGTATACATGCGAGATGAGCAATCCTCT GGGCACCGAGAGGGCACACTCCCAGCTGTCTATCCTGCCTACCCCAGGCTGCCGGAAGGATGGCA CCACA GGATCC Gagcctcggggccctaccatcaagccctgcccccttgcaagtgccctgcccctaatctgctggg cggaccctccgtgttc atcttcccacccaagatcaaggacgtgctgatgatctccctgtcccccatcgtgacctgcgtggt ggtggacgtgtccgaggacgacccggacgtgcagatcagttggttcgtgaacaacgtggaagtgc acaccgcccagacccagacccacagagaggactacaactccaccctgcgggtggtgtccgccctg cccatccagcaccaggactggatgtccggcaaagaattcaagtgcaaagtgaacaacaaggacct gcctgcccccatcgagcggaccatctccaagcccaagggctccgtgcgggctccccaggtgtacg tgctgcccccctccagaggaagagatgaccaagaagcaggtcacactgacctgcatggtcaccgac ttcatgcccgaggacatctacgtggaatggaccaacaatggcaagaccgagctgaactacaagaa caccgagcctgtgctggactccgacggctcctacttcatgtactccaagctgcgggtggaaaaga agaactgggtcgagcggaactcctactcctgctccgtggtgcacgagggcctgcacaaccaccac accaccaagtccttctcccggacccccggcaaa |
| SEQ ID NO: 59 | GCTCAGGCTGGACCTAGGGCTCCTTGGGCTGCCGCCTGCACCTGTGCAGGCGATTCTCTGGACTG CAGCGGCCGGGGCCTGGCCACACTGCCCAGGGACCTGCCTTCCTGGACCAGATCTCTGAACCTGA GCTACAATCGGCTGTCCGAGATCGATTCTGCCGCCTTTGAGGACCTGACAAATCTGCAGGAGGTG |
| | TATCTGAACAGCAATGAGCTGACCGCAATCCCTCCTGGAGCAGCCTCTATCGCGTGGTGAG CCTGTTCCTGCAGCACAACAAGATCCTGAGCGTGGATGGCTCCCAGCTGAAGAGCTACCTGTCTC TGGAGGTGCTGGACCTGAGCTCCAACAATATCACCGAGATCAGATCTAGCTGTTTTCCTAATGGC CTGCGGATCAGAGAGCTGAACCTGGCCTCTAATCGGATCAGCATCCTGGAGTCCGGCGCCTTCGA TGGCCTGAGCAGATCCCTGCTGACACTGCGCCTGTCCAAGAACCGGATCACCCAGCTGCCCGTGA AGGCCTTTAAGCTGCCTAGGCTGACACAGCTGGACCTGAACCGGAATAGAATCAGGCTGATCGAG GGCCTGACCTTCCAGGGCCTGGATAGCCTGGAGGTGCTGCGCCTGCAGCGGAACAATATCTCCCG CCTGACAGACGGAGCATTTTGGGGCCTGTCTAAGATGCACGTGCTGCACCTGGAGTACAATAGCC TGGTGGAGGTGAACTCTGGCAGCCTGTATGGCCTGACCGCCCTGCACCAGCTGCACCTGTCCAAC AATAGCATCAGCAGAATCCAGAGGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCCTTCAC AGGCCTGGACAATCTGTCCAAGCTGACCCTGTTTGGCAACAAGATCAAGTCTGTGGCCAAGCGGG CCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCACCTTGGCTGATGGGAAGGATGCTGCAGGCCTTTGTGACCG CCACATGCGCCCACCCAGAGAGCCTGAAGGGCCAGAGCATCTTCTCCGTGCTGCCCGATAGCTTC GTGTGCGACGATTTTCCTAAGCCACAGATCATCACCCAGCCAGAGACAACAATGGCCGTGGTGGG CAAGGACATCCGGTTTACATGTTCCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCTGCACCGCACAGAACAGCGCCGGCAGCGTGTCC GCCAACGCCACCCTGACCGTGCTGGAGACACCATCCCTGGCCGTGCCCCTGGAGGACAGGGTGGT GACCGTGGGCGAGACAGTGGCCTTTCAGTGTAAGGCCACCGGCTCTCCAACACCAAGGATCACCT GGCTGAAGGGCGGCAGGCCCCTGAGCCTGACAGAGCGCCACCACTTCACCCCTGGCAATCAGCTG CTGGTGGTGCAGAACGTGATGATCGATGACGCCGGCAGGTATACATGCGAGATGAGCAATCCTCT GGGCACCGAGAGGGCACACTCCCAGCTGTCTATCCTGCCTACCCCAGGCTGCCGGAAGGATGGCA CCACA GGATCC cgcaacaccggcccgcggcggcgaggagaagaagaaggagaaggagaaggaggagcaggaggagcg cgagaccaagacccccgagtgccccagccacacccagcccctgggcgtgttc atcttcccacccaagatcaaggacgtgctgatgatctccctgtcccccatcgtgacctgcgtggt ggtggacgtgtccgaggacgaccccgacgtgcagatcagttggttcgtgaacaacgtggaagtgc acaccgcccagacccagacccacagagaggactacaactccaccctgcgggtggtgtccgccctg cccatccagcaccaggactggatgtccggcaaagaattcaagtgcaaagtgaacaacaaggacct gcctgcccccatcgagcggaccatctccaagcccaagggctccgtgcgggctccccaggtgtacg tgctgcccctccagaggaagagatgaccaagaagcaggtcacactgacctgcatggtcaccgac ttcatgcccgaggacatctacgtggaatggaccaacaatggcaagaccgagctgaactacaagaa caccgagcctgtgctggactccgacggctcctacttcatgtactccaagctgcgggtggaaaaga agaactgggtcgagcggaactcctactcctgctccgtggtgcacgagggcctgcacaaccaccac accaccaagtccttctcccggacccccggcaaa |
| SEQ ID NO: 60 | GCTCAGGCTGGACCTAGGGCTCCTTGGGCTGGCGCCTGCACCTGTGCAGGCGATTCTCTGGACTG CAGCGGCCGGGGCCTGGCCACACTGCCCAGGGACCTGCCTTCCTGGACCAGATCTCTGAACCTGA GCTACAATGGCTGTCCGAGATGGATTCTGCGGCTTTGAGGACCTGACAAATCTGCAGGAGGTG TATCTGAACAGCAATGAGCTGACCGCAATCCCCTCCCTGGGAGCAGCCTCTATCGGCGTGGTGAG CCTGTTCCTGCAGCACAACAAGATCCTGAGCGTGGATGGCTCCCAGCTGAAGAGCTACCTGTCTC |
| | TGGAGGTGCTGGACCTGAGCTCCAACAATATCACCGAGATCAGATCTAGCTGTTTTCCTAATGGC CTGCGGATCAGAGAGCTGAACCTGGCCTCTAATCGGATCAGCATCCTGGAGTCCGGCGCCTTCGA TGGCCTGAGCAGATCCCTGCTGACACTGCGCCTGTCCAAGAACCGGATCACCCAGCTGCCCGTGA AGGCCTTTAAGCTGCCTAGGCTGACACAGCTGGACCTGAACCGGAATAGAATCAGGCTGATCGAG GGCCTGACCTTCCAGGGCCTGGATAGCCTGGAGGTGCTGCGCCTGCAGCGGAACAATATCTCCCG CCTGACAGACGGAGCATTTTGGGGCCTGTCTAAGATGCACGTGCTGCACCTGGAGTACAATAGCC TGGTGGAGGTGAACTCTGGCAGCCTGTATGGCCTGACCGCCCTGCACCAGCTGCACCTGTCCAAC AATAGCATCAGCAGAATCCAGAGGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCCTTCAC AGGCCTGGACAATCTGTCCAAGCTGACCCTGTTTGGCAACAAGATCAAGTCTGTGGCCAAGCGGG CCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCACCTTGGCTGATGGGAAGGATGCTGCAGGCCTTTGTGACCG CCACATGCGCCCACCCAGAGAGCCTGAAGGGCCAGAGCATCTTCTCCGTGCTGCCCGATAGCTTC GTGTGCGACGATTTTCCTAAGCCACAGATCATCACCCAGCCAGAGACAACAATGGCCGTGGTGGG CAAGGACATCCGGTTTACATGTTCCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCCTGCACCGCACAGAACAGCGCCGGCAGCGTGTCC GCCAACGCCACCCTGACCGTGCTGGAGACACCATCCTGGCCGTGCCCCTGGAGGACAGGGTGGT GACCGTGGGCGAGACAGTGGCCTTTCAGTGTAAGGCCACCGGCTCTCCAACACCAAGGATCACCT GGCTGAAGGGCGGCAGGCCCCTGAGCCTGACAGAGCGCCACCACTTCACCCCTGGCAATCAGCTG CTGGTGGTGCAGAACGTGATGATCGATGACGCCGGCAGGTATACATGCGAGATGAGCAATCCTCT GGGCACCGAGAGGGCACACTCCCAGCTGTCTATCCTGCCTACCCCAGGCTGCCGGAAGGATGGCA CCACA GGATCC gaaccgaaatcttctgacaaaacccacacctctccgccgtctccggctccggaactgctgggtgg ttcttctgtttc atcttcccacccaagatcaaggacgtgctgatgatctccctgtcccccatcgtgacctgcgtggt ggtggacgtgtccgaggacgaccccgacgtgcagatcagttggttcgtgaacaacgtggaagtgc acaccgcccagacccagacccacagagaggactacaactccaccctgcgggtggtgtccgccctg cccatccagcaccaggactggatgtccggcaaagaattcaagtgcaaagtgaacaacaaggacct gcctgcccccatcgagcggaccatctccaagcccaagggctccgtgcgggctcccaggtgtacg tgctgcccctccagaggaagagatgaccaagaagcaggtcacactgacctgcatggtcaccgac ttcatgcccgaggacatctacgtggaatggaccaacaatggcaagaccgagctgaactacaagaa caccgagcctgtgctggactccgacggctcctacttcatgtactccaagctgcgggtggaaaaga agaactgggtcgagcggaactcctactcctgctccgtggtgcacgagggcctgcacaaccaccac accaccaagtccttctcccggacccccggcaaa |

As still yet another example of the fusion protein provided in the present invention, a fusion protein, including the extracellular domain of the Lrig-1 protein represented by SEQ ID NO: 1; the linker represented by SEQ ID NO: 12; the linker represented by SEQ ID NO: 11; the hinge region represented by any one of SEQ ID NOs: 7 to 10; and the human IgG1-derived heavy chain constant region 2 (CH2) and heavy chain constant region 3 (CH3) which are represented by SEQ ID NO: 5, may be mentioned (see Table 18 below). The fusion proteins represented by SEQ ID NOs: 61 to 64 in Table 18 below may be encoded by the nucleic acid sequences represented by SEQ ID NOs: 65 to 68 in Table 19 (see Table 19 below).

**[Table 18]**

| SEQ ID NO | Sequence of information |
|---|---|
| SEQ ID NO: 61 | AGPRAFCAAACTCAGISLIDCGGRGLAALPGDLPSWTRSLNLSYNKLSEIDPAGFEDLPNLQEVYL NNNELTAVPSLGAASSHVVSLFLCHNKIRSVEGSQLKAYLSLEVLDLSLNNITEVRNTCFPHGPP IKELNLAGNRIGTLELGAFDGLSRSLLTLRLSKNRITQLPVRAFKLPRLTQLDLNRNRIRLIEGL TFQGLNSLEVLKLQRNNISKLTDGAFWGLSKMHVLHLEYNSLVEVNSGSLYGLTALHQLHLSNNS IARIHRKGWSFCQKLHELVLSFNNLTRLDEESLAELSSLSVLRLSHNSISHIAEGAFKGLRSLRV LDLDHNEISGTIEDTSGAFSGLDSLSKLTLFGNKIKSVAKRAFSGLEGLEHLNLGGNAIRSVQFD AFVKMKNLKELHISSDSFLCDCQLKWLPPWLIGRMLQAFVTATCAHPESLKGQSIFSVPPESFVC DDFLKPQIITQPETTMAMVGKDIRFTCSAASSSSSPMTFAWKKDNEVLTNADMENFVHVHAQDGE VMEYTTILHLRQVTFGHEGRYQCVITNHFGSTYSHKARLTVNVLPSFTKTPHDITIRTTTVARLE CAATGHPNPQIAWQKDGGTDFPAARERRMHVMPDDDVFFITDVKIDDAGVYSCTAQNSAGSISAN ATLTVLETPSLVVPLEDRVVSVGETVALQCKATGNPPPRITWFKGDRPLSLTERHHLTPDNQLLV VQNVVAEDAGRYTCEMSNTLGTERAHSQLSVLPAAGCRKDGTTVGIF GGG GS EPKSSDKTHT SPPCPAPELL GGPSVF LFPPKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHY TQKSLSLSPGK |
| SEQ ID NO: 62 | AGPRAPCAAACTCAGDSLDCGGRGLAALPGDLPSWTRSLNLSYNKLSEIDPAGFEDLPNLQEVYL NNNELTAVPSLGAASSHVVSLFLQHNKIRSVEGSQLKAYLSLEVLDLSLNNITEVRNTCFPHGPP IKELNLAGNRIGTLELGAFDGLSRSLLTLRLSKNRITQLPVRAFKLPRLTQLDLNRNRIRLIEGL TFQGLNSLEVLKLQRNNISKLTDGAFWGLSKMHVLHLEYNSLVEVNSGSLYGLTALHQLHLSNNS IARIHRKGWSFCQKLHELVLSFNNLTRLDEESLAELSSLSVLRLSHNSISHIAEGAFKGLRSLRV LDLDHNEISGTIEDTSGAFSGLDSLSKLTLFGNKIKSVAKRAFSGLEGLEHLNLGGNAIRSVQFD AFVKMKNLKELHISSDSFLCDCQLKWLPPWLIGRMLQAFVTATCAHPESLKGQSIFSVPPESFVC DDFLKPQIITQPETTMAMVGKDIRFTCSAASSSSSPMTFAWKKDNEVLTNADMENFVHVHAQDGE VMEYTTILHLRQVTFGHEGRYQCVITNHFGSTYSHKARLTVNVLPSFTKTPHDITIRTTTVARLE CAATGHPNPQIAWQKDGGTDFPAARERRMHVMPDDDVFFITDVKIDDAGVYSCTAQNSAGSISAN ATLTVLETPSLVVPLEDRVVSVGETVALQCKATGNPPPRITWFKGDRPLSLTERHHLTPDNQLLV VQNVVAEDAGRYTCEMSNTLGTERAHSQLSVLPAAGCRKDGTTVGIF GGG GS EPRGPTIKPCPPCKCPAPNLLGGPSVF LFPPKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHY TQKSLSLSPGK |
| SEQ ID NO: 63 | AGPRAPCAAACTCAGDSLDCGGRGLAALPGDLPSWTRSLNLSYNKLSEIDPAGFEDLPNLQEVYL NNNELTAVPSLGAASSHVVSLFLQHNKIRSVEGSQLKAYLSLEVLDLSLNNITEVRNTCFPHGPP IKELNLAGNRIGTLELGAFDGLSRSLLTLRLSKNRITQLPVRAFKLPRLTQLDLNRNRIRLIEGL TFQGLNSLEVLKLQRNNISKLTDGAFWGLSKMHVLHLEYNSLVEVNSGSLYGLTALHQLHLSNNS IARIHRKGWSFCQKLHELVLSFNNLTRLDEESLAELSSLSVLRLSHNSISHIAEGAFKGLRSLRV LDLDHNEISGTIEDTSGAFSGLDSLSKLTLFGNKIKSVAKRAFSGLEGLEHLNLGGNAIRSVQFD AFVKMKNLKELHISSDSFLCDCQLKWLPPWLIGRMLQAFVTATCAHPESLKGQSIFSVPPESFVC DDFLKPQIITQPETTMAMVGKDIRFTCSAASSSSSPMTFAWKKDNEVLTNADMENFVHVHAQDGE VMEYTTILHLRQVTFGHEGRYQCVITNHFGSTYSHKARLTVNVLPSFTKTPHDITIRTTTVARLE CAATGHPNPQIAWQKDGGTDFPAARERRMHVMPDDDVFFITDVKIDDAGVYSCTAQNSAGSISAN ATLTVLETPSLVVPLEDRVVSVGETVALQCKATGNPPPRITWFKGDRPLSLTERHHLTPDNQLLV VQNVVAEDAGRYTCEMSNTLGTERAHSQLSVLPAAGCRKDGTTVGIF |
| | GGG GS RNTGRGGEEKKKEKEKEEQEERETKTPECPSHTQPLGVF LFPPKPKDTLMI SRTPEVTCVVVDVSHEDPEVKFNWYVDGVEVHNAKTKPREEQYNSTYRVVSVL TVLHQDWLNGKEYKCKVSNKALPAPIEKTI SKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHY TQKSLSLSPGK |
| SEQ ID NO: 64 | AGPRAPCAAACTCAGDSLDCGGRGLAALPGDLPSWTRSLNLSYNKLSE IDPAGFEDLPNLQEVYL NNNELTAVPSLGAASSHVVSLFLQHNKI RSVEGSQLKAYLSLEVLDLSLNN ITEVRNTCFPHGPP IKELNLAGNR IGTLELGAFDGLSRSLLTLRLSKNR ITQLPVRAFKLPRLTQLDLNRNR IRL IEGL TFQGLNSLEVLKLQRNN ISKLTDGAFWGLSKMHVLHLEYNSLVEVNSGSLYGLTALHQLHLSNNS IAR IHRKGWSFCQKLHELVLSFNNLTRLDEESLAELSSLSVLRLSHNS ISH IAEGAFKGLRSLRV LDLDHNE ISGT IEDTSGAFSGLDSLSKLTLFGNK IKSVAKRAFSGLEGLEHLNLGGNA IRSVQFD AFVKMKNLKELH ISSDSFLCDCQLKWLPPWL IGRMLQAFVTATCAHPESLKGQS IFSVPPESFVC DDFLKPQ I ITQPETTMAMVGKD IRFTCSAASSSSSPMTFAWKKDNEVLTNADMENFVHVHAQDGE VMEYTT ILHLRQVTFGHEGRYQCV ITNHFGSTYSHKARLTVNVLPSFTKTPHD IT IRTTTVARLE CAATGHPNPQ IAWQKDGGTDFPAARERRMHVMPDDDVFF ITDVK IDDAGVYSCTAQNSAGS ISAN ATLTVLETPSLVVPLEDRVVSVGETVALQCKATGNPPPR ITWFKGDRPLSLTERHHLTPDNQLLV VQNVVAEDAGRYTCEMSNTLGTERAHSQLSVLPAAGCRKDGTTVG I F GGG GS EPKSSDKTHTSPPSPAPELLGGSSVF LFPPKPKDTLM ISRTPEVTCVVVDVSHEDPEVKFNWYVDGVEVHNAKTKPREEQYNSTYRVVSVL TVLHQDWLNGKEYKCKVSNKALPAP IEKT ISKAKGQPREPQVYTLPPSRDELTKNQVSLTCLVKG FYPSD IAVEWESNGQPENNYKTTPPVLDSDGSFFLYSKLTVDKSRWQQGNVFSCSVMHEALHNHY TQKSLSLSPGK |

**[Table 19]**

| SEO ID NO | Sequence information |
|---|---|
| SEQ ID NO: 65 | GCTGGGCCTGGGCTCCTTGTGCTGCCGCTGCACATGTGCAGGCGATTCCCTGGACTGGGCGG CAGAGGCTGGCCGCCCTGCCTGGCGATCTGCCATCCTGGACCGGAGCCTGAACCTGAGCTACA ACAAGCTGAGCGAGATCGATCCGCCGGCTTTGAGGACCTGCCTAACCTGCAGGAGGTGTATCTG AACAATAACGAGCTGACCGCGGTACCatccctggcgctgcttcatcacatgtcgtctctctctt tctgcagcacaacaagattcgcagcgtggagggagccagctgaaggcctacctttccttagaag tgttagatctgagtttgaacaacatcacggaagtgcggaacacctgctttccacacggaccgcct ataaaggagctcaacctggcaggcaatcggattggcaccctggagttggagcatttgatggtct gtcacggtcgctgctaactcttcgcctgagcaaaacaggatcacccagcttcctgtaagagcat tcaagctacccaggctgacacaactggacctcaatcggaacaggattcggctgatagagggcctc accttccaggggctcaacagcttggaggtgctgaagcttcagcgaaacaacatcagcaaactgac agatggggccttctggggactgtccaagatgcatgtgctgcacctggagtacaacagcctggtag aagtgaacagcggctcgctctacggcctcacggccctgcatcagctccacctcagcaacaattcc atcgctcgcattcaccgcaagggctggagcttctgccagaagctgcatgagttggtcctgtcctt caacaacctgacacggctggacgaggagagcctggccgagctgagcagcctgagtgtcctgcgtc tcagccacaattccatcagccacattgcggagggtgccttcaagggactcaggagcctgcgagtc ttggatctggaccataacgagatttcgggcacaatagaggacacgagcggcgccttctcagggct cgacagcctcagcaagctgactctgtttggaaacaagatcaagtctgtggctaagagagcattct cggggctggaaggcctggagcacctgaaccttggagggaatgcgatcagatctgtccagtttgat gcctttgtgaagatgaagaatcttaaagagctccatatcagcagcgacagcttcctgtgtgactg ccagctgaagtggctgccccgtggctaattggcaggatgctgcagcctttgtgacagccacct gtgcccacccagaatcactgaagggtcagagcatttctctgtgccaccagagagtttcgtgtgc gatgacttcctgaagccacagatcatcacccagccagaaaccaccatggctatggtgggcaagga catccggtttacatgctcagcagccagcagcagcagctcccccatgacctttgcctggaagaaag acaatgaagtcctgaccaatgcagacatggagaactttgtccacgtccacgcgcaggacggggaa gtgatggagtacaccaccatcctgcacctccgtcaggtcactttcgggcacgagggccgctacca atgtgtcatcaccaaccactttggctccacctattcacataaggccaggctcaccgtgaatgtg tgccatcattcaccaaaacgccccacgacataaccatccggaccaccaccgtggcccgcctcgaa tgtgctgccacaggtcacccaaaccctcagattgcctggcagaaggatggaggcacggatttccc cgctgcccgtgagcgacgcatgcatgtcatgccggatgacgacgtgtttttcatcactgatgtga aaatagatgacgcaggggtttacagctgtactgctcagaactcagccggttctattcagctaat gccaccctgactgtcctagagacccatccttggtggtccccttggaagaccgtgtggtatctgt gggagaaacagtggccctccaatgcaaagccacggggaaccctccgccccgcatcacctggttca aggggaccgcccgctgagcctcactgagcggcaccacctgaccctgacaaccagctcctggtg gttcagaacgtggtggcagaggatgcggggccgatatacctgtgagatgtccaacaccctgggcac ggagcgagctcacagccagctgagcgtcctgcccgcagcaggctgcaggaaggatgggaccacgg taggcatcttc GGCGGTGGC GGATCC GAGCCAAAGTCCTCTGATAAGACACACACCTCTCCACCATGCCCAGCACCAGAGCTGCTGGAGG ACCAAGCGTGTTC CTGTTTCCTCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCCTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAAGCACTGCACAATCACTAC ACCCAGAAATCACTGTCACTGAGCCCTGGCAAA |
| SEQ ID NO: 66 | GCTGGGCCTCGGGCTCCTTGTGCTGCGGCCTGCACATGTGCAGGCGATTCCCTGGACTGCGCGG CAGAGGCCTGGCCGCCCTGCCTGGCGATCTGCCATCCTGGACCGGAGCCTGAACCTGAGCTACA ACAAGCTGAGCGAGATCGATCCGCCGGCTTTGAGGACCTGCCTAACCTGCAGGAGGTGTATCTG AACAATAACGAGCTGACCGCGGTACCatccctgggcgctgcttcatcacatgtcgtctctctctt tctgcagcacaacaagattcgcagcgtggaggggagccagctgaaggcctacctttccttagaag tgttagatctgagtttgaacaacatcacggaagtgcggaacacctgctttccacacggaccgcct ataaaggagctcaacctggcaggcaatcggattggcaccctggagttgggagcatttgatggtct gtcacggtcgctgctaactcttcgcctgagcaaaaacaggatcacccagcttcctgtaagagcat tcaagctacccaggctgacacaactggacctcaatcggaacaggattcggctgatagagggcctc accttccaggggctcaacagcttggaggtgctgaagcttcagcgaaacaacatcagcaaactgac agatggggccttctgggactgtccaagatgcatgtgctgcacctggagtacaacagcctggtag aagtgaacagcggctcgctctacggcctcacggccctgcatcagctccacctcagcaacaattcc atcgctcgcattcaccgcaagggctggagcttctgccagaagctgcatgagttggtcctgtcctt caacaacctgacacggctggacgaggagagcctggccgagctgagcagcctgagtgtcctgcgtc tcagccacaattccatcagccacattgcggagggtgccttcaagggactcaggagcctgcgagtc ttggatctggaccataacgagatttcgggcacaatagaggacacgagcggcgccttctcagggct cgacagcctcagcaagctgactctgtttggaaacaagatcaagtctgtggctaagagagcattct cggggctggaaggcctggagcacctgaaccttggagggaatgcgatcagatctgtccagtttgat gcctttgtgaagatgaagaatcttaaagagctccatatcagcagcgacagcttcctgtgtgactg ccagctgaagtggctgcccccgtggctaattggcaggatgctgcaggcctttgtgacagccacct gtgcccacccagaatcactgaagggtcagagcatttctctgtgccaccagagagtttcgtgtgc gatgacttcctgaagccacagatcatcacccagccagaaaccaccatggctatggtgggcaagga catccggtttacatgctcagcagccagcagcagcagctcccccatgacctttgcctggaagaaag acaatgaagtcctgaccaatgcagacatggagaactttgtccacgtccacgcgcaggacggggaa gtgatggagtacaccaccatcctgcacctccgtcaggtcactttcgggcacgagggccgctacca atgtgtcatcaccaaccactttggctccacctattcacataaggccaggctcaccgtgaatgtgt tgccatcattcaccaaaacgccccacgacataaccatccggaccaccaccgtggcccgcctcgaa tgtgctgccacaggtcacccaaaccctcagattgcctggcagaaggatggaggcacggatttccc cgctgcccgtgagcgacgcatgcatgtcatgccggatgacgacgtgtttttcatcactgatgtga aaatagatgacgcaggggtttacagctgtactgctcagaactcagccggttctatttcagctaat gccaccctgactgtcctagagacccatccttggtggtccccttggaagaccgtgtggtatctgt gggagaaacagtggccctccaatgcaaagccacggggaacctccgcccccgcatcacctggttca aggggaaccgcccgctgagcctcactgagcggcaccacctgacccctgacaaccagctcctggtg gttcagaacgtggtggcagaggatgcgggccgatatacctgtgagatgtccaacaccctgggcac ggagcgagctcacagccagctgagcgtcctgcccgcagcaggctgcaggaaggatgggaccacgg taggcatcttc GGCGGTGGC GGATCC Gagcctcgggccctaccatcaagccctgccccccttgcaagtgccctgcccctaatctgctggg cggaccctccgtgttc CTGTTTCCTCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCCTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAAGCACTGCACAATCACTAC ACCCAGAAATCACTGTCACTGAGCCCTGGCAAA |
| SEQ ID NO: 67 | GCTGGGCCTCGGGCTCCTTGTGCTGCCGCCTGCACATGTGCAGGCGATTCCCTGGACTGCGGCGG |
| | CAGAGGCCTGGCCGCCCTGCCTGGCGATCTGCCATCCTGGACCCGAGCCTGAACCTGAGCTACA ACAAGCTGAGCGAGATCGATCCGGCCGGCTTTGAGGACCTGCCTAACCTGCAGGAGGTGTATCTG AACAATAACGAGCTGACCGCGGTACCatccctggcgctgcttcatcacatgtcgtctctctctt tctgcagcacaacaagattcgcagcgtggagggagccagctgaaggcctacctttccttagaag tgttagatctgagtttgaacaacatcacggaagtgcggaacacctgctttccacacggaccgcct ataaaggagctcaacctggcaggcaatcggattggcaccctggagttgggagcatttgatggtct gtcacggtcgctgctaactcttcgcctgagcaaaaacaggatcacccagcttcctgtaagagcat tcaagctacccaggctgacacaactggacctcaatcggaacaggattcggctgatagagggcctc accttccagggggctcaacagcttggaggtgctgaagcttcagcgaaacaacatcagcaaactgac agatggggccttctgggggactgtccaagatgcatgtgctgcacctggagtacaacagcctggtag aagtgaacagcggctcgctctacggcctcacggccctgcatcagctccacctcagcaacaattcc atcgctcgcattcaccgcaaggctggagcttctgccagaagctgcatgagttggtcctgtcctt caacaacctgacacggctggacgaggagagcctggccgagctgagcagcctgagtgtcctgcgtc tcagccacaattccatcagccacattgcggagggtgccttcaagggactcaggagcctgcgagtc ttggatctggaccataacgagatttcgggcacaatagaggacacgagcggcgccttctcagggct cgacagcctcagcaagctgactctgtttggaaacaagatcaagtctgtggctaagagagcattct cgggctggaaggcctggagcacctgaaccttggagggaatgcgatcagatctgtccagtttgat gcctttgtgaagatgaagaatcttaaagagctccatatcagcagcgacagcttcctgtgtgactg ccagctgaagtggctgccccgtggctaattggcaggatgctgcaggcctttgtgacagccacct gtgcccacccagaatcactgaagggtcagagcatttctctgtgccaccagagagtttcgtgtgc gatgacttcctgaagccacagatcatcacccagccagaaaccaccatggctatggtgggcaagga catccggtttacatgctcagcagccagcagcagcagctcccccatgacctttgcctggaagaaag acaatgaagtcctgaccaatgcagacatggagaacttgtccacgtccacgcaggacggggaa gtgatggagtacaccaccatcctgcacctccgtcaggtcactttcgggcacgagggccgctacca atgtgtcatcaccaaccactttggctccacctattcacataaggccaggctcaccgtgaatgtg tgccatcattcaccaaacgcccacgacataaccatccggaccaccaccgtggcccgcctcgaa tgtgctgccacaggtcacccaaaccctcagattgcctggcagaaggatggaggcacggatttccc cgctgcccgtgagcgacgcatgcatgtcatgccggatgacgacgtgtttttcatcactgatgtga aaatagatgacgcaggggtttacagctgtactgctcagaactcagccggttctatttcagctaat gccaccctgactgtcctagagaccccatccttggtggtccccttggaagaccgtgtggtatctgt gggagaaacagtggccctccaatgcaaagccacggggaaccctccgcccgcatcacctggttca agggggaccgcccgctgagcctcactgagcggcaccacctgaccctgacaaccagctcctggtg gttcagaacgtggtggcagaggatgcgggccgatatacctgtgagatgtccaacaccctgggcac ggagcgagctcacagccagctgagcgtcctgcccgcagcaggctgcaggaaggatgggaccacgg taggcatcttc GGCGGTGGC GGATCC cgcaacaccgacccggcggcgaggagaagaagaaggagaaggagaaggaggagcaggaggagcg cgagaccaagaccccgagtgcccagccacacccagccctggcgtgttc CTGTTCCTCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCCTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAAGCACTGCACAATCACTAC ACCCAGAAATCACTGTCACTGAGCCCTGGCAAA |
| SEQ ID NO: 68 | GCTGGGCCTCGGGCTCCTTGTGCTGCCGCCTGCACATGTGCAGGCCGATTCCCTGGACTGCGCGG CAGAGGCCTGGCCGCCCTGCCTGGCGATCTGCCATCCTGGACCCGGAGCCTGAACCTGAGCTACA |
| | ACAAGCTGAGCGAGATCGATCCGCCGGCTTTGAGGACCTGCCTAACCTGCAGGAGGTGTATCTG AACAATAACGAGCTGACCGCGGTACCatccctgggcgctgcttcatcacatgtcgtctctcttt tctgcagcacaacaagattcgcagcgtggagggagccagctgaaggcctacctttccttagaag tgttagatctgagtttgaacaacatcacggaagtgcggaacacctgctttccacacggaccgcct ataaaggagctcaacctggcaggcaatcggattggcaccctggagttgggagcatttgatggtct gtcacggtcctgctaactcttcgcctgagcaaaacaggatcacccagcttcctgtaagagcat tcaagctacccaggctgacacaactggacctcaatcggaacaggattcggctgatagagggcctc accttccagggggctcaacagcttggaggtgctgaagcttcagcgaaacaacatcagcaaactgac agatgggggccttctgggactgtccaagatgcatgtgctgcacctggagtacaacagcctggtag aagtgaacagcggctcgctctacggcctcacggccctgcatcagctccacctcagcaacaattcc atcgctcgcattcaccgcaagggctggagcttctgccagaagctgcatgagttggtcctgtcctt caacaacctgacacggctggacgaggagagcctggccgagctgagcagcctgagtgtcctgcgtc tcagccacaattccatcagccacattgcggagggtgccttcaagggactcaggagcctgcgagtc ttggatctggaccataacgagatttcgggcacaatagaggacacgagcggcgccttctcagggct cgacagcctcagcaagctgactctgtttggaaacaagatcaagtctgtggctaagagagcattct cggggctggaaggcctggagcacctgaaccttggagggaatgcgatcagatctgtccagtttgat gcctttgtgaagatgaagaatcttaaagagctccatatcagcagcgacagcttcctgtgtgactg ccagctgaagtggctgccccgtggctaattggcaggatgctgcaggcctttgtgacagccacct gtgcccacccagaatcactgaagggtcagagcatttctctgtgccaccagagagtttcgtgtgc gatgacttcctgaagccacagatcatcacccagccagaaaccaccatggctatggtgggcaagga catccggtttacatgctcagcagccagcagcagcagctccccatgacctttgcctggaagaaag acaatgaagtcctgaccaatgcagacatggagaactttgtccacgtccacgcgcaggacggggaa gtgatggagtacaccaccatcctgcacctccgtcaggtcactttcgggcacgagggccgctacca atgtgtcatcaccaaccactttggctccacctattcacataaggccaggctcaccgtgaatgtgt tgccatcattcaccaaaacgccccacgacataaccatccggaccaccaccgtggcccgcctcgaa tgtgctgccacaggtcacccaaaccctcagattgcctggcagaaggatggaggcacggatttccc cgctgcccgtgagcgacgcatgcatgtcatgccggatgacgacgtgtttttcatcactgatgtga aaatagatgacgcaggggtttacagctgtactgctcagaactcagccggttctatttcagctaat gccaccctgactgtcctagagacccatccttggtggtccccttggaagaccgtgtggtatctgt gggagaaacagtggccctccaatgcaaagccacggggaaccctccgcccgcatcacctggttca agggggaccgcccgctgagcctcactgagcggcaccacctgaccctgacaaccagctcctggtg gttcagaacgtggtggcagaggatgcggccgatatacctgtgagatgtccaacaccctgggcac ggagcgagctcacagccagctgagcgtcctgcccgcagcaggctgcaggaaggatggaccacgg taggcatcttc GGCGGTGGC GGATCC gaaccgaaatcttctgacaaaacccacacctctccgccgtctccggctccggaactgctgggtgg ttcttctgttttc CTGTTTCCTCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCCTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAAGCACTGCACAATCACTAC ACCCAGAAATCACTGTCACTGAGCCTGGCAAA |

As still yet another example of the fusion protein provided in the present invention, a fusion protein, including the extracellular domain of the Lrig-1 protein represented by SEQ ID NO: 3; the linker represented by SEQ ID NO: 12; the linker represented by SEQ ID NO: 11; the hinge region represented by any one of SEQ ID NOs: 7 to 10; and the human IgG1-derived heavy chain constant region 2 (CH2) and heavy chain constant region 3 (CH3) which are represented by SEQ ID NO: 5, may be mentioned (see Table 20 below). The fusion proteins represented by SEQ ID NOs: 69 to 72 in Table 20 below may be encoded by the nucleic acid sequences represented by SEQ ID NOs: 73 to 76 in Table 21 below (see Table 21 below).

**[Table 20]**

| SEQ ID NO | Sequence information |
|---|---|
| SEQ ID NO: 69 | [?]QAGPRAFCAAACTCAGISLLDCSGRGLATLPRDLPSWTRSLNLSYNRLSEIISAAFEDLTNLQEV [?]LNSNELTAIPSLGAASIGVVSLFLQHNKILSVDGSQLKSYLSLEVLDLSSNNITEIRSSCFPNG [?]RIRELNLASNRISILESGAFDGLSRSLLTLRLSKNRITQLPVKAFKLPRLTQLDLNRNRIRLIE [?]LTFQGLDSLEVLRLQRNNISRLTDGAFWGLSKMHVLHLEYNSLVEVNSGSLYGLTALHQLHLSN [?]SISRIQRDGWSFCQKLHELILSFNNLTRLDEESLAELSSLSILRLSHNAISHIAEGAFKGLKSL [?]VLDLDHNEISGTIEDTSGAFTGLDNLSKLTLFGNKIKSVAKRAFSGLESLEHLNLGENAIRSVQ [?]DAFAKMKNLKELYISSESFLCDCQLKWLPPWLMGRMLQAFVTATCAHPESLKGQSIFSVLPDSF [?]CDDFPKPQIITQPETTMAVVGKDIRFTCSAASSSSSPMTFAWKKDNEVLANADMENFAHVRAQD [?]EVMEYTTILHLRHVTFGHEGRYQCIITNHFGSTYSHKARLTVNVLPSFTKIPHDIAIRTGTTAR [?]ECAATGHPNPQIAWQKDGGTDFPAARERRMHVMPDDDVFFITDVKIDDMGVYSCTAQNSAGSVS [?]NATLTVLETPSLAVPLEDRVVTVGETVAFQCKATGSPTPRITWLKGGRPLSLTERHHFTPGNQL [?]VVQNVMIDDAGRYTCEMSNPLGTERAHSQLSILPTPGCRKDGTT [?]GG [?]S [?]PKSSDKTHT SPPCPAPELL GGPSVF [?]FPPKPKDTLMISRTPEVTCVVVDVSHEDPEVKFNWYVDGVEVHNAKTKPREEQYNSTYRVVSVL [?]VLHQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHY TQKSLSLSPGK |
| SEQ ID NO: 70 | AQAGPRAPCAAACTCAGDSLDCSGRGLATLPRDLPSWTRSLNLSYNRLSEIDSAAFEDLTNLQEV YLNSNELTAIPSLGAASIGVVSLFLQHNKILSVDGSQLKSYLSLEVLDLSSNNITEIRSSCFPNG LRIRELNLASNRISILESGAFDGLSRSLLTLRLSKNRITQLPVKAFKLPRLTQLDLNRNRIRLIE GLTFQGLDSLEVLRLQRNNISRLTDGAFWGLSKMHVLHLEYNSLVEVNSGSLYGLTALHQLHLSN NSISRIQRDGWSFCQKLHELILSFNNLTRLDEESLAELSSLSILRLSHNAISHIAEGAFKGLKSL RVLDLDHNEISGTIEDTSGAFTGLDNLSKLTLFGNKIKSVAKRAFSGLESLEHLNLGENAIRSVQ FDAFAKMKNLKELYISSESFLCDCQLKWLPPWLMGRMLQAFVTATCAHPESLKGQSIFSVLPDSF VCDDFPKPQIITQPETTMAVVGKDIRFTCSAASSSSSPMTFAWKKDNEVLANADMENFAHVRAQD GEVMEYTTILHLRHVTFGHEGRYQCIITNHFGSTYSHKARLTVNVLPSFTKIPHDIAIRTGTTAR LECAATGHPNPQIAWQKDGGTDFPAARERRMHVMPDDDVFFITDVKIDDMGVYSCTAQNSAGSVS ANATLTVLETPSLAVPLEDRVVTVGETVAFQCKATGSPTPRITWLKGGRPLSLTERHHFTPGNQL LVVQNVMIDDAGRYTCEMSNPLGTERAHSQLSILPTPGCRKDGTT GGG GS EPRGPTIKPCPPCKCPAPNLLGGPSVF LFPPKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHY TQKSLSLSPGK |
| SEQ ID NO: 71 | AQAGPRAPCAAACTCAGDSLDCSGRGLATLPRDLPSWTRSLNLSYNRLSEI DSAAFEDLTNLQEV YLNSNELTA I PSLGAAS I GVVSLFLQHNKI LSVDGSQLKSYLSLEVLDLSSNNI TE I RSSCFPNG LRI RELNLASNRI SI LESGAFDGLSRSLLTLRLSKNRI TQLPVKAFKLPRLTQLDLNRNRI RLI E GLTFQGLDSLEVLRLQRNNI SRLTDGAFWGLSKMHVLHLEYNSLVEVNSGSLYGLTALHQLHLSN NSI SRI QRDGWSFCQKLHELI LSFNNLTRLDEESLAELSSLSI LRLSHNAI SHI AEGAFKGLKSL RVLDLDHNEI SGTI EDTSGAFTGLDNLSKLTLFGNKI KSVAKRAFSGLESLEHLNLGENAI RSVQ FDAFAKMKNLKELYI SSESFLCDCQLKWLPPWLMGRMLQAFVTATCAHPESLKGQSI FSVLPDSF VCDDFPKPQI I TQPETTMAVVGKDI RFTCSAASSSSSPMTFAWKKDNEVLANADMENFAHVRAQD GEVMEYTTI LHLRHVTFGHEGRYQCI I TNHFGSTYSHKARLTVNVLPSFTKI PHDI AI RTGTTAR LECAATGHPNPQI AWQKDGGTDFPAARERRMHVMPDDDVFFI TDVKI DDMGVYSCTAQNSAGSVS ANATLTVLETPSLAVPLEDRVVTVGETVAFQCKATGSPTPRI TWLKGGRPLSLTERHHFTPGNQL LVVQNVMI DDAGRYTCEMSNPLGTERAHSQLSI LPTPGCRKDGTT |
| | GGG GS RNTGRGGEEKKKEKEKEEQEERETKTPECPSHTQPLGVF LFPPKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHY TQKSLSLSPGK |
| SEQ ID NO: 72 | AQAGPRAPCAAACTCAGDSLDCSGRGLATLPRDLPSWTRSLNLSYNRLSEIDSAAFEDLTNLQEV YLNSNELTAIPSLGAASIGVVSLFLQHNKILSVDGSQLKSYLSLEVLDLSSNNITEIRSSCFPNG LRIRELNLASNRISILESGAFDGLSRSLLTLRLSKNRITQLPVKAFKLPRLTQLDLNRNRIRLIE GLTFQGLDSLEVLRLQRNNISRLTDGAFWGLSKMHVLHLEYNSLVEVNSGSLYGLTALHQLHLSN NSISRIQRDGWSFCQKLHELILSFNNLTRLDEESLAELSSLSILRLSHNAISHIAEGAFKGLKSL RVLDLDHNEISGTIEDTSGAFTGLDNLSKLTLFGNKIKSVAKRAFSGLESLEHLNLGENAIRSVQ FDAFAKMKNLKELYISSESFLCDCQLKWLPPWLMGRMLQAFVTATCAHPESLKGQSIFSVLPDSF VCDDFPKPQIITQPETTMAVVGKDIRFTCSAASSSSSPMTFAWKKDNEVLANADMENFAHVRAQD GEVMEYTTILHLRHVTFGHEGRYQCIITNHFGSTYSHKARLTVNVLPSFTKIPHDIAIRTGTTAR LECAATGHPNPQIAWQKDGGTDFPAARERRMHVMPDDDVFFITDVKIDDMGVYSCTAQNSAGSVS ANATLTVLETPSLAVPLEDRVVTVGETVAFQCKATGSPTPRITWLKGGRPLSLTERHHFTPGNQL LVVQNVMIDDAGRYTCEMSNPLGTERAHSQLSILPTPGCRKDGTT GGG GS EPKSSDKTHTSPPSPAPELLGGSSVF LFPPKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSRDELTKNQVSLTCLVKG FYPSDIAVEWESNGQPENNYKTTPPVLDSDGSFFLYSKLTVDKSRWQQGNVFSCSVMHEALHNHY TQKSLSLSPGK |

**[Table 21]**

| SEQ ID NO | Sequence information |
|---|---|
| SEQ ID NO: 73 | GCTCAGGCTGGACCTAGGGCTCCTTGGGCTGCCGCCTGCACCTGTGCAGGCGATTCTCTGGACTG CAGCGGCCGGGGCCTGGCCACACTGCCCAGGGACCTGCCTTCCTGGACCAGATCTCTGAACCTGA GCTACAATGGCTGTCCGAGATGGATTCTGCGGCCTTTGAGGACCTGACAAATCTGCAGGAGGTG TATCTGAACAGCAATGAGCTGACCGCAATCCCCTCCCTGGGAGCAGCCTCTATCGGCGTGGTGAG CCTGTTCCTGCAGCACAACAAGATCCTGAGCGTGGATGGCTCCCAGCTGAAGAGCTACCTGTCTC TGGAGGTGCTGGACCTGAGCTCCAACAATATCACCGAGATCAGATCTAGCTGTTTTCCTAATGGC CTGCGGATCAGAGAGCTGAACCTGGCCTCTAATCGGATCAGCATCCTGGAGTCCGGCGCCTTCGA TGGCCTGAGCAGATCCCTGCTGACACTGCGCCTGTCCAAGAACCGGATCACCCAGCTGCCCGTGA AGGCCTTTAAGCTGCCTAGGCTGACACAGCTGGACCTGAACCGGAATAGAATCAGGCTGATCGAG GGCCTGACCTTCCAGGGCCTGGATAGCCTGGAGGTGCTGCGCCTGCAGCGGAACAATATCTCCCG CCTGACAGACGGAGCATTTTGGGGCCTGTCTAAGATGCACGTGCTGCACCTGGAGTACAATAGCC TGGTGGAGGTGAACTCTGGCAGCCTGTATGGCCTGACCGCCCTGCACCAGCTGCACCTGTCCAAC AATAGCATCAGCAGAATCCAGAGGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCCTTCAC AGGCCTGGACAATCTGTCCAAGCTGACCCTGTTTGGCAACAAGATCAAGTCTGTGGCCAAGCGGG CCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCACCTTGGCTGATGGGAAGGATGCTGCAGGCCTTTGTGACCG CCACATGCGCCCACCCAGAGAGCCTGAAGGGCCAGAGCATCTTCTCCGTGCTGCCCGATAGCTTC GTGTGCGACGATTTCCCTAAGCCACAGATCATCACCCAGCCAGAGACAACAATGGCCGTGGTGGG CAAGGACATCCGGTTTACATGTTCCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCCTGCACCGCACAGAACAGCGCCGGCAGCGTGTCC GCCAACGCCACCCTGACCGTGCTGGAGACACCATCCCTGGCCGTGCCCCTGGAGGACAGGGTGGT GACCGTGGGCGAGACAGTGGCCTTTCAGTGTAAGGCCACCGGCTCTCCAACACCAAGGATCACCT GGCTGAAGGGCGGCAGGCCCCTGAGCCTGACAGAGCGCCACCACTTCACCCCTGGCAATCAGCTG CTGGTGGTGCAGAACGTGATGATCGATGACGCCGGCAGGTATACATGCGAGATGAGCAATCCTCT GGGCACCGAGAGGGCACACTCCCAGCTGTCTATCCTGCCTACCCCAGGCTGCCGGAAGGATGGCA CCACA GGCGGTGGC GGATCC GAGCCAAAGTCCTCTGATAAGACACACACCTCTCCACCATGCCCAGCACCAGAGCTGCTGGGAGG ACCAAGCGTGTTC CTGTTCCCTCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCCTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAAGCACTGCACAATCACTAC ACCCAGAAATCACTGTCACTGAGCCCTGGCAAA |
| SEQ ID NO: 74 | GCTCAGGCTGGACCTAGGCTCCTTGGCTGCCGCCTGCACCTGTGCAGGCGATTCTCTGGACTG CAGCGGCGGGGCTGGCCACACTGGCCAGGGACCTGCCTTCCTGGACCAGATCTCTGAACCTGA GCTACAATGGCTGTCCGAGATCGATTCTGCGGCTTTGAGGACCTGACAAATCTGCAGGAGGTG TATCTGAACAGCAATGAGCTGACCGCAATCCCCTCCCTGGGAGCAGCCTCTATCGGCGTGGTGAG CCTGTTCCTGCAGCACAACAAGATCCTGAGCGTGGATGGCTCCCAGCTGAAGAGCTACCTGTCTC TGGAGGTGCTGGACCTGAGCTCCAACAATATCACCGAGATCAGATCTAGCTGTTTTCCTAATGGC CTGCGGATCAGAGAGCTGAACCTGGCCTCTAATCGGATCAGCATCCTGGAGTCCGGCGCTTCGA TGGCCTGAGCAGATCCCTGCTGACACTGCGCCTGTCCAAGAACCGGATCACCCAGCTGCCCGTGA AGGCCTTTAAGCTGCCTAGGCTGACACAGCTGGACCTGAACCGGAATAGAATCAGGCTGATCGAG GGCCTGACCTTCCAGGGCCTGGATAGCCTGGAGGTGCTGCGCCTGCAGCGGAACAATATCTCCG CCTGACAGACGGAGCATTTGGGGCCTGTCTAAGATGCACGTGCTGCACCTGGAGTACAATAGCC TGGTGGAGGTGAACTCTGGCAGCCTGTATGGCCTGACCGCCCTGCACCAGCTGCACCTGTCCAAC AATAGCATCAGCAGAATCCAGAGGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCCTTCAC AGGCCTGGACAATCTGTCCAAGCTGACCCTGTTTGGCAACAAGATCAAGTCTGTGGCCAAGCGGG CCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCACCTTGGCTGATGGGAAGCATGCTGCAGGCCTTTGTGACCG CCACATGCGCCCACCCAGAGAGCCTGAAGGGCCAGAGCATCTTCTCCGTGCTGCCCGATAGCTTC GTGTGCGACGATTTTCCTAAGCCACAGATCATCACCCAGCCAGAGACAACAATGGCCGTGGTGGG CAAGGACATCCGGTTTACATGTTCCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCCTGCACCGCACAGAACAGCGCCGGCAGCGTGTCC GCCAACGCCACCCTGACCGTGCTGGAGACACCATCCCTGGCCGTGCCCCTGGAGGACAGGTGGT GACCGTGGGCGAGACAGTGGCCTTTCAGTGTAAGGCCACCGGCTCTCCAACACCAAGGATCACCT GGCTGAAGGGCGGCAGGCCCCTGAGCCTGACAGAGCGCCACCACTTCACCCCTGGCAATCAGCTG CTGGTGGTGCAGAACGTGATGATCGATGACGCCGGCAGGTATACATGCGAGATGAGCAATCCTCT GGGCACCGAGAGGGCACACTCCCAGCTGTCTATCCTGCCTACCCCAGGCTGCCGGAAGGATGGCA CCACA GGCGGTGGC GGATCC Gagcctcggaccctaccatcaagccctgccccccttgcaagtgccctgcccctaatctgctggg cggaccctccgtgttc CTGTTTCCTCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCCTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAAGCACTGCACAATCACTAC ACCCAGAAATCACTGTCACTGAGCCCTGGCAAA |
| SEQ ID NO: 75 | GCTCAGGCTGGACCTAGGGCTCCTTGGCTGGCGCCTGCACCTGTGCAGGCGATTCTCTGGACTG |
| | CAGCGGCCGGGGCCTGGCCACACTGCCCAGGGACCTGCCTTCCTGGACCAGATCTCTGAACCTGA GCTACAATGGCTGTCCGAGATCGATTCTGCCGCCTTTGAGGACCTGACAAATCTGCAGGAGGTG TATCTGAACAGCAATGAGCTGACCGCAATCCCCTCCCTGGGAGCAGCCTCTATCGGCGTGGTGAG CCTGTTCCTGCAGCACAACAAGATCCTGAGCGTGGATGGCTCCCAGCTGAAGAGCTACCTGTCTC TGGAGGTGCTGGACCTGAGCTCCAACAATATCACCGAGATCAGATCTAGCTGTTTTCCTAATGGC CTGCGGATCAGAGAGCTGAACCTGGCCTCTAATCGGATCAGCATCCTGGAGTCCGGCGCCTTCGA TGGCCTGAGCAGATCCCTGCTGACACTGCGCCTGTCCAAGAACCGGATCACCCAGCTGCCCGTGA AGGCCTTTAAGCTGCCTAGGCTGACACAGCTGGACCTGAACCGGAATAGAATCAGGCTGATCGAG GGCCTGACCTTCCAGGGCCTGGATAGCCTGGAGGTGCTGCGCCTGCAGCGGAACAATATCTCCCG CCTGACAGACGGAGCATTTTGGGGCCTGTCTAAGATGCACGTGCTGCACCTGGAGTACAATAGCC TGGTGGAGGTGAACTCTGGCAGCCTGTATGGCCTGACCGCCCTGCACCAGCTGCACCTGTCCAAC AATAGCATCAGCAGAATCCAGAGGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCCTTCAC AGGCCTGGACAATCTGTCCAAGCTGACCCTGTTTGGCAACAAGATCAAGTCTGTGGCCAAGCGGG CCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCACCTTGGCTGATGGGAAGGATGCTGCAGGCCTTTGTGACCG CCACATGCGCCCACCCAGAGAGCCTGAAGGGCCAGAGCATCTTCTCCGTGCTGCCCGATAGCTTC GTGTGCGACGATTTTCCTAAGCCACAGATCATCACCCAGCCAGAGACAACAATGGCCGTGGTGGG CAAGGACATCCGGTTTACATGTTCCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCTTGCACCGCACAGAACAGCGCCGGCAGCGTGTCC GCCAACGCCACCCTGACCGTGCTGGAGACACCATCCCTGGCCGTGCCCCTGGAGGACAGGGTGGT GACCGTGGGCGAGACAGTGGCCTTTCAGTGTAAGGCCACCGGCTCTCCAACACCAAGGATCACCT GGCTGAAGGGCGGCAGGCCCCTGAGCCTGACAGAGCGCCACCACTTCACCCCTGGCAATCAGCTG CTGGTGGTGCAGAACGTGATGATCGATGACGCCGGCAGGTATACATGCGAGATGAGCAATCCTCT GGGCACCGAGAGGGCACACTCCCAGCTGTCTATCCTGCCTACCCCAGGCTGCCGGAAGGATGGCA CCACA GGCGGTGGC GGATCC cgcaacaccgacccacgacgcgaggagaagaagaaggagaaggagaaggaggaacaggaggagcg cgagaccaagaccccgagtgccccagccacacccagccctggacgattc CTGTTCCTCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCCTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAAGCACTGCACAATCACTAC ACCCAGAAATCACTGTCACTGAGCCCTGGCAAA |
| SEQ ID NO: 76 | GCTCAGGCTGGACCTAGGGCTCCTTGGGCTGCCGCCTGCACCTGTGCAGGCGATTCTCTGGACTG CAGCGGCCGGGGCCTGGCCACACTGCCCAGGGACCTGCCTTCCTGGACCAGATCTCTGAACCTGA |
| | GCTACAATGGCTGTCCGAGATGGATTCTGCCGCTTTGAGGACCTGACAAATCTGCAGGAGGTG TATCTGAACAGCAATGAGCTGACCGCAATCCCCTCCCTGGGAGCAGCCTCTATCGGCGTGGTGAG CCTGTTCCTGCAGCACAACAAGATCCTGAGCGTGGATGGCTCCCAGCTGAAGAGCTACCTGTCTC TGGAGGTGCTGGACCTGAGCTCCAACAATATCACCGAGATCAGATCTAGCTGTTTTCCTAATGGC CTGCGGATCAGAGAGCTGAACCTGGCCTCTAATCGGATCAGCATCCTGGAGTCCGGCGCTTCGA TGGCCTGAGCAGATCCCTGCTGACACTGCGCCTGTCCAAGAACCGGATCACCCAGCTGCCCGTGA AGGCCTTTAAGCTGCCTAGGCTGACACAGCTGGACCTGAACCGGAATAGAATCAGGCTGATCGAG GGCCTGACCTTCCAGGGCCTGGATAGCCTGGAGGTGCTGCGCCTGCAGCGGAACAATATCTCCCG CCTGACAGACGGAGCATTTTGGGGCCTGTCTAAGATGCACGTGCTGCACCTGGAGTACAATAGCC TGGTGGAGGTGAACTCTGGCAGCCTGTATGGCCTGACCGCCCTGCACCAGCTGCACCTGTCCAAC AATAGCATCAGCAGAATCCAGAGGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCCTTCAC AGGCCTGGACAATCTGTCCAAGCTGACCCTGTTTGGCAACAAGATCAAGTCTGTGGCCAAGCGGG CCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCACCTTGGCTGATGGGAAGGATGCTGCAGGCCTTTGTGACCG CCACATGCGCCCACCCAGAGAGCCTGAAGGGCCAGAGCATCTTCTCCGTGCTGCCCGATAGCTTC GTGTGCGACGATTTTCCTAAGCCACAGATCATCACCCAGCCAGAGACAACAATGGCCGTGGTGGG CAAGGACATCCGGTTTACATGTTCCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCCTGCACCGCACAGAACAGCGCCGGCAGCGTGTCC GCCAACGCCACCCTGACCGTGCTGGAGACACCATCCCTGGCCGTGCCCCTGGAGGACAGGGTGGT GACCGTGGGCGAGACAGTGGCCTTTCAGTGTAAGGCCACCGGCTCTCCAACACCAAGGATCACCT GGCTGAAGGGCGGCAGGCCCCTGAGCCTGACAGAGCGCCACCACTTCACCCCTGGCAATCAGCTG CTGGTGGTGCAGAACGTGATGATCGATGACGCCGGCAGGTATACATGCGAGATGAGCAATCCTCT GGGCACCGAGAGGGCACACTCCCAGCTGTCTATCCTGCCTACCCCAGGCTGCCGGAAGGATGGCA CCACA GGCGGTGGC GGATCC gaaccgaaatcttctgacaaaacccacacctctccgccgtctccggctccggaactgctgggtgg ttcttctgtttc CTGTTCCTCCAAAGCCCAAGGACACACTGATGATCTCCAGGACACCAGAGGTGACCTGCGTGGT GGTGGACGTGAGCCACGAGGACCCCGAGGTGAAGTTCAACTGGTACGTGGATGGCGTGGAGGTGC ACAATGCCAAGACCAAGCCCAGAGAGGAGCAGTACAACTCTACCTATAGGGTGGTGAGCGTGCTG ACAGTGCTGCACCAGGACTGGCTGAACGGCAAGGAGTATAAGTGCAAGGTGAGCAATAAGGCCCT GCCTGCCCCAATCGAGAAGACAATCTCCAAGGCCAAGGGCCAGCCAAGAGAGCCCCAGGTGTACA CCCTGCCCCCTAGCAGGGATGAGCTGACAAAGAACCAGGTGTCCCTGACCTGTCTGGTGAAGGGC TTTTATCCCTCCGACATCGCCGTGGAGTGGGAGTCTAATGGCCAGCCTGAGAATAACTACAAGAC AACCCCACCCGTGCTGGATTCTGACGGCAGCTTCTTTCTGTATTCTAAGCTGACCGTGGACAAGA GCAGGTGGCAGCAGGGCAACGTGTTCAGCTGCTCCGTGATGCACGAAGCACTGCACAATCACTAC ACCCAGAAATCACTGTCACTGAGCCCTGGCAAA |

As still yet another example of the fusion protein provided in the present invention, a fusion protein, including the extracellular domain of the Lrig-1 protein represented by SEQ ID NO: 3; the linker represented by SEQ ID NO: 12; the linker represented by SEQ ID NO: 11; the hinge region represented by any one of SEQ ID NOs: 7 to 10; and the mouse IgG2-derived heavy chain constant region 2 (CH2) and heavy chain constant region 3 (CH3) which are represented by SEQ ID NO: 6, may be mentioned (see Table 22 below). The fusion proteins represented by SEQ ID NOs: 77 to 80 in Table 22 below may be encoded by the nucleic acid sequences represented by SEQ ID NOs: 81 to 84 in Table 23 below (see Table 23 below).

**[Table 22]**

| SEQ ID NO | Sequence information |
|---|---|
| SEQ ID NO: 77 | AQAGPRAPCAAACTCAGDSLDCSGRGLATLPRDLPSWTRSLNLSYNRLSEI DSAAFEDLTNLQEV YLNSNELTA I PSLGAAS IGVVS LFLQHNK I LSVDGSQLKSYLS LEVLDLSSNN ITE I RSSCFPNG LR I RELNLASNR IS I LESGAFDGLS RSLLTLRLSKNR I TQLPVKAFKLPRLTQLDLNRNR I RL IE GLTFQGLDSLEVLRLQRNN I SRLTDGAFWGLSKMHVLHLEYNSLVEVNSGSLYGLTALHQLHLSN NS IS R IQRDGWS FCQKLHEL I LSFNNLTRLDEESLAELSS LS I LRLSHNA I SH IAEGAFKGLKSL RVLDLDHNE ISGT IEDTSGAFTGLDNLSKLTLFGNK IKSVAKRAFSGLESLEHLNLGENA I RSVQ FDAFAKMKNLKELY I SS ESFLCDCQLKWLPPWLMGRMLQAFVTATCAHPES LKGQS I FSVLPDSF VCDDFPKPQ I I TQPETTMAVVGKD I RFTCSAASSSSSPMTFAWKKDNEVLANADMENFAHVRAQD GEVMEYTT I LHLRHVTFGHEGRYQC I I TNHFGSTYSHKARLTVNVLPSFTK I PHD I A I RTGTTAR LECAATGHPNPQ IAWQKDGGTDFPAARERRMHVMPDDDVFF I TDVK I DDMGVYSCTAQNSAGSVS ANATLTVLETPSLAVPLEDRVVTVGETVAFQCKATGSPTPR I TWLKGGRPLSLTERHHFTPGNQL LVVQNVM I DDAGRYTCEMSNPLGTERAHSQLS I LPTPGCRKDGTT GGG GS EPKSSDKTHTSPPCPAPELLGGPSVF I FPPK I KDVLM I SLSP I VTCVVVDVSEDDPDVQ I SWFVNNVEVHTAQTQTHREDYNSTLRVVSAL P I QHQDWMSGKEFKCKVNNKDLPAP I ERT I SKPKGSVRAPQVYVLPPPEEEMTKKQVTLTCMVTD FMPED I YVEWTNNGKTELNYKNTEPVLDSDGSYFMYSKLRVEKKNWVERNSYSCSVVHEGLHNHH TTKSFSRTPGK |
| SEQ ID NO: 78 | AQAGPRAPCAAACTCAGDSLDCSGRGLATLPRDLPSWTRSLNLSYNRLSEIDSAAFEDLTNLQEV YLNSNELTAIPSLGAASIGVVSLFLQHNKILSVDGSQLKSYLSLEVLDLSSNNITEIRSSCFPNG LRIRELNLASNRISILESGAFDGLSRSLLTLRLSKNRITQLPVKAFKLPRLTQLDLNRNRIRLIE GLTFQGLDSLEVLRLQRNNISRLTDGAFWGLSKMHVLHLEYNSLVEVNSGSLYGLTALHQLHLSN NSISRIQRDGWSFCQKLHELILSFNNLTRLDEESLAELSSLSILRLSHNAISHIAEGAFKGLKSL RVLDLDHNEISGTIEDTSGAFTGLDNLSKLTLFGNKIKSVAKRAFSGLESLEHLNLGENAIRSVQ FDAFAKMKNLKELYISSESFLCDCQLKWLPPWLMGRMLQAFVTATCAHPESLKGQSIFSVLPDSF VCDDFPKPQIITQPETTMAVVGKDIRFTCSAASSSSSPMTFAWKKDNEVLANADMENFAHVRAQD GEVMEYTTILHLRHVTFGHEGRYQCIITNHFGSTYSHKARLTVNVLPSFTKIPHDIAIRTGTTAR LECAATGHPNPQIAWQKDGGTDFPAARERRMHVMPDDDVFFITDVKIDDMGVYSCTAQNSAGSVS ANATLTVLETPSLAVPLEDRVVTVGETVAFQCKATGSPTPRITWLKGGRPLSLTERHHFTPGNQL LVVQNVMIDDAGRYTCEMSNPLGTERAHSQLSILPTPGCRKDGTT GGG GS EPRGPTIKPCPPCKCPAPNLLGGPSVF IFPPKIKDVLMISLSPIVTCVVVDVSEDDPDVQISWFVNNVEVHTAQTQTHREDYNSTLRVVSAL PIQHQDWMSGKEFKCKVNNKDLPAPIERTISKPKGSVRAPQVYVLPPPEEEMTKKQVTLTCMVTD FMPEDIYVEWTNNGKTELNYKNTEPVLDSDGSYFMYSKLRVEKKNWVERNSYSCSVVHEGLHNHH TTKSFSRTPGK |
| SEQ ID NO: 79 | NJAGPRAPCAAACTCAG ISLDCSGRGLATLPRDLPSWTRSLNLSYNRLSE IDSAAFEDLTNLQEV YLNSNELTA IPSLGAAS IGVVSLFLQHNKI LSVDGSQLKSYLSLEVLDLSSNNITE IRSSCFPNG LR IRELNLASNR IS I LESGAFDGLS RSLLTLRLSKNR I TQLPVKAFKLPRLTQLDLNRNR I RL IE GLTFQGL DSLEVLRLQRNN I SRLTDGAFWGLSKMHVLHLEYNSLVEVNSGSLYGLTALHQLHLSN NS IS R IQRDGWSFCQKLHEL ILSFNNLTRLDEESLAELSSLS I LRLSHNA I SH IAEGAFKGLKSL RVLDLDHNE ISGT IEDTSGAFTGLDNLSKLTLFGNK IKSVAKRAFSGLESLEHLNLGENA I RSVQ FDAFAKMKNLKELY ISSESFLCDCQLKWLPPWLMGRMLQAFVTATCAHPESLKGQS IFSVLPDSF VCDDFPKPQI ITQPETTMAVVGKD I RFTCSAASSSSSPMTFAWKKDNEVLANA IMENFAHVRAQD GEVMEYTT ILHLRHVTFGHEGRYQC I ITNHFGSTYSHKARLTVNVLPSFTK IPHD IA IRTGTTAR LECAATGHPNPQ IAWQKDGGTDFPAARERRMHVMPDDDVFF ITDVK IDDMGVYSCTAQNSAGSVS ANATLTVLETPSLAVPLEDRVVTVGETVAFQCKATGSPTPR ITWLKGGRPLSLTERHHFTPGNQL LVVQNVM IDDAGRYTCEMSNPLGTERAHSQLS ILPTPGCRKDGTT |
| | GGG GS RNTGRGGEEKKKEKEKEEQEERETKTPECPSHTQPLGVF IFPPKIKDVLMISLSPIVTCVVVDVSEDDPDVQISWFVNNVEVHTAQTQTHREDYNSTLRVVSAL PIQHQDWMSGKEFKCKVNNKDLPAPIERTISKPKGSVRAPQVYVLPPPEEEMTKKQVTLTCMVTD FMPEDIYVEWTNNGKTELNYKNTEPVLDSDGSYFMYSKLRVEKKNWVERNSYSCSVVHEGLHNHH TTKSFSRTPGK |
| SEQ ID NO: 80 | AQAGPRAFCAAACTCAGDSL DCSGRGLATLPRDLPSWTRS LNLSYNRLSEI DSAAFEDLTNLQEV YLNSNELTAI PSLGAAS IGVVSLFLQHNKI LSVDGSQLKSYLSLEVLDLSSNNI TEI RSSCFPNG LRI RELNLASNRI SI LESGAFDGLSRSLLTLRLSKNRI TQLPVKAFKLPRLTQLDLNRNRI RLI E GLTFQGLDSLEVLRLQRNNI SRLTDGAFWGLSKMHVLHLEYNSLVEVNSGSLYGLTALHQLHLSN NS I SRI QRDGWSFCQKLHELI LSFNNLTRLDEESLAELSSLSI LRLSHNAI SHIAEGAFKGLKSL RVLDLDHNEI SGTI EDTSGAFTGLDNLSKLTLFGNKI KSVAKRAFSGLESLEHLNLGENAI RSVQ FDAFAKMKNLKELYI SSESFLCDCQLKWLPPWLMGRMLQAFVTATCAHPESLKGQSI FSVLPDSF VCDDFPKPQI I TQPETTMAVVGKDI RFTCSAASSSSSPMTFAWKKDNEVLANADMENFAHVRAQD GEVMEYTTI LHLRHVTFGHEGRYQCI I TNHFGSTYSHKARLTVNVLPSFTKI PHDI AI RTGTTAR LECAATGHPNPQI AWQKDGGTDFPAARERRMHVMPDDDVFFI TDVKI DDMGVYSCTAQNSAGSVS ANATLTVLETPSLAVPLEDRVVTVGETVAFQCKATGSPTPRI TWLKGGRPLSLTERHHFTPGNQL LVVQNVMI DDAGRYTCEMSNPLGTERAHSQLSI LPTPGCRKDGTT GGG GS EPKSSDKTHTSPPSPAPELLGGSSVF I FPPKI KDVLMI SLSPI VTCVVVDVSEDDPDVQI SWFVNNVEVHTAQTQTHREDYNSTLRVVSAL PI QHQDWMSGKEFKCKVNNKDLPAPI ERTI SKPKGSVRAPQVYVLPPPEEEMTKKQVTLTCMVTD FMPEDI YVEWTNNGKTELNYKNTEPVLDSDGSYFMYSKLRVEKKNWVERNSYSCSVVHEGLHNHH TTKSFSRTPGK |

**[Table 23]**

| SEQ ID NO | sequence information |
|---|---|
| SEQ ID NO: 81 | GCTCAGGCTGGACCTAGGGCTCCTTGCGCTGCCGCCTGCACCTGTGCAGGCGATTCTCTGGACTG CAGCGGCGGGGCTGGCCACACTGCCCAGGGACCTGCCTTCCTGGACCAGATCTCTGAACCTGA GCTACAATCGGCTGTCCGAGATCGATTCTGCGGCCTTTGAGGACCTGACAAATCTGCAGGAGGTG TATCTGAACAGCAATGAGCTGACCGCAATCCCCTCCCTGGGAGCAGCCTCTATCGGCGTGGTGAG CCTGTTCCTGCAGCACAACAAGATCCTGAGCGTGGATGCTCCCAGCTGAAGAGCTACCTGTCTC TGGAGGTGCTGGACCTGAGCTCCAACAATATCACCGAGATCAGATCTAGCTGTTTTCCTAATGGC CTGCGGATCAGAGAGCTGAACCTGGCCTCTAATCGGATCAGCATCCTGGAGTCGGGCGCTTCGA TGGCCTGAGCAGATCCCTGCTGACACTGCGCCTGTCCAAGAACCGGATCACCCAGCTGCCCGTGA AGGCCTTTAAGCTGCCTAGGCTGACACAGCTGGACCTGAACCGGAATAGAATCAGGCTGATCGAG GGCCTGACCTTCCAGGGCCTGGATAGCCTGGAGGTGCTGCGCCTGCAGCGGAACAATATCTCCG CCTGACAGACGGAGCATTTTGGGCCTGTCTAAGATGCACGTGCTGCACCTGGAGTACAATAGCC TGGTGGAGGTGAACTCTGGCAGCCTGTATGGCCTGACCGCCCTGCACCAGCTGCACCTGTCCAAC AATAGCATCAGCAGAATCCAGAGGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCTTCAC AGGCCTGGACAATCTGTCCAAGCTGACCCTGTTTGGCAACAAGATCAAGTCTGTGGCCAAGCGGG CCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCACCTTGGCTGATGGGAAGGATGCTGCAGGCCTTTGTGACCG CCACATGCGCCCACCCAGAGAGCCTGAAGGGCCAGAGCATCTTCTCCGTGCTGCCCGATAGCTTC GTGTGCGACGATTTTCCTAAGCCACAGATCATCACCCAGCCAGAGACAACAATGGCCGTGGTGGG CAAGGACATCCGGTTTACATGTTCCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCCTGCACCGCACAGAACAGCGCCGGCAGCGTGTCC GCCAACGCCACCCTGACCGTGCTGGAGACACCATCCCTGGCGTGCCCCTGGAGGACAGGGTGGT GACCGTGGGCGAGACAGTGGCCTTTCAGTGTAAGGCCACCGGCTCTCCAACACCAAGGATCACCT GGCTGAAGGGCGGCAGGCCCCTGAGCCTGACAGAGCGCCACCACTTCACCCCTGGCAATCAGCTG CTGGTGGTGCAGAACGTGATGATCGATGACGCCGGCAGGTATACATGCGAGATGAGCAATCCTCT GGGCACCGAGAGGGCACACTCCCAGCTGTCTATCCTGCCTACCCCAGGCTGCCGGAAGGATGGCA CCACA GGCGGTGGC GGATCC GAGCCAAAGTCCTCTGATAAGACACACACCTCTCCACCATGCCCAGCACCAGAGCTGCTGGAGG ACCAAGCGTGTTC atcttcccacccaagatcaaggacgtgctgatgatctccctgtcccccatcgtgacctgcgtggt ggtggacgtgtccgaggacgaccccgacgtgcagatcagttggttcgtgaacaacgtggaagtgc acaccgcccagacccagacccacagagaggactacaactccaccctgcgggtggtgtccgccctg cccatccagcaccaggactggatgtccggcaaagaattcaagtgcaaagtgaacaacaaggacct gcctgcccccatcgagcggaccatctccaagcccaagggctccgtgcgggctccccaggtgtacg tgctgcccctccagaggaagagatgaccaagaagcaggtcacactgacctgcatggtcaccgac ttcatgcccgaggacatctacgtggaatggaccaacaatggcaagaccgagctgaactacaagaa caccgagcctgtgctggactccgacggctcctacttcatgtactccaagctgcgggtggaaaaga agaactgggtcgagcggaactcctactcctgctccgtggtgcacgagggcctgcacaaccaccac accaccaagtccttctcccggacccccggcaaa |
| SEQ ID NO: 82 | GCTCAGGCTGGACCTAGGGCTCCTTGGGCTGCCGCCTGCACCTGTGCAGGCGATTCTCTGGACTG CAGCGGCCGGGGCCTGGCCACACTGGCCAGGGACCTGCCTTCCTGGACCAGATCTCTGAACCTGA GCTACAATGGCTGTCCGAGATCGATTCTGCCGCCTTTGAGGACCTGACAAATCTGCAGGAGGTG TATCTGAACAGCAATGAGCTGACCGCAATCCCCTCCCTGGGAGCAGCCTCTATCGGCGTGGTGAG CCTGTTCCTGCAGCACAACAAGATCCTGAGCGTGGATGGCTCCCAGCTGAAGAGCTACCTGTCTC TGGAGGTGCTGGACCTGAGCTCCAACAATATCACCGAGATCAGATCTAGCTGTTTTCCTAATGGC CTGCGGATCAGAGAGCTGAACCTGGCCTCTAATCGGATCAGCATCCTGGAGTCCGGCGCCTTCGA TGGCCTGAGCAGATCCCTGCTGACACTGCGCCTGTCCAAGAACCGGATCACCCAGCTGCCCGTGA AGGCCTTTAAGCTGCCTAGGCTGACACAGCTGGACCTGAACCGGAATAGAATCAGGCTGATCGAG GGCCTGACCTTCCAGGGCCTGGATAGCCTGGAGGTGCTGCGCCTGCAGCGGAACAATATCTCCCG CCTGACAGACGGAGCATTTTGGGGCCTGTCTAAGATGCACGTGCTGCACCTGGAGTACAATAGCC TGGTGGAGGTGAACTCTGGCAGCCTGTATGGCCTGACCGCCCTGCACCAGCTGCACCTGTCCAAC AATAGCATCAGCAGAATCCAGAGGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCCTTCAC AGGCCTGGACAATCTGTCCAAGCTGACCCTGTTTGGCAACAAGATCAAGTCTGTGGCCAAGCGG GCCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCACCTTGGCTGATGGGAAGGATGCTGCAGGCCTTTGTGACCG CCACATGCGCCCACCCAGAGAGCCTGAAGGGCCAGAGCATCTTCTCCGTGCTGCCCGATAGCTTC GTGTGCGACGATTTTCCTAAGCCACAGATCATCACCCAGCCAGAGACAACAATGGCCGTGGTGGG CAAGGACATCCGGTTTACATGTTCCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCCTGCACCGCACAGAACAGCGCCGGCAGCGTGTCC GCCAACGCCACCCTGACCGTGCTGGAGACACCATCCCTGGCCGTGCCCCTGGAGGACAGGGTGGT GACCGTGGGCGAGACAGTGGCCTTTCAGTGTAAGGCCACCGGCTCTCCAACACCAAGGATCACCT GGCTGAAGGGCGGCAGGCCCCTGAGCCTGACAGAGCGCCACCACTTCACCCCTGGCAATCAGCTG CTGGTGGTGCAGAACGTGATGATCGATGACGCCGGCAGGTATACATGCGAGATGAGCAATCCTCT GGGCACCGAGAGGGCACACTCCCAGCTGTCTATCCTGCCTACCCCAGGCTGCCGGAAGGATGGCA CCACA GGCGGTGGC GGATCC Gagcctcgggccctaccatcaagccctgcccccttgcaagtgccctgccccctaatctgctggg cggaccctccgtgttc atcttcccacccaagatcaaggacgtgctgatgatctccctgtcccccatcgtgacctgcgtggt ggtggacgtgtccgaggacgacccggacgtgcagatcagttggttcgtgaacaacgtggaagtgc acaccgcccagacccagacccacagagaggactacaactccaccctgcgggtggtgtccgccctg cccatccagcaccaggactggatgtccggcaaagaattcaagtgcaaagtgaacaacaaggacct gcctgcccccatcgagcggaccatctccaagcccaagggctccgtgcgggctccccaggtgtacg tgctgcccctccagaggaagagatgaccaagaagcaggtcacactgacctgcatggtcaccgac ttcatgcccgaggacatctacgtggaatggaccaacaatggcaagaccgagctgaactacaagaa caccgagcctgtgctggactccgacggctcctacttcatgtactccaagctgcgggtggaaaaga agaactgggtcgagcggaactcctactcctgctccgtggtgcacgagggcctgcacaaccaccac accaccaagtccttctcccggacccccggcaaa |
| SEQ ID NO: 83 | GCTCAGGCTGGACCTAGGCCTCCTTGGGCTGCCGCCTGCACCTGTGCAGGCGATTCTCTGGACTG |
| | CAGCGGCCGGGGCCTGGCCACACTGGCCAGGGACCTGCCTTCCTGGACCAGATCTCTGAACCTGA GCTACAATCGGCTGTCCGAGATCGATTCTGCCGCCTTTGAGGACCTGACAAATCTGCAGGAGGTG TATCTGAACAGCAATGAGCTGACCGCAATCCCCTCCCTGGGAGCAGCCTCTATCGGCGTGGTGAG CCTGTTCCTGCAGCACAACAAGATCCTGAGCGTGGATGGCTCCCAGCTGAAGAGCTACCTGTCTC TGGAGGTGCTGGACCTGAGCTCCAACAATATCACCGAGATCAGATCTAGCTGTTTTCCTAATGGC CTGCGGATCAGAGAGCTGAACCTGGCCTCTAATCGGATCAGCATCCTGGAGTCCGGCGCCTTCGA TGGCCTGAGCAGATCCCTGCTGACACTGCGCCTGTCCAAGAACCGGATCACCCAGCTGCCCGTGA AGGCCTTTAAGCTGCCTAGGCTGACACAGCTGGACCTGAACCGGAATAGAATCAGGCTGATCGAG GGCCTGACCTTCCAGGGCCTGGATAGCCTGGAGGTGCTGCGCCTGCAGCGGAACAATATCTCCCG CCTGACAGACGGAGCATTTTGGGGCCTGTCTAAGATGCACGTGCTGCACCTGGAGTACAATAGCC TGGTGGAGGTGAACTCTGGCAGCCTGTATGGCCTGACCGCCCTGCACCAGCTGCACCTGTCCAAC AATAGCATCAGCAGAATCCAGAGGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCCTTCAC AGGCCTGGACAATCTGTCCAAGCTGACCCTGTTTGGCAACAAGATCAAGTCTGTGGCCAAGCGGG CCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCACCTTGGCTGATGGGAAGGATGCTGCAGGCCTTTGTGACCG CCACATGCGCCCACCCAGAGAGCCTGAAGGGCCAGAGCATCTTCTCCGTGCTGCCCGATAGCTTC GTGTGCGACGATTTTCCTAAGCCACAGATCATCACCCAGCCAGAGACAACAATGGCCGTGGTGGG CAAGGACATCCGGTTTACATGTTCCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCCTGCACCGCACAGAACAGCGCCGGCAGCGTGTCC GCCAACGCCACCCTGACCGTGCTGGAGACACCATCCCTGGCCGTGCCCCTGGAGGACAGGGTGGT GACCGTGGGCGAGACAGTGGCCTTTCAGTGTAAGGCCACCGGCTCTCCAACACCAAGGATCACCT GGCTGAAGGGCGGCAGGCCCCTGAGCCTGACAGAGCGCCACCACTTCACCCCTGGCAATCAGCTG CTGGTGGTGCAGAACGTGATGATCGATGACGCCGGCAGGTATACATGCGAGATGAGCAATCCTCT GGGCACCGAGAGGGCACACTCCCAGCTGTCTATCCTGCCTACCCCAGGCTGCCGGAAGGATGGCA CCACA GGCGGTGGC GGATCC cgcaacaccggcccgcggcggcgaggagaagaagaaggagaaggagaaggaggagcaggaggagcg cgagaccaagaccccggagtgcccagccacacccagcccctggcgtgttc atcttcccacccaagatcaaggacgtgctgatgatctccctgtcccccatcgtgacctgcgtggt ggtggacgtgtcccaggacgaccccgacgtgcagatcagttggttcgtgaacaacgtggaagtgc acaccgcccagacccagacccacagagaggactacaactccaccctgcgggtggtgtccgccctg cccatccagcaccaggactggatgtccggcaaagaattcaagtgcaaagtgaacaacaaggacct gcctgcccccatcgagcggaccatctccaagcccaagggctccgtgcgggctccccaggtgtacg tgctgcccctccagaggaagagatgaccaagaagcaggtcacactgacctgcatggtcaccgac ttcatgcccgaggacatctacgtggaatggaccaacaatggcaagaccgagctgaactacaagaa caccgagcctgtgctggactccgacggctcctacttcatgtactccaagctgcgggtggaaaaga agaactgggtcgagcggaactcctactcctgctccgtggtgcacgagggcctgcacaaccaccac accaccaagtccttctcccggacccccggcaaa |
| SEQ ID NO: 84 | GCTCAGGCTGGACCTAGGGCTCCTTGGCTGCCGCCTGCACCTGTGCAGGCGATTCTCTGGACTG CAGCGGCCGGGGCCTGGCCACACTGCCCAGGGACCTGCCCTTCCTGGACCAGATCTCTGAACCTGA |
| | GCTACAATCGGCTGTCCGAGATCGATTCTGCGGCCTTTGAGGACCTGACAAATCTGCAGGAGGTG TATCTGAACAGCAATGAGCTGACCGCAATCCCCTCCCTGGGAGCAGCCTCTATCGGCGTGGTGAG CCTGTTCCTGCAGCACAACAAGATCCTGAGCGTGGATGGCTCCCAGCTGAAGAGCTACCTGTCTC TGGAGGTGCTGGACCTGAGCTCCAACAATATCACCGAGATCAGATCTAGCTGTTTTCCTAATGGC CTGCGGATCAGAGAGCTGAACCTGGCCTCTAATCGGATCAGCATCCTGGAGTCCGGCGCCTTCGA TGGCCTGAGCAGATCCCTGCTGACACTGCGCCTGTCCAAGAACCGGATCACCCAGCTGCCCGTGA AGGCCTTTAAGCTGCCTAGGCTGACACAGCTGGACCTGAACCGGAATAGAATCAGGCTGATCGAG GGCCTGACCTTCCAGGGCCTGGATAGCCTGGAGGTGCTGCGCCTGCAGCGGAACAATATCTCCCG CCTGACAGACGGAGCATTTTGGGGCCTGTCTAAGATGCACGTGCTGCACCTGGAGTACAATAGCC TGGTGGAGGTGAACTCTGGCAGCCTGTATGGCCTGACCGCCCTGCACCAGCTGCACCTGTCCAAC AATAGCATCAGCAGAATCCAGAGGGATGGCTGGTCCTTCTGCCAGAAGCTGCACGAGCTGATCCT GTCTTTTAACAATCTGACCAGGCTGGACGAGGAGAGCCTGGCAGAGCTGTCCTCTCTGTCCATCC TGCGCCTGTCTCACAATGCCATCAGCCACATCGCCGAGGGCGCCTTTAAGGGCCTGAAGAGCCTG AGGGTGCTGGATCTGGACCACAACGAGATCTCTGGCACCATCGAGGATACAAGCGGCGCCTTCAC AGGCCTGGACAATCTGTCCAAGCTGACCCTGTTTGGCAACAAGATCAAGTCTGTGGCCAAGCGGG CCTTCTCTGGCCTGGAGAGCCTGGAGCACCTGAACCTGGGCGAGAATGCCATCAGATCCGTGCAG TTCGATGCCTTTGCCAAGATGAAGAATCTGAAGGAGCTGTACATCAGCTCCGAGAGCTTCCTGTG CGACTGTCAGCTGAAGTGGCTGCCACCTTGGCTGATGGAAGGATGCTGCAGGCCTTTGTGACCG CCACATGCGCCCACCCAGAGAGCCTGAAGGGCCAGAGCATCTTCTCCGTGCTGCCCGATAGCTTC GTGTGCGACGATTTTCCTAAGCCACAGATCATCACCCAGCCAGAGACAACAATGGCCGTGGTGGG CAAGGACATCCGGTTTACATGTTCCGCCGCCTCTAGCTCCTCTAGCCCCATGACCTTCGCCTGGA AGAAGGATAACGAGGTGCTGGCCAATGCCGACATGGAGAACTTCGCCCACGTGAGAGCCCAGGAT GGCGAAGTGATGGAGTATACCACAATCCTGCACCTGCGGCACGTGACCTTTGGCCACGAGGGCAG ATACCAGTGCATCATCACAAATCACTTCGGCTCTACCTATAGCCACAAGGCCAGGCTGACAGTGA ACGTGCTGCCTAGCTTTACCAAGATCCCACACGACATCGCCATCAGAACAGGCACCACAGCAAGG CTGGAGTGTGCAGCAACCGGACACCCAAACCCTCAGATCGCATGGCAGAAGGATGGAGGCACAGA CTTCCCTGCAGCCCGCGAGAGGAGAATGCACGTGATGCCAGACGATGACGTGTTCTTTATCACAG ATGTGAAGATCGATGACATGGGCGTGTACTCCTGCACCGCACAGAACAGCGCCGGCAGCGTGTCC GCCAACGCCACCCTGACCGTGCTGGAGACACCATCCCTGGCCGTGCCCCTGGAGGACAGGTGGT GACCGTGGGCGAGACAGTGGCCTTTCAGTGTAAGGCCACCGGCTCTCCAACACCAAGGATCACCT GGCTGAAGGGCGCAGGCCCTGAGCTGACAGAGCGCCACCACTTCACCCCTGGCAATCAGCTG CTGGTGGTGCAGAACGTGATGATCGATGACGCCGGCAGGTATACATGCGAGATGAGCAATCCTCT GGGCACCGAGAGGGCACACTCCCAGCTGTCTATCCTGCCTACCCCAGGCTGCCGGAAGGATGGCA CCACA GGCGGTGGC GGATCC gaaccgaaatcttctgacaaaacccacacctctccgccgtctccggctccggaactgctgggtgg ttcttctgtttc atcttcccacccaagatcaaggacgtgctgatgatctccctgtcccccatcgtgacctgcgtggt ggtggacgtgtccgaggacgaccccgacgtgcagatcagttggttcgtgaacaacgtggaagtgc acaccgcccagacccagacccacagagaggactacaactccaccctgcgggtggtgtccgccctg cccatccagcaccaggactggatgtccggcaaagaattcaagtgcaaagtgaacaacaaggacct gcctgcccccatcgagcggaccatctccaagcccaagggctccgtgcgggctccccaggtgtacg tgctgcccccctccagaggaagagatgaccaagaagcaggtcacactgacctgcatggtcaccgac ttcatgcccgaggacatctacgtggaatggaccaacaatggcaagaccgagctgaactacaagaa caccgagcctgtgctggactccgacggctcctacttcatgtactccaagctgcgggtggaaaaga agaactgggtcgagcggaactcctactcctgctccgtggtgcacgagggcctgcacaaccaccac accaccaagtccttctcccggacccccggcaaa |

### 2) nucleic acid molecule

The composition of the present invention comprises a nucleic acid molecule encoding the fusion protein provided in the present invention.

The nucleic acid molecule of the present invention includes any nucleic acid molecule obtained by causing the amino acid sequence of the fusion protein provided in the present invention to be converted into a polynucleotide sequence as is known to those skilled in the art. Therefore, various polynucleotide sequences can be prepared due to open reading frame (ORF), all of which are also included in the nucleic acid molecule of the present invention.

### 3) Expression vector

The composition of the present invention comprises an expression vector into which the isolated nucleic acid molecule provided in the present invention is inserted.

In the present invention, the "vector" is a nucleic acid molecule that is capable of transporting another nucleic acid linked thereto. One type of vector is a "plasmid," which refers to circular double-stranded DNA into which additional DNA segments can be ligated. Another type of vector is a phage vector. Yet another type of vector is a viral vector, in which additional DNA segments can be ligated into the viral genome. Certain vectors are capable of autonomous replication in a host cell into which they are introduced (for example, bacterial vectors are episomal mammalian vectors having a bacterial origin of replication). Other vectors (for example, non-episomal mammalian vectors) can be integrated into the genome of a host cell upon introduction into the host cell, and thus are replicated along with the host genome. In addition, certain vectors are capable of directing expression of genes to which they are operatively linked. Such vectors are referred to herein as "recombinant expression vectors" or simply "expression vectors." In general, expression vectors useful in recombinant DNA techniques are often in the form of plasmids. In the present specification, "plasmid" and "vector" may be used interchangeably as the plasmid is the most commonly used form of vector.

Specific examples of the expression vector in the present invention may be selected from, but are not limited to, the group consisting of commercially widely used pCDNA vectors, F, R1, RP1, Col, pBR322, ToL, Ti vectors; cosmids; phages such as lambda, lambdoid, M13, Mu, p1 P22, Qµ, T-even, T2, T3, T7; plant viruses. Any expression vector known, to those skilled in the art, as an expression vector can be used in the present invention, and the expression vector is selected depending on the nature of the target host cell. Introduction of a vector into a host cell may be performed by calcium phosphate transfection, viral infection, DEAE-dextran-mediated transfection, lipofectamine transfection, or electroporation. However, the present invention is not limited thereto, and those skilled in the art may adopt and use an introduction method appropriate for the expression vector and the host cell which are used. The vector may preferably contain at least one selection marker. However, the present invention is not limited thereto, and selection can be made using the vector that contains no selection marker, depending on whether or not a product is produced. The selection marker is selected depending on the target host cell, which is done using methods already known to those skilled in the art, and thus the present invention has no limitation thereon.

To facilitate purification of the nucleic acid molecule of the present invention, a tag sequence may be inserted into and fused to an expression vector. The tag includes, but is not limited to, hexa-histidine tag, hemagglutinin tag, myc tag, or flag tag, and any tag known to those skilled in the art which facilitates purification can be used in the present invention.

### 4) Host cell

The composition of the present invention comprises a host cell transfected with the expression vector provided in the present invention.

In the present invention, the "host cell" includes individual cells or cell cultures which may be or have been recipients of the vector(s) for incorporation of a polypeptide insert. The host cell includes progeny of a single host cell, and the progeny may not necessarily be completely identical (in morphology or in genomic DNA complement) to the original parent cell due to natural, accidental, or intentional mutation. The host cell includes cells transfected *in vivo* with the polynucleotide(s) herein.

In the present invention, the host cell may include cells of mammalian, plant, insect, fungal, or cellular origin, and may be, for example, bacterial cells such as E. coli, Streptomyces, Salmonella typhimurium; fungal cells such as yeast cells and Pichia pastoris; insect cells such as Drosophila and Spodoptera Sf9 cells; animal cells such as Chinese hamster ovary (CHO) cells, SP2/0 (mouse myeloma), human lymphoblastoid, COS, NSO (mouse myeloma), 293T, Bowes melanoma cells, HT-1080, baby hamster kidney (BHK) cells, human embryonic kidney (HEK) cells, or PERC.6 (human retinal cells); or plant cells. However, the host cell is not limited thereto, and any cell known to those skilled in the art which can be used as a host cell line is available.

### 5) Combination with V-domain Ig suppressor of T cell activation (VISTA)

In the present invention, the composition may further comprise V-domain Ig suppressor of T cell activation (VISTA) or a fragment thereof.

In the present invention, the composition may further comprise a fusion protein that includes VISTA or a fragment thereof and an immunoglobulin Fc region. Here, a linker may be further included between the VISTA or a fragment thereof and the immunoglobulin Fc region.

In the present invention, the "V-domain Ig suppressor of T cell activation (VISTA)" corresponds to an immune checkpoint protein that functions to suppress immune activity against T cells. VISTA corresponds to a surface protein that includes an extracellular domain containing an IgG-V domain and a short cytoplasmic domain not containing ITAM and ITIM. VISTA is expressed in CD11b+ myeloid dendritic cells (DCs), CD4+ and CD8+ T cells, and Foxp3+CD4+ regulatory T cells, and can function to inhibit secretion and proliferation of cytokines following T cell activation. VISTA-deficient knock-out mice exhibit decreased organ function due to chronic inflammation, and show symptoms of multiple sclerosis much faster than normal mice.

In an example of the present invention, the VISTA may be derived from mammals, including primates such as humans and monkeys, rodents such as mice and rats, and the like.

In an example of the present invention, the VISTA may consist of the amino acid sequence represented by SEQ ID NO: 85, which is human-derived VISTA, or may be encoded by the nucleotide represented by SEQ ID NO: 87. However, the present invention is not limited thereto.

In an example of the present invention, the VISTA may consist of the amino acid sequence represented by SEQ ID NO: 86, which is mouse-derived VISTA, or may be encoded by the nucleotide represented by SEQ ID NO: 88. However, the present invention is not limited thereto.

In an example of the present invention, the fragment of VISTA may be, but is not limited to, an extracellular domain of VISTA.

In an example of the present invention, the extracellular domain of VISTA may consist of, but is not limited to, the amino acid sequence represented by SEQ ID NO: 89 that corresponds to a sequence of amino acids 33 to 194 of human-derived VISTA.

In an example of the present invention, the extracellular domain of VISTA may consist of, but is not limited to, the amino acid sequence represented by SEQ ID NO: 90 that corresponds to a sequence of amino acids 33 to 191 of mouse-derived VISTA.

In an example of the present invention, the fragment of VISTA may be, but is not limited to, a fragment of the extracellular domain of VISTA.

In the present invention, the fragment of the extracellular domain of VISTA may consist of an amino acid sequence represented by any one of SEQ ID NOs: 91 to 93 that are some fragments of the extracellular domain of human-derived VISTA. However, the present invention is not limited thereto.

In the present invention, the fragment of the extracellular domain of VISTA may consist of the amino acid sequence represented by SEQ ID NO: 94 or 95 that is a fragment of the extracellular domain of mouse-derived VISTA. However, the present invention is not limited thereto.

Here, description of the immunoglobulin Fc region and the linker overlaps with that described above, and thus will be omitted to avoid excessive complexity of the specification.

In the present invention, VISTA or a fragment thereof, or a fusion protein including the same, can be easily obtained by inserting, in a host cell, an expression vector, into which a nucleic acid molecule is inserted, the nucleic acid molecule being obtained by causing the amino acid sequence provided in the present invention to be converted into a polynucleotide sequence as is known to those skilled in the art, culturing the host cell, and then performing purification using chromatography or the like.

Here, description of the nucleic acid molecule, the expression vector, and the host cell also overlaps with that described above, and thus will be omitted to avoid excessive complexity of the specification.

### 6) Immune-related diseases and combination

### Immune-related diseases

In the present invention, the "immune related disease" is a disease caused by excessive activation and/or proliferation of various immune cells, for example, effector T cells or cytotoxic T cells and inflammatory cells, and may include, but is not limited to, autoimmune diseases; graft versus host disease; organ transplant rejection; asthma; atopy; or acute or chronic inflammatory diseases.

In the present invention, the "autoimmune disease" may be at least one selected from, but not limited to, the group consisting of rheumatoid arthritis, type 1 diabetes, systemic scleroderma, systemic lupus erythematosus, systemic lupus erythematosus, atopic dermatitis, psoriasis, alopecia areata, asthma, Crohn's disease, Behcet's disease, Sjogren's syndrome, Guillain-Barre syndrome, chronic thyroiditis, multiple sclerosis, multiple myositis, ankylosing spondylitis, fibromyalitis, and polyarteritis nodosa.

In addition, in the present invention, the "autoimmune disease" may be caused by a process in which activated T cells infiltrate cells constituting a nerve so that inflammation or apoptosis is induced, and may be, for example, a neurodegenerative disease or a neuroinflammatory disease (see Neuromolecular Med. 2005; 7(3): 255-64).

In the present invention, the neurodegenerative disease or neuroinflammatory disease may be selected from, but not limited to, the group consisting of stroke, dementia, Alzheimer's disease, Parkinson's disease, Huntington's disease, Niemann-Pick disease, prion disease, Creutzfeldt-Jakob disease, frontal temporal dementia, Lewis dementia, amyotrophic lateral sclerosis, paraneoplastic syndrome, corticobasal degeneration, multisystematic atrophy, progressive nuclear paralysis, autoimmune disease in nervous system, spinocerebellar ataxia, inflammatory and neuropathic pain, cerebrovascular disease, spinal cord injury, and tauopathy.

In the present invention, the immune-related disease, for example, the autoimmune disease may be caused by presence of a high frequency of autoimmune T cells (for example, effector T cells or cytotoxic T cells) or remarkably increased activity thereof in patients with disease, as compared with healthy controls, which, in turn, induces an excessive immune response or destroys cells of a specific organ (see Clinical & Experimental Immunology, 180: 353-360., Immunity, Vol. 17, 1-6, July, 2002., Ann Rheum Dis 2004;63(Suppl II): 96-101., Immunol Res. 2013 December; 57(0): 12-22).

In the present invention, the immune-related disease can be prevented, ameliorated, or treated by interfering with intracellular signaling pathways essential for activation and/or proliferation of T cells (for example, effector T cells or cytotoxic T cells), preferably autoreactive T cells (see Ann Rheum Dis 2004;63(Suppl II): 96-101). Specifically, the fusion protein according to the present invention can induce a co-inhibitory signal in effector T cells, thereby inhibiting proliferation of effector T cells and regulating activation thereof. Thus, the fusion protein can be used to effectively prevent, ameliorate, or treat various immune-related diseases that may be caused by excessive activation and/or proliferation of T cells (for example, effector T cells or cytotoxic T cells).

### Combination with therapeutic agents for immune-related diseases

The composition of the present invention may be used in combination with other drugs for treating the immune-related diseases. For example, the autoimmune diseases can be treated with molecules according to at least some embodiments of the present invention, together with (but not limited to) immunosuppressants such as corticosteroid, cyclosporine, cyclophosphamide, prednisone, azathioprine, methotrexate, rapamycin, and tacrolimus, biological agents such as TNF-α blocker or antagonist, or any other biological agents that target any inflammatory cytokine, nonsteroidal anti-inflammatory drug/Cox-2 inhibitor, hydroxychloroquine, sulphasalazopryine, gold salt, etanercept, infliximab, mycophenolate mofetil, basiliximab, atacicept, rituximab, cytoxan, interferon β-1a, interferon β-1b, glatiramer acetate, mitoxantrone hydrochloride, anakinra, and/or other biological substances and/or intravenous immunoglobulin (IVIG). Non-limiting examples of such known therapeutic agents include interferons such as IFN-β-1a (REBIF®, AVONEX®, and CINNOVEX® and IPN-β-1b (BETASERON®, EXTAVIA®, BETAFERON®, and ZIFERON®); glatiramer acetate (COPAXONE®) which is a polypeptide; natalizumab (TYSABRI®); and mitoxantrone (NOVANTRONE®) which is a cytotoxic agent.

In addition, the composition of the present invention may be used in combination with any known therapeutic agent or method for treating multiple sclerosis. Non-limiting examples of such a known therapeutic agent or method for treating multiple sclerosis include interferons such as IFN-β-1a (REBIF®, AVONEX®, and CINNOVEX®) and IPN-β-1b (BETASERON®, EXTAVIA®, BETAFERON®, and ZIFERON®); glatiramer acetate (COPAXONE®) which is a polypeptide; natalizumab (TYSABRI®); and mitoxantrone (NOVANTRONE®) which is a cytotoxic agent, fampridine (AMPYRA®). Other drugs include corticosteroid, methotrexate, cyclophosphamide, azathioprine and intravenous immunoglobulin (IVIG), inosine, ocrelizumab (R1594), Mylinax (Caldribine), alemtuzumab (Campath), daclizumab (Zenapax), Panaclar/dimethyl fumarate (BG-12), Teriflunomide (HMR1726), Fingolimod (FTY720), Laquinimod (ABR216062), as well as hematopoietic stem cell transplantation, Neurovx, rituximab (Rituxan), BCG vaccine, low-dose naltrexone, anthelmintic therapy, angioplasty, venous stent, and alternative therapies such as vitamin D, polyunsaturated fat, and medical marijuana.

In addition, the composition of the present invention may be used in combination with any known therapeutic agent or method for treating rheumatoid arthritis. Non-limiting examples of such a known therapeutic agent or method for treating rheumatoid arthritis include glucocorticoids, nonsteroidal anti-inflammatory drug (NSAID) such as salicylates, or cyclooxygenase-2 inhibitors, ibuprofen and naproxen, diclofenac, indomethacin, etodolac; disease-modifying antirheumatic drugs (DMARDs) - oral DMARDs: Auranofin (Ridaura®), Azathioprine (Imuran®), cyclosporine (Sandimmune®, Gengraf®, and Neoral®, each of which is generic), D-penicillamine (Cuprimine®), hydroxychloroquine (Plaquenil®), IM gold sodium thiomalate (Myochrysine®), aurothioglucose (Solganal®), leflunomide (Arava®), methotrexate (Rheumatrex®), minocycline (Minocin®), staphylococcal protein A immunoadsorption (Prosorba® column), sulfasalazine (Azulfidine®); biological DMARDs: TNF-α blockers including adalimumab (Humira®), etanercept (Enbrel®), infliximab (Remicade®), golimumab (Simponi®), certolizumab pegol (Cimzia®), and other biological DMARDs, such as anakinra (Kineret®), rituximab (Rituxan®), tocilizumab (Actemra®), CD28 inhibitors including Abatacept (Orencia®) and belatacept.

In addition, the composition of the present invention may be used in combination with any known therapeutic agent or method for type 1 diabetes. Non-limiting examples of such a known therapeutic agent or method for treating type 1 diabetes include insulin, insulin analogs, islet transplantation, stem cell therapy including PROCHYMAL®, non-insulin therapies such as IL-1β inhibitors including anakinra (Kineret®), Abatacept (Orencia®), diamyd, alefacept (Ameviv®), otelixizumab, DiaPep277 (Hsp60 derived peptide), alpha 1-antitrypsin, prednisone, azathioprine, ciclosporin, E1-INT (an injectable islet neogenesis therapy comprising an epidermal growth factor analog and a gastrin analog), statins including Zocor®, Simlup®, Simcard®, Simvacor®, Sitagliptin® (dipeptidyl peptidase (DPP-4) inhibitor), anti-CD3 mAb (for example, teplizumab); CTLA4-Ig (Abatacept), anti IL-1β (canakinumab), anti-CD20 mAb (for example, rituximab).

In addition, the composition of the present invention may be used in combination with any known therapeutic agent or method for treating Sjogren's syndrome. Non-limiting examples of such a known therapeutic agent or method for treating Sjogren's syndrome include cyclosporine, pilocarpine (Salagen®) and cevimeline (Evoxac®), hydroxychloroquine (Plaquenil®), cortisone (prednisone and others) and/or azathioprine (Imuran®) or cyclophosphamide (Cytoxan®), dexamethasone, thalidomide, dehydroepiandrosterone, NGX267, rebamipide, FID 114657, Etanercept®, Raptiva®, belimumab, MabThera® (rituximab); anakinra, intravenous immunoglobulin (IVIG), allogeneic mesenchymal stem cells (AlloMSCs), automatic neuro-electrostimulation by "Saliwell Crown".

In addition, the composition of the present invention may be used in combination with any known therapeutic agent or method for treating systemic lupus erythematosus. Non-limiting examples of such a known therapeutic agent or method for treating systemic lupus erythematosus include corticosteroids and disease-modifying antirheumatic drugs (DMARDs), commonly anti-malarial drugs such as plaquenil and immunosuppressants (for example, methotrexate and azathioprine) hydroxychloroquine, cytotoxic drugs (for example, cyclophosphamide and mycophenolate), hydroxychloroquine (HCQ), Benlysta® (belimumab), nonsteroidal anti-inflammatory drugs, prednisone, cellcept, prograf, atacicept, lupuzor, intravenous immunoglobulin (IVIG), CellCept® (mycophenolate mofetil), Orencia®, CTLA4-IgG4m (RG2077), rituximab, ocrelizumab, epratuzumab, CNTO 136, Sifalimumab (MEDI-545), A-623 (formerly AMG 623), AMG 557, rontalizumab, paquinimod (ABR-215757), LY2127399, CEP-33457, dehydroepiandrosterone, levothyroxine, abetimus sodium (LJP 394), memantine, opiates, rapamycin, renal transplantation, and stem cell transplantation.

### 7) Terms and composition

In the present invention, the "prevention" may include, without limitation, any act of blocking symptoms of a disease, or suppressing or delaying the symptoms, using the composition of the present invention.

In the present invention, the "amelioration" or "treatment" may include, without limitation, any act of ameliorating or beneficially altering symptoms of a disease, using the composition of the present invention.

In the present invention, the pharmaceutical composition may be characterized by being in the form of capsules, tablets, granules, injections, ointments, powders, or beverages, and the pharmaceutical composition may be characterized by being targeted to humans.

In the present invention, the pharmaceutical composition may be formulated in the form of oral preparations such as powders, granules, capsules, tablets, and aqueous suspensions, preparations for external use, suppositories, and sterile injectable solutions, respectively, according to conventional methods, and used. However, the pharmaceutical composition is not limited thereto. The pharmaceutical composition of the present invention may further comprise a pharmaceutically acceptable carrier. As the pharmaceutically acceptable carrier, a binder, a glidant, a disintegrant, an excipient, a solubilizer, a dispersant, a stabilizer, a suspending agent, a pigment, a flavor, and the like may be used for oral administration; a buffer, a preserving agent, a pain-relieving agent, a solubilizer, an isotonic agent, a stabilizer, and the like may be used in admixture for injections; and a base, an excipient, a lubricant, a preserving agent, and the like may be used for topical administration. The preparations of the pharmaceutical composition of the present invention may be prepared in various ways by being mixed with the pharmaceutically acceptable carrier as described above. For example, for oral administration, the pharmaceutical composition may be formulated in the form of tablets, troches, capsules, elixirs, suspensions, syrups, wafers, or the like. For injections, the pharmaceutical composition may be formulated in the form of unit dosage ampoules or multiple dosage forms. Alternatively, the pharmaceutical composition may be formulated into solutions, suspensions, tablets, capsules, sustained-release preparations, or the like.

Meanwhile, as examples of carriers, excipients, or diluents suitable for making preparations, lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, or the like may be used. In addition, a filler, an anti-coagulant, a lubricant, a wetting agent, a fragrance, an emulsifier, a preservative, and the like may further be included.

In the present invention, the route of administration of the pharmaceutical composition includes, but is not limited to, oral, intravenous, intramuscular, intraarterial, intramedullary, intradural, intracardiac, transdermal, subcutaneous, intraperitoneal, intranasal, intestinal, topical, sublingual, or rectal route. Oral or parenteral administration is preferred.

In the present invention, the "parenteral" includes subcutaneous, intradermal, intravenous, intramuscular, intraarticular, intrabursal, intrasternal, intradural, intralesional, and intracranial injection or infusion techniques. The pharmaceutical composition of the present invention may also be administered in the form of suppositories for rectal administration.

The pharmaceutical composition of the present invention may vary widely depending on a variety of factors, including activity of a certain compound used, the patient's age, body weight, general health status, sex, diet, time of administration, route of administration, rate of excretion, drug combination, and severity of a certain disease to be prevented or treated. A dose of the pharmaceutical composition may vary depending on the patient's condition, body weight, severity of disease, drug form, route of administration, and duration, and may be appropriately selected by those skilled in the art. The pharmaceutical composition may be administered in an amount of 0.0001 to 50 mg/kg or 0.001 to 50 mg/kg, per day. Administration may be made once a day or several times a day. The dose is not intended to limit the scope of the invention in any way. The pharmaceutical composition according to the present invention may be formulated in the form of pills, sugar-coated tablets, capsules, liquids, gels, syrups, slurries, or suspensions.

In the present invention, the food composition may be prepared in the form of various foods, for example, beverages, gums, tea, vitamin complexes, powders, granules, tablets, capsules, confections, rice cakes, bread, and the like.

In the present invention, in a case where the active ingredient is contained in the food composition, the amount thereof to be added may be, but is not limited to, 0.1% to 50% of a total weight of the food composition.

In the present invention, in a case where the food composition is prepared in the form of a beverage, there is no particular limitation except that the beverage contains the food composition at an indicated proportion, and the beverage may contain various flavoring agents or natural carbohydrates, or the like as additional ingredients similarly to conventional beverages. Specifically, examples of the natural carbohydrates may include monosaccharides such as glucose, disaccharides such as fructose, polysaccharides such as sucrose, conventional sugars such as dextrin and cyclodextrin, and sugar alcohol such as xylitol, sorbitol, and erythritol. Examples of the flavoring agents may include natural flavoring agents (thaumatin, stevia extracts (such as rebaudioside A), glycyrrhizin, and the like) and synthetic flavoring agents (saccharin, aspartame, and the like).

In the present invention, the food composition may further contain various nutrients, vitamins, minerals (electrolytes), flavorings such as synthetic flavorings and natural flavorings, colorants, pectic acid and salts thereof, alginic acid and salts thereof, organic acids, protective colloidal thickeners, pH adjusting agents, stabilizers, preservatives, glycerin, alcohol, carbonizing agents used in carbonated beverages, and the like.

In the present invention, the above-mentioned ingredients may be used individually or in combination. The proportion of such additives does not correspond to a key element of the present invention, and may be selected from, but is not limited to, the range of 0.1 to about 50 parts by weight per 100 parts by weight of the food composition of the present invention.

In the present invention, the cosmetic composition may be prepared in the form of skin softeners, nourishing lotions, nourishing essences, massage creams, cosmetic bath water additives, body lotions, body milks, bath oil, baby oil, baby powders, shower gels, shower creams, sun screen lotions, sun screen creams, suntan creams, skin lotions, skin creams, UV blocking cosmetics, cleansing milks, hair removing agents (for cosmetic purposes), face and body lotions, face and body creams, skin whitening creams, hand lotions, hair lotions, cosmetic creams, Jasmine oil, bath soaps, liquid soaps, cosmetic soaps, shampoos, hand cleaners, medicinal soaps (for non-medical purposes), cream soaps, facial washes, body cleansers, scalp cleansers, hair rinses, toilet soaps, tooth whitening gels, toothpastes, and the like. The composition of the present invention may further contain either a solvent which is conventionally used for the preparation of cosmetic compositions, or a suitable carrier, excipient, or diluent.

In the present invention, the type of solvent that may further be added to the cosmetic composition is not particularly limited, and examples of the solvent may include water, saline, DMSO, or a combination thereof. In addition, examples of the carrier, excipient, or diluent include, but are not limited to, purified water, oil, wax, fatty acids, fatty acid alcohol, fatty acid esters, surfactants, humectants, thickeners, antioxidants, viscosity stabilizers, chelating agents, buffers, lower alcohol, and the like. In addition, the cosmetic composition of the present invention may, if necessary, contain whitening agents, moisturizing agents, vitamins, UV blocking agents, fragrances, dyes, antibiotics, antibacterial agents, and antifungal agents.

In the present invention, examples of the oil may include hydrogenated vegetable oil, castor oil, cottonseed oil, olive oil, palm kernel oil, jojoba oil, and avocado oil, and examples of the wax may include beeswax, spermaceti, carnauba wax, candelilla wax, montan wax, ceresin wax, liquid paraffin, and lanolin.

In the present invention, examples of the fatty acids may include stearic acid, linoleic acid, linolenic acid, and oleic acid; examples of the fatty acid alcohol may include cetyl alcohol, octyl dodecanol, oleyl alcohol, panthenol, lanolin alcohol, stearyl alcohol, and hexadecanol; and examples of the fatty acid esters may include isopropyl myristate, isopropyl palmitate, and butyl stearate. Examples of the surfactants may include cationic surfactants, anionic surfactants, and nonionic surfactants, which are known in the art. Among these, if possible, surfactants derived from natural products are preferred. In addition, the cosmetic composition of the present invention may contain humectants, thickeners, antioxidants, and the like, which are widely known in the cosmetic field, and the types and amounts thereof are as known in the art.

### 2. Method for preventing, ameliorating, or treating immune-related diseases

According to another embodiment of the present invention, there is provided a method for preventing, ameliorating, or treating an immune-related disease, comprising a step of administering, to an individual, the fusion protein according to the present invention; a nucleic acid molecule encoding the same; an expression vector containing the nucleic acid molecule; or a host cell transformed with the expression vector; or a composition including any one of the foregoing.

In the present invention, descriptions related to the fusion protein, the nucleic acid molecule, the expression vector, the host cell, the composition, and the immune-related diseases overlap with those described above for the pharmaceutical composition, and thus will be omitted to avoid excessive complexity of the specification.

In the present invention, the "individual" is an individual suspected of developing an immune-related disease, and the individual suspected of developing an immune-related disease means a mammal, such as humans, mice, and domestic animals, who has developed or is likely to develop the disease in question. However, any individual, who is treatable with the fusion protein of the present invention or the composition comprising the same, is included therein without limitation.

The method of the present invention may comprise administering a fusion protein or a composition comprising the same in a pharmaceutically effective amount. An appropriate total daily amount used may be determined by an attending physician or veterinarian within the scope of sound medical judgment, and administration may be made once or several times. However, for the purposes of the present invention, a specific therapeutically effective amount for a particular patient is preferably applied differently depending on various factors, including type and degree of reaction to be achieved, the specific composition including whether other agents are used therewith as the case may be, the patient's age, body weight, general health status, sex, and diet, time of administration, route of administration, secretion rate of the composition, duration of treatment, and drugs used simultaneously or in combination with the specific composition, and similar factors well known in the medical field.

Meanwhile, the method for preventing, ameliorating, or treating immune-related diseases may be, but is not limited to, a combination therapy that further comprises administering a compound or substance having therapeutic activity against one or more immune-related diseases.

In the present invention, the "combination" should be understood to represent simultaneous, individual, or sequential administration. In a case where the administration is made in a sequential or individual manner, the second component should be administered at intervals such that beneficial effects of the combination are not lost.

In the present invention, the dosage of the fusion protein may be, but is not limited to, about 0.0001 µg to 500 mg per kg of patient's body weight.

### Advantageous Effects of Invention

The fusion protein provided in the present invention interacts with a ligand for the Lrig-1 protein, which is present in activated T cells, to give a co-inhibitory signal to the activated T cells, so that death or suppressed activity of the activated T cells is induced, which makes it possible to effectively prevent, ameliorate, or treat immune-related diseases.

### Brief Description of Drawings

FIG. 1 illustrates a structure of the Lrig-1 protein according to an embodiment of the present invention.
FIG. 2 illustrates a structure of the Lrig-1 protein according to an embodiment of the present invention.
FIG. 3 illustrates an expression level of Lrig-1 protein according to an embodiment of the present invention.
FIG. 4 illustrates an expression level of Lrig-1 mRNA according to an embodiment of the present invention.
FIG. 5 illustrates an expression level of Lrig-1 mRNA according to an embodiment of the present invention.
FIG. 6 graphically illustrates results obtained by comparing expression levels of Lrig-1, Lrig-2, and Lrig-3 mRNAs according to an embodiment of the present invention.
FIG. 7 illustrates results obtained by comparing expression levels of Lrig-1 protein in regulatory T cells and non-regulated T cells according to an embodiment of the present invention.
FIG. 8 illustrates results obtained by identifying expression levels of Lrig-1 protein on the surface of various immune cells according to an embodiment of the present invention.
FIG. 9 illustrates subsets of T cells in which a ligand recognized by the fusion protein according to an embodiment of the present invention is present.
FIG. 10 illustrates results obtained by subjecting naive T cells, and activated T cells obtained by activation with an anti-CD3/28 antibody to staining with the fusion protein and PD-L1-IgG at respective concentrations, according to an embodiment of the present invention.
FIG. 11 illustrates results obtained by subjecting activated T cells (Act) and Th1 cells (TH1) to immunoprecipitation, and then to Western blot analysis with an anti-VISTA antibody, according to an embodiment of the present invention.
FIGS. 12A and 12B illustrate results (A) obtained by activating T cells with an anti-CD3/28 antibody, allowing a VISTA antibody to be bound to the cell surface for 30 minutes, subjecting the cell surface to staining with Lrig-1-IgG, and then performing analysis through a flow cytometer, and results (B) obtained by allowing the fusion protein to be bound to the surface of the activated T cells for 30 minutes, subjecting the cell surface to staining with a VISTA antibody, and then performing analysis through a flow cytometer, respectively, according to an embodiment of the present invention.
FIGS. 13A and 13B illustrate results (A) obtained by activating naive T cells with an anti-CD3/28 antibody, subjecting the surface of the T cells to staining with a VISTA antibody every 24 hours, and then measuring changes in expression level of VISTA through a flow cytometer, and results (B) obtained by collecting a culture of the activated T cells every 24 hours, and then measuring, with a VISTA ELISA kit, an expression level of an extracellular domain of VISTA so that an amount of the extracellular domain present in the culture is measured.
FIGS. 14A and 14B graphically illustrate results obtained by collecting blood from normal mice and a mouse model for which rheumatoid arthritis (RA) or inflammatory bowel disease (IBD) has been induced, separating serum therefrom through centrifugation, and then measuring a concentration of an extracellular domain of VISTA in the serum using ELISA that targets the extracellular domain of VISTA, according to an embodiment of the present invention.
FIG. 15 illustrates results obtained by activating naive T cells, and then analyzing, with FACS, an expression level of CD25, which is a surface protein induced on the cell surface, according to an embodiment of the present invention.
FIGS. 16A, 16B, 16C, and 17 illustrate results obtained by identifying a proliferation inhibitory effect of the fusion protein according to the present invention in activated T cells, according to an embodiment of the present invention.

### Detailed Description of Invention

According to an embodiment of the present invention, there is provided a composition for preventing, ameliorating, or treating an immune-related disease, comprising, as an active ingredient, a fusion protein consisting of the amino acid sequence represented by SEQ ID NO: 21; a nucleic acid molecule encoding the same; an expression vector containing the nucleic acid molecule; or a host cell transformed with the expression vector.

According to another embodiment of the present invention, there is provided a composition for preventing, ameliorating, or treating an immune-related disease, comprising, as an active ingredient, a fusion protein encoded by the nucleotide sequence represented by SEQ ID NO: 25; a nucleic acid molecule encoding the same; an expression vector containing the nucleic acid molecule; or a host cell transformed with the expression vector.

Hereinafter, the present invention will be described in more detail by way of examples. These examples are only for describing the present invention in more detail, and it will be apparent to those skilled in the art that according to the gist of the present invention, the scope of the present invention is not limited by these examples.

### Examples

### [Preparation Example 1] T cell subtype cell culture

To identify whether the Lrig-1 protein is expressed only in regulatory T cells (Treg), the subsets of T cells, Th0, Th1, Th2, Th17, and iTreg, were prepared. The iTreg refers to cells whose differentiation has been artificially induced in a medium having the following composition, unlike nTreg which has been naturally isolated.

The subsets of the T cells were induced to differentiate into respective cells by first isolating naive T cells obtained from the spleen of mice, causing RPMI1640 (Invitrogen Gibco, Grand Island, NY) nutrient medium that contains 10% fetal bovine serum (FBS; HyClone, Logan, UT) to further contain the respective ingredients of Table 24 below, and performing 72-hour incubation in an incubator at 37°C, 5% CO₂.

**[Table 24]**

| Differentiated cell | Composition |
|---|---|
| Th0 | anti-CD3, anti-CD28 |
| Th1 | IL-12, anti-IL-4 antibody |
| Th2 | IL-4, anti-FPNβ |
| Th17 | IL-6, TGFβ, anti-IFNβ, anti-IL-4 |
| iTreg | IL-2, TGFβ |

### [Example 1] Structural analysis of Lrig-1

A three-dimensional steric structure of the extracellular domain of the Lrig-1 protein was predicted to produce a fusion protein including the extracellular domain of Lrig-1 protein, a surface protein of regulatory T cells.

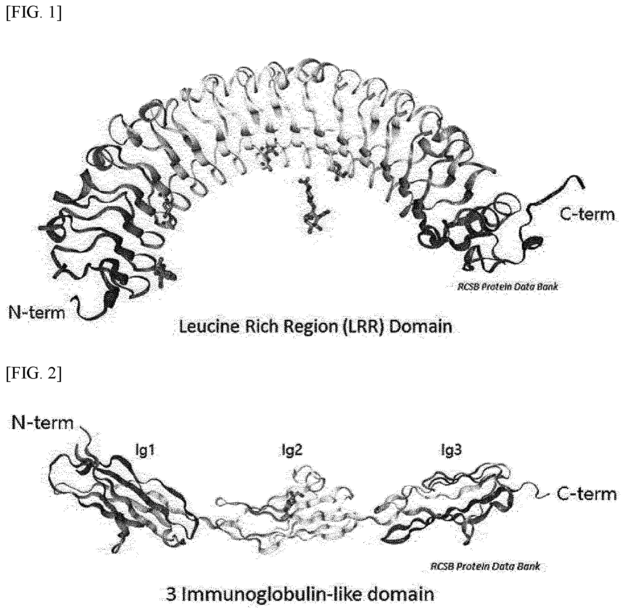

First, to predict base sequences of epitopes, tools of Uniprot (http://www.uniprot.org) and RCSB Protein Data Bank (http://www.rcsb.org/pdb) were used to predict a three-dimensional steric structure of the extracellular domain (ECD) of the Lrig-1 protein so that the structure of ECD is identified. Then, the results are illustrated in FIGS. 1 and 2.

As illustrated in FIG. 1, a total of 15 leucine-rich regions of LRR1 to LRR15 exist in the Lrig-LRR domain (amino acid sequence at positions 41 to 494) in the extracellular domain of the Lrig-1 protein. Each of the LRR domains is composed of 23 to 27 amino acids, with 3 to 5 leucine being present.

In addition, as illustrated in FIG. 2, three immunoglobulin-like domains exist in amino acid sequences at positions 494 to 781 of the Lrig-1 protein in the extracellular domain of the Lrig-1 protein.

### [Example 2] Identification of specific expression of Lrig-1 mRNAin regulatory T cells

Verification was made of whether the Lrig-1 protein can act as a biomarker specific for regulatory T cells.

For the verification, CD4⁺ T cells were isolated using magnet-activated cell sorting

(MACS), through CD4 beads, from the spleen of mice. Subsequently, regulatory T (CD4⁺CD25⁺ T) cells and non-regulatory T (CD4⁺CD25⁻ T) cells were isolated with a fluorescence-activated cell sorter (FACS) using a CD25 antibody. For the respective cells and the cells differentiated in Preparation Example 1, mRNA was extracted using Trizol, and then gDNA was removed from genomic RNA using gDNA extraction kit (Qiagen) according to the protocol provided by the manufacturer. The gDNA-removed mRNA was synthesized into cDNA through the BDsprint cDNA Synthesis Kit (Clonetech).

Real-time polymerase chain reaction (RT PCR) was performed to quantitatively identify an expression level of Lrig-1 mRNA in the cDNA.

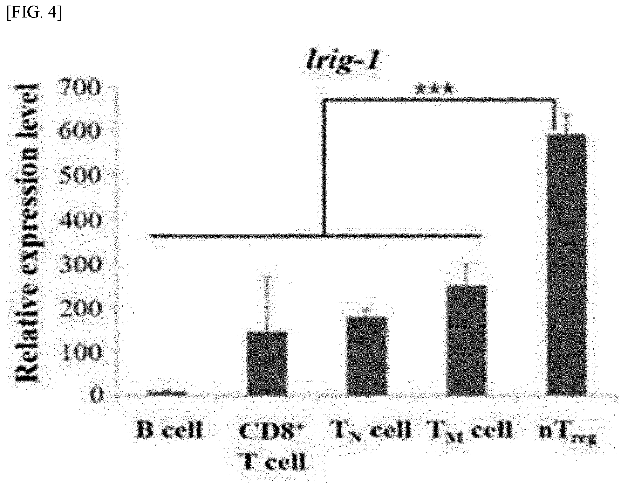

The real-time polymerase chain reaction was performed with primers shown in Table 25 below using SYBR Green (Molecular Probes) according to the protocol provided by the manufacturer under conditions of 40 cycles consisting of 95°C for 3 minutes, 61°C for 15 seconds, 72°C for 30 seconds, a relative gene expression level was calculated using the ΔCT method, and normalized using HPRT. The results are illustrated in FIGS. 4 to 6.

**[Table 25]**

| Primer | Sequence |
|---|---|
| Mouse Lrig-1 | Forward 5'- GAC GGA ATT CAG TGA GGA GAA CCT - 3' |
| | Reverse 5' - CAA CTG GTA GTG GCA GCT TGT AGG - 3' |
| Mouse Lrig-2 | forward 5' - TCA CAA GGA ACA TTG TCT GAA CCA- 3' |
| | reverse 5' - GCC TGA TCT AAC ACA TCC TCC TCA- 3' |
| Mouse Lrig-3 | forward 5' - CAG CAC CTT GAG CTG AAC AGA AAC - 3' |
| | reverse 5' - CCA GCC TTT GGT AAT CTC GGT TAG - 3' |
| Mouse FOXP3 | forward 5' - CTT TCA CCT ATC CCA CCC TTA TCC - 3' |
| | reverse 5' - ATT CAT CTA CGG TCC ACA CTG CTC - 3' |
| ACTG1 | forward 5' - GGC GTC ATG GTG GGC ATG GG - 3' |
| | reverse 5' - ATG GCG TGG GGA AGG GCG TA - 3' |

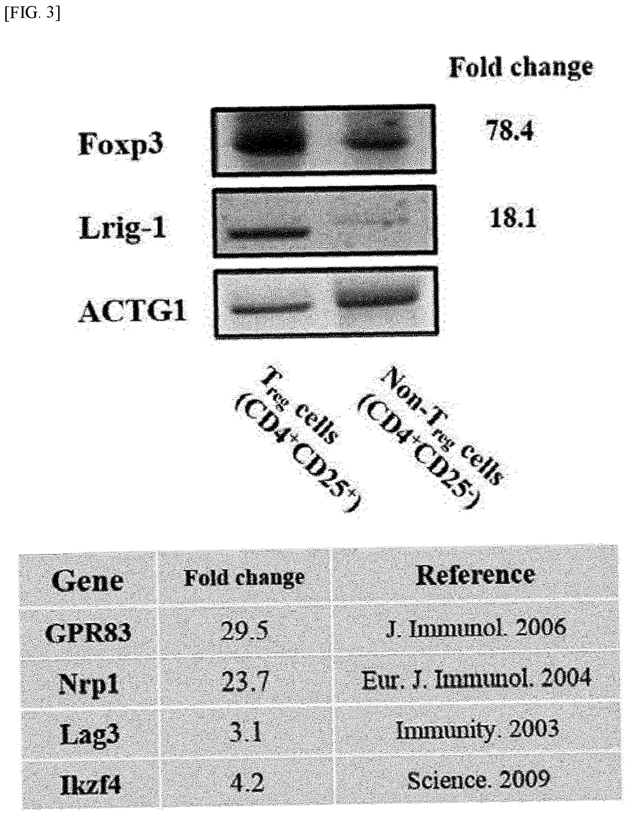

As illustrated in FIG. 3, it can be seen that the expression of Lrig-1 in regulatory T (CD4⁺CD25⁺ T) cells is 18.1 times higher than non-regulatory T (CD4⁺CD25⁻ T) cells. This was about 10 times higher expression level than Lag3 and Ikzf4, which are previously known markers for regulatory T cells.

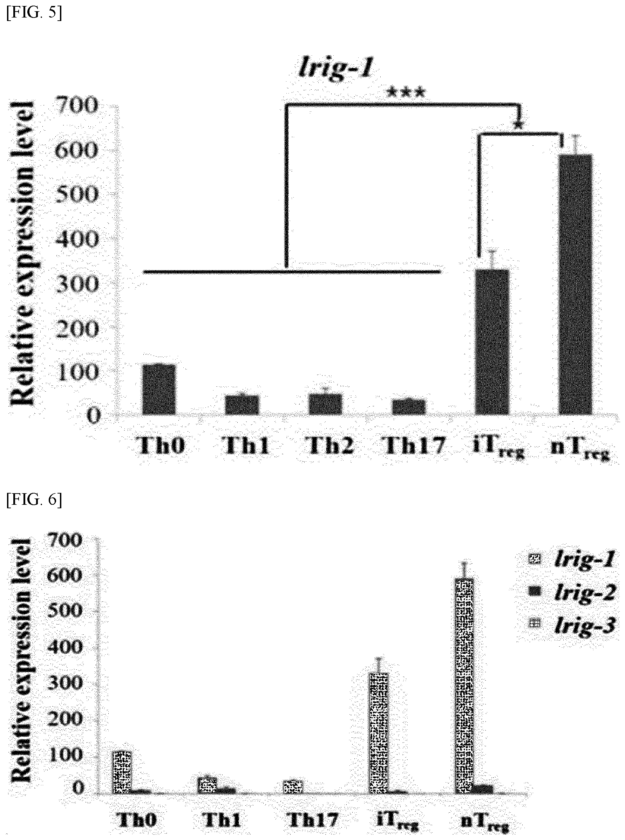

In addition, as illustrated in FIGS. 4 and 5, the expression of Lrig-1 mRNA was remarkably high in regulatory T cells as compared with other types of immune cells, and in particular, was remarkably high in naturally isolated regulatory T cells (nTreg) as compared with induced regulatory T cells (iTreg).

In addition, as illustrated in FIG. 6, the expression of Lrig-1 was the highest among Lrig-1, Lrig-2, and Lrig-3 which correspond to the Lrig family.

From the above results, it can be seen that the Lrig-1 protein according to the present invention is specifically expressed in regulatory T cells, in particular, naturally-occurring regulatory T cells.

### [Example 3] Identification of specific expression of Lrig-1 protein in regulatory T cells

It was identified whether the Lrig-1 protein expressed from Lrig-1 mRNA is specifically expressed only in regulatory T cells.

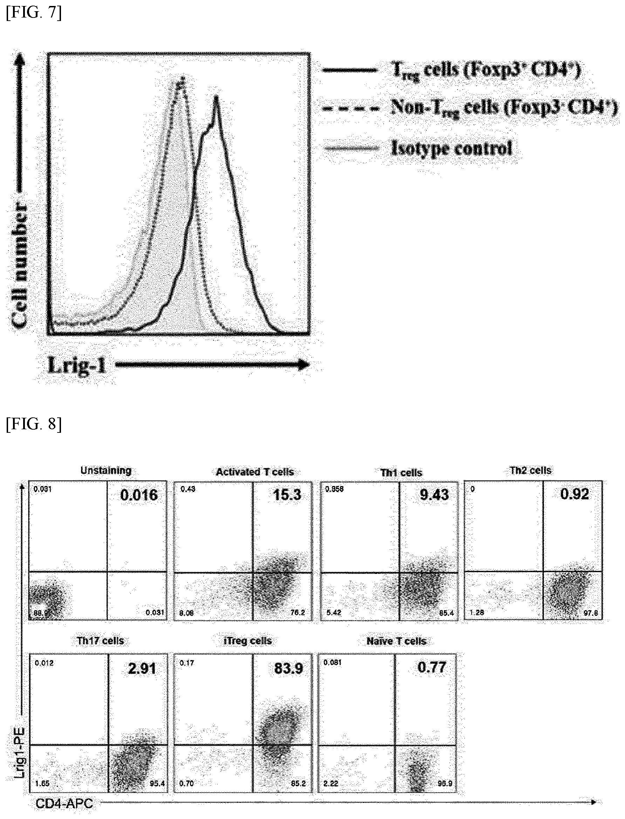

Using FOXP3-RFP-knocked-in mice, the FOXP3-RFP obtained by coupling red fluorescence protein (RFP) to FOXP3 promoter, which is a transcription factor specific for regulatory T cells, CD4⁺ T cells were isolated using magnet-activated cell sorting (MACS), through CD4 beads, from the spleen of the mice. Subsequently, using RFP protein, regulatory T (CD4⁺RFP⁺ T) cells and non-regulatory T (CD4⁺RFP⁻ T) cells were obtained by performing isolation through a fluorescence-activated cell sorter (FACS). The respective cells were stained with the purchased Lrig-1 antibody and a negative control was stained with an isotype-matched control antibody, to measure an expression level of Lrig-1 with the fluorescence-activated cell sorter. The results are illustrated in FIG. 7.

As illustrated in FIG. 7, the non-regulatory T cells indicated by a dotted line showed almost the same expression level of Lrig-1 as the negative control, whereas there were a large number of cells with high expression level of Lrig-1 in the regulatory T cells.

From the above results, it can be seen that the Lrig-1 protein according to the present invention is specifically expressed in regulatory T cells.

### [Example 4] Identification of specific expression of Lrig-1 protein on surface of regulatory T cells

From the viewpoint that in order to be a target of cell therapy, the Lrig-1 protein must be expressed on the surface of regulatory T cells, it was identified whether the Lrig-1 protein is expressed on the surface of the regulatory T cells.

The respective differentiated T cell subsets of Preparation Example 1 were stained with anti-CD4-APC and anti-Lrig-1-PE antibodies, and expression levels of Lrig-1 protein were measured at the respective cell surfaces using a fluorescence-activated cell sorter (FACS). The results are illustrated in FIG. 8.

As illustrated in FIG. 8, Lrig-1 was expressed in an amount of 0.77 to 15.3 in activated T cells, Th1 cells, Th2 cells, Th17 cells, and naive T cells, whereas Lrig-1 was expressed as high as 83.9 in differentiation-induced T cells (iTreg cells).

From the above results, it can be seen that the Lrig-1 protein according to the present invention is not only specifically expressed in regulatory T (Treg) cells, but also is, in particular, specifically expressed at a higher level on the surface of the Treg cells.

### [Preparation Example] Production of fusion protein

### 1. Construction of expression vector

To produce the fusion protein according to the present invention, respective nucleic acid sequences encoding respective fusion proteins as shown in Table 26 were synthesized. Nhel and EcoRI restriction enzyme sequences were added to the 5' and 3' ends of the nucleic acid sequence, respectively, and the following sequences were inserted after the restriction enzyme sequence at the 5' end: the Kozak's sequence (GCCACC), a start codon for protein translation, and the mouse IgG kappa light chain signal peptide (ATGGAAACCGATACTCTGCTGCTGTGGGTGCTGCTGCTGTGGGTGCCAGGCTCTA CCGGG) that allows an expressed protein to secrete outside the cell. Subsequently, a stop codon was inserted after the nucleic acid sequence encoding each of the fusion proteins as shown in Table 26, in which the each fusion protein includes the extracellular domain of human-derived Lrig-1 protein or VISTA protein; optionally a linker; a hinge region; human IgG1-derived heavy chain constant region 2 (CH2) and heavy chain constant region 3 (CH3). The nucleic acid sequence encoding the fusion protein of the present invention was cloned into pcDNA3.1(+) expression vector using the two restriction enzyme sequences, Nhel and EcoRI.

**[Table 26]**

| No. | Amino acid sequence of fusion protein | Nucleic acid sequence of fusion protein |
|---|---|---|
| Preparation Example 1 | SEQ ID NO: 13 | SEQ ID NO: 17 |
| Preparation Example 2 | SEQ ID NO: 14 | SEQ ID NO: 18 |
| Preparation Example 3 | SEQ ID NO: 15 | SEQ ID NO: 19 |
| Preparation Example 4 | SEQ ID NO: 16 | SEQ ID NO: 20 |
| Preparation Example 5 | SEQ ID NO: 21 | SEQ ID NO: 25 |
| Preparation Example 6 | SEQ ID NO: 22 | SEQ ID NO: 26 |
| Preparation Example 7 | SEQ ID NO: 23 | SEQ ID NO: 27 |
| Preparation Example 8 | SEQ ID NO: 24 | SEQ ID NO: 28 |
| Preparation Example 9 | SEQ ID NO: 29 | SEQ ID NO: 33 |
| Preparation Example 10 | SEQ ID NO: 30 | SEQ ID NO: 34 |
| Preparation Example 11 | SEQ ID NO: 31 | SEQ ID NO: 35 |
| Preparation Example 12 | SEQ ID NO: 32 | SEQ ID NO: 36 |
| Preparation Example 13 | SEQ ID NO: 37 | SEQ ID NO: 41 |
| Preparation Example 14 | SEQ ID NO: 38 | SEQ ID NO: 42 |
| Preparation Example 15 | SEQ ID NO: 39 | SEQ ID NO: 43 |
| Preparation Example 16 | SEQ ID NO: 40 | SEQ ID NO: 44 |
| Preparation Example 17 | SEQ ID NO: 45 | SEQ ID NO: 49 |
| Preparation Example 18 | SEQ ID NO: 46 | SEQ ID NO: 50 |
| Preparation Example 19 | SEQ ID NO: 47 | SEQ ID NO: 51 |
| Preparation Example 20 | SEQ ID NO: 48 | SEQ ID NO: 52 |
| Preparation Example 21 | SEQ ID NO: 53 | SEQ ID NO: 57 |
| Preparation Example 22 | SEQ ID NO: 54 | SEQ ID NO: 58 |
| Preparation Example 23 | SEQ ID NO: 55 | SEQ ID NO: 59 |
| Preparation Example 24 | SEQ ID NO: 56 | SEQ ID NO: 60 |
| Preparation Example 25 | SEQ ID NO: 61 | SEQ ID NO: 65 |
| Preparation Example 26 | SEQ ID NO: 62 | SEQ ID NO: 66 |
| Preparation Example 27 | SEQ ID NO: 63 | SEQ ID NO: 67 |
| Preparation Example 28 | SEQ ID NO: 64 | SEQ ID NO: 68 |
| Preparation Example 29 | SEQ ID NO: 69 | SEQ ID NO: 73 |
| Preparation | SEQ ID NO: 70 | SEQ ID NO: 74 |
| Example 30 | | |
| Preparation Example 31 | SEQ ID NO: 71 | SEQ ID NO: 75 |
| Preparation Example 32 | SEQ ID NO: 72 | SEQ ID NO: 76 |
| Preparation Example 33 | SEQ ID NO: 77 | SEQ ID NO: 81 |
| Preparation Example 34 | SEQ ID NO: 78 | SEQ ID NO: 82 |
| Preparation Example 35 | SEQ ID NO: 79 | SEQ ID NO: 83 |
| Preparation Example 36 | SEQ ID NO: 80 | SEQ ID NO: 84 |

### 2. Purification of fusion protein

293F cells were transformed with the expression vector as constructed in item no. 1. above using polyethyleneimine, and then cultured for 6 days under conditions of 37°C and 8% CO₂. The culture supernatant was filtered, passed through Protein A resin, and washed with 1× PBS. Elution was performed with a 0.1 M glycine solution at pH 3.5. Then, to the obtained solution was added a 1 M TRIS solution at pH 9.0 to neutralize the pH. Subsequently, the solution was dialyzed against a PBS solution, and then concentrated and used.

### [Example 5] Identification of distribution of Lrig-1 ligand recognized by fusion protein according to present invention

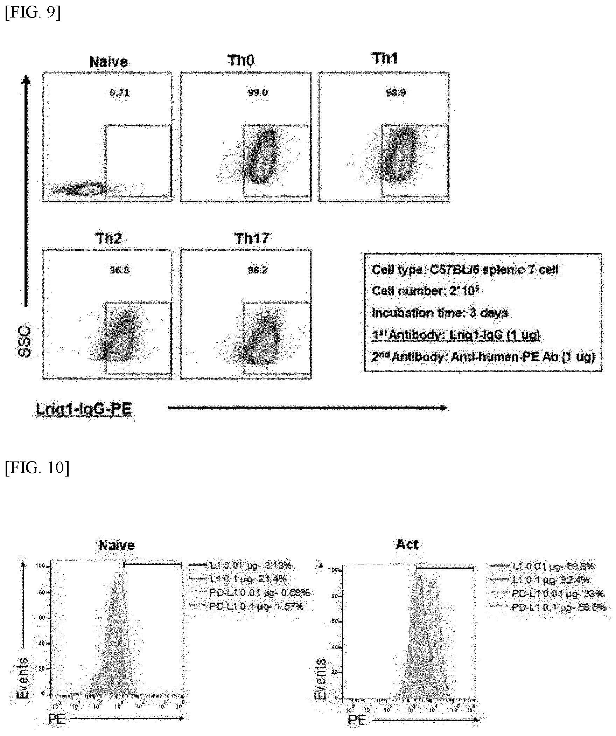

To discover immune cells in which a ligand for Lrig-1 protein is present, naive T cells, thought to be targets of regulatory T cells, were induced to differentiate into activated T cells, Th1 cells, Th2 cells, or Th17 cells, and then these differentiated cells were stained with the fusion protein (primary antibody) of Preparation Example 5 and anti-human-PE antibody (secondary antibody). Levels of the fusion protein of Preparation Example 5 bound to the respective cell surfaces was measured using a fluorescence-activated cell sorter (FACS). The results are illustrated in FIG. 9.

As illustrated in FIG. 9, the fusion protein of Preparation Example 5 hardly binds to the antigen on the surface of naive T cells, and thus weak staining was observed (0.71%); and the fusion protein of Preparation Example 5 binds to the antigen on the surface of activated T cells, Th1 cells, Th2 cells, and Th17 cells at a very high level, and thus staining of 96% or higher was observed.

From these results, it was found that the ligand for the Lrig-1 protein is a surface protein induced by stimulation with a T cell receptor.

### [Example 6] Identification of expression level of Lrig-1 protein in activated T cells

For comparison, the surface of naive T cells and activated T cells was stained in a concentration-dependent manner with a PD-L1-IgG fusion protein (product name: rmB7-H1/Fc Chimera, manufactured by R&D Systems, Cat No.: 1019-B7) (hereinafter referred to as 'PD-L1-IgG') that can recognize PD-1, which is previously known to be highly expressed in activated T cells and subsets of T cells, or the fusion protein of Preparation Example 5; and levels of the fusion protein of Preparation Example 5 bound to the respective cell surfaces were measured using a cell sorter. The results are illustrated in FIG. 10.

As illustrated in FIG. 10, it was identified that in a case of being stained with the fusion protein of Preparation Example 5, a remarkably high expression level of the fusion protein was observed, in a concentration-dependent manner, on the surface of activated T cells, as compared with the proportion observed in a case of being stained with PD-L1-IgG.

From these results, it was found that the ligand for the Lrig-1 protein was expressed at a high expression level on the surface of activated T cells or subsets of T cells, as compared with naive T cells.

### [Example 7] Identification of ligand for Lrig-1 protein in activated T cells

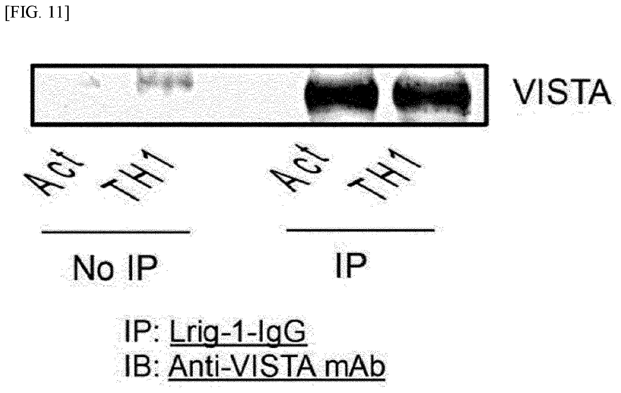

In Example 6, the fact that the ligand for Lrig-1 protein is highly expressed on the surface of activated T cells was identified. Based on this fact, to identify which molecule the ligand expressed on the activated T cells is, cell lysates of activated T cells and Th1 cells were subjected to immunoprecipitation with the fusion protein of Preparation Example 5. Identification was made using various antibodies among candidate ligands for Lrig-1 protein. As a result, Western blot was performed through a monoclonal antibody of V-domain Ig suppressor of T cell activation (VISTA), which is an immune checkpoint. The results are illustrated in FIG. 11.

As illustrated in FIG. 11, it was found that the molecule bound to the fusion protein of Preparation Example 5 was VISTA. In addition, it was found that VISTA was bound to the fusion protein of Preparation Example 5 at a very high level in both activated T cells (Act) and Th1 cells (TH1). From these results, it can be seen that the ligand for the Lrig-1 protein is VISTA.

### [Example 8] Identification of interaction between VISTA in activated T cells and Lrig-1 protein

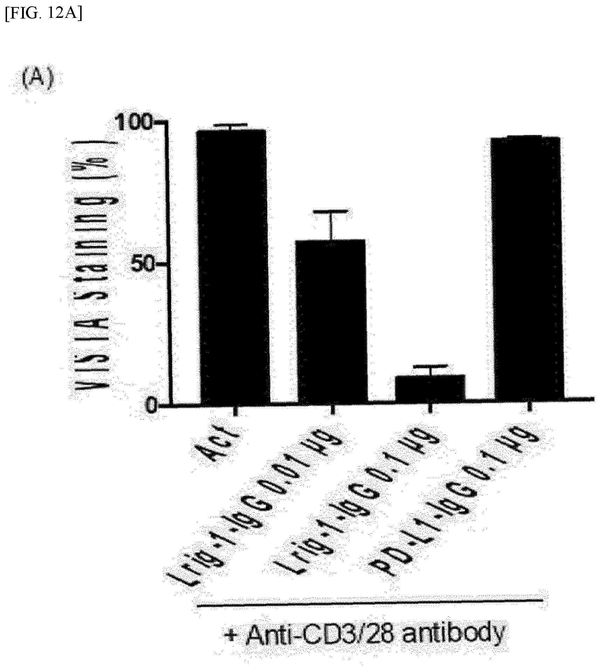

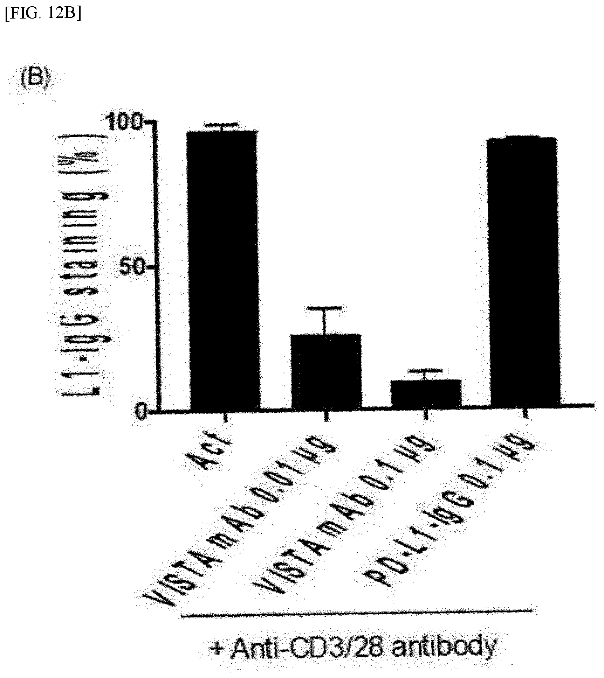

To identify whether VISTA existing on the surface of activated T cells directly interacts with Lrig-1 protein, VISTA mAb was bound to the surface of activated T cells in a concentration-dependent manner, and then the activated T cells were subjected to staining with the fusion protein of Preparation Example 5. Then, a degree of staining was checked using a fluorescence-activated cell sorter (FACS). The results are illustrated in FIGS. 12A and 12B.

As illustrated in FIG. 12A, it was found that in a case where the cell surface was stained only with the fusion protein of Preparation Example 5, the fusion protein exhibited a binding rate of about 95%, whereas in a case where the VISTA mAb was bound in a concentration-dependent manner before staining with the fusion protein of Preparation Example 5, the fusion protein exhibited a remarkably decreased binding rate due to competition.

On the contrary, as illustrated in FIG. 12B, it was found that even in a case where the fusion protein of Preparation Example 5 was first bound to the surface of activated T cells and then the VISTA mAb was bound thereto, a rate at which VISTA was stained decreased.

From these results, it was found that VISTA is expressed on the surface of the activated T cells, and the Lrig-1 protein recognizes and interacts with VISTA.

### [Example 9] Identification of expression pattern of VISTA on surface of T cells

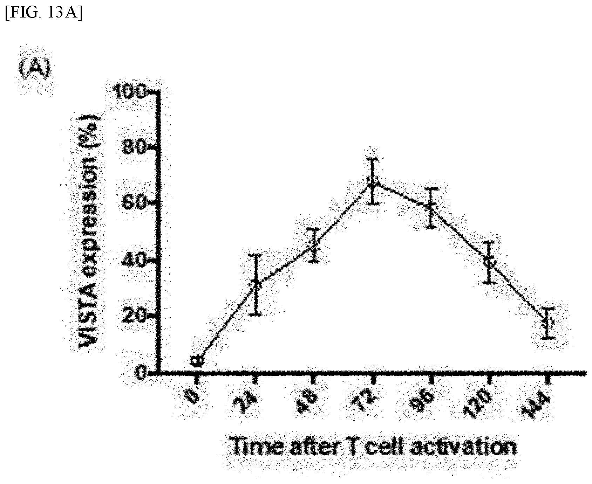

To identify an expression pattern of VISTA on the surface of T cells, mouse naive T cells were activated with an anti-CD3/28 antibody, and then expression levels of VISTA were checked at respective time points. The results are illustrated in FIG. 13A.

As illustrated in FIG. 13A, it was found that the expression level of VISTA gradually increased until 72 hours of activation; however, after 96 hours had elapsed, the expression level thereof on the surface gradually decreased.

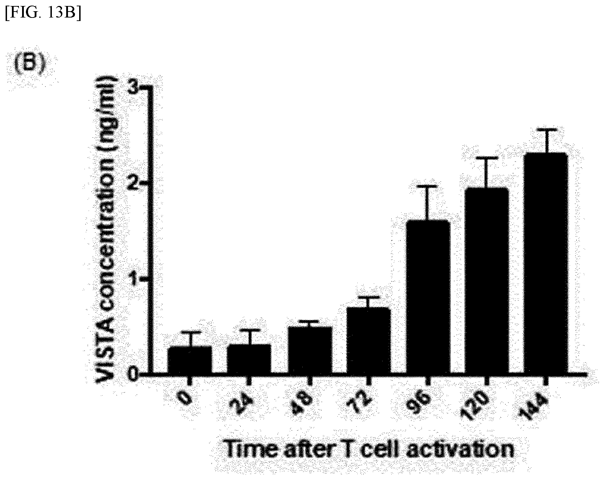

To identify the cause of the above-mentioned phenomenon, expression levels of an extracellular domain of VISTA present in a cell culture medium of activated T cells were measured at respective time points through ELISA that recognizes the extracellular domain of VISTA. The results are illustrated in FIG. 13B.

As illustrated in FIG. 13B, it was found that the extracellular domain of VISTA was hardly detected in the culture medium until 72 hours had elapsed after activation; however, the expression level of the extracellular domain of VISTA rapidly increased in the culture medium after 96 hours had elapsed (FIG. 13B). That is, from the viewpoint that the expression level of the extracellular domain of VISTA shows a pattern directly opposite to the expression level of VISTA present on the surface of activated T cells, it was found that the extracellular domain of VISTA in the culture medium is secreted by cleavage of VISTA present on the surface of the activated T cells.

### [Example 10] Identification of correlation between secretion of extracellular domain of VISTA and onset of autoimmune disease

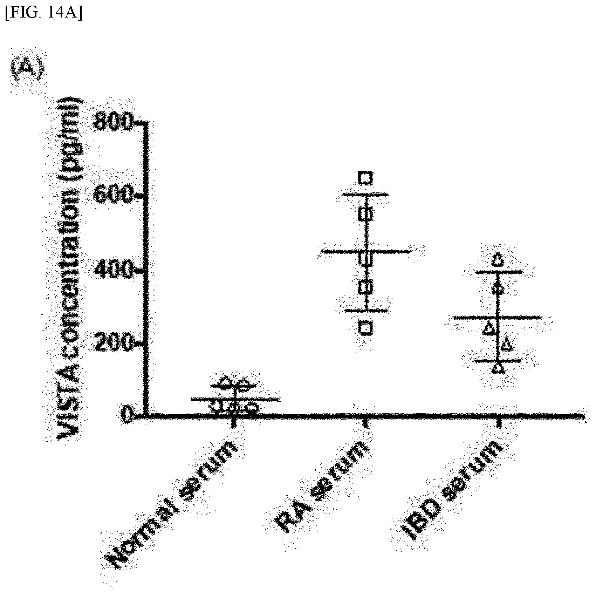

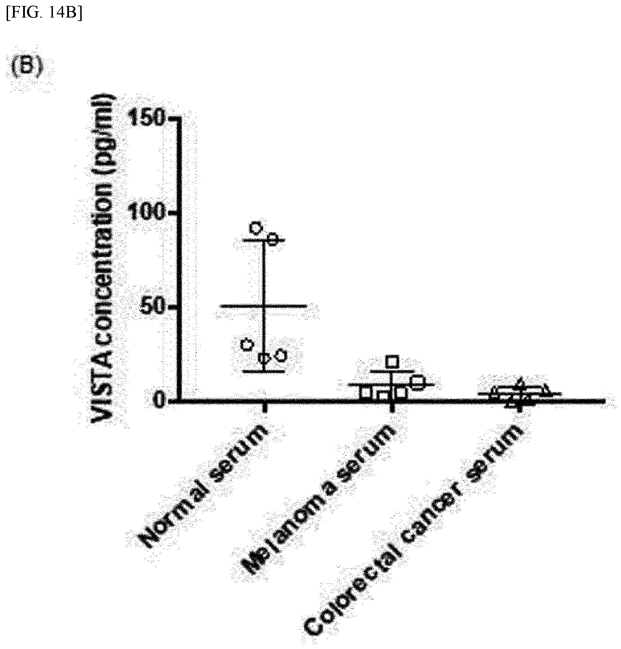

To investigate correlation between secretion of an extracellular domain of VISTA and onset of an autoimmune disease, serum was prepared from a mouse animal model, in which an autoimmune disease of rheumatoid arthritis (RA) or inflammatory bowel disease (IBD) was induced, and then an amount of an extracellular domain of VISTA present in the serum was analyzed by ELISA using a VISTA-specific antibody. The results are illustrated in FIG. 14.

As illustrated in FIG. 14, it was found that the extracellular domain of VISTA was detected in a larger amount in the serum of the mice, in which the autoimmune disease of RA or IBD was induced, than in the serum of normal mice.

### [Example 11] Identification of correlation between secretion of extracellular domain of VISTA and onset of autoimmune disease

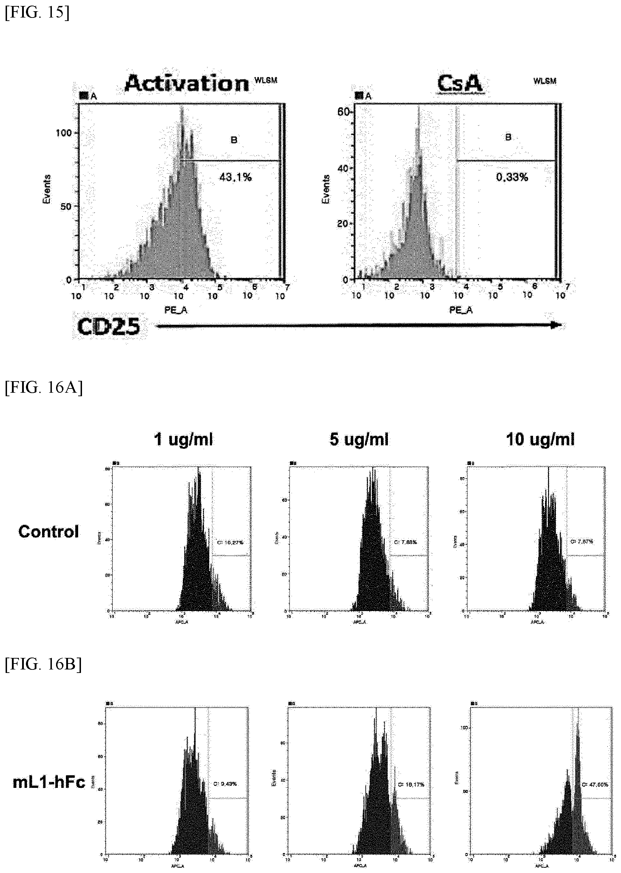

CD4+ CD62L+ T cells, which are naive T cells, were isolated from the spleen of 8-week-old C57BL/6 mice, and then the naive T cells were activated using an anti-CD3/anti-CD28 mAb immobilized on a plate. Thereafter, a degree of activation of the T cells was determined by analyzing, with FACS, an expression level of CD25 surface protein, which is expressed by induction on the surface of the T cells. The results are illustrated in FIG. 15. As illustrated in FIG. 15, for a control with suppressed activation of T cells, in a case where T cells were treated with cyclosporine (CsA), which is an immunosuppressant used in clinical practice, it can be seen that activation of the T cells was inhibited, resulting in decreased expression of CD25 from 43.1% to 0.33%.

From these results, it can be seen that in a case where VISTA was stimulated with Lrig1-IgG, which is a ligand for VISTA, a co-inhibitory signal was induced in activated T cells, thereby remarkably inhibiting activation of T cells, and the fusion protein of Preparation Example 5 exhibited a much better degree of inhibition of T cell activation than PD-L1-IgG that stimulates PD-1.

Furthermore, it can be seen that in a case where the fusion protein of Preparation Example 5 is administered in combination with the extracellular domain of VISTA or a fragment thereof; or a fusion protein that includes the extracellular domain of VISTA or a fragment thereof and immunoglobulin, a co-inhibition signal can be more efficiently induced in T cells, thereby more effectively inducing activation of the T cells.

### [Example 12] Therapeutic effect of fusion protein according to present invention on immune-related diseases

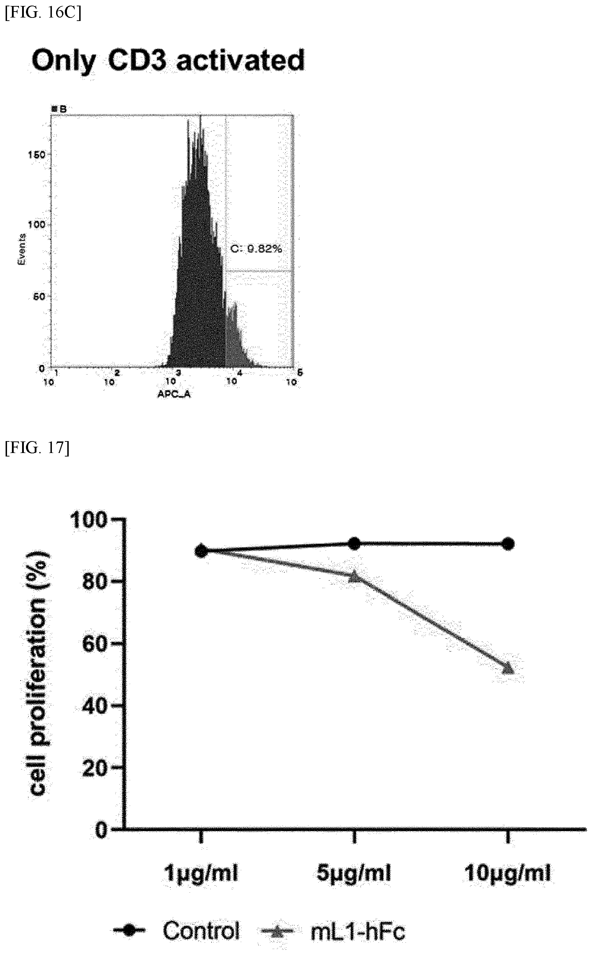

CD4+ CD62L+ T cells, which are naive T cells, were isolated from the spleen of 8-week-old C57BL/6 mice, and then the naive T cells were activated using an anti-CD3/anti-CD28 mAb immobilized on a plate. Thereafter, the activated T cells were dispensed into a 96-well plate. Then, the 96-well plate was treated with 1 µg/ml of anti-CD3 mAb and the fusion protein of Preparation Example 5 that corresponds to a concentration of 1 µg/ml, 5 µg/ml, or 10 µg/ml, and then subjected to staining with CSFE. Then, a degree of proliferation of the cells was analyzed under observation with FACS. The results are illustrated in FIGS. 16 and 17. Here, for a control, an anti-FLAG antibody was used in place of the fusion protein of Preparation Example 5.

As illustrated in FIGS. 17 and 18, in a case of being treated with the control and CD3 alone, the degree of inhibition of proliferation of the activated T cells corresponded to a maximum of 7.87% or 9.82%, whereas in a case of being treated with Lrig-1-IgG (mL1-hFc), which is the fusion protein of Preparation Example 5, proliferation of the activated T cells was inhibited by 18.17% at a concentration of 5 µg/ml and by 47.60% at a concentration of 10 µg/ml (FIGS. 17 and 18).

From these results, it can be seen that in a case where activated T cells are treated with the fusion protein according to the present invention, a co-inhibitory signal can be induced in the activated T cells, thereby remarkably inhibiting activation of the T cells. Thus, the fusion protein according to the present invention can be applied very effectively to prevention, amelioration, or treatment of immune-related diseases caused by activated T cells.

Although the present invention has been described in detail above, the scope of the present invention is not limited thereto. It will be obvious to those skilled in the art that various modifications and changes can be made without departing from the technical spirit of the present invention described in the claims.

### Industrial Applicability

The fusion protein provided in the present invention interacts with a ligand for Lrig-1 protein, which is present in activated T cells, to give a co-inhibitory signal to the activated T cells, so that death or suppressed activity of the activated T cells is induced, which allows the fusion protein to be very effectively used in the field of prevention, amelioration, or treatment of immune-related diseases.

## Claims

1. A pharmaceutical composition for preventing or treating an immune-related disease, comprising as an active ingredient:
a fusion protein that includes an extracellular domain of leucine-rich and immunoglobulin-like domains-1 (Lrig-1) protein and an immunoglobulin Fc region;
a nucleic acid molecule encoding the fusion protein;
an expression vector containing the nucleic acid molecule; or
a host cell transformed with the expression vector.

2. The pharmaceutical composition according to claim 1,
wherein the extracellular domain of the Lrig-1 protein consists of the amino acid sequence represented by SEQ ID NO: 1 or 3.

3. The pharmaceutical composition according to claim 1,
wherein the immunoglobulin Fc region includes 1 to 4 domains selected from the group consisting of CH1, CH2, CH3, and CH4 domains.

4. The pharmaceutical composition according to claim 1,
wherein the immunoglobulin Fc region is an IgG-, IgA-, IgD-, IgE-, or IgM-derived Fc region, heavy chain constant region 2 (CH2), heavy chain constant region 3 (CH3), hinge, a fragment thereof, or a combination thereof, or a hybrid Fc including the combination.

5. The pharmaceutical composition according to claim 1,
wherein the immunoglobulin Fc region includes IgG-, IgA-, IgD-, IgE-, or IgM-derived CH2 and CH3 domains.

6. The pharmaceutical composition according to claim 1,
wherein the immunoglobulin Fc region includes IgG1-, IgG2-, IgG3-, or IgG4-derived CH2 and CH3 domains.

7. The fusion protein according to claim 1,
wherein the immunoglobulin Fc region includes a hinge region.

8. The pharmaceutical composition according to claim 7,
wherein the immunoglobulin Fc region includes an IgG-, IgA-, IgM-, IgD-, IgE-, or Abatacept-derived hinge region.

9. The pharmaceutical composition according to claim 7,
wherein the hinge region includes an IgG1-, IgG2-, IgG3-, IgG4-, IgD-, or Abatacept-derived hinge region.

10. The pharmaceutical composition according to claim 1,
wherein the immunoglobulin Fc region includes CH2 and CH3 which are represented by SEQ ID NO: 5 or 6.

11. The pharmaceutical composition according to claim 1,
wherein the immunoglobulin Fc region includes a hinge region represented by any one of SEQ ID NOs: 7 to 10.

12. The pharmaceutical composition according to claim 1,
wherein the extracellular domain of the Lrig-1 protein is directly linked to the N-terminus or C-terminus of the immunoglobulin Fc region.

13. The pharmaceutical composition according to claim 1,
wherein the extracellular domain of the Lrig-1 protein is linked, via a linker, to the N-terminus or C-terminus of the immunoglobulin Fc region.

14. The pharmaceutical composition according to claim 13,
wherein the linker is at least one of a peptide linker represented by SEQ ID NO: 11 and a peptide linker represented by SEQ ID NO: 12.

15. The pharmaceutical composition according to claim 13,
wherein the linker is at least one selected from the group consisting of peptide linkers, each of which consists of an amino acid sequence represented by any one of Formulas 1 to 4:
[Formula 1] (G)ₙ
[Formula 2] (GGGGS)ₘ
[Formula 3] (EAAAK)ₚ
[Formula 4] (XP)_{q}
where n, m, and p are each independently an integer of 1 or higher;
q is an integer of 5 to 40; and
X is any one amino acid selected from the group consisting of A (Ala), C (Cys), D (Asp), E (Glu), F (Phe), G (Gly), H (His), I (Ile), K (Lys), L (Leu), M (Met), N (Asn), O (Pyl), P (Pro), Q (Gln), R (Arg), S (Ser), T (Thr), U (Sec), V (Val), W (Trp), and Y (Tyr).

16. The pharmaceutical composition according to claim 13,
wherein the linker is at least one selected from the group consisting of peptide linkers represented by SEQ ID NOs: 96 to 100.

17. The pharmaceutical composition according to claim 1, further comprising:
V-domain Ig suppressor of T cell activation (VISTA) or a fragment thereof; or a fusion protein that includes VISTA or a fragment thereof and an immunoglobulin Fc region.

18. The pharmaceutical composition according to claim 1,
wherein the immune-related disease is at least any one selected from the group consisting of autoimmune diseases; graft versus host disease; organ transplant rejection; asthma; atopy; or acute or chronic inflammatory diseases.

19. The pharmaceutical composition according to claim 18,
wherein the autoimmune disease is at least one selected from the group consisting of rheumatoid arthritis, type 1 diabetes, systemic scleroderma, systemic lupus erythematosus, systemic lupus erythematosus, atopic dermatitis, psoriasis, alopecia areata, asthma, Crohn's disease, Behcet's disease, Sjogren's syndrome, Guillain-Barre syndrome, chronic thyroiditis, multiple sclerosis, multiple myositis, ankylosing spondylitis, fibromyalitis, and polyarteritis nodosa.

20. The pharmaceutical composition according to claim 18,
wherein the autoimmune disease is a neurodegenerative disease or neuroinflammatory disease.

21. The pharmaceutical composition according to claim 20,
wherein the neurodegenerative disease or neuroinflammatory disease is at least one selected from the group consisting of stroke, dementia, Alzheimer's disease, Parkinson's disease, Huntington's disease, Niemann-Pick disease, prion disease, Creutzfeldt-Jakob disease, frontal temporal dementia, Lewis dementia, amyotrophic lateral sclerosis, paraneoplastic syndrome, corticobasal degeneration, multisystematic atrophy, progressive nuclear paralysis, autoimmune disease in nervous system, spinocerebellar ataxia, inflammatory and neuropathic pain, cerebrovascular disease, spinal cord injury, and tauopathy.

22. A method for preventing, ameliorating, or treating an immune-related disease, comprising:
a step of administering, to an individual, a fusion protein that includes an extracellular domain of leucine-rich and immunoglobulin-like domains-1 (Lrig-1) protein and an immunoglobulin Fc region; a nucleic acid molecule encoding the fusion protein; an expression vector containing the nucleic acid molecule; or a host cell transformed with the expression vector; or a composition including any one of the foregoing.


<110>    Good T Cells, Inc.

<120>    A composition for preventing or treating immune related diseases

<130>    PDPB187225k01

<150>    KR 2018-0085908
<151>    2018-07-24

<160>    100

<170>    KoPatentIn 3.0

<210>    1
<211>    762
<212>    PRT
<213>    Homo sapiens

<400>    1
   Ala Gly Pro Arg Ala Pro Cys Ala Ala Ala Cys Thr Cys Ala Gly Asp
     1               5                  10                  15
   Ser Leu Asp Cys Gly Gly Arg Gly Leu Ala Ala Leu Pro Gly Asp Leu
                20                  25                  30
   Pro Ser Trp Thr Arg Ser Leu Asn Leu Ser Tyr Asn Lys Leu Ser Glu
            35                  40                  45
   Ile Asp Pro Ala Gly Phe Glu Asp Leu Pro Asn Leu Gln Glu Val Tyr
        50                  55                  60
   Leu Asn Asn Asn Glu Leu Thr Ala Val Pro Ser Leu Gly Ala Ala Ser
    65                  70                  75                  80
   Ser His Val Val Ser Leu Phe Leu Gln His Asn Lys Ile Arg Ser Val
                    85                  90                  95
   Glu Gly Ser Gln Leu Lys Ala Tyr Leu Ser Leu Glu Val Leu Asp Leu
               100                 105                 110
   Ser Leu Asn Asn Ile Thr Glu Val Arg Asn Thr Cys Phe Pro His Gly
           115                 120                 125
   Pro Pro Ile Lys Glu Leu Asn Leu Ala Gly Asn Arg Ile Gly Thr Leu
       130                 135                 140
   Glu Leu Gly Ala Phe Asp Gly Leu Ser Arg Ser Leu Leu Thr Leu Arg
   145                 150                 155                 160
   Leu Ser Lys Asn Arg Ile Thr Gln Leu Pro Val Arg Ala Phe Lys Leu
                   165                 170                 175
   Pro Arg Leu Thr Gln Leu Asp Leu Asn Arg Asn Arg Ile Arg Leu Ile
               180                 185                 190
   Glu Gly Leu Thr Phe Gln Gly Leu Asn Ser Leu Glu Val Leu Lys Leu
           195                 200                 205
   Gln Arg Asn Asn Ile Ser Lys Leu Thr Asp Gly Ala Phe Trp Gly Leu
       210                 215                 220
   Ser Lys Met His Val Leu His Leu Glu Tyr Asn Ser Leu Val Glu Val
   225                 230                 235                 240
   Asn Ser Gly Ser Leu Tyr Gly Leu Thr Ala Leu His Gln Leu His Leu
                   245                 250                 255
   Ser Asn Asn Ser Ile Ala Arg Ile His Arg Lys Gly Trp Ser Phe Cys
               260                 265                 270
   Gln Lys Leu His Glu Leu Val Leu Ser Phe Asn Asn Leu Thr Arg Leu
           275                 280                 285
   Asp Glu Glu Ser Leu Ala Glu Leu Ser Ser Leu Ser Val Leu Arg Leu
       290                 295                 300
   Ser His Asn Ser Ile Ser His Ile Ala Glu Gly Ala Phe Lys Gly Leu
   305                 310                 315                 320
   Arg Ser Leu Arg Val Leu Asp Leu Asp His Asn Glu Ile Ser Gly Thr
                   325                 330                 335
   Ile Glu Asp Thr Ser Gly Ala Phe Ser Gly Leu Asp Ser Leu Ser Lys
               340                 345                 350
   Leu Thr Leu Phe Gly Asn Lys Ile Lys Ser Val Ala Lys Arg Ala Phe
           355                 360                 365
   Ser Gly Leu Glu Gly Leu Glu His Leu Asn Leu Gly Gly Asn Ala Ile
       370                 375                 380
   Arg Ser Val Gln Phe Asp Ala Phe Val Lys Met Lys Asn Leu Lys Glu
   385                 390                 395                 400
   Leu His Ile Ser Ser Asp Ser Phe Leu Cys Asp Cys Gln Leu Lys Trp
                   405                 410                 415
   Leu Pro Pro Trp Leu Ile Gly Arg Met Leu Gln Ala Phe Val Thr Ala
               420                 425                 430
   Thr Cys Ala His Pro Glu Ser Leu Lys Gly Gln Ser Ile Phe Ser Val
           435                 440                 445
   Pro Pro Glu Ser Phe Val Cys Asp Asp Phe Leu Lys Pro Gln Ile Ile
       450                 455                 460
   Thr Gln Pro Glu Thr Thr Met Ala Met Val Gly Lys Asp Ile Arg Phe
   465                 470                 475                 480
   Thr Cys Ser Ala Ala Ser Ser Ser Ser Ser Pro Met Thr Phe Ala Trp
                   485                 490                 495
   Lys Lys Asp Asn Glu Val Leu Thr Asn Ala Asp Met Glu Asn Phe Val
               500                 505                 510
   His Val His Ala Gln Asp Gly Glu Val Met Glu Tyr Thr Thr Ile Leu
           515                 520                 525
   His Leu Arg Gln Val Thr Phe Gly His Glu Gly Arg Tyr Gln Cys Val
       530                 535                 540
   Ile Thr Asn His Phe Gly Ser Thr Tyr Ser His Lys Ala Arg Leu Thr
   545                 550                 555                 560
   Val Asn Val Leu Pro Ser Phe Thr Lys Thr Pro His Asp Ile Thr Ile
                   565                 570                 575
   Arg Thr Thr Thr Val Ala Arg Leu Glu Cys Ala Ala Thr Gly His Pro
               580                 585                 590
   Asn Pro Gln Ile Ala Trp Gln Lys Asp Gly Gly Thr Asp Phe Pro Ala
           595                 600                 605
   Ala Arg Glu Arg Arg Met His Val Met Pro Asp Asp Asp Val Phe Phe
       610                 615                 620
   Ile Thr Asp Val Lys Ile Asp Asp Ala Gly Val Tyr Ser Cys Thr Ala
   625                 630                 635                 640
   Gln Asn Ser Ala Gly Ser Ile Ser Ala Asn Ala Thr Leu Thr Val Leu
                   645                 650                 655
   Glu Thr Pro Ser Leu Val Val Pro Leu Glu Asp Arg Val Val Ser Val
               660                 665                 670
   Gly Glu Thr Val Ala Leu Gln Cys Lys Ala Thr Gly Asn Pro Pro Pro
           675                 680                 685
   Arg Ile Thr Trp Phe Lys Gly Asp Arg Pro Leu Ser Leu Thr Glu Arg
       690                 695                 700
   His His Leu Thr Pro Asp Asn Gln Leu Leu Val Val Gln Asn Val Val
   705                 710                 715                 720
   Ala Glu Asp Ala Gly Arg Tyr Thr Cys Glu Met Ser Asn Thr Leu Gly
                   725                 730                 735
   Thr Glu Arg Ala His Ser Gln Leu Ser Val Leu Pro Ala Ala Gly Cys
               740                 745                 750
   Arg Lys Asp Gly Thr Thr Val Gly Ile Phe
           755                 760


<210>    2
<211>    2286
<212>    DNA
<213>    Homo sapiens

<400>    2
gctgggcctc gggctccttg tgctgccgcc tgcacatgtg caggcgattc cctggactgc      60

ggcggcagag gcctggccgc cctgcctggc gatctgccat cctggacccg gagcctgaac     120

ctgagctaca acaagctgag cgagatcgat cccgccggct ttgaggacct gcctaacctg     180

caggaggtgt atctgaacaa taacgagctg accgcggtac catccctggg cgctgcttca     240

tcacatgtcg tctctctctt tctgcagcac aacaagattc gcagcgtgga ggggagccag     300

ctgaaggcct acctttcctt agaagtgtta gatctgagtt tgaacaacat cacggaagtg     360

cggaacacct gctttccaca cggaccgcct ataaaggagc tcaacctggc aggcaatcgg     420

attggcaccc tggagttggg agcatttgat ggtctgtcac ggtcgctgct aactcttcgc     480

ctgagcaaaa acaggatcac ccagcttcct gtaagagcat tcaagctacc caggctgaca     540

caactggacc tcaatcggaa caggattcgg ctgatagagg gcctcacctt ccaggggctc     600

aacagcttgg aggtgctgaa gcttcagcga aacaacatca gcaaactgac agatggggcc 660

ttctggggac tgtccaagat gcatgtgctg cacctggagt acaacagcct ggtagaagtg 720

aacagcggct cgctctacgg cctcacggcc ctgcatcagc tccacctcag caacaattcc 780

atcgctcgca ttcaccgcaa gggctggagc ttctgccaga agctgcatga gttggtcctg 840

tccttcaaca acctgacacg gctggacgag gagagcctgg ccgagctgag cagcctgagt 900

gtcctgcgtc tcagccacaa ttccatcagc cacattgcgg agggtgcctt caagggactc 960

aggagcctgc gagtcttgga tctggaccat aacgagattt cgggcacaat agaggacacg 1020

agcggcgcct tctcagggct cgacagcctc agcaagctga ctctgtttgg aaacaagatc 1080

aagtctgtgg ctaagagagc attctcgggg ctggaaggcc tggagcacct gaaccttgga 1140

gggaatgcga tcagatctgt ccagtttgat gcctttgtga agatgaagaa tcttaaagag 1200

ctccatatca gcagcgacag cttcctgtgt gactgccagc tgaagtggct gcccccgtgg 1260

ctaattggca ggatgctgca ggcctttgtg acagccacct gtgcccaccc agaatcactg 1320

aagggtcaga gcattttctc tgtgccacca gagagtttcg tgtgcgatga cttcctgaag 1380

ccacagatca tcacccagcc agaaaccacc atggctatgg tgggcaagga catccggttt 1440

acatgctcag cagccagcag cagcagctcc cccatgacct ttgcctggaa gaaagacaat 1500

gaagtcctga ccaatgcaga catggagaac tttgtccacg tccacgcgca ggacggggaa 1560

gtgatggagt acaccaccat cctgcacctc cgtcaggtca ctttcggcca cgagggccgc 1620

taccaatgtg tcatcaccaa ccactttggc tccacctatt cacataaggc caggctcacc 1680

gtgaatgtgt tgccatcatt caccaaaacg ccccacgaca taaccatccg gaccaccacc 1740

gtggcccgcc tcgaatgtgc tgccacaggt cacccaaacc ctcagattgc ctggcagaag 1800

gatggaggca cggatttccc cgctgcccgt gagcgacgca tgcatgtcat gccggatgac 1860

gacgtgtttt tcatcactga tgtgaaaata gatgacgcag gggtttacag ctgtactgct 1920

cagaactcag ccggttctat ttcagctaat gccaccctga ctgtcctaga gaccccatcc 1980

ttggtggtcc ccttggaaga ccgtgtggta tctgtgggag aaacagtggc cctccaatgc 2040

aaagccacgg ggaaccctcc gccccgcatc acctggttca agggggaccg cccgctgagc 2100

ctcactgagc ggcaccacct gacccctgac aaccagctcc tggtggttca gaacgtggtg 2160

gcagaggatg cgggccgata tacctgtgag atgtccaaca ccctgggcac ggagcgagct 2220

cacagccagc tgagcgtcct gcccgcagca ggctgcagga aggatgggac cacggtaggc 2280

atcttc 2286

<210> 3
<211> 760
<212> PRT

<213>    Mus musculus

<400>    3
Ala Gln Ala Gly Pro Arg Ala Pro Cys Ala Ala Ala Cys Thr Cys Ala
1               5                   10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr
        755                 760

<210>    4
<211>    2280
<212>    DNA
<213>    Mus musculus

<400>    4
gctcaggctg gacctagggc tccttgcgct gccgcctgca cctgtgcagg cgattctctg      60

gactgcagcg gccggggcct ggccacactg cccagggacc tgccttcctg gaccagatct     120

ctgaacctga gctacaatcg gctgtccgag atcgattctg ccgcctttga ggacctgaca     180

aatctgcagg aggtgtatct gaacagcaat gagctgaccg caatcccctc cctgggagca     240

gcctctatcg gcgtggtgag cctgttcctg cagcacaaca agatcctgag cgtggatggc     300

tcccagctga agagctacct gtctctggag gtgctggacc tgagctccaa caatatcacc     360

gagatcagat ctagctgttt tcctaatggc ctgcggatca gagagctgaa cctggcctct     420

aatcggatca gcatcctgga gtccggcgcc ttcgatggcc tgagcagatc cctgctgaca     480

ctgcgcctgt ccaagaaccg gatcacccag ctgcccgtga aggcctttaa gctgcctagg     540

ctgacacagc tggacctgaa ccggaataga atcaggctga tcgagggcct gaccttccag     600

ggcctggata gcctggaggt gctgcgcctg cagcggaaca atatctcccg cctgacagac     660

ggagcatttt ggggcctgtc taagatgcac gtgctgcacc tggagtacaa tagcctggtg     720

gaggtgaact ctggcagcct gtatggcctg accgccctgc accagctgca cctgtccaac     780

aatagcatca gcagaatcca gagggatggc tggtccttct gccagaagct gcacgagctg     840

atcctgtctt ttaacaatct gaccaggctg gacgaggaga gcctggcaga gctgtcctct     900

ctgtccatcc tgcgcctgtc tcacaatgcc atcagccaca tcgccgaggg cgcctttaag     960

ggcctgaaga gcctgagggt gctggatctg gaccacaacg agatctctgg caccatcgag    1020

gatacaagcg gcgccttcac aggcctggac aatctgtcca agctgaccct gtttggcaac    1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

2280

<210> 5
<211> 206
<212> PRT
<213> Homo sapiens

<400> 5
Leu Phe Pro Pro Lys Pro Lys Asp Thr Leu Met Ile Ser Arg Thr Pro
1               5                   10                  15

Glu Val Thr Cys Val Val Val Asp Val Ser His Glu Asp Pro Glu Val
            20                  25                  30

Lys Phe Asn Trp Tyr Val Asp Gly Val Glu Val His Asn Ala Lys Thr
        35                  40                  45

Lys Pro Arg Glu Glu Gln Tyr Asn Ser Thr Tyr Arg Val Val Ser Val
    50                  55                  60

Leu Thr Val Leu His Gln Asp Trp Leu Asn Gly Lys Glu Tyr Lys Cys

65                  70                  75                  80

Lys Val Ser Asn Lys Ala Leu Pro Ala Pro Ile Glu Lys Thr Ile Ser
                85                  90                  95

Lys Ala Lys Gly Gln Pro Arg Glu Pro Gln Val Tyr Thr Leu Pro Pro
            100                 105                 110

Ser Arg Asp Glu Leu Thr Lys Asn Gln Val Ser Leu Thr Cys Leu Val
        115                 120                 125

Lys Gly Phe Tyr Pro Ser Asp Ile Ala Val Glu Trp Glu Ser Asn Gly
    130                 135                 140

Gln Pro Glu Asn Asn Tyr Lys Thr Thr Pro Pro Val Leu Asp Ser Asp
145                 150                 155                 160

Gly Ser Phe Phe Leu Tyr Ser Lys Leu Thr Val Asp Lys Ser Arg Trp
                165                 170                 175

Gln Gln Gly Asn Val Phe Ser Cys Ser Val Met His Glu Ala Leu His
            180                 185                 190

Asn His Tyr Thr Gln Lys Ser Leu Ser Leu Ser Pro Gly Lys
        195                 200                 205

<210>    6
<211>    206
<212>    PRT
<213>    Mus musculus

<400>    6
Ile Phe Pro Pro Lys Ile Lys Asp Val Leu Met Ile Ser Leu Ser Pro
 1               5                  10                  15

Ile Val Thr Cys Val Val Val Asp Val Ser Glu Asp Asp Pro Asp Val
            20                  25                  30

Gln Ile Ser Trp Phe Val Asn Asn Val Glu Val His Thr Ala Gln Thr
        35                  40                  45

Gln Thr His Arg Glu Asp Tyr Asn Ser Thr Leu Arg Val Val Ser Ala
    50                  55                  60

Leu Pro Ile Gln His Gln Asp Trp Met Ser Gly Lys Glu Phe Lys Cys
65                  70                  75                  80

Lys Val Asn Asn Lys Asp Leu Pro Ala Pro Ile Glu Arg Thr Ile Ser
                85                  90                  95

Lys Pro Lys Gly Ser Val Arg Ala Pro Gln Val Tyr Val Leu Pro Pro
            100                 105                 110

Pro Glu Glu Glu Met Thr Lys Lys Gln Val Thr Leu Thr Cys Met Val
        115                 120                 125

Thr Asp Phe Met Pro Glu Asp Ile Tyr Val Glu Trp Thr Asn Asn Gly
    130                 135                 140

Lys Thr Glu Leu Asn Tyr Lys Asn Thr Glu Pro Val Leu Asp Ser Asp
145                 150                 155                 160

Gly Ser Tyr Phe Met Tyr Ser Lys Leu Arg Val Glu Lys Lys Asn Trp
                165                 170                 175

Val Glu Arg Asn Ser Tyr Ser Cys Ser Val Val His Glu Gly Leu His
            180                 185                 190

Asn His His Thr Thr Lys Ser Phe Ser Arg Thr Pro Gly Lys
        195                 200                 205

<210>   7
<211>   26
<212>   PRT
<213>   Homo sapiens

<400>   7
Glu Pro Lys Ser Ser Asp Lys Thr His Thr Ser Pro Pro Cys Pro Ala
  1               5                  10                  15

Pro Glu Leu Leu Gly Gly Pro Ser Val Phe
            20                  25

<210>   8
<211>   27
<212>   PRT
<213>   Mus musculus

<400>   8
Glu Pro Arg Gly Pro Thr Ile Lys Pro Cys Pro Pro Cys Lys Cys Pro
  1               5                  10                  15

Ala Pro Asn Leu Leu Gly Gly Pro Ser Val Phe
            20                  25

<210>   9
<211>   39
<212>   PRT
<213>   Homo sapiens

<400>   9
Arg Asn Thr Gly Arg Gly Gly Glu Glu Lys Lys Lys Glu Lys Glu Lys
  1               5                  10                  15

Glu Glu Gln Glu Glu Arg Glu Thr Lys Thr Pro Glu Cys Pro Ser His
            20                  25                  30

Thr Gln Pro Leu Gly Val Phe
        35

<210>   10
<211>   26
<212>   PRT
<213>   Artificial Sequence

<220>
<223>   Abatacept hinge

<400>    10
Glu Pro Lys Ser Ser Asp Lys Thr His Thr Ser Pro Pro Ser Pro Ala
1               5                   10                  15

Pro Glu Leu Leu Gly Gly Ser Ser Val Phe
            20                  25

<210>    11
<211>    2
<212>    PRT
<213>    Artificial Sequence

<220>
<223>    peptide linker

<400>    11
Gly Ser
11

<210>    12
<211>    3
<212>    PRT
<213>    Artificial Sequence

<220>
<223>    peptide linker

<400>    12
Gly Gly Gly
1

<210>    13
<211>    994
<212>    PRT
<213>    Artificial Sequence

<220>
<223>    fusion protein

<400>    13
Ala Gly Pro Arg Ala Pro Cys Ala Ala Ala Cys Thr Cys Ala Gly Asp
1               5                   10                  15

Ser Leu Asp Cys Gly Gly Arg Gly Leu Ala Ala Leu Pro Gly Asp Leu
            20                  25                  30

Pro Ser Trp Thr Arg Ser Leu Asn Leu Ser Tyr Asn Lys Leu Ser Glu
        35                  40                  45

Ile Asp Pro Ala Gly Phe Glu Asp Leu Pro Asn Leu Gln Glu Val Tyr
    50                  55                  60

Leu Asn Asn Asn Glu Leu Thr Ala Val Pro Ser Leu Gly Ala Ala Ser
65                  70                  75                  80

Ser His Val Val Ser Leu Phe Leu Gln His Asn Lys Ile Arg Ser Val
                85                  90                  95

Glu Gly Ser Gln Leu Lys Ala Tyr Leu Ser Leu Glu Val Leu Asp Leu
        100                 105                 110

Ser Leu Asn Asn Ile Thr Glu Val Arg Asn Thr Cys Phe Pro His Gly
            115                 120                 125

Pro Pro Ile Lys Glu Leu Asn Leu Ala Gly Asn Arg Ile Gly Thr Leu
    130                 135                 140

Glu Leu Gly Ala Phe Asp Gly Leu Ser Arg Ser Leu Leu Thr Leu Arg
145                 150                 155                 160

Leu Ser Lys Asn Arg Ile Thr Gln Leu Pro Val Arg Ala Phe Lys Leu
                165                 170                 175

Pro Arg Leu Thr Gln Leu Asp Leu Asn Arg Asn Arg Ile Arg Leu Ile
        180                 185                 190

Glu Gly Leu Thr Phe Gln Gly Leu Asn Ser Leu Glu Val Leu Lys Leu
            195                 200                 205

Gln Arg Asn Asn Ile Ser Lys Leu Thr Asp Gly Ala Phe Trp Gly Leu
    210                 215                 220

Ser Lys Met His Val Leu His Leu Glu Tyr Asn Ser Leu Val Glu Val
225                 230                 235                 240

Asn Ser Gly Ser Leu Tyr Gly Leu Thr Ala Leu His Gln Leu His Leu
                245                 250                 255

Ser Asn Asn Ser Ile Ala Arg Ile His Arg Lys Gly Trp Ser Phe Cys
        260                 265                 270

Gln Lys Leu His Glu Leu Val Leu Ser Phe Asn Asn Leu Thr Arg Leu
            275                 280                 285

Asp Glu Glu Ser Leu Ala Glu Leu Ser Ser Leu Ser Val Leu Arg Leu
    290                 295                 300

Ser His Asn Ser Ile Ser His Ile Ala Glu Gly Ala Phe Lys Gly Leu
305                 310                 315                 320

Arg Ser Leu Arg Val Leu Asp Leu Asp His Asn Glu Ile Ser Gly Thr
                325                 330                 335

Ile Glu Asp Thr Ser Gly Ala Phe Ser Gly Leu Asp Ser Leu Ser Lys
        340                 345                 350

Leu Thr Leu Phe Gly Asn Lys Ile Lys Ser Val Ala Lys Arg Ala Phe
            355                 360                 365

Ser Gly Leu Glu Gly Leu Glu His Leu Asn Leu Gly Gly Asn Ala Ile
    370                 375                 380

Arg Ser Val Gln Phe Asp Ala Phe Val Lys Met Lys Asn Leu Lys Glu
385                 390                 395                 400

Leu His Ile Ser Ser Asp Ser Phe Leu Cys Asp Cys Gln Leu Lys Trp
                405                 410                 415

Leu Pro Pro Trp Leu Ile Gly Arg Met Leu Gln Ala Phe Val Thr Ala
        420                 425                 430

Thr Cys Ala His Pro Glu Ser Leu Lys Gly Gln Ser Ile Phe Ser Val
        435                 440                 445

Pro Pro Glu Ser Phe Val Cys Asp Asp Phe Leu Lys Pro Gln Ile Ile
    450                 455                 460

Thr Gln Pro Glu Thr Thr Met Ala Met Val Gly Lys Asp Ile Arg Phe
465                 470                 475                 480

Thr Cys Ser Ala Ala Ser Ser Ser Ser Ser Pro Met Thr Phe Ala Trp
                485                 490                 495

Lys Lys Asp Asn Glu Val Leu Thr Asn Ala Asp Met Glu Asn Phe Val
            500                 505                 510

His Val His Ala Gln Asp Gly Glu Val Met Glu Tyr Thr Thr Ile Leu
        515                 520                 525

His Leu Arg Gln Val Thr Phe Gly His Glu Gly Arg Tyr Gln Cys Val
    530                 535                 540

Ile Thr Asn His Phe Gly Ser Thr Tyr Ser His Lys Ala Arg Leu Thr
545                 550                 555                 560

Val Asn Val Leu Pro Ser Phe Thr Lys Thr Pro His Asp Ile Thr Ile
                565                 570                 575

Arg Thr Thr Thr Val Ala Arg Leu Glu Cys Ala Ala Thr Gly His Pro
            580                 585                 590

Asn Pro Gln Ile Ala Trp Gln Lys Asp Gly Gly Thr Asp Phe Pro Ala
        595                 600                 605

Ala Arg Glu Arg Arg Met His Val Met Pro Asp Asp Asp Val Phe Phe
    610                 615                 620

Ile Thr Asp Val Lys Ile Asp Asp Ala Gly Val Tyr Ser Cys Thr Ala
625                 630                 635                 640

Gln Asn Ser Ala Gly Ser Ile Ser Ala Asn Ala Thr Leu Thr Val Leu
                645                 650                 655

Glu Thr Pro Ser Leu Val Val Pro Leu Glu Asp Arg Val Val Ser Val
            660                 665                 670

Gly Glu Thr Val Ala Leu Gln Cys Lys Ala Thr Gly Asn Pro Pro Pro
        675                 680                 685

Arg Ile Thr Trp Phe Lys Gly Asp Arg Pro Leu Ser Leu Thr Glu Arg
    690                 695                 700

His His Leu Thr Pro Asp Asn Gln Leu Leu Val Val Gln Asn Val Val
705                 710                 715                 720

Ala Glu Asp Ala Gly Arg Tyr Thr Cys Glu Met Ser Asn Thr Leu Gly
                725                 730                 735

Thr Glu Arg Ala His Ser Gln Leu Ser Val Leu Pro Ala Ala Gly Cys
            740                 745                 750

Arg Lys Asp Gly Thr Thr Val Gly Ile Phe Glu Pro Lys Ser Ser Asp
        755                 760                 765

Lys Thr His Thr Ser Pro Pro Cys Pro Ala Pro Glu Leu Leu Gly Gly
    770                 775                 780

Pro Ser Val Phe Leu Phe Pro Pro Lys Pro Lys Asp Thr Leu Met Ile
785                 790                 795                 800

Ser Arg Thr Pro Glu Val Thr Cys Val Val Val Asp Val Ser His Glu
                805                 810                 815

Asp Pro Glu Val Lys Phe Asn Trp Tyr Val Asp Gly Val Glu Val His
            820                 825                 830

Asn Ala Lys Thr Lys Pro Arg Glu Glu Gln Tyr Asn Ser Thr Tyr Arg
        835                 840                 845

Val Val Ser Val Leu Thr Val Leu His Gln Asp Trp Leu Asn Gly Lys
    850                 855                 860

Glu Tyr Lys Cys Lys Val Ser Asn Lys Ala Leu Pro Ala Pro Ile Glu
865                 870                 875                 880

Lys Thr Ile Ser Lys Ala Lys Gly Gln Pro Arg Glu Pro Gln Val Tyr
                885                 890                 895

Thr Leu Pro Pro Ser Arg Asp Glu Leu Thr Lys Asn Gln Val Ser Leu
            900                 905                 910

Thr Cys Leu Val Lys Gly Phe Tyr Pro Ser Asp Ile Ala Val Glu Trp
        915                 920                 925

Glu Ser Asn Gly Gln Pro Glu Asn Asn Tyr Lys Thr Thr Pro Pro Val
    930                 935                 940

Leu Asp Ser Asp Gly Ser Phe Phe Leu Tyr Ser Lys Leu Thr Val Asp
945                 950                 955                 960

Lys Ser Arg Trp Gln Gln Gly Asn Val Phe Ser Cys Ser Val Met His
                965                 970                 975

Glu Ala Leu His Asn His Tyr Thr Gln Lys Ser Leu Ser Leu Ser Pro
            980                 985                 990

Gly Lys



<210>    14
<211>    995
<212>    PRT
<213>    Artificial Sequence

<220>
<223>    fusion protein


<400>    14
Ala Gly Pro Arg Ala Pro Cys Ala Ala Ala Cys Thr Cys Ala Gly Asp
  1               5                  10                  15

Ser Leu Asp Cys Gly Gly Arg Gly Leu Ala Ala Leu Pro Gly Asp Leu
            20                  25                  30

Pro Ser Trp Thr Arg Ser Leu Asn Leu Ser Tyr Asn Lys Leu Ser Glu
            35                  40                  45

Ile Asp Pro Ala Gly Phe Glu Asp Leu Pro Asn Leu Gln Glu Val Tyr
    50                  55                  60

Leu Asn Asn Asn Glu Leu Thr Ala Val Pro Ser Leu Gly Ala Ala Ser
65                  70                  75                  80

Ser His Val Val Ser Leu Phe Leu Gln His Asn Lys Ile Arg Ser Val
                85                  90                  95

Glu Gly Ser Gln Leu Lys Ala Tyr Leu Ser Leu Glu Val Leu Asp Leu
            100                 105                 110

Ser Leu Asn Asn Ile Thr Glu Val Arg Asn Thr Cys Phe Pro His Gly
        115                 120                 125

Pro Pro Ile Lys Glu Leu Asn Leu Ala Gly Asn Arg Ile Gly Thr Leu
    130                 135                 140

Glu Leu Gly Ala Phe Asp Gly Leu Ser Arg Ser Leu Leu Thr Leu Arg
145                 150                 155                 160

Leu Ser Lys Asn Arg Ile Thr Gln Leu Pro Val Arg Ala Phe Lys Leu
                165                 170                 175

Pro Arg Leu Thr Gln Leu Asp Leu Asn Arg Asn Arg Ile Arg Leu Ile
            180                 185                 190

Glu Gly Leu Thr Phe Gln Gly Leu Asn Ser Leu Glu Val Leu Lys Leu
        195                 200                 205

Gln Arg Asn Asn Ile Ser Lys Leu Thr Asp Gly Ala Phe Trp Gly Leu
    210                 215                 220

Ser Lys Met His Val Leu His Leu Glu Tyr Asn Ser Leu Val Glu Val
225                 230                 235                 240

Asn Ser Gly Ser Leu Tyr Gly Leu Thr Ala Leu His Gln Leu His Leu
                245                 250                 255

Ser Asn Asn Ser Ile Ala Arg Ile His Arg Lys Gly Trp Ser Phe Cys
            260                 265                 270

Gln Lys Leu His Glu Leu Val Leu Ser Phe Asn Asn Leu Thr Arg Leu
        275                 280                 285

Asp Glu Glu Ser Leu Ala Glu Leu Ser Ser Leu Ser Val Leu Arg Leu
    290                 295                 300

Ser His Asn Ser Ile Ser His Ile Ala Glu Gly Ala Phe Lys Gly Leu
305                 310                 315                 320

Arg Ser Leu Arg Val Leu Asp Leu Asp His Asn Glu Ile Ser Gly Thr
                325                 330                 335

Ile Glu Asp Thr Ser Gly Ala Phe Ser Gly Leu Asp Ser Leu Ser Lys
            340                 345                 350

Leu Thr Leu Phe Gly Asn Lys Ile Lys Ser Val Ala Lys Arg Ala Phe
        355                 360                 365

Ser Gly Leu Glu Gly Leu Glu His Leu Asn Leu Gly Gly Asn Ala Ile
    370                 375                 380

Arg Ser Val Gln Phe Asp Ala Phe Val Lys Met Lys Asn Leu Lys Glu
385                 390                 395                 400

Leu His Ile Ser Ser Asp Ser Phe Leu Cys Asp Cys Gln Leu Lys Trp
                405                 410                 415

Leu Pro Pro Trp Leu Ile Gly Arg Met Leu Gln Ala Phe Val Thr Ala
            420                 425                 430

Thr Cys Ala His Pro Glu Ser Leu Lys Gly Gln Ser Ile Phe Ser Val
        435                 440                 445

Pro Pro Glu Ser Phe Val Cys Asp Asp Phe Leu Lys Pro Gln Ile Ile
    450                 455                 460

Thr Gln Pro Glu Thr Thr Met Ala Met Val Gly Lys Asp Ile Arg Phe
465                 470                 475                 480

Thr Cys Ser Ala Ala Ser Ser Ser Ser Ser Pro Met Thr Phe Ala Trp
                485                 490                 495

Lys Lys Asp Asn Glu Val Leu Thr Asn Ala Asp Met Glu Asn Phe Val
            500                 505                 510

His Val His Ala Gln Asp Gly Glu Val Met Glu Tyr Thr Thr Ile Leu
        515                 520                 525

His Leu Arg Gln Val Thr Phe Gly His Glu Gly Arg Tyr Gln Cys Val
    530                 535                 540

Ile Thr Asn His Phe Gly Ser Thr Tyr Ser His Lys Ala Arg Leu Thr
545                 550                 555                 560

Val Asn Val Leu Pro Ser Phe Thr Lys Thr Pro His Asp Ile Thr Ile
                565                 570                 575

Arg Thr Thr Thr Val Ala Arg Leu Glu Cys Ala Ala Thr Gly His Pro
            580                 585                 590

Asn Pro Gln Ile Ala Trp Gln Lys Asp Gly Gly Thr Asp Phe Pro Ala
        595                 600                 605

Ala Arg Glu Arg Arg Met His Val Met Pro Asp Asp Asp Val Phe Phe
    610                 615                 620

Ile Thr Asp Val Lys Ile Asp Asp Ala Gly Val Tyr Ser Cys Thr Ala
625                 630                 635                 640

Gln Asn Ser Ala Gly Ser Ile Ser Ala Asn Ala Thr Leu Thr Val Leu
                645                 650                 655

Glu Thr Pro Ser Leu Val Val Pro Leu Glu Asp Arg Val Val Ser Val
            660                 665                 670

Gly Glu Thr Val Ala Leu Gln Cys Lys Ala Thr Gly Asn Pro Pro Pro
        675                 680                 685

Arg Ile Thr Trp Phe Lys Gly Asp Arg Pro Leu Ser Leu Thr Glu Arg
    690                 695                 700

His His Leu Thr Pro Asp Asn Gln Leu Leu Val Val Gln Asn Val Val
705                 710                 715                 720

Ala Glu Asp Ala Gly Arg Tyr Thr Cys Glu Met Ser Asn Thr Leu Gly
                725                 730                 735

Thr Glu Arg Ala His Ser Gln Leu Ser Val Leu Pro Ala Ala Gly Cys
            740                 745                 750

Arg Lys Asp Gly Thr Thr Val Gly Ile Phe Glu Pro Arg Gly Pro Thr
        755                 760                 765

Ile Lys Pro Cys Pro Pro Cys Lys Cys Pro Ala Pro Asn Leu Leu Gly
    770                 775                 780

Gly Pro Ser Val Phe Leu Phe Pro Pro Lys Pro Lys Asp Thr Leu Met
785                 790                 795                 800

Ile Ser Arg Thr Pro Glu Val Thr Cys Val Val Val Asp Val Ser His
                805                 810                 815

Glu Asp Pro Glu Val Lys Phe Asn Trp Tyr Val Asp Gly Val Glu Val
            820                 825                 830

His Asn Ala Lys Thr Lys Pro Arg Glu Glu Gln Tyr Asn Ser Thr Tyr
        835                 840                 845

Arg Val Val Ser Val Leu Thr Val Leu His Gln Asp Trp Leu Asn Gly
    850                 855                 860

Lys Glu Tyr Lys Cys Lys Val Ser Asn Lys Ala Leu Pro Ala Pro Ile
865                 870                 875                 880

Glu Lys Thr Ile Ser Lys Ala Lys Gly Gln Pro Arg Glu Pro Gln Val
                885                 890                 895

Tyr Thr Leu Pro Pro Ser Arg Asp Glu Leu Thr Lys Asn Gln Val Ser
            900                 905                 910

Leu Thr Cys Leu Val Lys Gly Phe Tyr Pro Ser Asp Ile Ala Val Glu
        915                 920                 925

Trp Glu Ser Asn Gly Gln Pro Glu Asn Asn Tyr Lys Thr Thr Pro Pro
    930                 935                 940

Val Leu Asp Ser Asp Gly Ser Phe Phe Leu Tyr Ser Lys Leu Thr Val
945                 950                 955                 960

Asp Lys Ser Arg Trp Gln Gln Gly Asn Val Phe Ser Cys Ser Val Met
                965                 970                 975

His Glu Ala Leu His Asn His Tyr Thr Gln Lys Ser Leu Ser Leu Ser
            980                 985                 990

Pro Gly Lys
        995


<210>   15
<211>   1007
<212>   PRT
<213>   Artificial Sequence

<220>
<223>    fusion protein

<400>    15
Ala Gly Pro Arg Ala Pro Cys Ala Ala Ala Cys Thr Cys Ala Gly Asp
1               5                   10                  15

Ser Leu Asp Cys Gly Gly Arg Gly Leu Ala Ala Leu Pro Gly Asp Leu
            20                  25                  30

Pro Ser Trp Thr Arg Ser Leu Asn Leu Ser Tyr Asn Lys Leu Ser Glu
        35                  40                  45

Ile Asp Pro Ala Gly Phe Glu Asp Leu Pro Asn Leu Gln Glu Val Tyr
    50                  55                  60

Leu Asn Asn Asn Glu Leu Thr Ala Val Pro Ser Leu Gly Ala Ala Ser
65                  70                  75                  80

Ser His Val Val Ser Leu Phe Leu Gln His Asn Lys Ile Arg Ser Val
                85                  90                  95

Glu Gly Ser Gln Leu Lys Ala Tyr Leu Ser Leu Glu Val Leu Asp Leu
            100                 105                 110

Ser Leu Asn Asn Ile Thr Glu Val Arg Asn Thr Cys Phe Pro His Gly
        115                 120                 125

Pro Pro Ile Lys Glu Leu Asn Leu Ala Gly Asn Arg Ile Gly Thr Leu
    130                 135                 140

Glu Leu Gly Ala Phe Asp Gly Leu Ser Arg Ser Leu Leu Thr Leu Arg
145                 150                 155                 160

Leu Ser Lys Asn Arg Ile Thr Gln Leu Pro Val Arg Ala Phe Lys Leu
                165                 170                 175

Pro Arg Leu Thr Gln Leu Asp Leu Asn Arg Asn Arg Ile Arg Leu Ile
            180                 185                 190

Glu Gly Leu Thr Phe Gln Gly Leu Asn Ser Leu Glu Val Leu Lys Leu
        195                 200                 205

Gln Arg Asn Asn Ile Ser Lys Leu Thr Asp Gly Ala Phe Trp Gly Leu
    210                 215                 220

Ser Lys Met His Val Leu His Leu Glu Tyr Asn Ser Leu Val Glu Val
225                 230                 235                 240

Asn Ser Gly Ser Leu Tyr Gly Leu Thr Ala Leu His Gln Leu His Leu
                245                 250                 255

Ser Asn Asn Ser Ile Ala Arg Ile His Arg Lys Gly Trp Ser Phe Cys
            260                 265                 270

Gln Lys Leu His Glu Leu Val Leu Ser Phe Asn Asn Leu Thr Arg Leu
        275                 280                 285

Asp Glu Glu Ser Leu Ala Glu Leu Ser Ser Leu Ser Val Leu Arg Leu
    290                 295                 300

Ser His Asn Ser Ile Ser His Ile Ala Glu Gly Ala Phe Lys Gly Leu

305                 310                 315                 320

Arg Ser Leu Arg Val Leu Asp Leu Asp His Asn Glu Ile Ser Gly Thr
                325                 330                 335

Ile Glu Asp Thr Ser Gly Ala Phe Ser Gly Leu Asp Ser Leu Ser Lys
            340                 345                 350

Leu Thr Leu Phe Gly Asn Lys Ile Lys Ser Val Ala Lys Arg Ala Phe
        355                 360                 365

Ser Gly Leu Glu Gly Leu Glu His Leu Asn Leu Gly Gly Asn Ala Ile
    370                 375                 380

Arg Ser Val Gln Phe Asp Ala Phe Val Lys Met Lys Asn Leu Lys Glu
385                 390                 395                 400

Leu His Ile Ser Ser Asp Ser Phe Leu Cys Asp Cys Gln Leu Lys Trp
                405                 410                 415

Leu Pro Pro Trp Leu Ile Gly Arg Met Leu Gln Ala Phe Val Thr Ala
            420                 425                 430

Thr Cys Ala His Pro Glu Ser Leu Lys Gly Gln Ser Ile Phe Ser Val
        435                 440                 445

Pro Pro Glu Ser Phe Val Cys Asp Asp Phe Leu Lys Pro Gln Ile Ile
    450                 455                 460

Thr Gln Pro Glu Thr Thr Met Ala Met Val Gly Lys Asp Ile Arg Phe
465                 470                 475                 480

Thr Cys Ser Ala Ala Ser Ser Ser Ser Ser Pro Met Thr Phe Ala Trp
                485                 490                 495

Lys Lys Asp Asn Glu Val Leu Thr Asn Ala Asp Met Glu Asn Phe Val
            500                 505                 510

His Val His Ala Gln Asp Gly Glu Val Met Glu Tyr Thr Thr Ile Leu
        515                 520                 525

His Leu Arg Gln Val Thr Phe Gly His Glu Gly Arg Tyr Gln Cys Val
    530                 535                 540

Ile Thr Asn His Phe Gly Ser Thr Tyr Ser His Lys Ala Arg Leu Thr
545                 550                 555                 560

Val Asn Val Leu Pro Ser Phe Thr Lys Thr Pro His Asp Ile Thr Ile
                565                 570                 575

Arg Thr Thr Thr Val Ala Arg Leu Glu Cys Ala Ala Thr Gly His Pro
            580                 585                 590

Asn Pro Gln Ile Ala Trp Gln Lys Asp Gly Gly Thr Asp Phe Pro Ala
        595                 600                 605

Ala Arg Glu Arg Arg Met His Val Met Pro Asp Asp Asp Val Phe Phe
    610                 615                 620

Ile Thr Asp Val Lys Ile Asp Asp Ala Gly Val Tyr Ser Cys Thr Ala
625                 630                 635                 640

Gln Asn Ser Ala Gly Ser Ile Ser Ala Asn Ala Thr Leu Thr Val Leu

645                 650                 655

Glu Thr Pro Ser Leu Val Val Pro Leu Glu Asp Arg Val Val Ser Val
            660                 665                 670

Gly Glu Thr Val Ala Leu Gln Cys Lys Ala Thr Gly Asn Pro Pro Pro
        675                 680                 685

Arg Ile Thr Trp Phe Lys Gly Asp Arg Pro Leu Ser Leu Thr Glu Arg
    690                 695                 700

His His Leu Thr Pro Asp Asn Gln Leu Leu Val Val Gln Asn Val Val
705                 710                 715                 720

Ala Glu Asp Ala Gly Arg Tyr Thr Cys Glu Met Ser Asn Thr Leu Gly
                725                 730                 735

Thr Glu Arg Ala His Ser Gln Leu Ser Val Leu Pro Ala Ala Gly Cys
            740                 745                 750

Arg Lys Asp Gly Thr Thr Val Gly Ile Phe Arg Asn Thr Gly Arg Gly
        755                 760                 765

Gly Glu Glu Lys Lys Lys Glu Lys Glu Lys Glu Glu Gln Glu Glu Arg
    770                 775                 780

Glu Thr Lys Thr Pro Glu Cys Pro Ser His Thr Gln Pro Leu Gly Val
785                 790                 795                 800

Phe Leu Phe Pro Pro Lys Pro Lys Asp Thr Leu Met Ile Ser Arg Thr
                805                 810                 815

Pro Glu Val Thr Cys Val Val Val Asp Val Ser His Glu Asp Pro Glu
            820                 825                 830

Val Lys Phe Asn Trp Tyr Val Asp Gly Val Glu Val His Asn Ala Lys
        835                 840                 845

Thr Lys Pro Arg Glu Glu Gln Tyr Asn Ser Thr Tyr Arg Val Val Ser
    850                 855                 860

Val Leu Thr Val Leu His Gln Asp Trp Leu Asn Gly Lys Glu Tyr Lys
865                 870                 875                 880

Cys Lys Val Ser Asn Lys Ala Leu Pro Ala Pro Ile Glu Lys Thr Ile
                885                 890                 895

Ser Lys Ala Lys Gly Gln Pro Arg Glu Pro Gln Val Tyr Thr Leu Pro
            900                 905                 910

Pro Ser Arg Asp Glu Leu Thr Lys Asn Gln Val Ser Leu Thr Cys Leu
        915                 920                 925

Val Lys Gly Phe Tyr Pro Ser Asp Ile Ala Val Glu Trp Glu Ser Asn
    930                 935                 940

Gly Gln Pro Glu Asn Asn Tyr Lys Thr Thr Pro Pro Val Leu Asp Ser
945                 950                 955                 960

Asp Gly Ser Phe Phe Leu Tyr Ser Lys Leu Thr Val Asp Lys Ser Arg
                965                 970                 975

Trp Gln Gln Gly Asn Val Phe Ser Cys Ser Val Met His Glu Ala Leu

980                 985                 990

His Asn His Tyr Thr Gln Lys Ser Leu Ser Leu Ser Pro Gly Lys
        995                 1000                1005

<210>   16
<211>   994
<212>   PRT
<213>   Artificial Sequence

<220>
<223>   fusion protein

<400>   16
   Ala Gly Pro Arg Ala Pro Cys Ala Ala Ala Cys Thr Cys Ala Gly Asp
   1               5                   10                  15
   Ser Leu Asp Cys Gly Gly Arg Gly Leu Ala Ala Leu Pro Gly Asp Leu
               20                  25                  30
   Pro Ser Trp Thr Arg Ser Leu Asn Leu Ser Tyr Asn Lys Leu Ser Glu
           35                  40                  45
   Ile Asp Pro Ala Gly Phe Glu Asp Leu Pro Asn Leu Gln Glu Val Tyr
       50                  55                  60
   Leu Asn Asn Asn Glu Leu Thr Ala Val Pro Ser Leu Gly Ala Ala Ser
   65                  70                  75                  80
   Ser His Val Val Ser Leu Phe Leu Gln His Asn Lys Ile Arg Ser Val
                   85                  90                  95
   Glu Gly Ser Gln Leu Lys Ala Tyr Leu Ser Leu Glu Val Leu Asp Leu
               100                 105                 110
   Ser Leu Asn Asn Ile Thr Glu Val Arg Asn Thr Cys Phe Pro His Gly
           115                 120                 125
   Pro Pro Ile Lys Glu Leu Asn Leu Ala Gly Asn Arg Ile Gly Thr Leu
       130                 135                 140
   Glu Leu Gly Ala Phe Asp Gly Leu Ser Arg Ser Leu Leu Thr Leu Arg
   145                 150                 155                 160
   Leu Ser Lys Asn Arg Ile Thr Gln Leu Pro Val Arg Ala Phe Lys Leu
                   165                 170                 175
   Pro Arg Leu Thr Gln Leu Asp Leu Asn Arg Asn Arg Ile Arg Leu Ile
               180                 185                 190
   Glu Gly Leu Thr Phe Gln Gly Leu Asn Ser Leu Glu Val Leu Lys Leu
           195                 200                 205
   Gln Arg Asn Asn Ile Ser Lys Leu Thr Asp Gly Ala Phe Trp Gly Leu
       210                 215                 220
   Ser Lys Met His Val Leu His Leu Glu Tyr Asn Ser Leu Val Glu Val
   225                 230                 235                 240
   Asn Ser Gly Ser Leu Tyr Gly Leu Thr Ala Leu His Gln Leu His Leu
                   245                 250                 255
   Ser Asn Asn Ser Ile Ala Arg Ile His Arg Lys Gly Trp Ser Phe Cys
               260                 265                 270
   Gln Lys Leu His Glu Leu Val Leu Ser Phe Asn Asn Leu Thr Arg Leu
           275                 280                 285
   Asp Glu Glu Ser Leu Ala Glu Leu Ser Ser Leu Ser Val Leu Arg Leu
       290                 295                 300
   Ser His Asn Ser Ile Ser His Ile Ala Glu Gly Ala Phe Lys Gly Leu
   305                 310                 315                 320
   Arg Ser Leu Arg Val Leu Asp Leu Asp His Asn Glu Ile Ser Gly Thr
                   325                 330                 335
   Ile Glu Asp Thr Ser Gly Ala Phe Ser Gly Leu Asp Ser Leu Ser Lys
               340                 345                 350
   Leu Thr Leu Phe Gly Asn Lys Ile Lys Ser Val Ala Lys Arg Ala Phe
           355                 360                 365
   Ser Gly Leu Glu Gly Leu Glu His Leu Asn Leu Gly Gly Asn Ala Ile
       370                 375                 380
   Arg Ser Val Gln Phe Asp Ala Phe Val Lys Met Lys Asn Leu Lys Glu
   385                 390                 395                 400
   Leu His Ile Ser Ser Asp Ser Phe Leu Cys Asp Cys Gln Leu Lys Trp
                   405                 410                 415
   Leu Pro Pro Trp Leu Ile Gly Arg Met Leu Gln Ala Phe Val Thr Ala
               420                 425                 430
   Thr Cys Ala His Pro Glu Ser Leu Lys Gly Gln Ser Ile Phe Ser Val
           435                 440                 445
   Pro Pro Glu Ser Phe Val Cys Asp Asp Phe Leu Lys Pro Gln Ile Ile
       450                 455                 460
   Thr Gln Pro Glu Thr Thr Met Ala Met Val Gly Lys Asp Ile Arg Phe
   465                 470                 475                 480
   Thr Cys Ser Ala Ala Ser Ser Ser Ser Ser Pro Met Thr Phe Ala Trp
                   485                 490                 495
   Lys Lys Asp Asn Glu Val Leu Thr Asn Ala Asp Met Glu Asn Phe Val
               500                 505                 510
   His Val His Ala Gln Asp Gly Glu Val Met Glu Tyr Thr Thr Ile Leu
           515                 520                 525
   His Leu Arg Gln Val Thr Phe Gly His Glu Gly Arg Tyr Gln Cys Val
       530                 535                 540
   Ile Thr Asn His Phe Gly Ser Thr Tyr Ser His Lys Ala Arg Leu Thr
   545                 550                 555                 560
   Val Asn Val Leu Pro Ser Phe Thr Lys Thr Pro His Asp Ile Thr Ile
                   565                 570                 575
   Arg Thr Thr Thr Val Ala Arg Leu Glu Cys Ala Ala Thr Gly His Pro
   580 585 590
   Asn Pro Gln Ile Ala Trp Gln Lys Asp Gly Gly Thr Asp Phe Pro Ala
   595 600 605
   Ala Arg Glu Arg Arg Met His Val Met Pro Asp Asp Asp Val Phe Phe
   610 615 620
   Ile Thr Asp Val Lys Ile Asp Asp Ala Gly Val Tyr Ser Cys Thr Ala
   625 630 635 640
   Gln Asn Ser Ala Gly Ser Ile Ser Ala Asn Ala Thr Leu Thr Val Leu
   645 650 655
   Glu Thr Pro Ser Leu Val Val Pro Leu Glu Asp Arg Val Val Ser Val
   660 665 670
   Gly Glu Thr Val Ala Leu Gln Cys Lys Ala Thr Gly Asn Pro Pro Pro
   675 680 685
   Arg Ile Thr Trp Phe Lys Gly Asp Arg Pro Leu Ser Leu Thr Glu Arg
   690 695 700
   His His Leu Thr Pro Asp Asn Gln Leu Leu Val Val Gln Asn Val Val
   705 710 715 720
   Ala Glu Asp Ala Gly Arg Tyr Thr Cys Glu Met Ser Asn Thr Leu Gly
   725 730 735
   Thr Glu Arg Ala His Ser Gln Leu Ser Val Leu Pro Ala Ala Gly Cys
   740 745 750
   Arg Lys Asp Gly Thr Thr Val Gly Ile Phe Glu Pro Lys Ser Ser Asp
   755 760 765
   Lys Thr His Thr Ser Pro Pro Ser Pro Ala Pro Glu Leu Leu Gly Gly
   770 775 780
   Ser Ser Val Phe Leu Phe Pro Pro Lys Pro Lys Asp Thr Leu Met Ile
   785 790 795 800
   Ser Arg Thr Pro Glu Val Thr Cys Val Val Val Asp Val Ser His Glu
   805 810 815
   Asp Pro Glu Val Lys Phe Asn Trp Tyr Val Asp Gly Val Glu Val His
   820 825 830
   Asn Ala Lys Thr Lys Pro Arg Glu Glu Gln Tyr Asn Ser Thr Tyr Arg
   835 840 845
   Val Val Ser Val Leu Thr Val Leu His Gln Asp Trp Leu Asn Gly Lys
   850 855 860
   Glu Tyr Lys Cys Lys Val Ser Asn Lys Ala Leu Pro Ala Pro Ile Glu
   865 870 875 880
   Lys Thr Ile Ser Lys Ala Lys Gly Gln Pro Arg Glu Pro Gln Val Tyr
   885 890 895
   Thr Leu Pro Pro Ser Arg Asp Glu Leu Thr Lys Asn Gln Val Ser Leu
   900 905 910
   Thr Cys Leu Val Lys Gly Phe Tyr Pro Ser Asp Ile Ala Val Glu Trp
           915                 920                 925
   Glu Ser Asn Gly Gln Pro Glu Asn Asn Tyr Lys Thr Thr Pro Pro Val
       930                 935                 940
   Leu Asp Ser Asp Gly Ser Phe Phe Leu Tyr Ser Lys Leu Thr Val Asp
   945                 950                 955                 960
   Lys Ser Arg Trp Gln Gln Gly Asn Val Phe Ser Cys Ser Val Met His
                   965                 970                 975
   Glu Ala Leu His Asn His Tyr Thr Gln Lys Ser Leu Ser Leu Ser Pro
               980                 985                 990
   Gly Lys

<210>    17
<211>    2982
<212>    DNA
<213>    Artificial Sequence

<220>
<223>    fusion protein

<400>    17
gctgggcctc gggctccttg tgctgccgcc tgcacatgtg caggcgattc cctggactgc      60

ggcggcagag gcctggccgc cctgcctggc gatctgccat cctggacccg gagcctgaac     120

ctgagctaca acaagctgag cgagatcgat cccgccggct ttgaggacct gcctaacctg     180

caggaggtgt atctgaacaa taacgagctg accgcggtac catccctggg cgctgcttca     240

tcacatgtcg tctctctctt tctgcagcac aacaagattc gcagcgtgga ggggagccag     300

ctgaaggcct acctttcctt agaagtgtta gatctgagtt tgaacaacat cacggaagtg     360

cggaacacct gctttccaca cggaccgcct ataaaggagc tcaacctggc aggcaatcgg     420

attggcaccc tggagttggg agcatttgat ggtctgtcac ggtcgctgct aactcttcgc     480

ctgagcaaaa acaggatcac ccagcttcct gtaagagcat tcaagctacc caggctgaca     540

caactggacc tcaatcggaa caggattcgg ctgatagagg gcctcacctt ccaggggctc     600

aacagcttgg aggtgctgaa gcttcagcga aacaacatca gcaaactgac agatggggcc     660

ttctggggac tgtccaagat gcatgtgctg cacctggagt acaacagcct ggtagaagtg     720

aacagcggct cgctctacgg cctcacggcc ctgcatcagc tccacctcag caacaattcc     780

atcgctcgca ttcaccgcaa gggctggagc ttctgccaga agctgcatga gttggtcctg     840

tccttcaaca acctgacacg gctggacgag gagagcctgg ccgagctgag cagcctgagt     900

gtcctgcgtc tcagccacaa ttccatcagc cacattgcgg agggtgcctt caagggactc      960

aggagcctgc gagtcttgga tctggaccat aacgagattt cgggcacaat agaggacacg    1020

agcggcgcct tctcagggct cgacagcctc agcaagctga ctctgtttgg aaacaagatc 1080

aagtctgtgg ctaagagagc attctcgggg ctggaaggcc tggagcacct gaaccttgga 1140

gggaatgcga tcagatctgt ccagtttgat gcctttgtga agatgaagaa tcttaaagag 1200

ctccatatca gcagcgacag cttcctgtgt gactgccagc tgaagtggct gcccccgtgg 1260

ctaattggca ggatgctgca ggcctttgtg acagccacct gtgcccaccc agaatcactg 1320

aagggtcaga gcattttctc tgtgccacca gagagtttcg tgtgcgatga cttcctgaag 1380

ccacagatca tcacccagcc agaaaccacc atggctatgg tgggcaagga catccggttt 1440

acatgctcag cagccagcag cagcagctcc cccatgacct ttgcctggaa gaaagacaat 1500

gaagtcctga ccaatgcaga catggagaac tttgtccacg tccacgcgca ggacggggaa 1560

gtgatggagt acaccaccat cctgcacctc cgtcaggtca ctttcgggca cgagggccgc 1620

taccaatgtg tcatcaccaa ccactttggc tccacctatt cacataaggc caggctcacc 1680

gtgaatgtgt tgccatcatt caccaaaacg ccccacgaca taaccatccg gaccaccacc 1740

gtggcccgcc tcgaatgtgc tgccacaggt cacccaaacc ctcagattgc ctggcagaag 1800

gatggaggca cggatttccc cgctgcccgt gagcgacgca tgcatgtcat gccggatgac 1860

gacgtgtttt tcatcactga tgtgaaaata gatgacgcag gggtttacag ctgtactgct 1920

cagaactcag ccggttctat ttcagctaat gccaccctga ctgtcctaga gaccccatcc 1980

ttggtggtcc ccttggaaga ccgtgtggta tctgtgggag aaacagtggc cctccaatgc 2040

aaagccacgg ggaaccctcc gccccgcatc acctggttca agggggaccg cccgctgagc 2100

ctcactgagc ggcaccacct gacccctgac aaccagctcc tggtggttca gaacgtggtg 2160

gcagaggatg cgggccgata tacctgtgag atgtccaaca ccctgggcac ggagcgagct 2220

cacagccagc tgagcgtcct gcccgcagca ggctgcagga aggatgggac cacggtaggc 2280

atcttcgagc caaagtcctc tgataagaca cacacctctc caccatgccc agcaccagag 2340

ctgctgggag gaccaagcgt gttcctgttt cctccaaagc ccaaggacac actgatgatc 2400

tccaggacac cagaggtgac ctgcgtggtg gtggacgtga gccacgagga ccccgaggtg 2460

aagttcaact ggtacgtgga tggcgtggag gtgcacaatg ccaagaccaa gcccagagag 2520

gagcagtaca actctaccta tagggtggtg agcgtgctga cagtgctgca ccaggactgg 2580

ctgaacggca aggagtataa gtgcaaggtg agcaataagg ccctgcctgc ccccatcgag 2640

aagacaatct ccaaggccaa gggccagcca agagagcccc aggtgtacac cctgccccct 2700

agcagggatg agctgacaaa gaaccaggtg tccctgacct gtctggtgaa gggcttttat 2760

ccctccgaca tcgccgtgga gtgggagtct aatggccagc ctgagaataa ctacaagaca 2820

accccacccg tgctggattc tgacggcagc ttctttctgt attctaagct gaccgtggac 2880

aagagcaggt ggcagcaggg caacgtgttc agctgctccg tgatgcacga agcactgcac 2940

aatcactaca cccagaaatc actgtcactg agccctggca aa 2982

<210> 18
<211> 2985
<212> DNA
<213> Artificial Sequence

<220>
<223> fusion protein

<400> 18
gctgggcctc gggctccttg tgctgccgcc tgcacatgtg caggcgattc cctggactgc 60

ggcggcagag gcctggccgc cctgcctggc gatctgccat cctggacccg gagcctgaac 120

ctgagctaca acaagctgag cgagatcgat cccgccggct ttgaggacct gcctaacctg 180

caggaggtgt atctgaacaa taacgagctg accgcggtac catccctggg cgctgcttca 240

tcacatgtcg tctctctctt tctgcagcac aacaagattc gcagcgtgga ggggagccag 300

ctgaaggcct acctttcctt agaagtgtta gatctgagtt tgaacaacat cacggaagtg 360

cggaacacct gctttccaca cggaccgcct ataaaggagc tcaacctggc aggcaatcgg 420

attggcaccc tggagttggg agcatttgat ggtctgtcac ggtcgctgct aactcttcgc 480

ctgagcaaaa acaggatcac ccagcttcct gtaagagcat tcaagctacc caggctgaca 540

caactggacc tcaatcggaa caggattcgg ctgatagagg gcctcacctt ccaggggctc 600

aacagcttgg aggtgctgaa gcttcagcga aacaacatca gcaaactgac agatggggcc 660

ttctggggac tgtccaagat gcatgtgctg cacctggagt acaacagcct ggtagaagtg 720

aacagcggct cgctctacgg cctcacggcc ctgcatcagc tccacctcag caacaattcc 780

atcgctcgca ttcaccgcaa gggctggagc ttctgccaga agctgcatga gttggtcctg 840

tccttcaaca acctgacacg gctggacgag gagagcctgg ccgagctgag cagcctgagt 900

gtcctgcgtc tcagccacaa ttccatcagc cacattgcgg agggtgcctt caagggactc 960

aggagcctgc gagtcttgga tctggaccat aacgagattt cgggcacaat agaggacacg 1020

agcggcgcct tctcagggct cgacagcctc agcaagctga ctctgtttgg aaacaagatc 1080

aagtctgtgg ctaagagagc attctcgggg ctggaaggcc tggagcacct gaaccttgga 1140

gggaatgcga tcagatctgt ccagtttgat gcctttgtga agatgaagaa tcttaaagag 1200

ctccatatca gcagcgacag cttcctgtgt gactgccagc tgaagtggct gcccccgtgg 1260

ctaattggca ggatgctgca ggcctttgtg acagccacct gtgcccaccc agaatcactg 1320

aagggtcaga gcattttctc tgtgccacca gagagtttcg tgtgcgatga cttcctgaag 1380

ccacagatca tcacccagcc agaaaccacc atggctatgg tgggcaagga catccggttt 1440

acatgctcag cagccagcag cagcagctcc cccatgacct ttgcctggaa gaaagacaat 1500

gaagtcctga ccaatgcaga catggagaac tttgtccacg tccacgcgca ggacggggaa 1560

gtgatggagt acaccaccat cctgcacctc cgtcaggtca ctttcggcca cgagggccgc 1620

taccaatgtg tcatcaccaa ccactttggc tccacctatt cacataaggc caggctcacc 1680

gtgaatgtgt tgccatcatt caccaaaacg ccccacgaca taaccatccg gaccaccacc 1740

gtggcccgcc tcgaatgtgc tgccacaggt cacccaaacc ctcagattgc ctggcagaag 1800

gatggaggca cggatttccc cgctgcccgt gagcgacgca tgcatgtcat gccggatgac 1860

gacgtgtttt tcatcactga tgtgaaaata gatgacgcag gggtttacag ctgtactgct 1920

cagaactcag ccggttctat ttcagctaat gccaccctga ctgtcctaga gaccccatcc 1980

ttggtggtcc ccttggaaga ccgtgtggta tctgtgggag aaacagtggc cctccaatgc 2040

aaagccacgg ggaaccctcc gccccgcatc acctggttca agggggaccg cccgctgagc 2100

ctcactgagc ggcaccacct gacccctgac aaccagctcc tggtggttca gaacgtggtg 2160

gcagaggatg cgggccgata tacctgtgag atgtccaaca ccctgggcac ggagcgagct 2220

cacagccagc tgagcgtcct gcccgcagca ggctgcagga aggatgggac cacggtaggc 2280

atcttcgagc ctcggggccc taccatcaag ccctgccccc cttgcaagtg ccctgcccct 2340

aatctgctgg gcggaccctc cgtgttcctg tttcctccaa agcccaagga cacactgatg 2400

atctccagga caccagaggt gacctgcgtg gtggtggacg tgagccacga ggaccccgag 2460

gtgaagttca actggtacgt ggatggcgtg gaggtgcaca atgccaagac caagcccaga 2520

gaggagcagt acaactctac ctatagggtg gtgagcgtgc tgacagtgct gcaccaggac 2580

tggctgaacg gcaaggagta taagtgcaag gtgagcaata aggccctgcc tgccccaatc 2640

gagaagacaa tctccaaggc caagggccag ccaagagagc cccaggtgta caccctgccc 2700

cctagcaggg atgagctgac aaagaaccag gtgtccctga cctgtctggt gaagggcttt 2760

tatccctccg acatcgccgt ggagtgggag tctaatggcc agcctgagaa taactacaag 2820

acaaccccac ccgtgctgga ttctgacggc agcttctttc tgtattctaa gctgaccgtg 2880

gacaagagca ggtggcagca gggcaacgtg ttcagctgct ccgtgatgca cgaagcactg 2940

cacaatcact acacccagaa atcactgtca ctgagccctg gcaaa 2985

<210> 19
<211> 3021
<212> DNA
<213> Artificial Sequence

<220>
<223> fusion protein

<400> 19
gctgggcctc gggctccttg tgctgccgcc tgcacatgtg caggcgattc cctggactgc 60

ggcggcagag gcctggccgc cctgcctggc gatctgccat cctggacccg gagcctgaac 120

ctgagctaca acaagctgag cgagatcgat cccgccggct ttgaggacct gcctaacctg 180

caggaggtgt atctgaacaa taacgagctg accgcggtac catccctggg cgctgcttca 240

tcacatgtcg tctctctctt tctgcagcac aacaagattc gcagcgtgga ggggagccag 300

ctgaaggcct acctttcctt agaagtgtta gatctgagtt tgaacaacat cacggaagtg 360

cggaacacct gctttccaca cggaccgcct ataaaggagc tcaacctggc aggcaatcgg 420

attggcaccc tggagttggg agcatttgat ggtctgtcac ggtcgctgct aactcttcgc 480

ctgagcaaaa acaggatcac ccagcttcct gtaagagcat tcaagctacc caggctgaca 540

caactggacc tcaatcggaa caggattcgg ctgatagagg gcctcacctt ccaggggctc 600

aacagcttgg aggtgctgaa gcttcagcga aacaacatca gcaaactgac agatggggcc 660

ttctggggac tgtccaagat gcatgtgctg cacctggagt acaacagcct ggtagaagtg 720

aacagcggct cgctctacgg cctcacggcc ctgcatcagc tccacctcag caacaattcc 780

atcgctcgca ttcaccgcaa gggctggagc ttctgccaga agctgcatga gttggtcctg 840

tccttcaaca acctgacacg gctggacgag gagagcctgg ccgagctgag cagcctgagt 900

gtcctgcgtc tcagccacaa ttccatcagc cacattgcgg agggtgcctt caagggactc 960

aggagcctgc gagtcttgga tctggaccat aacgagattt cgggcacaat agaggacacg 1020

agcggcgcct tctcagggct cgacagcctc agcaagctga ctctgtttgg aaacaagatc 1080

aagtctgtgg ctaagagagc attctcgggg ctggaaggcc tggagcacct gaaccttgga 1140

gggaatgcga tcagatctgt ccagtttgat gcctttgtga agatgaagaa tcttaaagag 1200

ctccatatca gcagcgacag cttcctgtgt gactgccagc tgaagtggct gcccccgtgg 1260

ctaattggca ggatgctgca ggcctttgtg acagccacct gtgcccaccc agaatcactg 1320

aagggtcaga gcattttctc tgtgccacca gagagtttcg tgtgcgatga cttcctgaag 1380

ccacagatca tcacccagcc agaaaccacc atggctatgg tgggcaagga catccggttt 1440

acatgctcag cagccagcag cagcagctcc cccatgacct ttgcctggaa gaaagacaat 1500

gaagtcctga ccaatgcaga catggagaac tttgtccacg tccacgcgca ggacggggaa 1560

gtgatggagt acaccaccat cctgcacctc cgtcaggtca ctttcggcca cgagggccgc 1620

taccaatgtg tcatcaccaa ccactttggc tccacctatt cacataaggc caggctcacc 1680

gtgaatgtgt tgccatcatt caccaaaacg ccccacgaca taaccatccg gaccaccacc 1740

gtggcccgcc tcgaatgtgc tgccacaggt cacccaaacc ctcagattgc ctggcagaag 1800

gatggaggca cggatttccc cgctgcccgt gagcgacgca tgcatgtcat gccggatgac 1860

gacgtgtttt tcatcactga tgtgaaaata gatgacgcag gggtttacag ctgtactgct 1920

cagaactcag ccggttctat ttcagctaat gccaccctga ctgtcctaga gaccccatcc 1980

ttggtggtcc ccttggaaga ccgtgtggta tctgtgggag aaacagtggc cctccaatgc 2040

aaagccacgg ggaaccctcc gccccgcatc acctggttca agggggaccg cccgctgagc 2100

ctcactgagc ggcaccacct gacccctgac aaccagctcc tggtggttca gaacgtggtg 2160

gcagaggatg cgggccgata tacctgtgag atgtccaaca ccctgggcac ggagcgagct 2220

cacagccagc tgagcgtcct gcccgcagca ggctgcagga aggatgggac cacggtaggc 2280

atcttccgca acaccggccg cggcggcgag gagaagaaga aggagaagga gaaggaggag 2340

caggaggagc gcgagaccaa gacccccgag tgccccagcc acacccagcc cctgggcgtg 2400

ttcctgtttc ctccaaagcc caaggacaca ctgatgatct ccaggacacc agaggtgacc 2460

tgcgtggtgg tggacgtgag ccacgaggac cccgaggtga agttcaactg gtacgtggat 2520

ggcgtggagg tgcacaatgc caagaccaag cccagagagg agcagtacaa ctctacctat 2580

agggtggtga gcgtgctgac agtgctgcac caggactggc tgaacggcaa ggagtataag 2640

tgcaaggtga gcaataaggc cctgcctgcc ccaatcgaga agacaatctc caaggccaag 2700

ggccagccaa gagagcccca ggtgtacacc ctgcccccta gcagggatga gctgacaaag 2760

aaccaggtgt ccctgacctg tctggtgaag ggcttttatc cctccgacat cgccgtggag 2820

tgggagtcta atggccagcc tgagaataac tacaagacaa ccccacccgt gctggattct 2880

gacggcagct tctttctgta ttctaagctg accgtggaca agagcaggtg gcagcagggc 2940

aacgtgttca gctgctccgt gatgcacgaa gcactgcaca atcactacac ccagaaatca 3000

ctgtcactga gccctggcaa a 3021

<210> 20
<211> 2982
<212> DNA
<213> Artificial Sequence

<220>
<223> fusion protein

<400> 20
gctgggcctc gggctccttg tgctgccgcc tgcacatgtg caggcgattc cctggactgc 60

ggcggcagag gcctggccgc cctgcctggc gatctgccat cctggacccg gagcctgaac 120

ctgagctaca acaagctgag cgagatcgat cccgccggct ttgaggacct gcctaacctg 180

caggaggtgt atctgaacaa taacgagctg accgcggtac catccctggg cgctgcttca 240

tcacatgtcg tctctctctt tctgcagcac aacaagattc gcagcgtgga ggggagccag 300

ctgaaggcct acctttcctt agaagtgtta gatctgagtt tgaacaacat cacggaagtg 360

cggaacacct gctttccaca cggaccgcct ataaaggagc tcaacctggc aggcaatcgg 420

attggcaccc tggagttggg agcatttgat ggtctgtcac ggtcgctgct aactcttcgc 480

ctgagcaaaa acaggatcac ccagcttcct gtaagagcat tcaagctacc caggctgaca 540

caactggacc tcaatcggaa caggattcgg ctgatagagg gcctcacctt ccaggggctc 600

aacagcttgg aggtgctgaa gcttcagcga aacaacatca gcaaactgac agatggggcc 660

ttctggggac tgtccaagat gcatgtgctg cacctggagt acaacagcct ggtagaagtg 720

aacagcggct cgctctacgg cctcacggcc ctgcatcagc tccacctcag caacaattcc 780

atcgctcgca ttcaccgcaa gggctggagc ttctgccaga agctgcatga gttggtcctg 840

tccttcaaca acctgacacg gctggacgag gagagcctgg ccgagctgag cagcctgagt 900

gtcctgcgtc tcagccacaa ttccatcagc cacattgcgg agggtgcctt caagggactc 960

aggagcctgc gagtcttgga tctggaccat aacgagattt cgggcacaat agaggacacg 1020

agcggcgcct tctcagggct cgacagcctc agcaagctga ctctgtttgg aaacaagatc 1080

aagtctgtgg ctaagagagc attctcgggg ctggaaggcc tggagcacct gaaccttgga 1140

gggaatgcga tcagatctgt ccagtttgat gcctttgtga agatgaagaa tcttaaagag 1200

ctccatatca gcagcgacag cttcctgtgt gactgccagc tgaagtggct gcccccgtgg 1260

ctaattggca ggatgctgca ggcctttgtg acagccacct gtgcccaccc agaatcactg 1320

aagggtcaga gcattttctc tgtgccacca gagagtttcg tgtgcgatga cttcctgaag 1380

ccacagatca tcacccagcc agaaaccacc atggctatgg tgggcaagga catccggttt 1440

acatgctcag cagccagcag cagcagctcc cccatgacct ttgcctggaa gaaagacaat 1500

gaagtcctga ccaatgcaga catggagaac tttgtccacg tccacgcgca ggacggggaa 1560

gtgatggagt acaccaccat cctgcacctc cgtcaggtca ctttcgggca cgagggccgc 1620

taccaatgtg tcatcaccaa ccactttggc tccacctatt cacataaggc caggctcacc 1680

gtgaatgtgt tgccatcatt caccaaaacg ccccacgaca taaccatccg gaccaccacc 1740

gtggcccgcc tcgaatgtgc tgccacaggt cacccaaacc ctcagattgc ctggcagaag 1800

gatggaggca cggatttccc cgctgcccgt gagcgacgca tgcatgtcat gccggatgac 1860

gacgtgtttt tcatcactga tgtgaaaata gatgacgcag gggtttacag ctgtactgct 1920

cagaactcag ccggttctat ttcagctaat gccaccctga ctgtcctaga gaccccatcc 1980

ttggtggtcc ccttggaaga ccgtgtggta tctgtgggag aaacagtggc cctccaatgc 2040

aaagccacgg ggaaccctcc gccccgcatc acctggttca agggggaccg cccgctgagc 2100

ctcactgagc ggcaccacct gacccctgac aaccagctcc tggtggttca gaacgtggtg 2160

gcagaggatg cgggccgata tacctgtgag atgtccaaca ccctgggcac ggagcgagct 2220

cacagccagc tgagcgtcct gcccgcagca ggctgcagga aggatgggac cacggtaggc 2280

atcttcgaac cgaaatcttc tgacaaaacc cacacctctc cgccgtctcc ggctccggaa 2340

ctgctgggtg gttcttctgt tttcctgttt cctccaaagc ccaaggacac actgatgatc 2400

tccaggacac cagaggtgac ctgcgtggtg gtggacgtga gccacgagga ccccgaggtg 2460

aagttcaact ggtacgtgga tggcgtggag gtgcacaatg ccaagaccaa gcccagagag 2520

gagcagtaca actctaccta tagggtggtg agcgtgctga cagtgctgca ccaggactgg 2580

ctgaacggca aggagtataa gtgcaaggtg agcaataagg ccctgcctgc cccaatcgag 2640

aagacaatct ccaaggccaa gggccagcca agagagcccc aggtgtacac cctgccccct 2700

agcagggatg agctgacaaa gaaccaggtg tccctgacct gtctggtgaa gggcttttat 2760

ccctccgaca tcgccgtgga gtgggagtct aatggccagc ctgagaataa ctacaagaca 2820

accccacccg tgctggattc tgacggcagc ttctttctgt attctaagct gaccgtggac 2880

aagagcaggt ggcagcaggg caacgtgttc agctgctccg tgatgcacga agcactgcac 2940

aatcactaca cccagaaatc actgtcactg agccctggca aa 2982

<210> 21
<211> 992
<212> PRT
<213> Artificial Sequence

<220>
<223> fusion protein

<400> 21
Ala Gln Ala Gly Pro Arg Ala Pro Cys Ala Ala Ala Cys Thr Cys Ala
1               5                   10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Glu Pro Lys Ser Ser Asp Lys Thr
        755                 760                 765

His Thr Ser Pro Pro Cys Pro Ala Pro Glu Leu Leu Gly Gly Pro Ser
    770                 775                 780

Val Phe Leu Phe Pro Pro Lys Pro Lys Asp Thr Leu Met Ile Ser Arg
785                 790                 795                 800

Thr Pro Glu Val Thr Cys Val Val Val Asp Val Ser His Glu Asp Pro
                805                 810                 815

Glu Val Lys Phe Asn Trp Tyr Val Asp Gly Val Glu Val His Asn Ala
            820                 825                 830

Lys Thr Lys Pro Arg Glu Glu Gln Tyr Asn Ser Thr Tyr Arg Val Val
        835                 840                 845

Ser Val Leu Thr Val Leu His Gln Asp Trp Leu Asn Gly Lys Glu Tyr
    850                 855                 860

Lys Cys Lys Val Ser Asn Lys Ala Leu Pro Ala Pro Ile Glu Lys Thr
865                 870                 875                 880

Ile Ser Lys Ala Lys Gly Gln Pro Arg Glu Pro Gln Val Tyr Thr Leu
                885                 890                 895

Pro Pro Ser Arg Asp Glu Leu Thr Lys Asn Gln Val Ser Leu Thr Cys
            900                 905                 910

Leu Val Lys Gly Phe Tyr Pro Ser Asp Ile Ala Val Glu Trp Glu Ser
        915                 920                 925

Asn Gly Gln Pro Glu Asn Asn Tyr Lys Thr Thr Pro Pro Val Leu Asp
    930                 935                 940

Ser Asp Gly Ser Phe Phe Leu Tyr Ser Lys Leu Thr Val Asp Lys Ser
945                 950                 955                 960

Arg Trp Gln Gln Gly Asn Val Phe Ser Cys Ser Val Met His Glu Ala
                965                 970                 975

Leu His Asn His Tyr Thr Gln Lys Ser Leu Ser Leu Ser Pro Gly Lys
            980                 985                 990

<210>    22
<211>    993
<212>    PRT
<213>    Artificial Sequence

<220>
<223>    fusion protein

<400>    22
Ala Gln Ala Gly Pro Arg Ala Pro Cys Ala Ala Ala Cys Thr Cys Ala
1               5                   10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu

            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe

435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Glu Pro Arg Gly Pro Thr Ile Lys
        755                 760                 765

Pro Cys Pro Pro Cys Lys Cys Pro Ala Pro Asn Leu Leu Gly Gly Pro

770                 775                 780

Ser Val Phe Leu Phe Pro Pro Lys Pro Lys Asp Thr Leu Met Ile Ser
785                 790                 795                 800

Arg Thr Pro Glu Val Thr Cys Val Val Val Asp Val Ser His Glu Asp
                805                 810                 815

Pro Glu Val Lys Phe Asn Trp Tyr Val Asp Gly Val Glu Val His Asn
            820                 825                 830

Ala Lys Thr Lys Pro Arg Glu Glu Gln Tyr Asn Ser Thr Tyr Arg Val
        835                 840                 845

Val Ser Val Leu Thr Val Leu His Gln Asp Trp Leu Asn Gly Lys Glu
    850                 855                 860

Tyr Lys Cys Lys Val Ser Asn Lys Ala Leu Pro Ala Pro Ile Glu Lys
865                 870                 875                 880

Thr Ile Ser Lys Ala Lys Gly Gln Pro Arg Glu Pro Gln Val Tyr Thr
                885                 890                 895

Leu Pro Pro Ser Arg Asp Glu Leu Thr Lys Asn Gln Val Ser Leu Thr
            900                 905                 910

Cys Leu Val Lys Gly Phe Tyr Pro Ser Asp Ile Ala Val Glu Trp Glu
        915                 920                 925

Ser Asn Gly Gln Pro Glu Asn Asn Tyr Lys Thr Thr Pro Pro Val Leu
    930                 935                 940

Asp Ser Asp Gly Ser Phe Phe Leu Tyr Ser Lys Leu Thr Val Asp Lys
945                 950                 955                 960

Ser Arg Trp Gln Gln Gly Asn Val Phe Ser Cys Ser Val Met His Glu
                965                 970                 975

Ala Leu His Asn His Tyr Thr Gln Lys Ser Leu Ser Leu Ser Pro Gly
            980                 985                 990

Lys

<210>   23
<211>   1005
<212>   PRT
<213>   Artificial Sequence

<220>
<223>   fusion protein

<400>   23
Ala Gln Ala Gly Pro Arg Ala Pro Cys Ala Ala Ala Cys Thr Cys Ala
1               5                   10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Arg Asn Thr Gly Arg Gly Gly Glu
        755                 760                 765

Glu Lys Lys Lys Glu Lys Glu Lys Glu Glu Gln Glu Glu Arg Glu Thr
    770                 775                 780

Lys Thr Pro Glu Cys Pro Ser His Thr Gln Pro Leu Gly Val Phe Leu
785                 790                 795                 800

Phe Pro Pro Lys Pro Lys Asp Thr Leu Met Ile Ser Arg Thr Pro Glu
                805                 810                 815

Val Thr Cys Val Val Val Asp Val Ser His Glu Asp Pro Glu Val Lys
            820                 825                 830

Phe Asn Trp Tyr Val Asp Gly Val Glu Val His Asn Ala Lys Thr Lys
        835                 840                 845

Pro Arg Glu Glu Gln Tyr Asn Ser Thr Tyr Arg Val Val Ser Val Leu
    850                 855                 860

Thr Val Leu His Gln Asp Trp Leu Asn Gly Lys Glu Tyr Lys Cys Lys
865                 870                 875                 880

Val Ser Asn Lys Ala Leu Pro Ala Pro Ile Glu Lys Thr Ile Ser Lys
                885                 890                 895

Ala Lys Gly Gln Pro Arg Glu Pro Gln Val Tyr Thr Leu Pro Pro Ser
            900                 905                 910

Arg Asp Glu Leu Thr Lys Asn Gln Val Ser Leu Thr Cys Leu Val Lys
        915                 920                 925

Gly Phe Tyr Pro Ser Asp Ile Ala Val Glu Trp Glu Ser Asn Gly Gln
    930                 935                 940

Pro Glu Asn Asn Tyr Lys Thr Thr Pro Pro Val Leu Asp Ser Asp Gly
945                 950                 955                 960

Ser Phe Phe Leu Tyr Ser Lys Leu Thr Val Asp Lys Ser Arg Trp Gln
                965                 970                 975

Gln Gly Asn Val Phe Ser Cys Ser Val Met His Glu Ala Leu His Asn
            980                 985                 990

His Tyr Thr Gln Lys Ser Leu Ser Leu Ser Pro Gly Lys
        995                 1000                1005


<210>   24
<211>   992
<212>   PRT
<213>   Artificial Sequence

<220>
<223>   fusion protein

<400>     24
Ala Gln Ala Gly Pro Arg Ala Pro Cys Ala Ala Ala Cys Thr Cys Ala
1               5                   10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Glu Pro Lys Ser Ser Asp Lys Thr
        755                 760                 765

His Thr Ser Pro Pro Ser Pro Ala Pro Glu Leu Leu Gly Gly Ser Ser
    770                 775                 780

Val Phe Leu Phe Pro Pro Lys Pro Lys Asp Thr Leu Met Ile Ser Arg
785                 790                 795                 800

Thr Pro Glu Val Thr Cys Val Val Val Asp Val Ser His Glu Asp Pro
                805                 810                 815

Glu Val Lys Phe Asn Trp Tyr Val Asp Gly Val Glu Val His Asn Ala
            820                 825                 830

Lys Thr Lys Pro Arg Glu Glu Gln Tyr Asn Ser Thr Tyr Arg Val Val
        835                 840                 845

Ser Val Leu Thr Val Leu His Gln Asp Trp Leu Asn Gly Lys Glu Tyr
    850                 855                 860

Lys Cys Lys Val Ser Asn Lys Ala Leu Pro Ala Pro Ile Glu Lys Thr
865                 870                 875                 880

Ile Ser Lys Ala Lys Gly Gln Pro Arg Glu Pro Gln Val Tyr Thr Leu
                885                 890                 895

Pro Pro Ser Arg Asp Glu Leu Thr Lys Asn Gln Val Ser Leu Thr Cys
            900                 905                 910

Leu Val Lys Gly Phe Tyr Pro Ser Asp Ile Ala Val Glu Trp Glu Ser
        915                 920                 925

Asn Gly Gln Pro Glu Asn Asn Tyr Lys Thr Thr Pro Pro Val Leu Asp
    930                 935                 940

Ser Asp Gly Ser Phe Phe Leu Tyr Ser Lys Leu Thr Val Asp Lys Ser
945                 950                 955                 960

Arg Trp Gln Gln Gly Asn Val Phe Ser Cys Ser Val Met His Glu Ala
                965                 970                 975

Leu His Asn His Tyr Thr Gln Lys Ser Leu Ser Leu Ser Pro Gly Lys
            980                 985                 990

<210>	25
<211>	2976
<212>	DNA
<213>	Artificial Sequence

<220>
<223>	fusion protein


<400>	25
gctcaggctg gacctagggc tccttgcgct gccgcctgca cctgtgcagg cgattctctg	60

gactgcagcg gccggggcct ggccacactg cccagggacc tgccttcctg gaccagatct	120

ctgaacctga gctacaatcg gctgtccgag atcgattctg ccgcctttga ggacctgaca	180

aatctgcagg aggtgtatct gaacagcaat gagctgaccg caatcccctc cctgggagca	240

gcctctatcg gcgtggtgag cctgttcctg cagcacaaca agatcctgag cgtggatggc	300

tcccagctga agagctacct gtctctggag gtgctggacc tgagctccaa caatatcacc	360

gagatcagat ctagctgttt tcctaatggc ctgcggatca gagagctgaa cctggcctct	420

aatcggatca gcatcctgga gtccggcgcc ttcgatggcc tgagcagatc cctgctgaca	480

ctgcgcctgt ccaagaaccg gatcacccag ctgcccgtga aggcctttaa gctgcctagg	540

ctgacacagc tggacctgaa ccggaataga atcaggctga tcgagggcct gaccttccag	600

ggcctggata gcctggaggt gctgcgcctg cagcggaaca atatctcccg cctgacagac	660

ggagcatttt ggggcctgtc taagatgcac gtgctgcacc tggagtacaa tagcctggtg	720

gaggtgaact ctggcagcct gtatggcctg accgccctgc accagctgca cctgtccaac	780

aatagcatca gcagaatcca gagggatggc tggtccttct gccagaagct gcacgagctg	840

atcctgtctt ttaacaatct gaccaggctg gacgaggaga gcctggcaga gctgtcctct	900

ctgtccatcc tgcgcctgtc tcacaatgcc atcagccaca tcgccgaggg cgcctttaag	960

ggcctgaaga gcctgagggt gctggatctg gaccacaacg agatctctgg caccatcgag	1020

gatacaagcg gcgccttcac aggcctggac aatctgtcca agctgaccct gtttggcaac	1080

aagatcaagt ctgtggccaa gcgggccttc tctggcctgg agagcctgga gcacctgaac	1140

ctgggcgaga atgccatcag atccgtgcag ttcgatgcct ttgccaagat gaagaatctg	1200

aaggagctgt acatcagctc cgagagcttc ctgtgcgact gtcagctgaa gtggctgcca	1260

ccttggctga tgggaaggat gctgcaggcc tttgtgaccg ccacatgcgc ccacccagag	1320

agcctgaagg gccagagcat cttctccgtg ctgcccgata gcttcgtgtg cgacgatttt	1380

cctaagccac agatcatcac ccagccagag acaacaatgg ccgtggtggg caaggacatc	1440

cggtttacat gttccgccgc ctctagctcc tctagcccca tgaccttcgc ctggaagaag	1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

gagccaaagt cctctgataa gacacacacc tctccaccat gcccagcacc agagctgctg 2340

ggaggaccaa gcgtgttcct gtttcctcca aagcccaagg acacactgat gatctccagg 2400

acaccagagg tgacctgcgt ggtggtggac gtgagccacg aggaccccga ggtgaagttc 2460

aactggtacg tggatggcgt ggaggtgcac aatgccaaga ccaagcccag agaggagcag 2520

tacaactcta cctatagggt ggtgagcgtg ctgacagtgc tgcaccagga ctggctgaac 2580

ggcaaggagt ataagtgcaa ggtgagcaat aaggccctgc ctgccccaat cgagaagaca 2640

atctccaagg ccaagggcca gccaagagag ccccaggtgt acaccctgcc ccctagcagg 2700

gatgagctga caaagaacca ggtgtccctg acctgtctgg tgaagggctt ttatccctcc 2760

gacatcgccg tggagtggga gtctaatggc cagcctgaga ataactacaa gacaacccca 2820

cccgtgctgg attctgacgg cagcttcttt ctgtattcta agctgaccgt ggacaagagc 2880

aggtggcagc agggcaacgt gttcagctgc tccgtgatgc acgaagcact gcacaatcac 2940

tacacccaga aatcactgtc actgagccct ggcaaa 2976

<210> 26
<211> 2979
<212> DNA
<213> Artificial Sequence

<220>
<223> fusion protein

<400> 26
gctcaggctg gacctagggc tccttgcgct gccgcctgca cctgtgcagg cgattctctg 60

gactgcagcg gccggggcct ggccacactg cccagggacc tgccttcctg gaccagatct 120

ctgaacctga gctacaatcg gctgtccgag atcgattctg ccgcctttga ggacctgaca 180

aatctgcagg aggtgtatct gaacagcaat gagctgaccg caatcccctc cctgggagca 240

gcctctatcg gcgtggtgag cctgttcctg cagcacaaca agatcctgag cgtggatggc 300

tcccagctga agagctacct gtctctggag gtgctggacc tgagctccaa caatatcacc 360

gagatcagat ctagctgttt tcctaatggc ctgcggatca gagagctgaa cctggcctct 420

aatcggatca gcatcctgga gtccggcgcc ttcgatggcc tgagcagatc cctgctgaca 480

ctgcgcctgt ccaagaaccg gatcacccag ctgcccgtga aggcctttaa gctgcctagg 540

ctgacacagc tggacctgaa ccggaataga atcaggctga tcgagggcct gaccttccag 600

ggcctggata gcctggaggt gctgcgcctg cagcggaaca atatctcccg cctgacagac 660

ggagcatttt ggggcctgtc taagatgcac gtgctgcacc tggagtacaa tagcctggtg 720

gaggtgaact ctggcagcct gtatggcctg accgccctgc accagctgca cctgtccaac 780

aatagcatca gcagaatcca gagggatggc tggtccttct gccagaagct gcacgagctg 840

atcctgtctt ttaacaatct gaccaggctg gacgaggaga gcctggcaga gctgtcctct 900

ctgtccatcc tgcgcctgtc tcacaatgcc atcagccaca tcgccgaggg cgcctttaag 960

ggcctgaaga gcctgagggt gctggatctg gaccacaacg agatctctgg caccatcgag 1020

gatacaagcg gcgccttcac aggcctggac aatctgtcca agctgaccct gtttggcaac 1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

gagcctcggg gccctaccat caagccctgc cccccttgca agtgccctgc ccctaatctg 2340

ctgggcggac cctccgtgtt cctgtttcct ccaaagccca aggacacact gatgatctcc 2400

aggacaccag aggtgacctg cgtggtggtg gacgtgagcc acgaggaccc cgaggtgaag 2460

ttcaactggt acgtggatgg cgtggaggtg cacaatgcca agaccaagcc cagagaggag 2520

cagtacaact ctacctatag ggtggtgagc gtgctgacag tgctgcacca ggactggctg 2580

aacggcaagg agtataagtg caaggtgagc aataaggccc tgcctgcccc aatcgagaag 2640

acaatctcca aggccaaggg ccagccaaga gagccccagg tgtacaccct gccccctagc 2700

agggatgagc tgacaaagaa ccaggtgtcc ctgacctgtc tggtgaaggg cttttatccc 2760

tccgacatcg ccgtggagtg ggagtctaat ggccagcctg agaataacta caagacaacc 2820

ccacccgtgc tggattctga cggcagcttc tttctgtatt ctaagctgac cgtggacaag 2880

agcaggtggc agcagggcaa cgtgttcagc tgctccgtga tgcacgaagc actgcacaat 2940

cactacaccc agaaatcact gtcactgagc cctggcaaa 2979

<210> 27
<211> 3015
<212> DNA
<213> Artificial Sequence

<220>
<223> fusion protein

<400> 27
gctcaggctg gacctagggc tccttgcgct gccgcctgca cctgtgcagg cgattctctg 60

gactgcagcg gccggggcct ggccacactg cccagggacc tgccttcctg gaccagatct 120

ctgaacctga gctacaatcg gctgtccgag atcgattctg ccgcctttga ggacctgaca 180

aatctgcagg aggtgtatct gaacagcaat gagctgaccg caatcccctc cctgggagca 240

gcctctatcg gcgtggtgag cctgttcctg cagcacaaca agatcctgag cgtggatggc 300

tcccagctga agagctacct gtctctggag gtgctggacc tgagctccaa caatatcacc 360

gagatcagat ctagctgttt tcctaatggc ctgcggatca gagagctgaa cctggcctct 420

aatcggatca gcatcctgga gtccggcgcc ttcgatggcc tgagcagatc cctgctgaca 480

ctgcgcctgt ccaagaaccg gatcacccag ctgcccgtga aggcctttaa gctgcctagg 540

ctgacacagc tggacctgaa ccggaataga atcaggctga tcgagggcct gaccttccag 600

ggcctggata gcctggaggt gctgcgcctg cagcggaaca atatctcccg cctgacagac 660

ggagcatttt ggggcctgtc taagatgcac gtgctgcacc tggagtacaa tagcctggtg 720

gaggtgaact ctggcagcct gtatggcctg accgccctgc accagctgca cctgtccaac 780

aatagcatca gcagaatcca gagggatggc tggtccttct gccagaagct gcacgagctg 840

atcctgtctt ttaacaatct gaccaggctg gacgaggaga gcctggcaga gctgtcctct 900

ctgtccatcc tgcgcctgtc tcacaatgcc atcagccaca tcgccgaggg cgcctttaag 960

ggcctgaaga gcctgagggt gctggatctg gaccacaacg agatctctgg caccatcgag 1020

gatacaagcg gcgccttcac aggcctggac aatctgtcca agctgaccct gtttggcaac 1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

cgcaacaccg gccgcggcgg cgaggagaag aagaaggaga aggagaagga ggagcaggag 2340

gagcgcgaga ccaagacccc cgagtgcccc agccacaccc agcccctggg cgtgttcctg 2400

tttcctccaa agcccaagga cacactgatg atctccagga caccagaggt gacctgcgtg 2460

gtggtggacg tgagccacga ggaccccgag gtgaagttca actggtacgt ggatggcgtg 2520

gaggtgcaca atgccaagac caagcccaga gaggagcagt acaactctac ctatagggtg 2580

gtgagcgtgc tgacagtgct gcaccaggac tggctgaacg gcaaggagta taagtgcaag 2640

gtgagcaata aggccctgcc tgccccaatc gagaagacaa tctccaaggc caagggccag 2700

ccaagagagc cccaggtgta caccctgccc cctagcaggg atgagctgac aaagaaccag 2760

gtgtccctga cctgtctggt gaagggcttt tatccctccg acatcgccgt ggagtgggag 2820

tctaatggcc agcctgagaa taactacaag acaaccccac ccgtgctgga ttctgacggc 2880

agcttctttc tgtattctaa gctgaccgtg gacaagagca ggtggcagca gggcaacgtg 2940

ttcagctgct ccgtgatgca cgaagcactg cacaatcact acacccagaa atcactgtca 3000

ctgagccctg gcaaa 3015

<210> 28
<211> 2976
<212> DNA
<213> Artificial Sequence

<220>
<223> fusion protein

<400> 28
gctcaggctg gacctagggc tccttgcgct gccgcctgca cctgtgcagg cgattctctg 60

gactgcagcg gccggggcct ggccacactg cccagggacc tgccttcctg gaccagatct 120

ctgaacctga gctacaatcg gctgtccgag atcgattctg ccgcctttga ggacctgaca 180

aatctgcagg aggtgtatct gaacagcaat gagctgaccg caatcccctc cctgggagca 240

gcctctatcg gcgtggtgag cctgttcctg cagcacaaca agatcctgag cgtggatggc 300

tcccagctga agagctacct gtctctggag gtgctggacc tgagctccaa caatatcacc 360

gagatcagat ctagctgttt tcctaatggc ctgcggatca gagagctgaa cctggcctct 420

aatcggatca gcatcctgga gtccggcgcc ttcgatggcc tgagcagatc cctgctgaca 480

ctgcgcctgt ccaagaaccg gatcacccag ctgcccgtga aggcctttaa gctgcctagg 540

ctgacacagc tggacctgaa ccggaataga atcaggctga tcgagggcct gaccttccag 600

ggcctggata gcctggaggt gctgcgcctg cagcggaaca atatctcccg cctgacagac 660

ggagcatttt ggggcctgtc taagatgcac gtgctgcacc tggagtacaa tagcctggtg 720

gaggtgaact ctggcagcct gtatggcctg accgccctgc accagctgca cctgtccaac 780

aatagcatca gcagaatcca gagggatggc tggtccttct gccagaagct gcacgagctg 840

atcctgtctt ttaacaatct gaccaggctg gacgaggaga gcctggcaga gctgtcctct 900

ctgtccatcc tgcgcctgtc tcacaatgcc atcagccaca tcgccgaggg cgcctttaag 960

ggcctgaaga gcctgagggt gctggatctg gaccacaacg agatctctgg caccatcgag 1020

gatacaagcg gcgccttcac aggcctggac aatctgtcca agctgaccct gtttggcaac 1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

gaaccgaaat cttctgacaa aacccacacc tctccgccgt ctccggctcc ggaactgctg 2340

ggtggttctt ctgttttcct gtttcctcca aagcccaagg acacactgat gatctccagg 2400

acaccagagg tgacctgcgt ggtggtggac gtgagccacg aggaccccga ggtgaagttc 2460

aactggtacg tggatggcgt ggaggtgcac aatgccaaga ccaagcccag agaggagcag 2520

tacaactcta cctatagggt ggtgagcgtg ctgacagtgc tgcaccagga ctggctgaac 2580

ggcaaggagt ataagtgcaa ggtgagcaat aaggccctgc ctgccccaat cgagaagaca 2640

atctccaagg ccaagggcca gccaagagag ccccaggtgt acaccctgcc ccctagcagg 2700

gatgagctga caaagaacca ggtgtccctg acctgtctgg tgaagggctt ttatccctcc 2760

gacatcgccg tggagtggga gtctaatggc cagcctgaga ataactacaa gacaacccca 2820

cccgtgctgg attctgacgg cagcttcttt ctgtattcta agctgaccgt ggacaagagc 2880

aggtggcagc agggcaacgt gttcagctgc tccgtgatgc acgaagcact gcacaatcac 2940

tacacccaga aatcactgtc actgagccct ggcaaa 2976

<210> 29
<211> 992
<212> PRT
<213> Artificial Sequence

<220>
<223> fusion protein

<400> 29
Ala Gln Ala Gly Pro Arg Ala Pro Cys Ala Ala Ala Cys Thr Cys Ala
1               5                   10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Glu Pro Lys Ser Ser Asp Lys Thr
        755                 760                 765

His Thr Ser Pro Pro Cys Pro Ala Pro Glu Leu Leu Gly Gly Pro Ser
    770                 775                 780

Val Phe Ile Phe Pro Pro Lys Ile Lys Asp Val Leu Met Ile Ser Leu
785                 790                 795                 800

Ser Pro Ile Val Thr Cys Val Val Val Asp Val Ser Glu Asp Asp Pro
                805                 810                 815

Asp Val Gln Ile Ser Trp Phe Val Asn Asn Val Glu Val His Thr Ala
            820                 825                 830

Gln Thr Gln Thr His Arg Glu Asp Tyr Asn Ser Thr Leu Arg Val Val
        835                 840                 845

Ser Ala Leu Pro Ile Gln His Gln Asp Trp Met Ser Gly Lys Glu Phe
    850                 855                 860

Lys Cys Lys Val Asn Asn Lys Asp Leu Pro Ala Pro Ile Glu Arg Thr
865                 870                 875                 880

Ile Ser Lys Pro Lys Gly Ser Val Arg Ala Pro Gln Val Tyr Val Leu
                885                 890                 895

Pro Pro Pro Glu Glu Glu Met Thr Lys Lys Gln Val Thr Leu Thr Cys
            900                 905                 910

Met Val Thr Asp Phe Met Pro Glu Asp Ile Tyr Val Glu Trp Thr Asn
        915                 920                 925

Asn Gly Lys Thr Glu Leu Asn Tyr Lys Asn Thr Glu Pro Val Leu Asp
    930                 935                 940

Ser Asp Gly Ser Tyr Phe Met Tyr Ser Lys Leu Arg Val Glu Lys Lys
945                 950                 955                 960

Asn Trp Val Glu Arg Asn Ser Tyr Ser Cys Ser Val Val His Glu Gly
                965                 970                 975

Leu His Asn His His Thr Thr Lys Ser Phe Ser Arg Thr Pro Gly Lys
            980                 985                 990

<210>    30
<211>    993
<212>    PRT
<213>    Artificial Sequence

<220>
<223>    fusion protein

<400>    30
Ala Gln Ala Gly Pro Arg Ala Pro Cys Ala Ala Ala Cys Thr Cys Ala
 1               5                  10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Glu Pro Arg Gly Pro Thr Ile Lys
        755                 760                 765

Pro Cys Pro Pro Cys Lys Cys Pro Ala Pro Asn Leu Leu Gly Gly Pro
    770                 775                 780

Ser Val Phe Ile Phe Pro Pro Lys Ile Lys Asp Val Leu Met Ile Ser
785                 790                 795                 800

Leu Ser Pro Ile Val Thr Cys Val Val Val Asp Val Ser Glu Asp Asp
                805                 810                 815

Pro Asp Val Gln Ile Ser Trp Phe Val Asn Asn Val Glu Val His Thr
            820                 825                 830

Ala Gln Thr Gln Thr His Arg Glu Asp Tyr Asn Ser Thr Leu Arg Val
        835                 840                 845

Val Ser Ala Leu Pro Ile Gln His Gln Asp Trp Met Ser Gly Lys Glu
    850                 855                 860

Phe Lys Cys Lys Val Asn Asn Lys Asp Leu Pro Ala Pro Ile Glu Arg
865                 870                 875                 880

Thr Ile Ser Lys Pro Lys Gly Ser Val Arg Ala Pro Gln Val Tyr Val
                885                 890                 895

Leu Pro Pro Pro Glu Glu Glu Met Thr Lys Lys Gln Val Thr Leu Thr
            900                 905                 910

Cys Met Val Thr Asp Phe Met Pro Glu Asp Ile Tyr Val Glu Trp Thr
        915                 920                 925

Asn Asn Gly Lys Thr Glu Leu Asn Tyr Lys Asn Thr Glu Pro Val Leu
    930                 935                 940

Asp Ser Asp Gly Ser Tyr Phe Met Tyr Ser Lys Leu Arg Val Glu Lys
945                 950                 955                 960

Lys Asn Trp Val Glu Arg Asn Ser Tyr Ser Cys Ser Val Val His Glu
                965                 970                 975

Gly Leu His Asn His His Thr Thr Lys Ser Phe Ser Arg Thr Pro Gly
            980                 985                 990

Lys

<210>    31
<211>    1005
<212>    PRT
<213>    Artificial Sequence

<220>
<223>    fusion protein

<400>    31
Ala Gln Ala Gly Pro Arg Ala Pro Cys Ala Ala Ala Cys Thr Cys Ala
 1               5                  10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Arg Asn Thr Gly Arg Gly Gly Glu
        755                 760                 765

Glu Lys Lys Lys Glu Lys Glu Lys Glu Glu Gln Glu Glu Arg Glu Thr
    770                 775                 780

Lys Thr Pro Glu Cys Pro Ser His Thr Gln Pro Leu Gly Val Phe Ile
785                 790                 795                 800

Phe Pro Pro Lys Ile Lys Asp Val Leu Met Ile Ser Leu Ser Pro Ile
                805                 810                 815

Val Thr Cys Val Val Val Asp Val Ser Glu Asp Asp Pro Asp Val Gln
            820                 825                 830

Ile Ser Trp Phe Val Asn Asn Val Glu Val His Thr Ala Gln Thr Gln
        835                 840                 845

Thr His Arg Glu Asp Tyr Asn Ser Thr Leu Arg Val Val Ser Ala Leu
    850                 855                 860

Pro Ile Gln His Gln Asp Trp Met Ser Gly Lys Glu Phe Lys Cys Lys
865                 870                 875                 880

Val Asn Asn Lys Asp Leu Pro Ala Pro Ile Glu Arg Thr Ile Ser Lys
                885                 890                 895

Pro Lys Gly Ser Val Arg Ala Pro Gln Val Tyr Val Leu Pro Pro Pro
            900                 905                 910

Glu Glu Glu Met Thr Lys Lys Gln Val Thr Leu Thr Cys Met Val Thr
        915                 920                 925

Asp Phe Met Pro Glu Asp Ile Tyr Val Glu Trp Thr Asn Asn Gly Lys
    930                 935                 940

Thr Glu Leu Asn Tyr Lys Asn Thr Glu Pro Val Leu Asp Ser Asp Gly
945                 950                 955                 960

Ser Tyr Phe Met Tyr Ser Lys Leu Arg Val Glu Lys Lys Asn Trp Val
                965                 970                 975

Glu Arg Asn Ser Tyr Ser Cys Ser Val Val His Glu Gly Leu His Asn
            980                 985                 990

His His Thr Thr Lys Ser Phe Ser Arg Thr Pro Gly Lys
        995                 1000                1005


<210>   32
<211>   992
<212>   PRT
<213>   Artificial Sequence

<220>
<223>   fusion protein


<400>   32
Ala Gln Ala Gly Pro Arg Ala Pro Cys Ala Ala Ala Cys Thr Cys Ala
1               5                   10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala

65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu

405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro

740                 745                 750

Gly Cys Arg Lys Asp Gly Thr Thr Glu Pro Lys Ser Ser Asp Lys Thr
        755                 760                 765

His Thr Ser Pro Pro Ser Pro Ala Pro Glu Leu Leu Gly Gly Ser Ser
    770                 775                 780

Val Phe Ile Phe Pro Pro Lys Ile Lys Asp Val Leu Met Ile Ser Leu
785                 790                 795                 800

Ser Pro Ile Val Thr Cys Val Val Val Asp Val Ser Glu Asp Asp Pro
                805                 810                 815

Asp Val Gln Ile Ser Trp Phe Val Asn Asn Val Glu Val His Thr Ala
            820                 825                 830

Gln Thr Gln Thr His Arg Glu Asp Tyr Asn Ser Thr Leu Arg Val Val
        835                 840                 845

Ser Ala Leu Pro Ile Gln His Gln Asp Trp Met Ser Gly Lys Glu Phe
    850                 855                 860

Lys Cys Lys Val Asn Asn Lys Asp Leu Pro Ala Pro Ile Glu Arg Thr
865                 870                 875                 880

Ile Ser Lys Pro Lys Gly Ser Val Arg Ala Pro Gln Val Tyr Val Leu
                885                 890                 895

Pro Pro Pro Glu Glu Glu Met Thr Lys Lys Gln Val Thr Leu Thr Cys
            900                 905                 910

Met Val Thr Asp Phe Met Pro Glu Asp Ile Tyr Val Glu Trp Thr Asn
        915                 920                 925

Asn Gly Lys Thr Glu Leu Asn Tyr Lys Asn Thr Glu Pro Val Leu Asp
    930                 935                 940

Ser Asp Gly Ser Tyr Phe Met Tyr Ser Lys Leu Arg Val Glu Lys Lys
945                 950                 955                 960

Asn Trp Val Glu Arg Asn Ser Tyr Ser Cys Ser Val Val His Glu Gly
                965                 970                 975

Leu His Asn His His Thr Thr Lys Ser Phe Ser Arg Thr Pro Gly Lys
            980                 985                 990

<210>    33
<211>    2976
<212>    DNA
<213>    Artificial Sequence

<220>
<223>    fusion protein

<400>    33
gctcaggctg gacctagggc tccttgcgct gccgcctgca cctgtgcagg cgattctctg        60

gactgcagcg gccggggcct ggccacactg cccagggacc tgccttcctg gaccagatct       120

ctgaacctga gctacaatcg gctgtccgag atcgattctg ccgcctttga ggacctgaca 180

aatctgcagg aggtgtatct gaacagcaat gagctgaccg caatcccctc cctgggagca 240

gcctctatcg gcgtggtgag cctgttcctg cagcacaaca agatcctgag cgtggatggc 300

tcccagctga agagctacct gtctctggag gtgctggacc tgagctccaa caatatcacc 360

gagatcagat ctagctgttt tcctaatggc ctgcggatca gagagctgaa cctggcctct 420

aatcggatca gcatcctgga gtccggcgcc ttcgatggcc tgagcagatc cctgctgaca 480

ctgcgcctgt ccaagaaccg gatcacccag ctgcccgtga aggcctttaa gctgcctagg 540

ctgacacagc tggacctgaa ccggaataga atcaggctga tcgagggcct gaccttccag 600

ggcctggata gcctggaggt gctgcgcctg cagcggaaca atatctcccg cctgacagac 660

ggagcatttt ggggcctgtc taagatgcac gtgctgcacc tggagtacaa tagcctggtg 720

gaggtgaact ctggcagcct gtatggcctg accgccctgc accagctgca cctgtccaac 780

aatagcatca gcagaatcca gagggatggc tggtccttct gccagaagct gcacgagctg 840

atcctgtctt ttaacaatct gaccaggctg gacgaggaga gcctggcaga gctgtcctct 900

ctgtccatcc tgcgcctgtc tcacaatgcc atcagccaca tcgccgaggg cgcctttaag 960

ggcctgaaga gcctgagggt gctggatctg gaccacaacg agatctctgg caccatcgag 1020

gatacaagcg gcgccttcac aggcctggac aatctgtcca agctgaccct gtttggcaac 1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

gagccaaagt cctctgataa gacacacacc tctccaccat gcccagcacc agagctgctg 2340

ggaggaccaa gcgtgttcat cttcccaccc aagatcaagg acgtgctgat gatctccctg 2400

tcccccatcg tgacctgcgt ggtggtggac gtgtccgagg acgaccccga cgtgcagatc 2460

agttggttcg tgaacaacgt ggaagtgcac accgcccaga cccagaccca cagagaggac 2520

tacaactcca ccctgcgggt ggtgtccgcc ctgcccatcc agcaccagga ctggatgtcc 2580

ggcaaagaat tcaagtgcaa agtgaacaac aaggacctgc ctgcccccat cgagcggacc 2640

atctccaagc ccaagggctc cgtgcgggct ccccaggtgt acgtgctgcc ccctccagag 2700

gaagagatga ccaagaagca ggtcacactg acctgcatgg tcaccgactt catgcccgag 2760

gacatctacg tggaatggac caacaatggc aagaccgagc tgaactacaa gaacaccgag 2820

cctgtgctgg actccgacgg ctcctacttc atgtactcca agctgcgggt ggaaaagaag 2880

aactgggtcg agcggaactc ctactcctgc tccgtggtgc acgagggcct gcacaaccac 2940

cacaccacca agtccttctc ccggaccccc ggcaaa 2976

<210> 34
<211> 2979
<212> DNA
<213> Artificial Sequence

<220>
<223> fusion protein


<400> 34
gctcaggctg gacctagggc tccttgcgct gccgcctgca cctgtgcagg cgattctctg 60

gactgcagcg gccggggcct ggccacactg cccagggacc tgccttcctg gaccagatct 120

ctgaacctga gctacaatcg gctgtccgag atcgattctg ccgcctttga ggacctgaca 180

aatctgcagg aggtgtatct gaacagcaat gagctgaccg caatcccctc cctgggagca 240

gcctctatcg gcgtggtgag cctgttcctg cagcacaaca agatcctgag cgtggatggc 300

tcccagctga agagctacct gtctctggag gtgctggacc tgagctccaa caatatcacc 360

gagatcagat ctagctgttt tcctaatggc ctgcggatca gagagctgaa cctggcctct 420

aatcggatca gcatcctgga gtccggcgcc ttcgatggcc tgagcagatc cctgctgaca 480

ctgcgcctgt ccaagaaccg gatcacccag ctgcccgtga aggcctttaa gctgcctagg 540

ctgacacagc tggacctgaa ccggaataga atcaggctga tcgagggcct gaccttccag 600

ggcctggata gcctggaggt gctgcgcctg cagcggaaca atatctcccg cctgacagac 660

ggagcatttt ggggcctgtc taagatgcac gtgctgcacc tggagtacaa tagcctggtg 720

gaggtgaact ctggcagcct gtatggcctg accgccctgc accagctgca cctgtccaac 780

aatagcatca gcagaatcca gagggatggc tggtccttct gccagaagct gcacgagctg 840

atcctgtctt ttaacaatct gaccaggctg gacgaggaga gcctggcaga gctgtcctct 900

ctgtccatcc tgcgcctgtc tcacaatgcc atcagccaca tcgccgaggg cgcctttaag 960

ggcctgaaga gcctgagggt gctggatctg gaccacaacg agatctctgg caccatcgag 1020

gatacaagcg gcgccttcac aggcctggac aatctgtcca agctgaccct gtttggcaac 1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

gagcctcggg gccctaccat caagccctgc cccccttgca agtgccctgc ccctaatctg 2340

ctgggcggac cctccgtgtt catcttccca cccaagatca aggacgtgct gatgatctcc 2400

ctgtccccca tcgtgacctg cgtggtggtg gacgtgtccg aggacgaccc cgacgtgcag 2460

atcagttggt tcgtgaacaa cgtggaagtg cacaccgccc agacccagac ccacagagag 2520

gactacaact ccaccctgcg ggtggtgtcc gccctgccca tccagcacca ggactggatg 2580

tccggcaaag aattcaagtg caaagtgaac aacaaggacc tgcctgcccc catcgagcgg 2640

accatctcca agcccaaggg ctccgtgcgg gctccccagg tgtacgtgct gccccctcca 2700

gaggaagaga tgaccaagaa gcaggtcaca ctgacctgca tggtcaccga cttcatgccc 2760

gaggacatct acgtggaatg gaccaacaat ggcaagaccg agctgaacta caagaacacc 2820

gagcctgtgc tggactccga cggctcctac ttcatgtact ccaagctgcg ggtggaaaag 2880

aagaactggg tcgagcggaa ctcctactcc tgctccgtgg tgcacgaggg cctgcacaac 2940

caccacacca ccaagtcctt ctcccggacc cccggcaaa 2979

<210> 35
<211> 3015
<212> DNA
<213> Artificial Sequence

<220>
<223> fusion protein

<400> 35
gctcaggctg gacctagggc tccttgcgct gccgcctgca cctgtgcagg cgattctctg 60

gactgcagcg gccggggcct ggccacactg cccagggacc tgccttcctg gaccagatct 120

ctgaacctga gctacaatcg gctgtccgag atcgattctg ccgcctttga ggacctgaca 180

aatctgcagg aggtgtatct gaacagcaat gagctgaccg caatcccctc cctgggagca 240

gcctctatcg gcgtggtgag cctgttcctg cagcacaaca agatcctgag cgtggatggc 300

tcccagctga agagctacct gtctctggag gtgctggacc tgagctccaa caatatcacc 360

gagatcagat ctagctgttt tcctaatggc ctgcggatca gagagctgaa cctggcctct 420

aatcggatca gcatcctgga gtccggcgcc ttcgatggcc tgagcagatc cctgctgaca 480

ctgcgcctgt ccaagaaccg gatcacccag ctgcccgtga aggcctttaa gctgcctagg 540

ctgacacagc tggacctgaa ccggaataga atcaggctga tcgagggcct gaccttccag 600

ggcctggata gcctggaggt gctgcgcctg cagcggaaca atatctcccg cctgacagac 660

ggagcatttt ggggcctgtc taagatgcac gtgctgcacc tggagtacaa tagcctggtg 720

gaggtgaact ctggcagcct gtatggcctg accgccctgc accagctgca cctgtccaac 780

aatagcatca gcagaatcca gagggatggc tggtccttct gccagaagct gcacgagctg 840

atcctgtctt ttaacaatct gaccaggctg gacgaggaga gcctggcaga gctgtcctct 900

ctgtccatcc tgcgcctgtc tcacaatgcc atcagccaca tcgccgaggg cgcctttaag 960

ggcctgaaga gcctgagggt gctggatctg gaccacaacg agatctctgg caccatcgag 1020

gatacaagcg gcgccttcac aggcctggac aatctgtcca agctgaccct gtttggcaac 1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

cgcaacaccg gccgcggcgg cgaggagaag aagaaggaga aggagaagga ggagcaggag 2340

gagcgcgaga ccaagacccc cgagtgcccc agccacaccc agcccctggg cgtgttcatc 2400

ttcccaccca agatcaagga cgtgctgatg atctccctgt cccccatcgt gacctgcgtg 2460

gtggtggacg tgtccgagga cgaccccgac gtgcagatca gttggttcgt gaacaacgtg 2520

gaagtgcaca ccgcccagac ccagacccac agagaggact acaactccac cctgcgggtg 2580

gtgtccgccc tgcccatcca gcaccaggac tggatgtccg gcaaagaatt caagtgcaaa 2640

gtgaacaaca aggacctgcc tgcccccatc gagcggacca tctccaagcc caagggctcc 2700

gtgcgggctc cccaggtgta cgtgctgccc cctccagagg aagagatgac caagaagcag 2760

gtcacactga cctgcatggt caccgacttc atgcccgagg acatctacgt ggaatggacc 2820

aacaatggca agaccgagct gaactacaag aacaccgagc ctgtgctgga ctccgacggc 2880

tcctacttca tgtactccaa gctgcgggtg gaaaagaaga actgggtcga gcggaactcc 2940

tactcctgct ccgtggtgca cgagggcctg cacaaccacc acaccaccaa gtccttctcc 3000

cggacccccg gcaaa 3015

<210> 36
<211> 2976
<212> DNA
<213> Artificial Sequence

<220>
<223> fusion protein

<400> 36
gctcaggctg gacctagggc tccttgcgct gccgcctgca cctgtgcagg cgattctctg 60

gactgcagcg gccggggcct ggccacactg cccagggacc tgccttcctg gaccagatct 120

ctgaacctga gctacaatcg gctgtccgag atcgattctg ccgcctttga ggacctgaca 180

aatctgcagg aggtgtatct gaacagcaat gagctgaccg caatcccctc cctgggagca 240

gcctctatcg gcgtggtgag cctgttcctg cagcacaaca agatcctgag cgtggatggc 300

tcccagctga agagctacct gtctctggag gtgctggacc tgagctccaa caatatcacc 360

gagatcagat ctagctgttt tcctaatggc ctgcggatca gagagctgaa cctggcctct 420

aatcggatca gcatcctgga gtccggcgcc ttcgatggcc tgagcagatc cctgctgaca 480

ctgcgcctgt ccaagaaccg gatcacccag ctgcccgtga aggcctttaa gctgcctagg 540

ctgacacagc tggacctgaa ccggaataga atcaggctga tcgagggcct gaccttccag 600

ggcctggata gcctggaggt gctgcgcctg cagcggaaca atatctcccg cctgacagac 660

ggagcatttt ggggcctgtc taagatgcac gtgctgcacc tggagtacaa tagcctggtg 720

gaggtgaact ctggcagcct gtatggcctg accgccctgc accagctgca cctgtccaac 780

aatagcatca gcagaatcca gagggatggc tggtccttct gccagaagct gcacgagctg 840

atcctgtctt ttaacaatct gaccaggctg gacgaggaga gcctggcaga gctgtcctct 900

ctgtccatcc tgcgcctgtc tcacaatgcc atcagccaca tcgccgaggg cgcctttaag 960

ggcctgaaga gcctgagggt gctggatctg gaccacaacg agatctctgg caccatcgag 1020

gatacaagcg gcgccttcac aggcctggac aatctgtcca agctgaccct gtttggcaac 1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

gaaccgaaat cttctgacaa aacccacacc tctccgccgt ctccggctcc ggaactgctg 2340

ggtggttctt ctgttttcat cttcccaccc aagatcaagg acgtgctgat gatctccctg 2400

tcccccatcg tgacctgcgt ggtggtggac gtgtccgagg acgaccccga cgtgcagatc 2460

agttggttcg tgaacaacgt ggaagtgcac accgcccaga cccagaccca cagagaggac 2520

tacaactcca ccctgcgggt ggtgtccgcc ctgcccatcc agcaccagga ctggatgtcc 2580

ggcaaagaat tcaagtgcaa agtgaacaac aaggacctgc ctgcccccat cgagcggacc 2640

atctccaagc ccaagggctc cgtgcgggct ccccaggtgt acgtgctgcc ccctccagag 2700

gaagagatga ccaagaagca ggtcacactg acctgcatgg tcaccgactt catgcccgag 2760

gacatctacg tggaatggac caacaatggc aagaccgagc tgaactacaa gaacaccgag 2820

cctgtgctgg actccgacgg ctcctacttc atgtactcca agctgcgggt ggaaaagaag 2880

aactgggtcg agcggaactc ctactcctgc tccgtggtgc acgagggcct gcacaaccac 2940

cacaccacca agtccttctc ccggaccccc ggcaaa 2976

<210> 37
<211> 996
<212> PRT
<213> Artificial Sequence

<220>
<223> fusion protein

<400>    37
Ala Gly Pro Arg Ala Pro Cys Ala Ala Ala Cys Thr Cys Ala Gly Asp
1               5                   10                  15

Ser Leu Asp Cys Gly Gly Arg Gly Leu Ala Ala Leu Pro Gly Asp Leu
            20                  25                  30

Pro Ser Trp Thr Arg Ser Leu Asn Leu Ser Tyr Asn Lys Leu Ser Glu
        35                  40                  45

Ile Asp Pro Ala Gly Phe Glu Asp Leu Pro Asn Leu Gln Glu Val Tyr
    50                  55                  60

Leu Asn Asn Asn Glu Leu Thr Ala Val Pro Ser Leu Gly Ala Ala Ser
65                  70                  75                  80

Ser His Val Val Ser Leu Phe Leu Gln His Asn Lys Ile Arg Ser Val
                85                  90                  95

Glu Gly Ser Gln Leu Lys Ala Tyr Leu Ser Leu Glu Val Leu Asp Leu
            100                 105                 110

Ser Leu Asn Asn Ile Thr Glu Val Arg Asn Thr Cys Phe Pro His Gly
        115                 120                 125

Pro Pro Ile Lys Glu Leu Asn Leu Ala Gly Asn Arg Ile Gly Thr Leu
    130                 135                 140

Glu Leu Gly Ala Phe Asp Gly Leu Ser Arg Ser Leu Leu Thr Leu Arg
145                 150                 155                 160

Leu Ser Lys Asn Arg Ile Thr Gln Leu Pro Val Arg Ala Phe Lys Leu
                165                 170                 175

Pro Arg Leu Thr Gln Leu Asp Leu Asn Arg Asn Arg Ile Arg Leu Ile
            180                 185                 190

Glu Gly Leu Thr Phe Gln Gly Leu Asn Ser Leu Glu Val Leu Lys Leu
        195                 200                 205

Gln Arg Asn Asn Ile Ser Lys Leu Thr Asp Gly Ala Phe Trp Gly Leu
    210                 215                 220

Ser Lys Met His Val Leu His Leu Glu Tyr Asn Ser Leu Val Glu Val
225                 230                 235                 240

Asn Ser Gly Ser Leu Tyr Gly Leu Thr Ala Leu His Gln Leu His Leu
                245                 250                 255

Ser Asn Asn Ser Ile Ala Arg Ile His Arg Lys Gly Trp Ser Phe Cys
            260                 265                 270

Gln Lys Leu His Glu Leu Val Leu Ser Phe Asn Asn Leu Thr Arg Leu
        275                 280                 285

Asp Glu Glu Ser Leu Ala Glu Leu Ser Ser Leu Ser Val Leu Arg Leu
    290                 295                 300

Ser His Asn Ser Ile Ser His Ile Ala Glu Gly Ala Phe Lys Gly Leu
305                 310                 315                 320

Arg Ser Leu Arg Val Leu Asp Leu Asp His Asn Glu Ile Ser Gly Thr

325                 330                 335

Ile Glu Asp Thr Ser Gly Ala Phe Ser Gly Leu Asp Ser Leu Ser Lys
            340                 345                 350

Leu Thr Leu Phe Gly Asn Lys Ile Lys Ser Val Ala Lys Arg Ala Phe
        355                 360                 365

Ser Gly Leu Glu Gly Leu Glu His Leu Asn Leu Gly Gly Asn Ala Ile
    370                 375                 380

Arg Ser Val Gln Phe Asp Ala Phe Val Lys Met Lys Asn Leu Lys Glu
385                 390                 395                 400

Leu His Ile Ser Ser Asp Ser Phe Leu Cys Asp Cys Gln Leu Lys Trp
                405                 410                 415

Leu Pro Pro Trp Leu Ile Gly Arg Met Leu Gln Ala Phe Val Thr Ala
            420                 425                 430

Thr Cys Ala His Pro Glu Ser Leu Lys Gly Gln Ser Ile Phe Ser Val
        435                 440                 445

Pro Pro Glu Ser Phe Val Cys Asp Asp Phe Leu Lys Pro Gln Ile Ile
    450                 455                 460

Thr Gln Pro Glu Thr Thr Met Ala Met Val Gly Lys Asp Ile Arg Phe
465                 470                 475                 480

Thr Cys Ser Ala Ala Ser Ser Ser Ser Ser Pro Met Thr Phe Ala Trp
                485                 490                 495

Lys Lys Asp Asn Glu Val Leu Thr Asn Ala Asp Met Glu Asn Phe Val
            500                 505                 510

His Val His Ala Gln Asp Gly Glu Val Met Glu Tyr Thr Thr Ile Leu
        515                 520                 525

His Leu Arg Gln Val Thr Phe Gly His Glu Gly Arg Tyr Gln Cys Val
    530                 535                 540

Ile Thr Asn His Phe Gly Ser Thr Tyr Ser His Lys Ala Arg Leu Thr
545                 550                 555                 560

Val Asn Val Leu Pro Ser Phe Thr Lys Thr Pro His Asp Ile Thr Ile
                565                 570                 575

Arg Thr Thr Thr Val Ala Arg Leu Glu Cys Ala Ala Thr Gly His Pro
            580                 585                 590

Asn Pro Gln Ile Ala Trp Gln Lys Asp Gly Gly Thr Asp Phe Pro Ala
        595                 600                 605

Ala Arg Glu Arg Arg Met His Val Met Pro Asp Asp Asp Val Phe Phe
    610                 615                 620

Ile Thr Asp Val Lys Ile Asp Asp Ala Gly Val Tyr Ser Cys Thr Ala
625                 630                 635                 640

Gln Asn Ser Ala Gly Ser Ile Ser Ala Asn Ala Thr Leu Thr Val Leu
                645                 650                 655

Glu Thr Pro Ser Leu Val Val Pro Leu Glu Asp Arg Val Val Ser Val

            660                 665                 670

Gly Glu Thr Val Ala Leu Gln Cys Lys Ala Thr Gly Asn Pro Pro Pro
        675                 680                 685

Arg Ile Thr Trp Phe Lys Gly Asp Arg Pro Leu Ser Leu Thr Glu Arg
    690                 695                 700

His His Leu Thr Pro Asp Asn Gln Leu Leu Val Val Gln Asn Val Val
705                 710                 715                 720

Ala Glu Asp Ala Gly Arg Tyr Thr Cys Glu Met Ser Asn Thr Leu Gly
                725                 730                 735

Thr Glu Arg Ala His Ser Gln Leu Ser Val Leu Pro Ala Ala Gly Cys
            740                 745                 750

Arg Lys Asp Gly Thr Thr Val Gly Ile Phe Gly Ser Glu Pro Lys Ser
        755                 760                 765

Ser Asp Lys Thr His Thr Ser Pro Pro Cys Pro Ala Pro Glu Leu Leu
    770                 775                 780

Gly Gly Pro Ser Val Phe Leu Phe Pro Pro Lys Pro Lys Asp Thr Leu
785                 790                 795                 800

Met Ile Ser Arg Thr Pro Glu Val Thr Cys Val Val Val Asp Val Ser
                805                 810                 815

His Glu Asp Pro Glu Val Lys Phe Asn Trp Tyr Val Asp Gly Val Glu
            820                 825                 830

Val His Asn Ala Lys Thr Lys Pro Arg Glu Glu Gln Tyr Asn Ser Thr
        835                 840                 845

Tyr Arg Val Val Ser Val Leu Thr Val Leu His Gln Asp Trp Leu Asn
    850                 855                 860

Gly Lys Glu Tyr Lys Cys Lys Val Ser Asn Lys Ala Leu Pro Ala Pro
865                 870                 875                 880

Ile Glu Lys Thr Ile Ser Lys Ala Lys Gly Gln Pro Arg Glu Pro Gln
                885                 890                 895

Val Tyr Thr Leu Pro Pro Ser Arg Asp Glu Leu Thr Lys Asn Gln Val
            900                 905                 910

Ser Leu Thr Cys Leu Val Lys Gly Phe Tyr Pro Ser Asp Ile Ala Val
        915                 920                 925

Glu Trp Glu Ser Asn Gly Gln Pro Glu Asn Asn Tyr Lys Thr Thr Pro
    930                 935                 940

Pro Val Leu Asp Ser Asp Gly Ser Phe Phe Leu Tyr Ser Lys Leu Thr
945                 950                 955                 960

Val Asp Lys Ser Arg Trp Gln Gln Gly Asn Val Phe Ser Cys Ser Val
                965                 970                 975

Met His Glu Ala Leu His Asn His Tyr Thr Gln Lys Ser Leu Ser Leu
            980                 985                 990

Ser Pro Gly Lys

        995

<210>    38
<211>    997
<212>    PRT
<213>    Artificial Sequence

<220>
<223>    fusion protein

<400>    38
Ala Gly Pro Arg Ala Pro Cys Ala Ala Ala Cys Thr Cys Ala Gly Asp
1               5                   10                  15

Ser Leu Asp Cys Gly Gly Arg Gly Leu Ala Ala Leu Pro Gly Asp Leu
            20                  25                  30

Pro Ser Trp Thr Arg Ser Leu Asn Leu Ser Tyr Asn Lys Leu Ser Glu
        35                  40                  45

Ile Asp Pro Ala Gly Phe Glu Asp Leu Pro Asn Leu Gln Glu Val Tyr
    50                  55                  60

Leu Asn Asn Asn Glu Leu Thr Ala Val Pro Ser Leu Gly Ala Ala Ser
65                  70                  75                  80

Ser His Val Val Ser Leu Phe Leu Gln His Asn Lys Ile Arg Ser Val
                85                  90                  95

Glu Gly Ser Gln Leu Lys Ala Tyr Leu Ser Leu Glu Val Leu Asp Leu
            100                 105                 110

Ser Leu Asn Asn Ile Thr Glu Val Arg Asn Thr Cys Phe Pro His Gly
        115                 120                 125

Pro Pro Ile Lys Glu Leu Asn Leu Ala Gly Asn Arg Ile Gly Thr Leu
    130                 135                 140

Glu Leu Gly Ala Phe Asp Gly Leu Ser Arg Ser Leu Leu Thr Leu Arg
145                 150                 155                 160

Leu Ser Lys Asn Arg Ile Thr Gln Leu Pro Val Arg Ala Phe Lys Leu
                165                 170                 175

Pro Arg Leu Thr Gln Leu Asp Leu Asn Arg Asn Arg Ile Arg Leu Ile
            180                 185                 190

Glu Gly Leu Thr Phe Gln Gly Leu Asn Ser Leu Glu Val Leu Lys Leu
        195                 200                 205

Gln Arg Asn Asn Ile Ser Lys Leu Thr Asp Gly Ala Phe Trp Gly Leu
    210                 215                 220

Ser Lys Met His Val Leu His Leu Glu Tyr Asn Ser Leu Val Glu Val
225                 230                 235                 240

Asn Ser Gly Ser Leu Tyr Gly Leu Thr Ala Leu His Gln Leu His Leu
                245                 250                 255

Ser Asn Asn Ser Ile Ala Arg Ile His Arg Lys Gly Trp Ser Phe Cys
            260                 265                 270

Gln Lys Leu His Glu Leu Val Leu Ser Phe Asn Asn Leu Thr Arg Leu
        275                 280                 285

Asp Glu Glu Ser Leu Ala Glu Leu Ser Ser Leu Ser Val Leu Arg Leu
    290                 295                 300

Ser His Asn Ser Ile Ser His Ile Ala Glu Gly Ala Phe Lys Gly Leu
305                 310                 315                 320

Arg Ser Leu Arg Val Leu Asp Leu Asp His Asn Glu Ile Ser Gly Thr
                325                 330                 335

Ile Glu Asp Thr Ser Gly Ala Phe Ser Gly Leu Asp Ser Leu Ser Lys
            340                 345                 350

Leu Thr Leu Phe Gly Asn Lys Ile Lys Ser Val Ala Lys Arg Ala Phe
        355                 360                 365

Ser Gly Leu Glu Gly Leu Glu His Leu Asn Leu Gly Gly Asn Ala Ile
    370                 375                 380

Arg Ser Val Gln Phe Asp Ala Phe Val Lys Met Lys Asn Leu Lys Glu
385                 390                 395                 400

Leu His Ile Ser Ser Asp Ser Phe Leu Cys Asp Cys Gln Leu Lys Trp
                405                 410                 415

Leu Pro Pro Trp Leu Ile Gly Arg Met Leu Gln Ala Phe Val Thr Ala
            420                 425                 430

Thr Cys Ala His Pro Glu Ser Leu Lys Gly Gln Ser Ile Phe Ser Val
        435                 440                 445

Pro Pro Glu Ser Phe Val Cys Asp Asp Phe Leu Lys Pro Gln Ile Ile
    450                 455                 460

Thr Gln Pro Glu Thr Thr Met Ala Met Val Gly Lys Asp Ile Arg Phe
465                 470                 475                 480

Thr Cys Ser Ala Ala Ser Ser Ser Ser Ser Pro Met Thr Phe Ala Trp
                485                 490                 495

Lys Lys Asp Asn Glu Val Leu Thr Asn Ala Asp Met Glu Asn Phe Val
            500                 505                 510

His Val His Ala Gln Asp Gly Glu Val Met Glu Tyr Thr Thr Ile Leu
        515                 520                 525

His Leu Arg Gln Val Thr Phe Gly His Glu Gly Arg Tyr Gln Cys Val
    530                 535                 540

Ile Thr Asn His Phe Gly Ser Thr Tyr Ser His Lys Ala Arg Leu Thr
545                 550                 555                 560

Val Asn Val Leu Pro Ser Phe Thr Lys Thr Pro His Asp Ile Thr Ile
                565                 570                 575

Arg Thr Thr Thr Val Ala Arg Leu Glu Cys Ala Ala Thr Gly His Pro
            580                 585                 590

Asn Pro Gln Ile Ala Trp Gln Lys Asp Gly Gly Thr Asp Phe Pro Ala
        595                 600                 605

Ala Arg Glu Arg Arg Met His Val Met Pro Asp Asp Asp Val Phe Phe
    610                 615                 620

Ile Thr Asp Val Lys Ile Asp Asp Ala Gly Val Tyr Ser Cys Thr Ala
625                 630                 635                 640

Gln Asn Ser Ala Gly Ser Ile Ser Ala Asn Ala Thr Leu Thr Val Leu
                645                 650                 655

Glu Thr Pro Ser Leu Val Val Pro Leu Glu Asp Arg Val Val Ser Val
            660                 665                 670

Gly Glu Thr Val Ala Leu Gln Cys Lys Ala Thr Gly Asn Pro Pro Pro
        675                 680                 685

Arg Ile Thr Trp Phe Lys Gly Asp Arg Pro Leu Ser Leu Thr Glu Arg
    690                 695                 700

His His Leu Thr Pro Asp Asn Gln Leu Leu Val Val Gln Asn Val Val
705                 710                 715                 720

Ala Glu Asp Ala Gly Arg Tyr Thr Cys Glu Met Ser Asn Thr Leu Gly
                725                 730                 735

Thr Glu Arg Ala His Ser Gln Leu Ser Val Leu Pro Ala Ala Gly Cys
            740                 745                 750

Arg Lys Asp Gly Thr Thr Val Gly Ile Phe Gly Ser Glu Pro Arg Gly
        755                 760                 765

Pro Thr Ile Lys Pro Cys Pro Pro Cys Lys Cys Pro Ala Pro Asn Leu
    770                 775                 780

Leu Gly Gly Pro Ser Val Phe Leu Phe Pro Pro Lys Pro Lys Asp Thr
785                 790                 795                 800

Leu Met Ile Ser Arg Thr Pro Glu Val Thr Cys Val Val Val Asp Val
                805                 810                 815

Ser His Glu Asp Pro Glu Val Lys Phe Asn Trp Tyr Val Asp Gly Val
            820                 825                 830

Glu Val His Asn Ala Lys Thr Lys Pro Arg Glu Glu Gln Tyr Asn Ser
        835                 840                 845

Thr Tyr Arg Val Val Ser Val Leu Thr Val Leu His Gln Asp Trp Leu
    850                 855                 860

Asn Gly Lys Glu Tyr Lys Cys Lys Val Ser Asn Lys Ala Leu Pro Ala
865                 870                 875                 880

Pro Ile Glu Lys Thr Ile Ser Lys Ala Lys Gly Gln Pro Arg Glu Pro
                885                 890                 895

Gln Val Tyr Thr Leu Pro Pro Ser Arg Asp Glu Leu Thr Lys Asn Gln
            900                 905                 910

Val Ser Leu Thr Cys Leu Val Lys Gly Phe Tyr Pro Ser Asp Ile Ala
        915                 920                 925

Val Glu Trp Glu Ser Asn Gly Gln Pro Glu Asn Asn Tyr Lys Thr Thr
    930                 935                 940

Pro Pro Val Leu Asp Ser Asp Gly Ser Phe Phe Leu Tyr Ser Lys Leu
945                 950                 955                 960

Thr Val Asp Lys Ser Arg Trp Gln Gln Gly Asn Val Phe Ser Cys Ser
                965                 970                 975

Val Met His Glu Ala Leu His Asn His Tyr Thr Gln Lys Ser Leu Ser
            980                 985                 990

Leu Ser Pro Gly Lys
        995

<210>   39
<211>   1009
<212>   PRT
<213>   Artificial Sequence

<220>
<223>   fusion protein

<400>   39
Ala Gly Pro Arg Ala Pro Cys Ala Ala Ala Cys Thr Cys Ala Gly Asp
1               5                   10                  15

Ser Leu Asp Cys Gly Gly Arg Gly Leu Ala Ala Leu Pro Gly Asp Leu
            20                  25                  30

Pro Ser Trp Thr Arg Ser Leu Asn Leu Ser Tyr Asn Lys Leu Ser Glu
        35                  40                  45

Ile Asp Pro Ala Gly Phe Glu Asp Leu Pro Asn Leu Gln Glu Val Tyr
    50                  55                  60

Leu Asn Asn Asn Glu Leu Thr Ala Val Pro Ser Leu Gly Ala Ala Ser
65                  70                  75                  80

Ser His Val Val Ser Leu Phe Leu Gln His Asn Lys Ile Arg Ser Val
                85                  90                  95

Glu Gly Ser Gln Leu Lys Ala Tyr Leu Ser Leu Glu Val Leu Asp Leu
            100                 105                 110

Ser Leu Asn Asn Ile Thr Glu Val Arg Asn Thr Cys Phe Pro His Gly
        115                 120                 125

Pro Pro Ile Lys Glu Leu Asn Leu Ala Gly Asn Arg Ile Gly Thr Leu
    130                 135                 140

Glu Leu Gly Ala Phe Asp Gly Leu Ser Arg Ser Leu Leu Thr Leu Arg
145                 150                 155                 160

Leu Ser Lys Asn Arg Ile Thr Gln Leu Pro Val Arg Ala Phe Lys Leu
                165                 170                 175

Pro Arg Leu Thr Gln Leu Asp Leu Asn Arg Asn Arg Ile Arg Leu Ile
            180                 185                 190

Glu Gly Leu Thr Phe Gln Gly Leu Asn Ser Leu Glu Val Leu Lys Leu
        195                 200                 205

Gln Arg Asn Asn Ile Ser Lys Leu Thr Asp Gly Ala Phe Trp Gly Leu
    210                 215                 220

Ser Lys Met His Val Leu His Leu Glu Tyr Asn Ser Leu Val Glu Val
225                 230                 235                 240

Asn Ser Gly Ser Leu Tyr Gly Leu Thr Ala Leu His Gln Leu His Leu
                245                 250                 255

Ser Asn Asn Ser Ile Ala Arg Ile His Arg Lys Gly Trp Ser Phe Cys
            260                 265                 270

Gln Lys Leu His Glu Leu Val Leu Ser Phe Asn Asn Leu Thr Arg Leu
        275                 280                 285

Asp Glu Glu Ser Leu Ala Glu Leu Ser Ser Leu Ser Val Leu Arg Leu
    290                 295                 300

Ser His Asn Ser Ile Ser His Ile Ala Glu Gly Ala Phe Lys Gly Leu
305                 310                 315                 320

Arg Ser Leu Arg Val Leu Asp Leu Asp His Asn Glu Ile Ser Gly Thr
                325                 330                 335

Ile Glu Asp Thr Ser Gly Ala Phe Ser Gly Leu Asp Ser Leu Ser Lys
            340                 345                 350

Leu Thr Leu Phe Gly Asn Lys Ile Lys Ser Val Ala Lys Arg Ala Phe
        355                 360                 365

Ser Gly Leu Glu Gly Leu Glu His Leu Asn Leu Gly Gly Asn Ala Ile
    370                 375                 380

Arg Ser Val Gln Phe Asp Ala Phe Val Lys Met Lys Asn Leu Lys Glu
385                 390                 395                 400

Leu His Ile Ser Ser Asp Ser Phe Leu Cys Asp Cys Gln Leu Lys Trp
                405                 410                 415

Leu Pro Pro Trp Leu Ile Gly Arg Met Leu Gln Ala Phe Val Thr Ala
            420                 425                 430

Thr Cys Ala His Pro Glu Ser Leu Lys Gly Gln Ser Ile Phe Ser Val
        435                 440                 445

Pro Pro Glu Ser Phe Val Cys Asp Asp Phe Leu Lys Pro Gln Ile Ile
    450                 455                 460

Thr Gln Pro Glu Thr Thr Met Ala Met Val Gly Lys Asp Ile Arg Phe
465                 470                 475                 480

Thr Cys Ser Ala Ala Ser Ser Ser Ser Ser Pro Met Thr Phe Ala Trp
                485                 490                 495

Lys Lys Asp Asn Glu Val Leu Thr Asn Ala Asp Met Glu Asn Phe Val
            500                 505                 510

His Val His Ala Gln Asp Gly Glu Val Met Glu Tyr Thr Thr Ile Leu
        515                 520                 525

His Leu Arg Gln Val Thr Phe Gly His Glu Gly Arg Tyr Gln Cys Val
    530                 535                 540

Ile Thr Asn His Phe Gly Ser Thr Tyr Ser His Lys Ala Arg Leu Thr
545                 550                 555                 560

Val Asn Val Leu Pro Ser Phe Thr Lys Thr Pro His Asp Ile Thr Ile
                565                 570                 575

Arg Thr Thr Thr Val Ala Arg Leu Glu Cys Ala Ala Thr Gly His Pro
            580                 585                 590

Asn Pro Gln Ile Ala Trp Gln Lys Asp Gly Gly Thr Asp Phe Pro Ala
        595                 600                 605

Ala Arg Glu Arg Arg Met His Val Met Pro Asp Asp Asp Val Phe Phe
    610                 615                 620

Ile Thr Asp Val Lys Ile Asp Asp Ala Gly Val Tyr Ser Cys Thr Ala
625                 630                 635                 640

Gln Asn Ser Ala Gly Ser Ile Ser Ala Asn Ala Thr Leu Thr Val Leu
                645                 650                 655

Glu Thr Pro Ser Leu Val Val Pro Leu Glu Asp Arg Val Val Ser Val
            660                 665                 670

Gly Glu Thr Val Ala Leu Gln Cys Lys Ala Thr Gly Asn Pro Pro Pro
        675                 680                 685

Arg Ile Thr Trp Phe Lys Gly Asp Arg Pro Leu Ser Leu Thr Glu Arg
    690                 695                 700

His His Leu Thr Pro Asp Asn Gln Leu Leu Val Val Gln Asn Val Val
705                 710                 715                 720

Ala Glu Asp Ala Gly Arg Tyr Thr Cys Glu Met Ser Asn Thr Leu Gly
                725                 730                 735

Thr Glu Arg Ala His Ser Gln Leu Ser Val Leu Pro Ala Ala Gly Cys
            740                 745                 750

Arg Lys Asp Gly Thr Thr Val Gly Ile Phe Gly Ser Arg Asn Thr Gly
        755                 760                 765

Arg Gly Gly Glu Glu Lys Lys Lys Glu Lys Glu Lys Glu Glu Gln Glu
    770                 775                 780

Glu Arg Glu Thr Lys Thr Pro Glu Cys Pro Ser His Thr Gln Pro Leu
785                 790                 795                 800

Gly Val Phe Leu Phe Pro Pro Lys Pro Lys Asp Thr Leu Met Ile Ser
                805                 810                 815

Arg Thr Pro Glu Val Thr Cys Val Val Val Asp Val Ser His Glu Asp
            820                 825                 830

Pro Glu Val Lys Phe Asn Trp Tyr Val Asp Gly Val Glu Val His Asn
        835                 840                 845

Ala Lys Thr Lys Pro Arg Glu Glu Gln Tyr Asn Ser Thr Tyr Arg Val
    850                 855                 860

Val Ser Val Leu Thr Val Leu His Gln Asp Trp Leu Asn Gly Lys Glu
865                 870                 875                 880

Tyr Lys Cys Lys Val Ser Asn Lys Ala Leu Pro Ala Pro Ile Glu Lys
                885                 890                 895

Thr Ile Ser Lys Ala Lys Gly Gln Pro Arg Glu Pro Gln Val Tyr Thr
            900                 905                 910

Leu Pro Pro Ser Arg Asp Glu Leu Thr Lys Asn Gln Val Ser Leu Thr
        915                 920                 925

Cys Leu Val Lys Gly Phe Tyr Pro Ser Asp Ile Ala Val Glu Trp Glu
    930                 935                 940

Ser Asn Gly Gln Pro Glu Asn Asn Tyr Lys Thr Thr Pro Pro Val Leu
945                 950                 955                 960

Asp Ser Asp Gly Ser Phe Phe Leu Tyr Ser Lys Leu Thr Val Asp Lys
                965                 970                 975

Ser Arg Trp Gln Gln Gly Asn Val Phe Ser Cys Ser Val Met His Glu
            980                 985                 990

Ala Leu His Asn His Tyr Thr Gln Lys Ser Leu Ser Leu Ser Pro Gly
        995                 1000                1005

Lys

<210>    40
<211>    996
<212>    PRT
<213>    Artificial Sequence

<220>
<223>    fusion protein

<400>    40
Ala Gly Pro Arg Ala Pro Cys Ala Ala Ala Cys Thr Cys Ala Gly Asp
 1               5                  10                  15

Ser Leu Asp Cys Gly Gly Arg Gly Leu Ala Ala Leu Pro Gly Asp Leu
            20                  25                  30

Pro Ser Trp Thr Arg Ser Leu Asn Leu Ser Tyr Asn Lys Leu Ser Glu
        35                  40                  45

Ile Asp Pro Ala Gly Phe Glu Asp Leu Pro Asn Leu Gln Glu Val Tyr
    50                  55                  60

Leu Asn Asn Asn Glu Leu Thr Ala Val Pro Ser Leu Gly Ala Ala Ser
65                  70                  75                  80

Ser His Val Val Ser Leu Phe Leu Gln His Asn Lys Ile Arg Ser Val
                85                  90                  95

Glu Gly Ser Gln Leu Lys Ala Tyr Leu Ser Leu Glu Val Leu Asp Leu
            100                 105                 110

Ser Leu Asn Asn Ile Thr Glu Val Arg Asn Thr Cys Phe Pro His Gly
        115                 120                 125

Pro Pro Ile Lys Glu Leu Asn Leu Ala Gly Asn Arg Ile Gly Thr Leu

130                 135                 140

Glu Leu Gly Ala Phe Asp Gly Leu Ser Arg Ser Leu Leu Thr Leu Arg
145                 150                 155                 160

Leu Ser Lys Asn Arg Ile Thr Gln Leu Pro Val Arg Ala Phe Lys Leu
                165                 170                 175

Pro Arg Leu Thr Gln Leu Asp Leu Asn Arg Asn Arg Ile Arg Leu Ile
            180                 185                 190

Glu Gly Leu Thr Phe Gln Gly Leu Asn Ser Leu Glu Val Leu Lys Leu
        195                 200                 205

Gln Arg Asn Asn Ile Ser Lys Leu Thr Asp Gly Ala Phe Trp Gly Leu
    210                 215                 220

Ser Lys Met His Val Leu His Leu Glu Tyr Asn Ser Leu Val Glu Val
225                 230                 235                 240

Asn Ser Gly Ser Leu Tyr Gly Leu Thr Ala Leu His Gln Leu His Leu
                245                 250                 255

Ser Asn Asn Ser Ile Ala Arg Ile His Arg Lys Gly Trp Ser Phe Cys
            260                 265                 270

Gln Lys Leu His Glu Leu Val Leu Ser Phe Asn Asn Leu Thr Arg Leu
        275                 280                 285

Asp Glu Glu Ser Leu Ala Glu Leu Ser Ser Leu Ser Val Leu Arg Leu
    290                 295                 300

Ser His Asn Ser Ile Ser His Ile Ala Glu Gly Ala Phe Lys Gly Leu
305                 310                 315                 320

Arg Ser Leu Arg Val Leu Asp Leu Asp His Asn Glu Ile Ser Gly Thr
                325                 330                 335

Ile Glu Asp Thr Ser Gly Ala Phe Ser Gly Leu Asp Ser Leu Ser Lys
            340                 345                 350

Leu Thr Leu Phe Gly Asn Lys Ile Lys Ser Val Ala Lys Arg Ala Phe
        355                 360                 365

Ser Gly Leu Glu Gly Leu Glu His Leu Asn Leu Gly Gly Asn Ala Ile
    370                 375                 380

Arg Ser Val Gln Phe Asp Ala Phe Val Lys Met Lys Asn Leu Lys Glu
385                 390                 395                 400

Leu His Ile Ser Ser Asp Ser Phe Leu Cys Asp Cys Gln Leu Lys Trp
                405                 410                 415

Leu Pro Pro Trp Leu Ile Gly Arg Met Leu Gln Ala Phe Val Thr Ala
            420                 425                 430

Thr Cys Ala His Pro Glu Ser Leu Lys Gly Gln Ser Ile Phe Ser Val
        435                 440                 445

Pro Pro Glu Ser Phe Val Cys Asp Asp Phe Leu Lys Pro Gln Ile Ile
    450                 455                 460

Thr Gln Pro Glu Thr Thr Met Ala Met Val Gly Lys Asp Ile Arg Phe

465                 470                 475                 480

Thr Cys Ser Ala Ala Ser Ser Ser Ser Ser Pro Met Thr Phe Ala Trp
                485                 490                 495

Lys Lys Asp Asn Glu Val Leu Thr Asn Ala Asp Met Glu Asn Phe Val
            500                 505                 510

His Val His Ala Gln Asp Gly Glu Val Met Glu Tyr Thr Thr Ile Leu
        515                 520                 525

His Leu Arg Gln Val Thr Phe Gly His Glu Gly Arg Tyr Gln Cys Val
    530                 535                 540

Ile Thr Asn His Phe Gly Ser Thr Tyr Ser His Lys Ala Arg Leu Thr
545                 550                 555                 560

Val Asn Val Leu Pro Ser Phe Thr Lys Thr Pro His Asp Ile Thr Ile
                565                 570                 575

Arg Thr Thr Thr Val Ala Arg Leu Glu Cys Ala Ala Thr Gly His Pro
            580                 585                 590

Asn Pro Gln Ile Ala Trp Gln Lys Asp Gly Gly Thr Asp Phe Pro Ala
        595                 600                 605

Ala Arg Glu Arg Arg Met His Val Met Pro Asp Asp Asp Val Phe Phe
    610                 615                 620

Ile Thr Asp Val Lys Ile Asp Asp Ala Gly Val Tyr Ser Cys Thr Ala
625                 630                 635                 640

Gln Asn Ser Ala Gly Ser Ile Ser Ala Asn Ala Thr Leu Thr Val Leu
                645                 650                 655

Glu Thr Pro Ser Leu Val Val Pro Leu Glu Asp Arg Val Val Ser Val
            660                 665                 670

Gly Glu Thr Val Ala Leu Gln Cys Lys Ala Thr Gly Asn Pro Pro Pro
        675                 680                 685

Arg Ile Thr Trp Phe Lys Gly Asp Arg Pro Leu Ser Leu Thr Glu Arg
    690                 695                 700

His His Leu Thr Pro Asp Asn Gln Leu Leu Val Val Gln Asn Val Val
705                 710                 715                 720

Ala Glu Asp Ala Gly Arg Tyr Thr Cys Glu Met Ser Asn Thr Leu Gly
                725                 730                 735

Thr Glu Arg Ala His Ser Gln Leu Ser Val Leu Pro Ala Ala Gly Cys
            740                 745                 750

Arg Lys Asp Gly Thr Thr Val Gly Ile Phe Gly Ser Glu Pro Lys Ser
        755                 760                 765

Ser Asp Lys Thr His Thr Ser Pro Pro Ser Pro Ala Pro Glu Leu Leu
    770                 775                 780

Gly Gly Ser Ser Val Phe Leu Phe Pro Pro Lys Pro Lys Asp Thr Leu
785                 790                 795                 800

Met Ile Ser Arg Thr Pro Glu Val Thr Cys Val Val Val Asp Val Ser

805                 810                 815

His Glu Asp Pro Glu Val Lys Phe Asn Trp Tyr Val Asp Gly Val Glu
            820                 825                 830

Val His Asn Ala Lys Thr Lys Pro Arg Glu Glu Gln Tyr Asn Ser Thr
        835                 840                 845

Tyr Arg Val Val Ser Val Leu Thr Val Leu His Gln Asp Trp Leu Asn
    850                 855                 860

Gly Lys Glu Tyr Lys Cys Lys Val Ser Asn Lys Ala Leu Pro Ala Pro
865                 870                 875                 880

Ile Glu Lys Thr Ile Ser Lys Ala Lys Gly Gln Pro Arg Glu Pro Gln
                885                 890                 895

Val Tyr Thr Leu Pro Pro Ser Arg Asp Glu Leu Thr Lys Asn Gln Val
            900                 905                 910

Ser Leu Thr Cys Leu Val Lys Gly Phe Tyr Pro Ser Asp Ile Ala Val
        915                 920                 925

Glu Trp Glu Ser Asn Gly Gln Pro Glu Asn Asn Tyr Lys Thr Thr Pro
    930                 935                 940

Pro Val Leu Asp Ser Asp Gly Ser Phe Phe Leu Tyr Ser Lys Leu Thr
945                 950                 955                 960

Val Asp Lys Ser Arg Trp Gln Gln Gly Asn Val Phe Ser Cys Ser Val
                965                 970                 975

Met His Glu Ala Leu His Asn His Tyr Thr Gln Lys Ser Leu Ser Leu
            980                 985                 990

Ser Pro Gly Lys
        995


<210>   41
<211>   2988
<212>   DNA
<213>   Artificial Sequence

<220>
<223>   fusion protein


<400>   41
gctgggcctc gggctccttg tgctgccgcc tgcacatgtg caggcgattc cctggactgc      60

ggcggcagag gcctggccgc cctgcctggc gatctgccat cctggacccg gagcctgaac     120

ctgagctaca acaagctgag cgagatcgat cccgccggct ttgaggacct gcctaacctg     180

caggaggtgt atctgaacaa taacgagctg accgcggtac catccctggg cgctgcttca     240

tcacatgtcg tctctctctt tctgcagcac aacaagattc gcagcgtgga ggggagccag     300

ctgaaggcct acctttcctt agaagtgtta gatctgagtt tgaacaacat cacggaagtg     360

cggaacacct gctttccaca cggaccgcct ataaaggagc tcaacctggc aggcaatcgg     420

attggcaccc tggagttggg agcatttgat ggtctgtcac ggtcgctgct aactcttcgc 480

ctgagcaaaa acaggatcac ccagcttcct gtaagagcat tcaagctacc caggctgaca 540

caactggacc tcaatcggaa caggattcgg ctgatagagg gcctcacctt ccaggggctc 600

aacagcttgg aggtgctgaa gcttcagcga aacaacatca gcaaactgac agatggggcc 660

ttctggggac tgtccaagat gcatgtgctg cacctggagt acaacagcct ggtagaagtg 720

aacagcggct cgctctacgg cctcacggcc ctgcatcagc tccacctcag caacaattcc 780

atcgctcgca ttcaccgcaa gggctggagc ttctgccaga agctgcatga gttggtcctg 840

tccttcaaca acctgacacg gctggacgag gagagcctgg ccgagctgag cagcctgagt 900

gtcctgcgtc tcagccacaa ttccatcagc cacattgcgg agggtgcctt caagggactc 960

aggagcctgc gagtcttgga tctggaccat aacgagattt cgggcacaat agaggacacg 1020

agcggcgcct tctcagggct cgacagcctc agcaagctga ctctgtttgg aaacaagatc 1080

aagtctgtgg ctaagagagc attctcgggg ctggaaggcc tggagcacct gaaccttgga 1140

gggaatgcga tcagatctgt ccagtttgat gcctttgtga agatgaagaa tcttaaagag 1200

ctccatatca gcagcgacag cttcctgtgt gactgccagc tgaagtggct gcccccgtgg 1260

ctaattggca ggatgctgca ggcctttgtg acagccacct gtgcccaccc agaatcactg 1320

aagggtcaga gcattttctc tgtgccacca gagagtttcg tgtgcgatga cttcctgaag 1380

ccacagatca tcacccagcc agaaaccacc atggctatgg tgggcaagga catccggttt 1440

acatgctcag cagccagcag cagcagctcc cccatgacct ttgcctggaa gaaagacaat 1500

gaagtcctga ccaatgcaga catggagaac tttgtccacg tccacgcgca ggacggggaa 1560

gtgatggagt acaccaccat cctgcacctc cgtcaggtca ctttcgggca cgagggccgc 1620

taccaatgtg tcatcaccaa ccactttggc tccacctatt cacataaggc caggctcacc 1680

gtgaatgtgt tgccatcatt caccaaaacg ccccacgaca taaccatccg gaccaccacc 1740

gtggcccgcc tcgaatgtgc tgccacaggt cacccaaacc ctcagattgc ctggcagaag 1800

gatggaggca cggatttccc cgctgcccgt gagcgacgca tgcatgtcat gccggatgac 1860

gacgtgtttt tcatcactga tgtgaaaata gatgacgcag gggtttacag ctgtactgct 1920

cagaactcag ccggttctat ttcagctaat gccaccctga ctgtcctaga gaccccatcc 1980

ttggtggtcc ccttggaaga ccgtgtggta tctgtgggag aaacagtggc cctccaatgc 2040

aaagccacgg ggaaccctcc gccccgcatc acctggttca agggggaccg cccgctgagc 2100

ctcactgagc ggcaccacct gacccctgac aaccagctcc tggtggttca gaacgtggtg 2160

gcagaggatg cgggccgata tacctgtgag atgtccaaca ccctgggcac ggagcgagct 2220

cacagccagc tgagcgtcct gcccgcagca ggctgcagga aggatgggac cacggtaggc 2280

atcttcggat ccgagccaaa gtcctctgat aagacacaca cctctccacc atgcccagca 2340

ccagagctgc tgggaggacc aagcgtgttc ctgtttcctc caaagcccaa ggacacactg 2400

atgatctcca ggacaccaga ggtgacctgc gtggtggtgg acgtgagcca cgaggacccc 2460

gaggtgaagt tcaactggta cgtggatggc gtggaggtgc acaatgccaa gaccaagccc 2520

agagaggagc agtacaactc tacctatagg gtggtgagcg tgctgacagt gctgcaccag 2580

gactggctga acggcaagga gtataagtgc aaggtgagca ataaggccct gcctgcccca 2640

atcgagaaga caatctccaa ggccaagggc cagccaagag agccccaggt gtacaccctg 2700

ccccctagca gggatgagct gacaaagaac caggtgtccc tgacctgtct ggtgaagggc 2760

ttttatccct ccgacatcgc cgtggagtgg gagtctaatg gccagcctga gaataactac 2820

aagacaaccc cacccgtgct ggattctgac ggcagcttct ttctgtattc taagctgacc 2880

gtggacaaga gcaggtggca gcagggcaac gtgttcagct gctccgtgat gcacgaagca 2940

ctgcacaatc actacaccca gaaatcactg tcactgagcc ctggcaaa 2988

<210> 42
<211> 2991
<212> DNA
<213> Artificial Sequence

<220>
<223> fusion protein

<400> 42
gctgggcctc gggctccttg tgctgccgcc tgcacatgtg caggcgattc cctggactgc 60

ggcggcagag gcctggccgc cctgcctggc gatctgccat cctggacccg gagcctgaac 120

ctgagctaca acaagctgag cgagatcgat cccgccggct ttgaggacct gcctaacctg 180

caggaggtgt atctgaacaa taacgagctg accgcggtac catccctggg cgctgcttca 240

tcacatgtcg tctctctctt tctgcagcac aacaagattc gcagcgtgga ggggagccag 300

ctgaaggcct acctttcctt agaagtgtta gatctgagtt tgaacaacat cacggaagtg 360

cggaacacct gctttccaca cggaccgcct ataaaggagc tcaacctggc aggcaatcgg 420

attggcaccc tggagttggg agcatttgat ggtctgtcac ggtcgctgct aactcttcgc 480

ctgagcaaaa acaggatcac ccagcttcct gtaagagcat tcaagctacc caggctgaca 540

caactggacc tcaatcggaa caggattcgg ctgatagagg gcctcacctt ccaggggctc 600

aacagcttgg aggtgctgaa gcttcagcga aacaacatca gcaaactgac agatggggcc 660

ttctggggac tgtccaagat gcatgtgctg cacctggagt acaacagcct ggtagaagtg 720

aacagcggct cgctctacgg cctcacggcc ctgcatcagc tccacctcag caacaattcc 780

atcgctcgca ttcaccgcaa gggctggagc ttctgccaga agctgcatga gttggtcctg 840

tccttcaaca acctgacacg gctggacgag gagagcctgg ccgagctgag cagcctgagt 900

gtcctgcgtc tcagccacaa ttccatcagc cacattgcgg agggtgcctt caagggactc 960

aggagcctgc gagtcttgga tctggaccat aacgagattt cgggcacaat agaggacacg 1020

agcggcgcct tctcagggct cgacagcctc agcaagctga ctctgtttgg aaacaagatc 1080

aagtctgtgg ctaagagagc attctcgggg ctggaaggcc tggagcacct gaaccttgga 1140

gggaatgcga tcagatctgt ccagtttgat gcctttgtga agatgaagaa tcttaaagag 1200

ctccatatca gcagcgacag cttcctgtgt gactgccagc tgaagtggct gcccccgtgg 1260

ctaattggca ggatgctgca ggcctttgtg acagccacct gtgcccaccc agaatcactg 1320

aagggtcaga gcattttctc tgtgccacca gagagtttcg tgtgcgatga cttcctgaag 1380

ccacagatca tcacccagcc agaaaccacc atggctatgg tgggcaagga catccggttt 1440

acatgctcag cagccagcag cagcagctcc cccatgacct ttgcctggaa gaaagacaat 1500

gaagtcctga ccaatgcaga catggagaac tttgtccacg tccacgcgca ggacggggaa 1560

gtgatggagt acaccaccat cctgcacctc cgtcaggtca ctttcgggca cgagggccgc 1620

taccaatgtg tcatcaccaa ccactttggc tccacctatt cacataaggc caggctcacc 1680

gtgaatgtgt tgccatcatt caccaaaacg ccccacgaca taaccatccg gaccaccacc 1740

gtggcccgcc tcgaatgtgc tgccacaggt cacccaaacc ctcagattgc ctggcagaag 1800

gatggaggca cggatttccc cgctgcccgt gagcgacgca tgcatgtcat gccggatgac 1860

gacgtgtttt tcatcactga tgtgaaaata gatgacgcag gggtttacag ctgtactgct 1920

cagaactcag ccggttctat ttcagctaat gccaccctga ctgtcctaga gaccccatcc 1980

ttggtggtcc ccttggaaga ccgtgtggta tctgtgggag aaacagtggc cctccaatgc 2040

aaagccacgg ggaaccctcc gccccgcatc acctggttca agggggaccg cccgctgagc 2100

ctcactgagc ggcaccacct gacccctgac aaccagctcc tggtggttca gaacgtggtg 2160

gcagaggatg cgggccgata tacctgtgag atgtccaaca ccctgggcac ggagcgagct 2220

cacagccagc tgagcgtcct gcccgcagca ggctgcagga aggatgggac cacggtaggc 2280

atcttcggat ccgagcctcg gggccctacc atcaagccct gccccccttg caagtgccct 2340

gcccctaatc tgctgggcgg accctccgtg ttcctgtttc ctccaaagcc caaggacaca 2400

ctgatgatct ccaggacacc agaggtgacc tgcgtggtgg tggacgtgag ccacgaggac 2460

cccgaggtga agttcaactg gtacgtggat ggcgtggagg tgcacaatgc caagaccaag 2520

cccagagagg agcagtacaa ctctacctat agggtggtga gcgtgctgac agtgctgcac 2580

caggactggc tgaacggcaa ggagtataag tgcaaggtga gcaataaggc cctgcctgcc 2640

ccaatcgaga agacaatctc caaggccaag ggccagccaa gagagcccca ggtgtacacc 2700

ctgcccccta gcagggatga gctgacaaag aaccaggtgt ccctgacctg tctggtgaag 2760

ggcttttatc cctccgacat cgccgtggag tgggagtcta atggccagcc tgagaataac 2820

tacaagacaa ccccacccgt gctggattct gacggcagct tctttctgta ttctaagctg 2880

accgtggaca agagcaggtg gcagcagggc aacgtgttca gctgctccgt gatgcacgaa 2940

gcactgcaca atcactacac ccagaaatca ctgtcactga gccctggcaa a 2991

<210> 43
<211> 3027
<212> DNA
<213> Artificial Sequence

<220>
<223> fusion protein

<400> 43
gctgggcctc gggctccttg tgctgccgcc tgcacatgtg caggcgattc cctggactgc 60

ggcggcagag gcctggccgc cctgcctggc gatctgccat cctggacccg gagcctgaac 120

ctgagctaca acaagctgag cgagatcgat cccgccggct ttgaggacct gcctaacctg 180

caggaggtgt atctgaacaa taacgagctg accgcggtac catccctggg cgctgcttca 240

tcacatgtcg tctctctctt tctgcagcac aacaagattc gcagcgtgga ggggagccag 300

ctgaaggcct acctttcctt agaagtgtta gatctgagtt tgaacaacat cacggaagtg 360

cggaacacct gctttccaca cggaccgcct ataaaggagc tcaacctggc aggcaatcgg 420

attggcaccc tggagttggg agcatttgat ggtctgtcac ggtcgctgct aactcttcgc 480

ctgagcaaaa acaggatcac ccagcttcct gtaagagcat tcaagctacc caggctgaca 540

caactggacc tcaatcggaa caggattcgg ctgatagagg gcctcacctt ccaggggctc 600

aacagcttgg aggtgctgaa gcttcagcga aacaacatca gcaaactgac agatggggcc 660

ttctggggac tgtccaagat gcatgtgctg cacctggagt acaacagcct ggtagaagtg 720

aacagcggct cgctctacgg cctcacggcc ctgcatcagc tccacctcag caacaattcc 780

atcgctcgca ttcaccgcaa gggctggagc ttctgccaga agctgcatga gttggtcctg 840

tccttcaaca acctgacacg gctggacgag gagagcctgg ccgagctgag cagcctgagt 900

gtcctgcgtc tcagccacaa ttccatcagc cacattgcgg agggtgcctt caagggactc 960

aggagcctgc gagtcttgga tctggaccat aacgagattt cgggcacaat agaggacacg 1020

agcggcgcct tctcagggct cgacagcctc agcaagctga ctctgtttgg aaacaagatc 1080

aagtctgtgg ctaagagagc attctcgggg ctggaaggcc tggagcacct gaaccttgga 1140

gggaatgcga tcagatctgt ccagtttgat gcctttgtga agatgaagaa tcttaaagag 1200

ctccatatca gcagcgacag cttcctgtgt gactgccagc tgaagtggct gcccccgtgg 1260

ctaattggca ggatgctgca ggcctttgtg acagccacct gtgcccaccc agaatcactg 1320

aagggtcaga gcattttctc tgtgccacca gagagtttcg tgtgcgatga cttcctgaag 1380

ccacagatca tcacccagcc agaaaccacc atggctatgg tgggcaagga catccggttt 1440

acatgctcag cagccagcag cagcagctcc cccatgacct ttgcctggaa gaaagacaat 1500

gaagtcctga ccaatgcaga catggagaac tttgtccacg tccacgcgca ggacggggaa 1560

gtgatggagt acaccaccat cctgcacctc cgtcaggtca ctttcgggca cgagggccgc 1620

taccaatgtg tcatcaccaa ccactttggc tccacctatt cacataaggc caggctcacc 1680

gtgaatgtgt tgccatcatt caccaaaacg ccccacgaca taaccatccg gaccaccacc 1740

gtggcccgcc tcgaatgtgc tgccacaggt cacccaaacc ctcagattgc ctggcagaag 1800

gatggaggca cggatttccc cgctgcccgt gagcgacgca tgcatgtcat gccggatgac 1860

gacgtgtttt tcatcactga tgtgaaaata gatgacgcag gggtttacag ctgtactgct 1920

cagaactcag ccggttctat ttcagctaat gccaccctga ctgtcctaga gaccccatcc 1980

ttggtggtcc ccttggaaga ccgtgtggta tctgtgggag aaacagtggc cctccaatgc 2040

aaagccacgg ggaaccctcc gccccgcatc acctggttca agggggaccg cccgctgagc 2100

ctcactgagc ggcaccacct gacccctgac aaccagctcc tggtggttca gaacgtggtg 2160

gcagaggatg cgggccgata tacctgtgag atgtccaaca ccctgggcac ggagcgagct 2220

cacagccagc tgagcgtcct gcccgcagca ggctgcagga aggatgggac cacggtaggc 2280

atcttcggat cccgcaacac cggccgcggc ggcgaggaga agaagaagga gaaggagaag 2340

gaggagcagg aggagcgcga gaccaagacc cccgagtgcc ccagccacac ccagcccctg 2400

ggcgtgttcc tgtttcctcc aaagcccaag gacacactga tgatctccag gacaccagag 2460

gtgacctgcg tggtggtgga cgtgagccac gaggaccccg aggtgaagtt caactggtac 2520

gtggatggcg tggaggtgca caatgccaag accaagccca gagaggagca gtacaactct 2580

acctataggg tggtgagcgt gctgacagtg ctgcaccagg actggctgaa cggcaaggag 2640

tataagtgca aggtgagcaa taaggccctg cctgccccaa tcgagaagac aatctccaag 2700

gccaagggcc agccaagaga gccccaggtg tacaccctgc cccctagcag ggatgagctg 2760

acaaagaacc aggtgtccct gacctgtctg gtgaagggct tttatccctc cgacatcgcc 2820

gtggagtggg agtctaatgg ccagcctgag aataactaca agacaacccc acccgtgctg 2880

gattctgacg gcagcttctt tctgtattct aagctgaccg tggacaagag caggtggcag 2940

cagggcaacg tgttcagctg ctccgtgatg cacgaagcac tgcacaatca ctacacccag 3000

aaatcactgt cactgagccc tggcaaa 3027

<210> 44
<211> 2988
<212> DNA
<213> Artificial Sequence

<220>
<223>    fusion protein

<400>    44
gctgggcctc gggctccttg tgctgccgcc tgcacatgtg caggcgattc cctggactgc      60

ggcggcagag gcctggccgc cctgcctggc gatctgccat cctggacccg gagcctgaac     120

ctgagctaca acaagctgag cgagatcgat cccgccggct ttgaggacct gcctaacctg     180

caggaggtgt atctgaacaa taacgagctg accgcggtac catccctggg cgctgcttca     240

tcacatgtcg tctctctctt tctgcagcac aacaagattc gcagcgtgga ggggagccag     300

ctgaaggcct acctttcctt agaagtgtta gatctgagtt tgaacaacat cacggaagtg     360

cggaacacct gctttccaca cggaccgcct ataaaggagc tcaacctggc aggcaatcgg     420

attggcaccc tggagttggg agcatttgat ggtctgtcac ggtcgctgct aactcttcgc     480

ctgagcaaaa acaggatcac ccagcttcct gtaagagcat tcaagctacc caggctgaca     540

caactggacc tcaatcggaa caggattcgg ctgatagagg gcctcacctt ccaggggctc     600

aacagcttgg aggtgctgaa gcttcagcga aacaacatca gcaaactgac agatggggcc     660

ttctggggac tgtccaagat gcatgtgctg cacctggagt acaacagcct ggtagaagtg     720

aacagcggct cgctctacgg cctcacggcc ctgcatcagc tccacctcag caacaattcc     780

atcgctcgca ttcaccgcaa gggctggagc ttctgccaga agctgcatga gttggtcctg     840

tccttcaaca acctgacacg gctggacgag gagagcctgg ccgagctgag cagcctgagt     900

gtcctgcgtc tcagccacaa ttccatcagc cacattgcgg agggtgcctt caagggactc     960

aggagcctgc gagtcttgga tctggaccat aacgagattt cgggcacaat agaggacacg    1020

agcggcgcct tctcagggct cgacagcctc agcaagctga ctctgtttgg aaacaagatc    1080

aagtctgtgg ctaagagagc attctcgggg ctggaaggcc tggagcacct gaaccttgga    1140

gggaatgcga tcagatctgt ccagtttgat gcctttgtga agatgaagaa tcttaaagag    1200

ctccatatca gcagcgacag cttcctgtgt gactgccagc tgaagtggct gcccccgtgg    1260

ctaattggca ggatgctgca ggcctttgtg acagccacct gtgcccaccc agaatcactg    1320

aagggtcaga gcattttctc tgtgccacca gagagtttcg tgtgcgatga cttcctgaag    1380

ccacagatca tcacccagcc agaaaccacc atggctatgg tgggcaagga catccggttt    1440

acatgctcag cagccagcag cagcagctcc cccatgacct ttgcctggaa gaaagacaat    1500

gaagtcctga ccaatgcaga catggagaac tttgtccacg tccacgcgca ggacggggaa    1560

gtgatggagt acaccaccat cctgcacctc cgtcaggtca ctttcgggca cgagggccgc    1620

taccaatgtg tcatcaccaa ccactttggc tccacctatt cacataaggc caggctcacc    1680

gtgaatgtgt tgccatcatt caccaaaacg ccccacgaca taaccatccg gaccaccacc    1740

gtggcccgcc tcgaatgtgc tgccacaggt cacccaaacc ctcagattgc ctggcagaag 1800

gatggaggca cggatttccc cgctgcccgt gagcgacgca tgcatgtcat gccggatgac 1860

gacgtgtttt tcatcactga tgtgaaaata gatgacgcag gggtttacag ctgtactgct 1920

cagaactcag ccggttctat ttcagctaat gccaccctga ctgtcctaga gaccccatcc 1980

ttggtggtcc ccttggaaga ccgtgtggta tctgtgggag aaacagtggc cctccaatgc 2040

aaagccacgg ggaaccctcc gccccgcatc acctggttca agggggaccg cccgctgagc 2100

ctcactgagc ggcaccacct gacccctgac aaccagctcc tggtggttca gaacgtggtg 2160

gcagaggatg cgggccgata tacctgtgag atgtccaaca ccctgggcac ggagcgagct 2220

cacagccagc tgagcgtcct gcccgcagca ggctgcagga aggatgggac cacggtaggc 2280

atcttcggat ccgaaccgaa atcttctgac aaaacccaca cctctccgcc gtctccggct 2340

ccggaactgc tgggtggttc ttctgttttc ctgtttcctc caaagcccaa ggacacactg 2400

atgatctcca ggacaccaga ggtgacctgc gtggtggtgg acgtgagcca cgaggacccc 2460

gaggtgaagt tcaactggta cgtggatggc gtggaggtgc acaatgccaa gaccaagccc 2520

agagaggagc agtacaactc tacctatagg gtggtgagcg tgctgacagt gctgcaccag 2580

gactggctga acggcaagga gtataagtgc aaggtgagca ataaggccct gcctgcccca 2640

atcgagaaga caatctccaa ggccaagggc cagccaagag agccccaggt gtacaccctg 2700

ccccctagca gggatgagct gacaaagaac caggtgtccc tgacctgtct ggtgaagggc 2760

ttttatccct ccgacatcgc cgtggagtgg gagtctaatg gccagcctga gaataactac 2820

aagacaaccc cacccgtgct ggattctgac ggcagcttct ttctgtattc taagctgacc 2880

gtggacaaga gcaggtggca gcagggcaac gtgttcagct gctccgtgat gcacgaagca 2940

ctgcacaatc actacaccca gaaatcactg tcactgagcc ctggcaaa 2988

<210> 45
<211> 994
<212> PRT
<213> Artificial Sequence

<220>
<223> fusion protein

<400> 45
Ala Gln Ala Gly Pro Arg Ala Pro Cys Ala Ala Ala Cys Thr Cys Ala
1               5                   10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Gly Ser Glu Pro Lys Ser Ser Asp
        755                 760                 765

Lys Thr His Thr Ser Pro Pro Cys Pro Ala Pro Glu Leu Leu Gly Gly
    770                 775                 780

Pro Ser Val Phe Leu Phe Pro Pro Lys Pro Lys Asp Thr Leu Met Ile
785                 790                 795                 800

Ser Arg Thr Pro Glu Val Thr Cys Val Val Val Asp Val Ser His Glu
                805                 810                 815

Asp Pro Glu Val Lys Phe Asn Trp Tyr Val Asp Gly Val Glu Val His
            820                 825                 830

Asn Ala Lys Thr Lys Pro Arg Glu Glu Gln Tyr Asn Ser Thr Tyr Arg
        835                 840                 845

Val Val Ser Val Leu Thr Val Leu His Gln Asp Trp Leu Asn Gly Lys
    850                 855                 860

Glu Tyr Lys Cys Lys Val Ser Asn Lys Ala Leu Pro Ala Pro Ile Glu
865                 870                 875                 880

Lys Thr Ile Ser Lys Ala Lys Gly Gln Pro Arg Glu Pro Gln Val Tyr
                885                 890                 895

Thr Leu Pro Pro Ser Arg Asp Glu Leu Thr Lys Asn Gln Val Ser Leu
            900                 905                 910

Thr Cys Leu Val Lys Gly Phe Tyr Pro Ser Asp Ile Ala Val Glu Trp
        915                 920                 925

Glu Ser Asn Gly Gln Pro Glu Asn Asn Tyr Lys Thr Thr Pro Pro Val
    930                 935                 940

Leu Asp Ser Asp Gly Ser Phe Phe Leu Tyr Ser Lys Leu Thr Val Asp
945                 950                 955                 960

Lys Ser Arg Trp Gln Gln Gly Asn Val Phe Ser Cys Ser Val Met His
                965                 970                 975

Glu Ala Leu His Asn His Tyr Thr Gln Lys Ser Leu Ser Leu Ser Pro
            980                 985                 990

Gly Lys



<210>   46
<211>   995
<212>   PRT
<213>   Artificial Sequence

<220>
<223>   fusion protein

<400>    46
   Ala Gln Ala Gly Pro Arg Ala Pro Cys Ala Ala Ala Cys Thr Cys Ala
   1               5                   10                  15
   Gly Asp Ser Leu Asp Cys Ser Gly Arg Gly Leu Ala Thr Leu Pro Arg
               20                  25                  30
   Asp Leu Pro Ser Trp Thr Arg Ser Leu Asn Leu Ser Tyr Asn Arg Leu
           35                  40                  45
   Ser Glu Ile Asp Ser Ala Ala Phe Glu Asp Leu Thr Asn Leu Gln Glu
       50                  55                  60
   Val Tyr Leu Asn Ser Asn Glu Leu Thr Ala Ile Pro Ser Leu Gly Ala
   65                  70                  75                  80
   Ala Ser Ile Gly Val Val Ser Leu Phe Leu Gln His Asn Lys Ile Leu
                   85                  90                  95
   Ser Val Asp Gly Ser Gln Leu Lys Ser Tyr Leu Ser Leu Glu Val Leu
               100                 105                 110
   Asp Leu Ser Ser Asn Asn Ile Thr Glu Ile Arg Ser Ser Cys Phe Pro
           115                 120                 125
   Asn Gly Leu Arg Ile Arg Glu Leu Asn Leu Ala Ser Asn Arg Ile Ser
       130                 135                 140
   Ile Leu Glu Ser Gly Ala Phe Asp Gly Leu Ser Arg Ser Leu Leu Thr
   145                 150                 155                 160
   Leu Arg Leu Ser Lys Asn Arg Ile Thr Gln Leu Pro Val Lys Ala Phe
                   165                 170                 175
   Lys Leu Pro Arg Leu Thr Gln Leu Asp Leu Asn Arg Asn Arg Ile Arg
               180                 185                 190
   Leu Ile Glu Gly Leu Thr Phe Gln Gly Leu Asp Ser Leu Glu Val Leu
           195                 200                 205
   Arg Leu Gln Arg Asn Asn Ile Ser Arg Leu Thr Asp Gly Ala Phe Trp
       210                 215                 220
   Gly Leu Ser Lys Met His Val Leu His Leu Glu Tyr Asn Ser Leu Val
   225                 230                 235                 240
   Glu Val Asn Ser Gly Ser Leu Tyr Gly Leu Thr Ala Leu His Gln Leu
                   245                 250                 255
   His Leu Ser Asn Asn Ser Ile Ser Arg Ile Gln Arg Asp Gly Trp Ser
               260                 265                 270
   Phe Cys Gln Lys Leu His Glu Leu Ile Leu Ser Phe Asn Asn Leu Thr
           275                 280                 285
   Arg Leu Asp Glu Glu Ser Leu Ala Glu Leu Ser Ser Leu Ser Ile Leu
       290                 295                 300
   Arg Leu Ser His Asn Ala Ile Ser His Ile Ala Glu Gly Ala Phe Lys
   305                 310                 315                 320
   Gly Leu Lys Ser Leu Arg Val Leu Asp Leu Asp His Asn Glu Ile Ser
   325 330 335
   Gly Thr Ile Glu Asp Thr Ser Gly Ala Phe Thr Gly Leu Asp Asn Leu
   340 345 350
   Ser Lys Leu Thr Leu Phe Gly Asn Lys Ile Lys Ser Val Ala Lys Arg
   355 360 365
   Ala Phe Ser Gly Leu Glu Ser Leu Glu His Leu Asn Leu Gly Glu Asn
   370 375 380
   Ala Ile Arg Ser Val Gln Phe Asp Ala Phe Ala Lys Met Lys Asn Leu
   385 390 395 400
   Lys Glu Leu Tyr Ile Ser Ser Glu Ser Phe Leu Cys Asp Cys Gln Leu
   405 410 415
   Lys Trp Leu Pro Pro Trp Leu Met Gly Arg Met Leu Gln Ala Phe Val
   420 425 430
   Thr Ala Thr Cys Ala His Pro Glu Ser Leu Lys Gly Gln Ser Ile Phe
   435 440 445
   Ser Val Leu Pro Asp Ser Phe Val Cys Asp Asp Phe Pro Lys Pro Gln
   450 455 460
   Ile Ile Thr Gln Pro Glu Thr Thr Met Ala Val Val Gly Lys Asp Ile
   465 470 475 480
   Arg Phe Thr Cys Ser Ala Ala Ser Ser Ser Ser Ser Pro Met Thr Phe
   485 490 495
   Ala Trp Lys Lys Asp Asn Glu Val Leu Ala Asn Ala Asp Met Glu Asn
   500 505 510
   Phe Ala His Val Arg Ala Gln Asp Gly Glu Val Met Glu Tyr Thr Thr
   515 520 525
   Ile Leu His Leu Arg His Val Thr Phe Gly His Glu Gly Arg Tyr Gln
   530 535 540
   Cys Ile Ile Thr Asn His Phe Gly Ser Thr Tyr Ser His Lys Ala Arg
   545 550 555 560
   Leu Thr Val Asn Val Leu Pro Ser Phe Thr Lys Ile Pro His Asp Ile
   565 570 575
   Ala Ile Arg Thr Gly Thr Thr Ala Arg Leu Glu Cys Ala Ala Thr Gly
   580 585 590
   His Pro Asn Pro Gln Ile Ala Trp Gln Lys Asp Gly Gly Thr Asp Phe
   595 600 605
   Pro Ala Ala Arg Glu Arg Arg Met His Val Met Pro Asp Asp Asp Val
   610 615 620
   Phe Phe Ile Thr Asp Val Lys Ile Asp Asp Met Gly Val Tyr Ser Cys
   625 630 635 640
   Thr Ala Gln Asn Ser Ala Gly Ser Val Ser Ala Asn Ala Thr Leu Thr
   645 650 655
   Val Leu Glu Thr Pro Ser Leu Ala Val Pro Leu Glu Asp Arg Val Val
   660 665 670
   Thr Val Gly Glu Thr Val Ala Phe Gln Cys Lys Ala Thr Gly Ser Pro
   675 680 685
   Thr Pro Arg Ile Thr Trp Leu Lys Gly Gly Arg Pro Leu Ser Leu Thr
   690 695 700
   Glu Arg His His Phe Thr Pro Gly Asn Gln Leu Leu Val Val Gln Asn
   705 710 715 720
   Val Met Ile Asp Asp Ala Gly Arg Tyr Thr Cys Glu Met Ser Asn Pro
   725 730 735
   Leu Gly Thr Glu Arg Ala His Ser Gln Leu Ser Ile Leu Pro Thr Pro
   740 745 750
   Gly Cys Arg Lys Asp Gly Thr Thr Gly Ser Glu Pro Arg Gly Pro Thr
   755 760 765
   Ile Lys Pro Cys Pro Pro Cys Lys Cys Pro Ala Pro Asn Leu Leu Gly
   770 775 780
   Gly Pro Ser Val Phe Leu Phe Pro Pro Lys Pro Lys Asp Thr Leu Met
   785 790 795 800
   Ile Ser Arg Thr Pro Glu Val Thr Cys Val Val Val Asp Val Ser His
   805 810 815
   Glu Asp Pro Glu Val Lys Phe Asn Trp Tyr Val Asp Gly Val Glu Val
   820 825 830
   His Asn Ala Lys Thr Lys Pro Arg Glu Glu Gln Tyr Asn Ser Thr Tyr
   835 840 845
   Arg Val Val Ser Val Leu Thr Val Leu His Gln Asp Trp Leu Asn Gly
   850 855 860
   Lys Glu Tyr Lys Cys Lys Val Ser Asn Lys Ala Leu Pro Ala Pro Ile
   865 870 875 880
   Glu Lys Thr Ile Ser Lys Ala Lys Gly Gln Pro Arg Glu Pro Gln Val
   885 890 895
   Tyr Thr Leu Pro Pro Ser Arg Asp Glu Leu Thr Lys Asn Gln Val Ser
   900 905 910
   Leu Thr Cys Leu Val Lys Gly Phe Tyr Pro Ser Asp Ile Ala Val Glu
   915 920 925
   Trp Glu Ser Asn Gly Gln Pro Glu Asn Asn Tyr Lys Thr Thr Pro Pro
   930 935 940
   Val Leu Asp Ser Asp Gly Ser Phe Phe Leu Tyr Ser Lys Leu Thr Val
   945 950 955 960
   Asp Lys Ser Arg Trp Gln Gln Gly Asn Val Phe Ser Cys Ser Val Met
   965 970 975
   His Glu Ala Leu His Asn His Tyr Thr Gln Lys Ser Leu Ser Leu Ser
   980 985 990
   Pro Gly Lys
   995

<210>   47
<211>   1007
<212>   PRT
<213>   Artificial Sequence

<220>
<223>   fusion protein

<400>   47
Ala Gln Ala Gly Pro Arg Ala Pro Cys Ala Ala Ala Cys Thr Cys Ala
1               5                   10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Gly Ser Arg Asn Thr Gly Arg Gly
        755                 760                 765

Gly Glu Glu Lys Lys Lys Glu Lys Glu Lys Glu Glu Gln Glu Glu Arg
    770                 775                 780

Glu Thr Lys Thr Pro Glu Cys Pro Ser His Thr Gln Pro Leu Gly Val
785                 790                 795                 800

Phe Leu Phe Pro Pro Lys Pro Lys Asp Thr Leu Met Ile Ser Arg Thr
                805                 810                 815

Pro Glu Val Thr Cys Val Val Val Asp Val Ser His Glu Asp Pro Glu
            820                 825                 830

Val Lys Phe Asn Trp Tyr Val Asp Gly Val Glu Val His Asn Ala Lys
        835                 840                 845

Thr Lys Pro Arg Glu Glu Gln Tyr Asn Ser Thr Tyr Arg Val Val Ser
    850                 855                 860

Val Leu Thr Val Leu His Gln Asp Trp Leu Asn Gly Lys Glu Tyr Lys
865                 870                 875                 880

Cys Lys Val Ser Asn Lys Ala Leu Pro Ala Pro Ile Glu Lys Thr Ile
                885                 890                 895

Ser Lys Ala Lys Gly Gln Pro Arg Glu Pro Gln Val Tyr Thr Leu Pro
            900                 905                 910

Pro Ser Arg Asp Glu Leu Thr Lys Asn Gln Val Ser Leu Thr Cys Leu
        915                 920                 925

Val Lys Gly Phe Tyr Pro Ser Asp Ile Ala Val Glu Trp Glu Ser Asn
    930                 935                 940

Gly Gln Pro Glu Asn Asn Tyr Lys Thr Thr Pro Pro Val Leu Asp Ser
945                 950                 955                 960

Asp Gly Ser Phe Phe Leu Tyr Ser Lys Leu Thr Val Asp Lys Ser Arg
                965                 970                 975

Trp Gln Gln Gly Asn Val Phe Ser Cys Ser Val Met His Glu Ala Leu
            980                 985                 990

His Asn His Tyr Thr Gln Lys Ser Leu Ser Leu Ser Pro Gly Lys
        995                 1000                1005

<210>    48
<211>    994
<212>    PRT
<213>    Artificial Sequence

<220>
<223>    fusion protein

<400>    48
Ala Gln Ala Gly Pro Arg Ala Pro Cys Ala Ala Ala Cys Thr Cys Ala
1               5                   10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu

195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln

530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Gly Ser Glu Pro Lys Ser Ser Asp
        755                 760                 765

Lys Thr His Thr Ser Pro Pro Ser Pro Ala Pro Glu Leu Leu Gly Gly
    770                 775                 780

Ser Ser Val Phe Leu Phe Pro Pro Lys Pro Lys Asp Thr Leu Met Ile
785                 790                 795                 800

Ser Arg Thr Pro Glu Val Thr Cys Val Val Val Asp Val Ser His Glu
                805                 810                 815

Asp Pro Glu Val Lys Phe Asn Trp Tyr Val Asp Gly Val Glu Val His
            820                 825                 830

Asn Ala Lys Thr Lys Pro Arg Glu Glu Gln Tyr Asn Ser Thr Tyr Arg
        835                 840                 845

Val Val Ser Val Leu Thr Val Leu His Gln Asp Trp Leu Asn Gly Lys
    850                 855                 860

Glu Tyr Lys Cys Lys Val Ser Asn Lys Ala Leu Pro Ala Pro Ile Glu

865                 870                 875                 880

Lys Thr Ile Ser Lys Ala Lys Gly Gln Pro Arg Glu Pro Gln Val Tyr
                885                 890                 895

Thr Leu Pro Pro Ser Arg Asp Glu Leu Thr Lys Asn Gln Val Ser Leu
            900                 905                 910

Thr Cys Leu Val Lys Gly Phe Tyr Pro Ser Asp Ile Ala Val Glu Trp
        915                 920                 925

Glu Ser Asn Gly Gln Pro Glu Asn Asn Tyr Lys Thr Thr Pro Pro Val
    930                 935                 940

Leu Asp Ser Asp Gly Ser Phe Phe Leu Tyr Ser Lys Leu Thr Val Asp
945                 950                 955                 960

Lys Ser Arg Trp Gln Gln Gly Asn Val Phe Ser Cys Ser Val Met His
                965                 970                 975

Glu Ala Leu His Asn His Tyr Thr Gln Lys Ser Leu Ser Leu Ser Pro
            980                 985                 990

Gly Lys

<210>    49
<211>    2982
<212>    DNA
<213>    Artificial Sequence

<220>
<223>    fusion protein

<400>    49
gctcaggctg gacctagggc tccttgcgct gccgcctgca cctgtgcagg cgattctctg      60

gactgcagcg gccggggcct ggccacactg cccagggacc tgccttcctg gaccagatct     120

ctgaacctga gctacaatcg gctgtccgag atcgattctg ccgcctttga ggacctgaca     180

aatctgcagg aggtgtatct gaacagcaat gagctgaccg caatcccctc cctgggagca     240

gcctctatcg gcgtggtgag cctgttcctg cagcacaaca agatcctgag cgtggatggc     300

tcccagctga agagctacct gtctctggag gtgctggacc tgagctccaa caatatcacc     360

gagatcagat ctagctgttt tcctaatggc ctgcggatca gagagctgaa cctggcctct     420

aatcggatca gcatcctgga gtccggcgcc ttcgatggcc tgagcagatc cctgctgaca     480

ctgcgcctgt ccaagaaccg gatcacccag ctgcccgtga aggcctttaa gctgcctagg     540

ctgacacagc tggacctgaa ccggaataga atcaggctga tcgagggcct gaccttccag     600

ggcctggata gcctggaggt gctgcgcctg cagcggaaca atatctcccg cctgacagac     660

ggagcatttt ggggcctgtc taagatgcac gtgctgcacc tggagtacaa tagcctggtg     720

gaggtgaact ctggcagcct gtatggcctg accgccctgc accagctgca cctgtccaac     780

aatagcatca gcagaatcca gagggatggc tggtccttct gccagaagct gcacgagctg 840

atcctgtctt ttaacaatct gaccaggctg gacgaggaga gcctggcaga gctgtcctct 900

ctgtccatcc tgcgcctgtc tcacaatgcc atcagccaca tcgccgaggg cgcctttaag 960

ggcctgaaga gcctgagggt gctggatctg gaccacaacg agatctctgg caccatcgag 1020

gatacaagcg gcgccttcac aggcctggac aatctgtcca agctgaccct gtttggcaac 1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

ggatccgagc caaagtcctc tgataagaca cacacctctc caccatgccc agcaccagag 2340

ctgctgggag gaccaagcgt gttcctgttt cctccaaagc ccaaggacac actgatgatc 2400

tccaggacac cagaggtgac ctgcgtggtg gtggacgtga gccacgagga ccccgaggtg 2460

aagttcaact ggtacgtgga tggcgtggag gtgcacaatg ccaagaccaa gcccagagag 2520

gagcagtaca actctaccta tagggtggtg agcgtgctga cagtgctgca ccaggactgg 2580

ctgaacggca aggagtataa gtgcaaggtg agcaataagg ccctgcctgc ccccatcgag 2640

aagacaatct ccaaggccaa gggccagcca agagagcccc aggtgtacac cctgccccct 2700

agcagggatg agctgacaaa gaaccaggtg tccctgacct gtctggtgaa gggcttttat 2760

ccctccgaca tcgccgtgga gtgggagtct aatggccagc ctgagaataa ctacaagaca 2820

accccacccg tgctggattc tgacggcagc ttctttctgt attctaagct gaccgtggac 2880

aagagcaggt ggcagcaggg caacgtgttc agctgctccg tgatgcacga agcactgcac 2940

aatcactaca cccagaaatc actgtcactg agccctggca aa 2982

<210> 50
<211> 2985
<212> DNA
<213> Artificial Sequence

<220>
<223> fusion protein

<400> 50
gctcaggctg gacctagggc tccttgcgct gccgcctgca cctgtgcagg cgattctctg 60

gactgcagcg gccggggcct ggccacactg cccagggacc tgccttcctg gaccagatct 120

ctgaacctga gctacaatcg gctgtccgag atcgattctg ccgcctttga ggacctgaca 180

aatctgcagg aggtgtatct gaacagcaat gagctgaccg caatcccctc cctgggagca 240

gcctctatcg gcgtggtgag cctgttcctg cagcacaaca agatcctgag cgtggatggc 300

tcccagctga agagctacct gtctctggag gtgctggacc tgagctccaa caatatcacc 360

gagatcagat ctagctgttt tcctaatggc ctgcggatca gagagctgaa cctggcctct 420

aatcggatca gcatcctgga gtccggcgcc ttcgatggcc tgagcagatc cctgctgaca 480

ctgcgcctgt ccaagaaccg gatcacccag ctgcccgtga aggcctttaa gctgcctagg 540

ctgacacagc tggacctgaa ccggaataga atcaggctga tcgagggcct gaccttccag 600

ggcctggata gcctggaggt gctgcgcctg cagcggaaca atatctcccg cctgacagac 660

ggagcatttt ggggcctgtc taagatgcac gtgctgcacc tggagtacaa tagcctggtg 720

gaggtgaact ctggcagcct gtatggcctg accgccctgc accagctgca cctgtccaac 780

aatagcatca gcagaatcca gagggatggc tggtccttct gccagaagct gcacgagctg 840

atcctgtctt ttaacaatct gaccaggctg gacgaggaga gcctggcaga gctgtcctct 900

ctgtccatcc tgcgcctgtc tcacaatgcc atcagccaca tcgccgaggg cgcctttaag 960

ggcctgaaga gcctgagggt gctggatctg gaccacaacg agatctctgg caccatcgag 1020

gatacaagcg gcgccttcac aggcctggac aatctgtcca agctgaccct gtttggcaac 1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

ggatccgagc ctcgggggcc taccatcaag ccctgccccc cttgcaagtg ccctgcccct 2340

aatctgctgg gcggaccctc cgtgttcctg tttcctccaa agcccaagga cacactgatg 2400

atctccagga caccagaggt gacctgcgtg gtggtggacg tgagccacga ggaccccgag 2460

gtgaagttca actggtacgt ggatggcgtg gaggtgcaca atgccaagac caagcccaga 2520

gaggagcagt acaactctac ctatagggtg gtgagcgtgc tgacagtgct gcaccaggac 2580

tggctgaacg gcaaggagta taagtgcaag gtgagcaata aggccctgcc tgccccaatc 2640

gagaagacaa tctccaaggc caagggccag ccaagagagc cccaggtgta caccctgccc 2700

cctagcaggg atgagctgac aaagaaccag gtgtccctga cctgtctggt gaagggcttt 2760

tatccctccg acatcgccgt ggagtgggag tctaatggcc agcctgagaa taactacaag 2820

acaaccccac ccgtgctgga ttctgacggc agcttctttc tgtattctaa gctgaccgtg 2880

gacaagagca ggtggcagca gggcaacgtg ttcagctgct ccgtgatgca cgaagcactg 2940

cacaatcact acacccagaa atcactgtca ctgagccctg gcaaa 2985

<210> 51
<211> 3021
<212> DNA

<213>    Artificial Sequence

<220>
<223>    fusion protein

<400>    51
gctcaggctg gacctagggc tccttgcgct gccgcctgca cctgtgcagg cgattctctg      60

gactgcagcg gccggggcct ggccacactg cccagggacc tgccttcctg gaccagatct     120

ctgaacctga gctacaatcg gctgtccgag atcgattctg ccgcctttga ggacctgaca     180

aatctgcagg aggtgtatct gaacagcaat gagctgaccg caatcccctc cctgggagca     240

gcctctatcg gcgtggtgag cctgttcctg cagcacaaca agatcctgag cgtggatggc     300

tcccagctga agagctacct gtctctggag gtgctggacc tgagctccaa caatatcacc     360

gagatcagat ctagctgttt tcctaatggc ctgcggatca gagagctgaa cctggcctct     420

aatcggatca gcatcctgga gtccggcgcc ttcgatggcc tgagcagatc cctgctgaca     480

ctgcgcctgt ccaagaaccg gatcacccag ctgcccgtga aggcctttaa gctgcctagg     540

ctgacacagc tggacctgaa ccggaataga atcaggctga tcgagggcct gaccttccag     600

ggcctggata gcctggaggt gctgcgcctg cagcggaaca atatctcccg cctgacagac     660

ggagcatttt ggggcctgtc taagatgcac gtgctgcacc tggagtacaa tagcctggtg     720

gaggtgaact ctggcagcct gtatggcctg accgccctgc accagctgca cctgtccaac     780

aatagcatca gcagaatcca gagggatggc tggtccttct gccagaagct gcacgagctg     840

atcctgtctt ttaacaatct gaccaggctg gacgaggaga gcctggcaga gctgtcctct     900

ctgtccatcc tgcgcctgtc tcacaatgcc atcagccaca tcgccgaggg cgcctttaag     960

ggcctgaaga gcctgagggt gctggatctg gaccacaacg agatctctgg caccatcgag    1020

gatacaagcg gcgccttcac aggcctggac aatctgtcca agctgaccct gtttggcaac    1080

aagatcaagt ctgtggccaa gcgggccttc tctggcctgg agagcctgga gcacctgaac    1140

ctgggcgaga atgccatcag atccgtgcag ttcgatgcct ttgccaagat gaagaatctg    1200

aaggagctgt acatcagctc cgagagcttc ctgtgcgact gtcagctgaa gtggctgcca    1260

ccttggctga tgggaaggat gctgcaggcc tttgtgaccg ccacatgcgc ccacccagag    1320

agcctgaagg gccagagcat cttctccgtg ctgcccgata gcttcgtgtg cgacgatttt    1380

cctaagccac agatcatcac ccagccagag acaacaatgg ccgtggtggg caaggacatc    1440

cggtttacat gttccgccgc ctctagctcc tctagcccca tgaccttcgc ctggaagaag    1500

gataacgagg tgctggccaa tgccgacatg gagaacttcg cccacgtgag agcccaggat    1560

ggcgaagtga tggagtatac cacaatcctg cacctgcggc acgtgacctt tggccacgag    1620

ggcagatacc agtgcatcat cacaaatcac ttcggctcta cctatagcca caaggccagg    1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

ggatcccgca acaccggccg cggcggcgag gagaagaaga aggagaagga gaaggaggag 2340

caggaggagc gcgagaccaa gacccccgag tgccccagcc acacccagcc cctgggcgtg 2400

ttcctgtttc ctccaaagcc caaggacaca ctgatgatct ccaggacacc agaggtgacc 2460

tgcgtggtgg tggacgtgag ccacgaggac cccgaggtga agttcaactg gtacgtggat 2520

ggcgtggagg tgcacaatgc caagaccaag cccagagagg agcagtacaa ctctacctat 2580

agggtggtga gcgtgctgac agtgctgcac caggactggc tgaacggcaa ggagtataag 2640

tgcaaggtga gcaataaggc cctgcctgcc ccaatcgaga agacaatctc caaggccaag 2700

ggccagccaa gagagcccca ggtgtacacc ctgcccccta gcagggatga gctgacaaag 2760

aaccaggtgt ccctgacctg tctggtgaag ggcttttatc cctccgacat cgccgtggag 2820

tgggagtcta atggccagcc tgagaataac tacaagacaa ccccacccgt gctggattct 2880

gacggcagct tctttctgta ttctaagctg accgtggaca agagcaggtg gcagcagggc 2940

aacgtgttca gctgctccgt gatgcacgaa gcactgcaca atcactacac ccagaaatca 3000

ctgtcactga gccctggcaa a 3021

<210> 52
<211> 2982
<212> DNA
<213> Artificial Sequence

<220>
<223> fusion protein

<400> 52
gctcaggctg gacctagggc tccttgcgct gccgcctgca cctgtgcagg cgattctctg 60

gactgcagcg gccgggccct ggccacactg cccagggacc tgccttcctg gaccagatct 120

ctgaacctga gctacaatcg gctgtccgag atcgattctg ccgcctttga ggacctgaca 180

aatctgcagg aggtgtatct gaacagcaat gagctgaccg caatcccctc cctgggagca 240

gcctctatcg gcgtggtgag cctgttcctg cagcacaaca agatcctgag cgtggatggc 300

tcccagctga agagctacct gtctctggag gtgctggacc tgagctccaa caatatcacc 360

gagatcagat ctagctgttt tcctaatggc ctgcggatca gagagctgaa cctggcctct 420

aatcggatca gcatcctgga gtccggcgcc ttcgatggcc tgagcagatc cctgctgaca 480

ctgcgcctgt ccaagaaccg gatcacccag ctgcccgtga aggcctttaa gctgcctagg 540

ctgacacagc tggacctgaa ccggaataga atcaggctga tcgagggcct gaccttccag 600

ggcctggata gcctggaggt gctgcgcctg cagcggaaca atatctcccg cctgacagac 660

ggagcatttt ggggcctgtc taagatgcac gtgctgcacc tggagtacaa tagcctggtg 720

gaggtgaact ctggcagcct gtatggcctg accgccctgc accagctgca cctgtccaac 780

aatagcatca gcagaatcca gagggatggc tggtccttct gccagaagct gcacgagctg 840

atcctgtctt ttaacaatct gaccaggctg gacgaggaga gcctggcaga gctgtcctct 900

ctgtccatcc tgcgcctgtc tcacaatgcc atcagccaca tcgccgaggg cgcctttaag 960

ggcctgaaga gcctgagggt gctggatctg gaccacaacg agatctctgg caccatcgag 1020

gatacaagcg gcgccttcac aggcctggac aatctgtcca agctgaccct gtttggcaac 1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

ggatccgaac cgaaatcttc tgacaaaacc cacacctctc cgccgtctcc ggctccggaa 2340

ctgctgggtg gttcttctgt tttcctgttt cctccaaagc ccaaggacac actgatgatc 2400

tccaggacac cagaggtgac ctgcgtggtg gtggacgtga gccacgagga ccccgaggtg 2460

aagttcaact ggtacgtgga tggcgtggag gtgcacaatg ccaagaccaa gcccagagag 2520

gagcagtaca actctaccta tagggtggtg agcgtgctga cagtgctgca ccaggactgg 2580

ctgaacggca aggagtataa gtgcaaggtg agcaataagg ccctgcctgc cccaatcgag 2640

aagacaatct ccaaggccaa gggccagcca agagagcccc aggtgtacac cctgccccct 2700

agcagggatg agctgacaaa gaaccaggtg tccctgacct gtctggtgaa gggcttttat 2760

ccctccgaca tcgccgtgga gtgggagtct aatggccagc ctgagaataa ctacaagaca 2820

accccacccg tgctggattc tgacggcagc ttctttctgt attctaagct gaccgtggac 2880

aagagcaggt ggcagcaggg caacgtgttc agctgctccg tgatgcacga agcactgcac 2940

aatcactaca cccagaaatc actgtcactg agccctggca aa 2982

<210> 53
<211> 994
<212> PRT
<213> Artificial Sequence

<220>
<223> fusion protein

<400> 53
Ala Gln Ala Gly Pro Arg Ala Pro Cys Ala Ala Ala Cys Thr Cys Ala
1               5                   10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Gly Ser Glu Pro Lys Ser Ser Asp
        755                 760                 765

Lys Thr His Thr Ser Pro Pro Cys Pro Ala Pro Glu Leu Leu Gly Gly
    770                 775                 780

Pro Ser Val Phe Ile Phe Pro Pro Lys Ile Lys Asp Val Leu Met Ile
785                 790                 795                 800

Ser Leu Ser Pro Ile Val Thr Cys Val Val Val Asp Val Ser Glu Asp
                805                 810                 815

Asp Pro Asp Val Gln Ile Ser Trp Phe Val Asn Asn Val Glu Val His
            820                 825                 830

Thr Ala Gln Thr Gln Thr His Arg Glu Asp Tyr Asn Ser Thr Leu Arg
        835                 840                 845

Val Val Ser Ala Leu Pro Ile Gln His Gln Asp Trp Met Ser Gly Lys
    850                 855                 860

Glu Phe Lys Cys Lys Val Asn Asn Lys Asp Leu Pro Ala Pro Ile Glu
865                 870                 875                 880

Arg Thr Ile Ser Lys Pro Lys Gly Ser Val Arg Ala Pro Gln Val Tyr
                885                 890                 895

Val Leu Pro Pro Pro Glu Glu Glu Met Thr Lys Lys Gln Val Thr Leu
            900                 905                 910

Thr Cys Met Val Thr Asp Phe Met Pro Glu Asp Ile Tyr Val Glu Trp
        915                 920                 925

Thr Asn Asn Gly Lys Thr Glu Leu Asn Tyr Lys Asn Thr Glu Pro Val
    930                 935                 940

Leu Asp Ser Asp Gly Ser Tyr Phe Met Tyr Ser Lys Leu Arg Val Glu
945                 950                 955                 960

Lys Lys Asn Trp Val Glu Arg Asn Ser Tyr Ser Cys Ser Val Val His
                965                 970                 975

Glu Gly Leu His Asn His His Thr Thr Lys Ser Phe Ser Arg Thr Pro
            980                 985                 990

Gly Lys

<210>    54
<211>    995
<212>    PRT
<213>    Artificial Sequence

<220>
<223>    fusion protein

<400>    54
Ala Gln Ala Gly Pro Arg Ala Pro Cys Ala Ala Ala Cys Thr Cys Ala
  1               5                  10                  15

Gly Asp Ser Leu Asp Cys Ser Gly Arg Gly Leu Ala Thr Leu Pro Arg
             20                  25                  30

Asp Leu Pro Ser Trp Thr Arg Ser Leu Asn Leu Ser Tyr Asn Arg Leu
         35                  40                  45

Ser Glu Ile Asp Ser Ala Ala Phe Glu Asp Leu Thr Asn Leu Gln Glu

50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu

385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro

725                 730                 735

Leu Gly Thr Glu Arg Ala His Ser Gln Leu Ser Ile Leu Pro Thr Pro
            740                 745                 750

Gly Cys Arg Lys Asp Gly Thr Thr Gly Ser Glu Pro Arg Gly Pro Thr
        755                 760                 765

Ile Lys Pro Cys Pro Pro Cys Lys Cys Pro Ala Pro Asn Leu Leu Gly
    770                 775                 780

Gly Pro Ser Val Phe Ile Phe Pro Pro Lys Ile Lys Asp Val Leu Met
785                 790                 795                 800

Ile Ser Leu Ser Pro Ile Val Thr Cys Val Val Val Asp Val Ser Glu
                805                 810                 815

Asp Asp Pro Asp Val Gln Ile Ser Trp Phe Val Asn Asn Val Glu Val
            820                 825                 830

His Thr Ala Gln Thr Gln Thr His Arg Glu Asp Tyr Asn Ser Thr Leu
        835                 840                 845

Arg Val Val Ser Ala Leu Pro Ile Gln His Gln Asp Trp Met Ser Gly
    850                 855                 860

Lys Glu Phe Lys Cys Lys Val Asn Asn Lys Asp Leu Pro Ala Pro Ile
865                 870                 875                 880

Glu Arg Thr Ile Ser Lys Pro Lys Gly Ser Val Arg Ala Pro Gln Val
                885                 890                 895

Tyr Val Leu Pro Pro Pro Glu Glu Glu Met Thr Lys Lys Gln Val Thr
            900                 905                 910

Leu Thr Cys Met Val Thr Asp Phe Met Pro Glu Asp Ile Tyr Val Glu
        915                 920                 925

Trp Thr Asn Asn Gly Lys Thr Glu Leu Asn Tyr Lys Asn Thr Glu Pro
    930                 935                 940

Val Leu Asp Ser Asp Gly Ser Tyr Phe Met Tyr Ser Lys Leu Arg Val
945                 950                 955                 960

Glu Lys Lys Asn Trp Val Glu Arg Asn Ser Tyr Ser Cys Ser Val Val
                965                 970                 975

His Glu Gly Leu His Asn His His Thr Thr Lys Ser Phe Ser Arg Thr
            980                 985                 990

Pro Gly Lys
        995

<210>   55
<211>   1007
<212>   PRT
<213>   Artificial Sequence

<220>
<223>   fusion protein

<400> 55
Ala Gln Ala Gly Pro Arg Ala Pro Cys Ala Ala Ala Cys Thr Cys Ala
1               5                   10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Gly Ser Arg Asn Thr Gly Arg Gly
        755                 760                 765

Gly Glu Glu Lys Lys Lys Glu Lys Glu Lys Glu Glu Gln Glu Glu Arg
    770                 775                 780

Glu Thr Lys Thr Pro Glu Cys Pro Ser His Thr Gln Pro Leu Gly Val
785                 790                 795                 800

Phe Ile Phe Pro Pro Lys Ile Lys Asp Val Leu Met Ile Ser Leu Ser
                805                 810                 815

Pro Ile Val Thr Cys Val Val Val Asp Val Ser Glu Asp Asp Pro Asp
            820                 825                 830

Val Gln Ile Ser Trp Phe Val Asn Asn Val Glu Val His Thr Ala Gln
        835                 840                 845

Thr Gln Thr His Arg Glu Asp Tyr Asn Ser Thr Leu Arg Val Val Ser
    850                 855                 860

Ala Leu Pro Ile Gln His Gln Asp Trp Met Ser Gly Lys Glu Phe Lys
865                 870                 875                 880

Cys Lys Val Asn Asn Lys Asp Leu Pro Ala Pro Ile Glu Arg Thr Ile
                885                 890                 895

Ser Lys Pro Lys Gly Ser Val Arg Ala Pro Gln Val Tyr Val Leu Pro
            900                 905                 910

Pro Pro Glu Glu Glu Met Thr Lys Lys Gln Val Thr Leu Thr Cys Met
        915                 920                 925

Val Thr Asp Phe Met Pro Glu Asp Ile Tyr Val Glu Trp Thr Asn Asn
    930                 935                 940

Gly Lys Thr Glu Leu Asn Tyr Lys Asn Thr Glu Pro Val Leu Asp Ser
945                 950                 955                 960

Asp Gly Ser Tyr Phe Met Tyr Ser Lys Leu Arg Val Glu Lys Lys Asn
                965                 970                 975

Trp Val Glu Arg Asn Ser Tyr Ser Cys Ser Val Val His Glu Gly Leu
            980                 985                 990

His Asn His His Thr Thr Lys Ser Phe Ser Arg Thr Pro Gly Lys
        995                 1000                1005

<210>    56
<211>    994
<212>    PRT
<213>    Artificial Sequence

<220>
<223>    fusion protein


<400>    56
Ala Gln Ala Gly Pro Arg Ala Pro Cys Ala Ala Ala Cys Thr Cys Ala
1               5                   10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser

260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe

595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Gly Ser Glu Pro Lys Ser Ser Asp
        755                 760                 765

Lys Thr His Thr Ser Pro Pro Ser Pro Ala Pro Glu Leu Leu Gly Gly
    770                 775                 780

Ser Ser Val Phe Ile Phe Pro Pro Lys Ile Lys Asp Val Leu Met Ile
785                 790                 795                 800

Ser Leu Ser Pro Ile Val Thr Cys Val Val Val Asp Val Ser Glu Asp
                805                 810                 815

Asp Pro Asp Val Gln Ile Ser Trp Phe Val Asn Asn Val Glu Val His
            820                 825                 830

Thr Ala Gln Thr Gln Thr His Arg Glu Asp Tyr Asn Ser Thr Leu Arg
        835                 840                 845

Val Val Ser Ala Leu Pro Ile Gln His Gln Asp Trp Met Ser Gly Lys
    850                 855                 860

Glu Phe Lys Cys Lys Val Asn Asn Lys Asp Leu Pro Ala Pro Ile Glu
865                 870                 875                 880

Arg Thr Ile Ser Lys Pro Lys Gly Ser Val Arg Ala Pro Gln Val Tyr
                885                 890                 895

Val Leu Pro Pro Pro Glu Glu Glu Met Thr Lys Lys Gln Val Thr Leu
            900                 905                 910

Thr Cys Met Val Thr Asp Phe Met Pro Glu Asp Ile Tyr Val Glu Trp
        915                 920                 925

Thr Asn Asn Gly Lys Thr Glu Leu Asn Tyr Lys Asn Thr Glu Pro Val

930                 935                 940

Leu Asp Ser Asp Gly Ser Tyr Phe Met Tyr Ser Lys Leu Arg Val Glu
945                 950                 955                 960

Lys Lys Asn Trp Val Glu Arg Asn Ser Tyr Ser Cys Ser Val Val His
                965                 970                 975

Glu Gly Leu His Asn His His Thr Thr Lys Ser Phe Ser Arg Thr Pro
            980                 985                 990

Gly Lys

<210>    57
<211>    2982
<212>    DNA
<213>    Artificial Sequence

<220>
<223>    fusion protein

<400>    57
gctcaggctg gacctagggc tccttgcgct gccgcctgca cctgtgcagg cgattctctg      60

gactgcagcg gccggggcct ggccacactg cccagggacc tgccttcctg gaccagatct      120

ctgaacctga gctacaatcg gctgtccgag atcgattctg ccgcctttga ggacctgaca      180

aatctgcagg aggtgtatct gaacagcaat gagctgaccg caatcccctc cctgggagca      240

gcctctatcg gcgtggtgag cctgttcctg cagcacaaca agatcctgag cgtggatggc      300

tcccagctga agagctacct gtctctggag gtgctggacc tgagctccaa caatatcacc      360

gagatcagat ctagctgttt tcctaatggc ctgcggatca gagagctgaa cctggcctct      420

aatcggatca gcatcctgga gtccggcgcc ttcgatggcc tgagcagatc cctgctgaca      480

ctgcgcctgt ccaagaaccg gatcacccag ctgcccgtga aggcctttaa gctgcctagg      540

ctgacacagc tggacctgaa ccggaataga atcaggctga tcgagggcct gaccttccag      600

ggcctggata gcctggaggt gctgcgcctg cagcggaaca atatctcccg cctgacagac      660

ggagcatttt ggggcctgtc taagatgcac gtgctgcacc tggagtacaa tagcctggtg      720

gaggtgaact ctggcagcct gtatggcctg accgccctgc accagctgca cctgtccaac      780

aatagcatca gcagaatcca gagggatggc tggtccttct gccagaagct gcacgagctg      840

atcctgtctt ttaacaatct gaccaggctg gacgaggaga gcctggcaga gctgtcctct      900

ctgtccatcc tgcgcctgtc tcacaatgcc atcagccaca tcgccgaggg cgcctttaag      960

ggcctgaaga gcctgagggt gctggatctg gaccacaacg agatctctgg caccatcgag      1020

gatacaagcg gcgccttcac aggcctggac aatctgtcca agctgaccct gtttggcaac      1080

aagatcaagt ctgtggccaa gcgggccttc tctggcctgg agagcctgga gcacctgaac      1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

ggatccgagc caaagtcctc tgataagaca cacacctctc caccatgccc agcaccagag 2340

ctgctgggag gaccaagcgt gttcatcttc ccacccaaga tcaaggacgt gctgatgatc 2400

tccctgtccc ccatcgtgac ctgcgtggtg gtggacgtgt ccgaggacga ccccgacgtg 2460

cagatcagtt ggttcgtgaa caacgtggaa gtgcacaccg cccagaccca gacccacaga 2520

gaggactaca actccaccct gcgggtggtg tccgccctgc ccatccagca ccaggactgg 2580

atgtccggca aagaattcaa gtgcaaagtg aacaacaagg acctgcctgc ccccatcgag 2640

cggaccatct ccaagcccaa gggctccgtg cgggctcccc aggtgtacgt gctgccccct 2700

ccagaggaag agatgaccaa gaagcaggtc acactgacct gcatggtcac cgacttcatg 2760

cccgaggaca tctacgtgga atggaccaac aatggcaaga ccgagctgaa ctacaagaac 2820

accgagcctg tgctggactc cgacggctcc tacttcatgt actccaagct gcgggtggaa 2880

aagaagaact gggtcgagcg gaactcctac tcctgctccg tggtgcacga gggcctgcac 2940

aaccaccaca ccaccaagtc cttctcccgg acccccggca aa 2982

<210>    58
<211>    2985
<212>    DNA
<213>    Artificial Sequence

<220>
<223>    fusion protein


<400>    58
gctcaggctg gacctagggc tccttgcgct gccgcctgca cctgtgcagg cgattctctg      60

gactgcagcg gccggggcct ggccacactg cccagggacc tgccttcctg gaccagatct     120

ctgaacctga gctacaatcg gctgtccgag atcgattctg ccgcctttga ggacctgaca     180

aatctgcagg aggtgtatct gaacagcaat gagctgaccg caatcccctc cctgggagca     240

gcctctatcg gcgtggtgag cctgttcctg cagcacaaca agatcctgag cgtggatggc     300

tcccagctga agagctacct gtctctggag gtgctggacc tgagctccaa caatatcacc     360

gagatcagat ctagctgttt tcctaatggc ctgcggatca gagagctgaa cctggcctct     420

aatcggatca gcatcctgga gtccggcgcc ttcgatggcc tgagcagatc cctgctgaca     480

ctgcgcctgt ccaagaaccg gatcacccag ctgcccgtga aggcctttaa gctgcctagg     540

ctgacacagc tggacctgaa ccggaataga atcaggctga tcgagggcct gaccttccag     600

ggcctggata gcctggaggt gctgcgcctg cagcggaaca atatctcccg cctgacagac     660

ggagcatttt ggggcctgtc taagatgcac gtgctgcacc tggagtacaa tagcctggtg     720

gaggtgaact ctggcagcct gtatggcctg accgccctgc accagctgca cctgtccaac     780

aatagcatca gcagaatcca gagggatggc tggtccttct gccagaagct gcacgagctg     840

atcctgtctt ttaacaatct gaccaggctg gacgaggaga gcctggcaga gctgtcctct     900

ctgtccatcc tgcgcctgtc tcacaatgcc atcagccaca tcgccgaggg cgcctttaag     960

ggcctgaaga gcctgagggt gctggatctg gaccacaacg agatctctgg caccatcgag    1020

gatacaagcg gcgccttcac aggcctggac aatctgtcca agctgaccct gtttggcaac    1080

aagatcaagt ctgtggccaa gcgggccttc tctggcctgg agagcctgga gcacctgaac    1140

ctgggcgaga atgccatcag atccgtgcag ttcgatgcct ttgccaagat gaagaatctg    1200

aaggagctgt acatcagctc cgagagcttc ctgtgcgact gtcagctgaa gtggctgcca    1260

ccttggctga tgggaaggat gctgcaggcc tttgtgaccg ccacatgcgc ccacccagag    1320

agcctgaagg gccagagcat cttctccgtg ctgcccgata gcttcgtgtg cgacgatttt    1380

cctaagccac agatcatcac ccagccagag acaacaatgg ccgtggtggg caaggacatc    1440

cggtttacat gttccgccgc ctctagctcc tctagcccca tgaccttcgc ctggaagaag    1500

gataacgagg tgctggccaa tgccgacatg gagaacttcg cccacgtgag agcccaggat    1560

ggcgaagtga tggagtatac cacaatcctg cacctgcggc acgtgacctt tggccacgag    1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

ggatccgagc ctcggggccc taccatcaag ccctgccccc cttgcaagtg ccctgcccct 2340

aatctgctgg gcggaccctc cgtgttcatc ttcccaccca agatcaagga cgtgctgatg 2400

atctccctgt cccccatcgt gacctgcgtg gtggtggacg tgtccgagga cgaccccgac 2460

gtgcagatca gttggttcgt gaacaacgtg gaagtgcaca ccgcccagac ccagacccac 2520

agagaggact acaactccac cctgcgggtg gtgtccgccc tgcccatcca gcaccaggac 2580

tggatgtccg gcaaagaatt caagtgcaaa gtgaacaaca aggacctgcc tgcccccatc 2640

gagcggacca tctccaagcc caagggctcc gtgcgggctc cccaggtgta cgtgctgccc 2700

cctccagagg aagagatgac caagaagcag gtcacactga cctgcatggt caccgacttc 2760

atgcccgagg acatctacgt ggaatggacc aacaatggca agaccgagct gaactacaag 2820

aacaccgagc ctgtgctgga ctccgacggc tcctacttca tgtactccaa gctgcgggtg 2880

gaaaagaaga actgggtcga gcggaactcc tactcctgct ccgtggtgca cgagggcctg 2940

cacaaccacc acaccaccaa gtccttctcc cggacccccg gcaaa 2985

<210> 59
<211> 3021
<212> DNA
<213> Artificial Sequence

<220>
<223> fusion protein

<400> 59
gctcaggctg gacctagggc tccttgcgct gccgcctgca cctgtgcagg cgattctctg 60

gactgcagcg gccggggcct ggccacactg cccagggacc tgccttcctg gaccagatct 120

ctgaacctga gctacaatcg gctgtccgag atcgattctg ccgcctttga ggacctgaca 180

aatctgcagg aggtgtatct gaacagcaat gagctgaccg caatcccctc cctgggagca 240

gcctctatcg gcgtggtgag cctgttcctg cagcacaaca agatcctgag cgtggatggc 300

tcccagctga agagctacct gtctctggag gtgctggacc tgagctccaa caatatcacc 360

gagatcagat ctagctgttt tcctaatggc ctgcggatca gagagctgaa cctggcctct 420

aatcggatca gcatcctgga gtccggcgcc ttcgatggcc tgagcagatc cctgctgaca 480

ctgcgcctgt ccaagaaccg gatcacccag ctgcccgtga aggcctttaa gctgcctagg 540

ctgacacagc tggacctgaa ccggaataga atcaggctga tcgagggcct gaccttccag 600

ggcctggata gcctggaggt gctgcgcctg cagcggaaca atatctcccg cctgacagac 660

ggagcatttt ggggcctgtc taagatgcac gtgctgcacc tggagtacaa tagcctggtg 720

gaggtgaact ctggcagcct gtatggcctg accgccctgc accagctgca cctgtccaac 780

aatagcatca gcagaatcca gagggatggc tggtccttct gccagaagct gcacgagctg 840

atcctgtctt ttaacaatct gaccaggctg gacgaggaga gcctggcaga gctgtcctct 900

ctgtccatcc tgcgcctgtc tcacaatgcc atcagccaca tcgccgaggg cgcctttaag 960

ggcctgaaga gcctgagggt gctggatctg gaccacaacg agatctctgg caccatcgag 1020

gatacaagcg gcgccttcac aggcctggac aatctgtcca agctgaccct gtttggcaac 1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

ggatcccgca acaccggccg cggcggcgag gagaagaaga aggagaagga gaaggaggag 2340

caggaggagc gcgagaccaa gacccccgag tgccccagcc acacccagcc cctgggcgtg 2400

ttcatcttcc cacccaagat caaggacgtg ctgatgatct ccctgtcccc catcgtgacc 2460

tgcgtggtgg tggacgtgtc cgaggacgac cccgacgtgc agatcagttg gttcgtgaac 2520

aacgtggaag tgcacaccgc ccagacccag acccacagag aggactacaa ctccaccctg 2580

cgggtggtgt ccgccctgcc catccagcac caggactgga tgtccggcaa agaattcaag 2640

tgcaaagtga acaacaagga cctgcctgcc cccatcgagc ggaccatctc caagcccaag 2700

ggctccgtgc gggctcccca ggtgtacgtg ctgccccctc cagaggaaga gatgaccaag 2760

aagcaggtca cactgacctg catggtcacc gacttcatgc ccgaggacat ctacgtggaa 2820

tggaccaaca atggcaagac cgagctgaac tacaagaaca ccgagcctgt gctggactcc 2880

gacggctcct acttcatgta ctccaagctg cgggtggaaa agaagaactg ggtcgagcgg 2940

aactcctact cctgctccgt ggtgcacgag ggcctgcaca accaccacac caccaagtcc 3000

ttctcccgga cccccggcaa a 3021

<210> 60
<211> 2982
<212> DNA
<213> Artificial Sequence

<220>
<223> fusion protein

<400> 60
gctcaggctg gacctagggc tccttgcgct gccgcctgca cctgtgcagg cgattctctg 60

gactgcagcg gccggggcct ggccacactg cccagggacc tgccttcctg gaccagatct 120

ctgaacctga gctacaatcg gctgtccgag atcgattctg ccgcctttga ggacctgaca 180

aatctgcagg aggtgtatct gaacagcaat gagctgaccg caatcccctc cctgggagca 240

gcctctatcg gcgtggtgag cctgttcctg cagcacaaca agatcctgag cgtggatggc 300

tcccagctga agagctacct gtctctggag gtgctggacc tgagctccaa caatatcacc 360

gagatcagat ctagctgttt tcctaatggc ctgcggatca gagagctgaa cctggcctct 420

aatcggatca gcatcctgga gtccggcgcc ttcgatggcc tgagcagatc cctgctgaca 480

ctgcgcctgt ccaagaaccg gatcacccag ctgcccgtga aggcctttaa gctgcctagg 540

ctgacacagc tggacctgaa ccggaataga atcaggctga tcgagggcct gaccttccag 600

ggcctggata gcctggaggt gctgcgcctg cagcggaaca atatctcccg cctgacagac 660

ggagcatttt ggggcctgtc taagatgcac gtgctgcacc tggagtacaa tagcctggtg 720

gaggtgaact ctggcagcct gtatggcctg accgccctgc accagctgca cctgtccaac 780

aatagcatca gcagaatcca gagggatggc tggtccttct gccagaagct gcacgagctg 840

atcctgtctt ttaacaatct gaccaggctg gacgaggaga gcctggcaga gctgtcctct 900

ctgtccatcc tgcgcctgtc tcacaatgcc atcagccaca tcgccgaggg cgcctttaag 960

ggcctgaaga gcctgagggt gctggatctg gaccacaacg agatctctgg caccatcgag 1020

gatacaagcg gcgccttcac aggcctggac aatctgtcca agctgaccct gtttggcaac 1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

ggatccgaac cgaaatcttc tgacaaaacc cacacctctc cgccgtctcc ggctccggaa 2340

ctgctgggtg gttcttctgt tttcatcttc ccacccaaga tcaaggacgt gctgatgatc 2400

tccctgtccc ccatcgtgac ctgcgtggtg gtggacgtgt ccgaggacga ccccgacgtg 2460

cagatcagtt ggttcgtgaa caacgtggaa gtgcacaccg cccagaccca gacccacaga 2520

gaggactaca actccaccct gcgggtggtg tccgccctgc ccatccagca ccaggactgg 2580

atgtccggca aagaattcaa gtgcaaagtg aacaacaagg acctgcctgc ccccatcgag 2640

cggaccatct ccaagcccaa gggctccgtg cgggctcccc aggtgtacgt gctgccccct 2700

ccagaggaag agatgaccaa gaagcaggtc acactgacct gcatggtcac cgacttcatg 2760

cccgaggaca tctacgtgga atggaccaac aatggcaaga ccgagctgaa ctacaagaac 2820

accgagcctg tgctggactc cgacggctcc tacttcatgt actccaagct gcgggtggaa 2880

aagaagaact gggtcgagcg gaactcctac tcctgctccg tggtgcacga gggcctgcac 2940

aaccaccaca ccaccaagtc cttctcccgg acccccggca aa 2982

<210> 61
<211> 999
<212> PRT
<213> Artificial Sequence

<220>
<223> fusion protein

<400> 61
Ala Gly Pro Arg Ala Pro Cys Ala Ala Ala Cys Thr Cys Ala Gly Asp
1               5                   10                  15

Ser Leu Asp Cys Gly Gly Arg Gly Leu Ala Ala Leu Pro Gly Asp Leu
            20                  25                  30

Pro Ser Trp Thr Arg Ser Leu Asn Leu Ser Tyr Asn Lys Leu Ser Glu
        35                  40                  45

Ile Asp Pro Ala Gly Phe Glu Asp Leu Pro Asn Leu Gln Glu Val Tyr
    50                  55                  60

Leu Asn Asn Asn Glu Leu Thr Ala Val Pro Ser Leu Gly Ala Ala Ser
65                  70                  75                  80

Ser His Val Val Ser Leu Phe Leu Gln His Asn Lys Ile Arg Ser Val
                85                  90                  95

Glu Gly Ser Gln Leu Lys Ala Tyr Leu Ser Leu Glu Val Leu Asp Leu
            100                 105                 110

Ser Leu Asn Asn Ile Thr Glu Val Arg Asn Thr Cys Phe Pro His Gly
        115                 120                 125

Pro Pro Ile Lys Glu Leu Asn Leu Ala Gly Asn Arg Ile Gly Thr Leu
    130                 135                 140

Glu Leu Gly Ala Phe Asp Gly Leu Ser Arg Ser Leu Leu Thr Leu Arg
145                 150                 155                 160

Leu Ser Lys Asn Arg Ile Thr Gln Leu Pro Val Arg Ala Phe Lys Leu
                165                 170                 175

Pro Arg Leu Thr Gln Leu Asp Leu Asn Arg Asn Arg Ile Arg Leu Ile
            180                 185                 190

Glu Gly Leu Thr Phe Gln Gly Leu Asn Ser Leu Glu Val Leu Lys Leu
        195                 200                 205

Gln Arg Asn Asn Ile Ser Lys Leu Thr Asp Gly Ala Phe Trp Gly Leu
    210                 215                 220

Ser Lys Met His Val Leu His Leu Glu Tyr Asn Ser Leu Val Glu Val
225                 230                 235                 240

Asn Ser Gly Ser Leu Tyr Gly Leu Thr Ala Leu His Gln Leu His Leu
                245                 250                 255

Ser Asn Asn Ser Ile Ala Arg Ile His Arg Lys Gly Trp Ser Phe Cys
            260                 265                 270

Gln Lys Leu His Glu Leu Val Leu Ser Phe Asn Asn Leu Thr Arg Leu
        275                 280                 285

Asp Glu Glu Ser Leu Ala Glu Leu Ser Ser Leu Ser Val Leu Arg Leu
    290                 295                 300

Ser His Asn Ser Ile Ser His Ile Ala Glu Gly Ala Phe Lys Gly Leu
305                 310                 315                 320

Arg Ser Leu Arg Val Leu Asp Leu Asp His Asn Glu Ile Ser Gly Thr
                325                 330                 335

Ile Glu Asp Thr Ser Gly Ala Phe Ser Gly Leu Asp Ser Leu Ser Lys
            340                 345                 350

Leu Thr Leu Phe Gly Asn Lys Ile Lys Ser Val Ala Lys Arg Ala Phe
        355                 360                 365

Ser Gly Leu Glu Gly Leu Glu His Leu Asn Leu Gly Gly Asn Ala Ile
    370                 375                 380

Arg Ser Val Gln Phe Asp Ala Phe Val Lys Met Lys Asn Leu Lys Glu
385                 390                 395                 400

Leu His Ile Ser Ser Asp Ser Phe Leu Cys Asp Cys Gln Leu Lys Trp
                405                 410                 415

Leu Pro Pro Trp Leu Ile Gly Arg Met Leu Gln Ala Phe Val Thr Ala
            420                 425                 430

Thr Cys Ala His Pro Glu Ser Leu Lys Gly Gln Ser Ile Phe Ser Val
        435                 440                 445

Pro Pro Glu Ser Phe Val Cys Asp Asp Phe Leu Lys Pro Gln Ile Ile
    450                 455                 460

Thr Gln Pro Glu Thr Thr Met Ala Met Val Gly Lys Asp Ile Arg Phe
465                 470                 475                 480

Thr Cys Ser Ala Ala Ser Ser Ser Ser Ser Pro Met Thr Phe Ala Trp
                485                 490                 495

Lys Lys Asp Asn Glu Val Leu Thr Asn Ala Asp Met Glu Asn Phe Val
            500                 505                 510

His Val His Ala Gln Asp Gly Glu Val Met Glu Tyr Thr Thr Ile Leu
        515                 520                 525

His Leu Arg Gln Val Thr Phe Gly His Glu Gly Arg Tyr Gln Cys Val
    530                 535                 540

Ile Thr Asn His Phe Gly Ser Thr Tyr Ser His Lys Ala Arg Leu Thr
545                 550                 555                 560

Val Asn Val Leu Pro Ser Phe Thr Lys Thr Pro His Asp Ile Thr Ile
                565                 570                 575

Arg Thr Thr Thr Val Ala Arg Leu Glu Cys Ala Ala Thr Gly His Pro
            580                 585                 590

Asn Pro Gln Ile Ala Trp Gln Lys Asp Gly Gly Thr Asp Phe Pro Ala
        595                 600                 605

Ala Arg Glu Arg Arg Met His Val Met Pro Asp Asp Asp Val Phe Phe
    610                 615                 620

Ile Thr Asp Val Lys Ile Asp Asp Ala Gly Val Tyr Ser Cys Thr Ala
625                 630                 635                 640

Gln Asn Ser Ala Gly Ser Ile Ser Ala Asn Ala Thr Leu Thr Val Leu
                645                 650                 655

Glu Thr Pro Ser Leu Val Val Pro Leu Glu Asp Arg Val Val Ser Val
            660                 665                 670

Gly Glu Thr Val Ala Leu Gln Cys Lys Ala Thr Gly Asn Pro Pro Pro
        675                 680                 685

Arg Ile Thr Trp Phe Lys Gly Asp Arg Pro Leu Ser Leu Thr Glu Arg
    690                 695                 700

His His Leu Thr Pro Asp Asn Gln Leu Leu Val Val Gln Asn Val Val
705                 710                 715                 720

Ala Glu Asp Ala Gly Arg Tyr Thr Cys Glu Met Ser Asn Thr Leu Gly
                725                 730                 735

Thr Glu Arg Ala His Ser Gln Leu Ser Val Leu Pro Ala Ala Gly Cys
            740                 745                 750

Arg Lys Asp Gly Thr Thr Val Gly Ile Phe Gly Gly Gly Gly Ser Glu
        755                 760                 765

Pro Lys Ser Ser Asp Lys Thr His Thr Ser Pro Pro Cys Pro Ala Pro
    770                 775                 780

Glu Leu Leu Gly Gly Pro Ser Val Phe Leu Phe Pro Pro Lys Pro Lys
785                 790                 795                 800

Asp Thr Leu Met Ile Ser Arg Thr Pro Glu Val Thr Cys Val Val Val
                805                 810                 815

Asp Val Ser His Glu Asp Pro Glu Val Lys Phe Asn Trp Tyr Val Asp
            820                 825                 830

Gly Val Glu Val His Asn Ala Lys Thr Lys Pro Arg Glu Glu Gln Tyr
        835                 840                 845

Asn Ser Thr Tyr Arg Val Val Ser Val Leu Thr Val Leu His Gln Asp
    850                 855                 860

Trp Leu Asn Gly Lys Glu Tyr Lys Cys Lys Val Ser Asn Lys Ala Leu
865                 870                 875                 880

Pro Ala Pro Ile Glu Lys Thr Ile Ser Lys Ala Lys Gly Gln Pro Arg
                885                 890                 895

Glu Pro Gln Val Tyr Thr Leu Pro Pro Ser Arg Asp Glu Leu Thr Lys
            900                 905                 910

Asn Gln Val Ser Leu Thr Cys Leu Val Lys Gly Phe Tyr Pro Ser Asp
        915                 920                 925

Ile Ala Val Glu Trp Glu Ser Asn Gly Gln Pro Glu Asn Asn Tyr Lys
    930                 935                 940

Thr Thr Pro Pro Val Leu Asp Ser Asp Gly Ser Phe Phe Leu Tyr Ser
945                 950                 955                 960

Lys Leu Thr Val Asp Lys Ser Arg Trp Gln Gln Gly Asn Val Phe Ser
                965                 970                 975

Cys Ser Val Met His Glu Ala Leu His Asn His Tyr Thr Gln Lys Ser
            980                 985                 990

Leu Ser Leu Ser Pro Gly Lys
        995

<210>   62
<211>   1000
<212>   PRT
<213>   Artificial Sequence

<220>
<223>   fusion protein

<400>   62
Ala Gly Pro Arg Ala Pro Cys Ala Ala Ala Cys Thr Cys Ala Gly Asp
1               5                   10                  15

Ser Leu Asp Cys Gly Gly Arg Gly Leu Ala Ala Leu Pro Gly Asp Leu
            20                  25                  30

Pro Ser Trp Thr Arg Ser Leu Asn Leu Ser Tyr Asn Lys Leu Ser Glu
        35                  40                  45

Ile Asp Pro Ala Gly Phe Glu Asp Leu Pro Asn Leu Gln Glu Val Tyr
    50                  55                  60

Leu Asn Asn Asn Glu Leu Thr Ala Val Pro Ser Leu Gly Ala Ala Ser
65                  70                  75                  80

Ser His Val Val Ser Leu Phe Leu Gln His Asn Lys Ile Arg Ser Val
                85                  90                  95

Glu Gly Ser Gln Leu Lys Ala Tyr Leu Ser Leu Glu Val Leu Asp Leu
            100                 105                 110

Ser Leu Asn Asn Ile Thr Glu Val Arg Asn Thr Cys Phe Pro His Gly

        115                 120                 125

Pro Pro Ile Lys Glu Leu Asn Leu Ala Gly Asn Arg Ile Gly Thr Leu
    130                 135                 140

Glu Leu Gly Ala Phe Asp Gly Leu Ser Arg Ser Leu Leu Thr Leu Arg
145                 150                 155                 160

Leu Ser Lys Asn Arg Ile Thr Gln Leu Pro Val Arg Ala Phe Lys Leu
                165                 170                 175

Pro Arg Leu Thr Gln Leu Asp Leu Asn Arg Asn Arg Ile Arg Leu Ile
            180                 185                 190

Glu Gly Leu Thr Phe Gln Gly Leu Asn Ser Leu Glu Val Leu Lys Leu
        195                 200                 205

Gln Arg Asn Asn Ile Ser Lys Leu Thr Asp Gly Ala Phe Trp Gly Leu
    210                 215                 220

Ser Lys Met His Val Leu His Leu Glu Tyr Asn Ser Leu Val Glu Val
225                 230                 235                 240

Asn Ser Gly Ser Leu Tyr Gly Leu Thr Ala Leu His Gln Leu His Leu
                245                 250                 255

Ser Asn Asn Ser Ile Ala Arg Ile His Arg Lys Gly Trp Ser Phe Cys
            260                 265                 270

Gln Lys Leu His Glu Leu Val Leu Ser Phe Asn Asn Leu Thr Arg Leu
        275                 280                 285

Asp Glu Glu Ser Leu Ala Glu Leu Ser Ser Leu Ser Val Leu Arg Leu
    290                 295                 300

Ser His Asn Ser Ile Ser His Ile Ala Glu Gly Ala Phe Lys Gly Leu
305                 310                 315                 320

Arg Ser Leu Arg Val Leu Asp Leu Asp His Asn Glu Ile Ser Gly Thr
                325                 330                 335

Ile Glu Asp Thr Ser Gly Ala Phe Ser Gly Leu Asp Ser Leu Ser Lys
            340                 345                 350

Leu Thr Leu Phe Gly Asn Lys Ile Lys Ser Val Ala Lys Arg Ala Phe
        355                 360                 365

Ser Gly Leu Glu Gly Leu Glu His Leu Asn Leu Gly Gly Asn Ala Ile
    370                 375                 380

Arg Ser Val Gln Phe Asp Ala Phe Val Lys Met Lys Asn Leu Lys Glu
385                 390                 395                 400

Leu His Ile Ser Ser Asp Ser Phe Leu Cys Asp Cys Gln Leu Lys Trp
                405                 410                 415

Leu Pro Pro Trp Leu Ile Gly Arg Met Leu Gln Ala Phe Val Thr Ala
            420                 425                 430

Thr Cys Ala His Pro Glu Ser Leu Lys Gly Gln Ser Ile Phe Ser Val
        435                 440                 445

Pro Pro Glu Ser Phe Val Cys Asp Asp Phe Leu Lys Pro Gln Ile Ile

450                 455                 460

Thr Gln Pro Glu Thr Thr Met Ala Met Val Gly Lys Asp Ile Arg Phe
465                 470                 475                 480

Thr Cys Ser Ala Ala Ser Ser Ser Ser Ser Pro Met Thr Phe Ala Trp
                485                 490                 495

Lys Lys Asp Asn Glu Val Leu Thr Asn Ala Asp Met Glu Asn Phe Val
            500                 505                 510

His Val His Ala Gln Asp Gly Glu Val Met Glu Tyr Thr Thr Ile Leu
        515                 520                 525

His Leu Arg Gln Val Thr Phe Gly His Glu Gly Arg Tyr Gln Cys Val
    530                 535                 540

Ile Thr Asn His Phe Gly Ser Thr Tyr Ser His Lys Ala Arg Leu Thr
545                 550                 555                 560

Val Asn Val Leu Pro Ser Phe Thr Lys Thr Pro His Asp Ile Thr Ile
                565                 570                 575

Arg Thr Thr Thr Val Ala Arg Leu Glu Cys Ala Ala Thr Gly His Pro
            580                 585                 590

Asn Pro Gln Ile Ala Trp Gln Lys Asp Gly Gly Thr Asp Phe Pro Ala
        595                 600                 605

Ala Arg Glu Arg Arg Met His Val Met Pro Asp Asp Asp Val Phe Phe
    610                 615                 620

Ile Thr Asp Val Lys Ile Asp Asp Ala Gly Val Tyr Ser Cys Thr Ala
625                 630                 635                 640

Gln Asn Ser Ala Gly Ser Ile Ser Ala Asn Ala Thr Leu Thr Val Leu
                645                 650                 655

Glu Thr Pro Ser Leu Val Val Pro Leu Glu Asp Arg Val Val Ser Val
            660                 665                 670

Gly Glu Thr Val Ala Leu Gln Cys Lys Ala Thr Gly Asn Pro Pro Pro
        675                 680                 685

Arg Ile Thr Trp Phe Lys Gly Asp Arg Pro Leu Ser Leu Thr Glu Arg
    690                 695                 700

His His Leu Thr Pro Asp Asn Gln Leu Leu Val Val Gln Asn Val Val
705                 710                 715                 720

Ala Glu Asp Ala Gly Arg Tyr Thr Cys Glu Met Ser Asn Thr Leu Gly
                725                 730                 735

Thr Glu Arg Ala His Ser Gln Leu Ser Val Leu Pro Ala Ala Gly Cys
            740                 745                 750

Arg Lys Asp Gly Thr Thr Val Gly Ile Phe Gly Gly Gly Gly Ser Glu
        755                 760                 765

Pro Arg Gly Pro Thr Ile Lys Pro Cys Pro Pro Cys Lys Cys Pro Ala
    770                 775                 780

Pro Asn Leu Leu Gly Gly Pro Ser Val Phe Leu Phe Pro Pro Lys Pro

785                 790                 795                 800

Lys Asp Thr Leu Met Ile Ser Arg Thr Pro Glu Val Thr Cys Val Val
                805                 810                 815

Val Asp Val Ser His Glu Asp Pro Glu Val Lys Phe Asn Trp Tyr Val
            820                 825                 830

Asp Gly Val Glu Val His Asn Ala Lys Thr Lys Pro Arg Glu Glu Gln
        835                 840                 845

Tyr Asn Ser Thr Tyr Arg Val Val Ser Val Leu Thr Val Leu His Gln
    850                 855                 860

Asp Trp Leu Asn Gly Lys Glu Tyr Lys Cys Lys Val Ser Asn Lys Ala
865                 870                 875                 880

Leu Pro Ala Pro Ile Glu Lys Thr Ile Ser Lys Ala Lys Gly Gln Pro
                885                 890                 895

Arg Glu Pro Gln Val Tyr Thr Leu Pro Pro Ser Arg Asp Glu Leu Thr
            900                 905                 910

Lys Asn Gln Val Ser Leu Thr Cys Leu Val Lys Gly Phe Tyr Pro Ser
        915                 920                 925

Asp Ile Ala Val Glu Trp Glu Ser Asn Gly Gln Pro Glu Asn Asn Tyr
    930                 935                 940

Lys Thr Thr Pro Pro Val Leu Asp Ser Asp Gly Ser Phe Phe Leu Tyr
945                 950                 955                 960

Ser Lys Leu Thr Val Asp Lys Ser Arg Trp Gln Gln Gly Asn Val Phe
                965                 970                 975

Ser Cys Ser Val Met His Glu Ala Leu His Asn His Tyr Thr Gln Lys
            980                 985                 990

Ser Leu Ser Leu Ser Pro Gly Lys
        995                 1000


<210>   63
<211>   1012
<212>   PRT
<213>   Artificial Sequence

<220>
<223>   fusion protein


<400>   63
Ala Gly Pro Arg Ala Pro Cys Ala Ala Ala Cys Thr Cys Ala Gly Asp
1               5                   10                  15

Ser Leu Asp Cys Gly Gly Arg Gly Leu Ala Ala Leu Pro Gly Asp Leu
            20                  25                  30

Pro Ser Trp Thr Arg Ser Leu Asn Leu Ser Tyr Asn Lys Leu Ser Glu
        35                  40                  45

Ile Asp Pro Ala Gly Phe Glu Asp Leu Pro Asn Leu Gln Glu Val Tyr
    50                  55                  60

Leu Asn Asn Asn Glu Leu Thr Ala Val Pro Ser Leu Gly Ala Ala Ser
65                  70                  75                  80

Ser His Val Val Ser Leu Phe Leu Gln His Asn Lys Ile Arg Ser Val
                85                  90                  95

Glu Gly Ser Gln Leu Lys Ala Tyr Leu Ser Leu Glu Val Leu Asp Leu
            100                 105                 110

Ser Leu Asn Asn Ile Thr Glu Val Arg Asn Thr Cys Phe Pro His Gly
        115                 120                 125

Pro Pro Ile Lys Glu Leu Asn Leu Ala Gly Asn Arg Ile Gly Thr Leu
    130                 135                 140

Glu Leu Gly Ala Phe Asp Gly Leu Ser Arg Ser Leu Leu Thr Leu Arg
145                 150                 155                 160

Leu Ser Lys Asn Arg Ile Thr Gln Leu Pro Val Arg Ala Phe Lys Leu
                165                 170                 175

Pro Arg Leu Thr Gln Leu Asp Leu Asn Arg Asn Arg Ile Arg Leu Ile
            180                 185                 190

Glu Gly Leu Thr Phe Gln Gly Leu Asn Ser Leu Glu Val Leu Lys Leu
        195                 200                 205

Gln Arg Asn Asn Ile Ser Lys Leu Thr Asp Gly Ala Phe Trp Gly Leu
    210                 215                 220

Ser Lys Met His Val Leu His Leu Glu Tyr Asn Ser Leu Val Glu Val
225                 230                 235                 240

Asn Ser Gly Ser Leu Tyr Gly Leu Thr Ala Leu His Gln Leu His Leu
                245                 250                 255

Ser Asn Asn Ser Ile Ala Arg Ile His Arg Lys Gly Trp Ser Phe Cys
            260                 265                 270

Gln Lys Leu His Glu Leu Val Leu Ser Phe Asn Asn Leu Thr Arg Leu
        275                 280                 285

Asp Glu Glu Ser Leu Ala Glu Leu Ser Ser Leu Ser Val Leu Arg Leu
    290                 295                 300

Ser His Asn Ser Ile Ser His Ile Ala Glu Gly Ala Phe Lys Gly Leu
305                 310                 315                 320

Arg Ser Leu Arg Val Leu Asp Leu Asp His Asn Glu Ile Ser Gly Thr
                325                 330                 335

Ile Glu Asp Thr Ser Gly Ala Phe Ser Gly Leu Asp Ser Leu Ser Lys
            340                 345                 350

Leu Thr Leu Phe Gly Asn Lys Ile Lys Ser Val Ala Lys Arg Ala Phe
        355                 360                 365

Ser Gly Leu Glu Gly Leu Glu His Leu Asn Leu Gly Gly Asn Ala Ile
    370                 375                 380

Arg Ser Val Gln Phe Asp Ala Phe Val Lys Met Lys Asn Leu Lys Glu
385                 390                 395                 400

Leu His Ile Ser Ser Asp Ser Phe Leu Cys Asp Cys Gln Leu Lys Trp
                405                 410                 415

Leu Pro Pro Trp Leu Ile Gly Arg Met Leu Gln Ala Phe Val Thr Ala
            420                 425                 430

Thr Cys Ala His Pro Glu Ser Leu Lys Gly Gln Ser Ile Phe Ser Val
        435                 440                 445

Pro Pro Glu Ser Phe Val Cys Asp Asp Phe Leu Lys Pro Gln Ile Ile
    450                 455                 460

Thr Gln Pro Glu Thr Thr Met Ala Met Val Gly Lys Asp Ile Arg Phe
465                 470                 475                 480

Thr Cys Ser Ala Ala Ser Ser Ser Ser Ser Pro Met Thr Phe Ala Trp
                485                 490                 495

Lys Lys Asp Asn Glu Val Leu Thr Asn Ala Asp Met Glu Asn Phe Val
            500                 505                 510

His Val His Ala Gln Asp Gly Glu Val Met Glu Tyr Thr Thr Ile Leu
        515                 520                 525

His Leu Arg Gln Val Thr Phe Gly His Glu Gly Arg Tyr Gln Cys Val
    530                 535                 540

Ile Thr Asn His Phe Gly Ser Thr Tyr Ser His Lys Ala Arg Leu Thr
545                 550                 555                 560

Val Asn Val Leu Pro Ser Phe Thr Lys Thr Pro His Asp Ile Thr Ile
                565                 570                 575

Arg Thr Thr Thr Val Ala Arg Leu Glu Cys Ala Ala Thr Gly His Pro
            580                 585                 590

Asn Pro Gln Ile Ala Trp Gln Lys Asp Gly Gly Thr Asp Phe Pro Ala
        595                 600                 605

Ala Arg Glu Arg Arg Met His Val Met Pro Asp Asp Asp Val Phe Phe
    610                 615                 620

Ile Thr Asp Val Lys Ile Asp Asp Ala Gly Val Tyr Ser Cys Thr Ala
625                 630                 635                 640

Gln Asn Ser Ala Gly Ser Ile Ser Ala Asn Ala Thr Leu Thr Val Leu
                645                 650                 655

Glu Thr Pro Ser Leu Val Val Pro Leu Glu Asp Arg Val Val Ser Val
            660                 665                 670

Gly Glu Thr Val Ala Leu Gln Cys Lys Ala Thr Gly Asn Pro Pro Pro
        675                 680                 685

Arg Ile Thr Trp Phe Lys Gly Asp Arg Pro Leu Ser Leu Thr Glu Arg
    690                 695                 700

His His Leu Thr Pro Asp Asn Gln Leu Leu Val Val Gln Asn Val Val
705                 710                 715                 720

Ala Glu Asp Ala Gly Arg Tyr Thr Cys Glu Met Ser Asn Thr Leu Gly
                725                 730                 735

Thr Glu Arg Ala His Ser Gln Leu Ser Val Leu Pro Ala Ala Gly Cys
            740                 745                 750

Arg Lys Asp Gly Thr Thr Val Gly Ile Phe Gly Gly Gly Gly Ser Arg
        755                 760                 765

Asn Thr Gly Arg Gly Gly Glu Glu Lys Lys Lys Glu Lys Glu Lys Glu
    770                 775                 780

Glu Gln Glu Glu Arg Glu Thr Lys Thr Pro Glu Cys Pro Ser His Thr
785                 790                 795                 800

Gln Pro Leu Gly Val Phe Leu Phe Pro Pro Lys Pro Lys Asp Thr Leu
                805                 810                 815

Met Ile Ser Arg Thr Pro Glu Val Thr Cys Val Val Val Asp Val Ser
            820                 825                 830

His Glu Asp Pro Glu Val Lys Phe Asn Trp Tyr Val Asp Gly Val Glu
        835                 840                 845

Val His Asn Ala Lys Thr Lys Pro Arg Glu Glu Gln Tyr Asn Ser Thr
    850                 855                 860

Tyr Arg Val Val Ser Val Leu Thr Val Leu His Gln Asp Trp Leu Asn
865                 870                 875                 880

Gly Lys Glu Tyr Lys Cys Lys Val Ser Asn Lys Ala Leu Pro Ala Pro
                885                 890                 895

Ile Glu Lys Thr Ile Ser Lys Ala Lys Gly Gln Pro Arg Glu Pro Gln
            900                 905                 910

Val Tyr Thr Leu Pro Pro Ser Arg Asp Glu Leu Thr Lys Asn Gln Val
        915                 920                 925

Ser Leu Thr Cys Leu Val Lys Gly Phe Tyr Pro Ser Asp Ile Ala Val
    930                 935                 940

Glu Trp Glu Ser Asn Gly Gln Pro Glu Asn Asn Tyr Lys Thr Thr Pro
945                 950                 955                 960

Pro Val Leu Asp Ser Asp Gly Ser Phe Phe Leu Tyr Ser Lys Leu Thr
                965                 970                 975

Val Asp Lys Ser Arg Trp Gln Gln Gly Asn Val Phe Ser Cys Ser Val
            980                 985                 990

Met His Glu Ala Leu His Asn His Tyr Thr Gln Lys Ser Leu Ser Leu
        995                 1000                1005

Ser Pro Gly Lys
    1010


<210>   64
<211>   999
<212>   PRT
<213>   Artificial Sequence

<220>
<223>   fusion protein

<400>    64
Ala Gly Pro Arg Ala Pro Cys Ala Ala Ala Cys Thr Cys Ala Gly Asp
1               5                   10                  15

Ser Leu Asp Cys Gly Gly Arg Gly Leu Ala Ala Leu Pro Gly Asp Leu
            20                  25                  30

Pro Ser Trp Thr Arg Ser Leu Asn Leu Ser Tyr Asn Lys Leu Ser Glu
        35                  40                  45

Ile Asp Pro Ala Gly Phe Glu Asp Leu Pro Asn Leu Gln Glu Val Tyr
    50                  55                  60

Leu Asn Asn Asn Glu Leu Thr Ala Val Pro Ser Leu Gly Ala Ala Ser
65                  70                  75                  80

Ser His Val Val Ser Leu Phe Leu Gln His Asn Lys Ile Arg Ser Val
                85                  90                  95

Glu Gly Ser Gln Leu Lys Ala Tyr Leu Ser Leu Glu Val Leu Asp Leu
            100                 105                 110

Ser Leu Asn Asn Ile Thr Glu Val Arg Asn Thr Cys Phe Pro His Gly
        115                 120                 125

Pro Pro Ile Lys Glu Leu Asn Leu Ala Gly Asn Arg Ile Gly Thr Leu
    130                 135                 140

Glu Leu Gly Ala Phe Asp Gly Leu Ser Arg Ser Leu Leu Thr Leu Arg
145                 150                 155                 160

Leu Ser Lys Asn Arg Ile Thr Gln Leu Pro Val Arg Ala Phe Lys Leu
                165                 170                 175

Pro Arg Leu Thr Gln Leu Asp Leu Asn Arg Asn Arg Ile Arg Leu Ile
            180                 185                 190

Glu Gly Leu Thr Phe Gln Gly Leu Asn Ser Leu Glu Val Leu Lys Leu
        195                 200                 205

Gln Arg Asn Asn Ile Ser Lys Leu Thr Asp Gly Ala Phe Trp Gly Leu
    210                 215                 220

Ser Lys Met His Val Leu His Leu Glu Tyr Asn Ser Leu Val Glu Val
225                 230                 235                 240

Asn Ser Gly Ser Leu Tyr Gly Leu Thr Ala Leu His Gln Leu His Leu
                245                 250                 255

Ser Asn Asn Ser Ile Ala Arg Ile His Arg Lys Gly Trp Ser Phe Cys
            260                 265                 270

Gln Lys Leu His Glu Leu Val Leu Ser Phe Asn Asn Leu Thr Arg Leu
        275                 280                 285

Asp Glu Glu Ser Leu Ala Glu Leu Ser Ser Leu Ser Val Leu Arg Leu
    290                 295                 300

Ser His Asn Ser Ile Ser His Ile Ala Glu Gly Ala Phe Lys Gly Leu
305                 310                 315                 320

Arg Ser Leu Arg Val Leu Asp Leu Asp His Asn Glu Ile Ser Gly Thr
                325                 330                 335

Ile Glu Asp Thr Ser Gly Ala Phe Ser Gly Leu Asp Ser Leu Ser Lys
            340                 345                 350

Leu Thr Leu Phe Gly Asn Lys Ile Lys Ser Val Ala Lys Arg Ala Phe
        355                 360                 365

Ser Gly Leu Glu Gly Leu Glu His Leu Asn Leu Gly Gly Asn Ala Ile
    370                 375                 380

Arg Ser Val Gln Phe Asp Ala Phe Val Lys Met Lys Asn Leu Lys Glu
385                 390                 395                 400

Leu His Ile Ser Ser Asp Ser Phe Leu Cys Asp Cys Gln Leu Lys Trp
                405                 410                 415

Leu Pro Pro Trp Leu Ile Gly Arg Met Leu Gln Ala Phe Val Thr Ala
            420                 425                 430

Thr Cys Ala His Pro Glu Ser Leu Lys Gly Gln Ser Ile Phe Ser Val
        435                 440                 445

Pro Pro Glu Ser Phe Val Cys Asp Asp Phe Leu Lys Pro Gln Ile Ile
    450                 455                 460

Thr Gln Pro Glu Thr Thr Met Ala Met Val Gly Lys Asp Ile Arg Phe
465                 470                 475                 480

Thr Cys Ser Ala Ala Ser Ser Ser Ser Ser Pro Met Thr Phe Ala Trp
                485                 490                 495

Lys Lys Asp Asn Glu Val Leu Thr Asn Ala Asp Met Glu Asn Phe Val
            500                 505                 510

His Val His Ala Gln Asp Gly Glu Val Met Glu Tyr Thr Thr Ile Leu
        515                 520                 525

His Leu Arg Gln Val Thr Phe Gly His Glu Gly Arg Tyr Gln Cys Val
    530                 535                 540

Ile Thr Asn His Phe Gly Ser Thr Tyr Ser His Lys Ala Arg Leu Thr
545                 550                 555                 560

Val Asn Val Leu Pro Ser Phe Thr Lys Thr Pro His Asp Ile Thr Ile
                565                 570                 575

Arg Thr Thr Thr Val Ala Arg Leu Glu Cys Ala Ala Thr Gly His Pro
            580                 585                 590

Asn Pro Gln Ile Ala Trp Gln Lys Asp Gly Gly Thr Asp Phe Pro Ala
        595                 600                 605

Ala Arg Glu Arg Arg Met His Val Met Pro Asp Asp Asp Val Phe Phe
    610                 615                 620

Ile Thr Asp Val Lys Ile Asp Asp Ala Gly Val Tyr Ser Cys Thr Ala
625                 630                 635                 640

Gln Asn Ser Ala Gly Ser Ile Ser Ala Asn Ala Thr Leu Thr Val Leu
                645                 650                 655

Glu Thr Pro Ser Leu Val Val Pro Leu Glu Asp Arg Val Val Ser Val
            660                 665                 670

Gly Glu Thr Val Ala Leu Gln Cys Lys Ala Thr Gly Asn Pro Pro Pro
        675                 680                 685

Arg Ile Thr Trp Phe Lys Gly Asp Arg Pro Leu Ser Leu Thr Glu Arg
    690                 695                 700

His His Leu Thr Pro Asp Asn Gln Leu Leu Val Val Gln Asn Val Val
705                 710                 715                 720

Ala Glu Asp Ala Gly Arg Tyr Thr Cys Glu Met Ser Asn Thr Leu Gly
                725                 730                 735

Thr Glu Arg Ala His Ser Gln Leu Ser Val Leu Pro Ala Ala Gly Cys
            740                 745                 750

Arg Lys Asp Gly Thr Thr Val Gly Ile Phe Gly Gly Gly Gly Ser Glu
        755                 760                 765

Pro Lys Ser Ser Asp Lys Thr His Thr Ser Pro Pro Ser Pro Ala Pro
    770                 775                 780

Glu Leu Leu Gly Gly Ser Ser Val Phe Leu Phe Pro Pro Lys Pro Lys
785                 790                 795                 800

Asp Thr Leu Met Ile Ser Arg Thr Pro Glu Val Thr Cys Val Val Val
                805                 810                 815

Asp Val Ser His Glu Asp Pro Glu Val Lys Phe Asn Trp Tyr Val Asp
            820                 825                 830

Gly Val Glu Val His Asn Ala Lys Thr Lys Pro Arg Glu Glu Gln Tyr
        835                 840                 845

Asn Ser Thr Tyr Arg Val Val Ser Val Leu Thr Val Leu His Gln Asp
    850                 855                 860

Trp Leu Asn Gly Lys Glu Tyr Lys Cys Lys Val Ser Asn Lys Ala Leu
865                 870                 875                 880

Pro Ala Pro Ile Glu Lys Thr Ile Ser Lys Ala Lys Gly Gln Pro Arg
                885                 890                 895

Glu Pro Gln Val Tyr Thr Leu Pro Pro Ser Arg Asp Glu Leu Thr Lys
            900                 905                 910

Asn Gln Val Ser Leu Thr Cys Leu Val Lys Gly Phe Tyr Pro Ser Asp
        915                 920                 925

Ile Ala Val Glu Trp Glu Ser Asn Gly Gln Pro Glu Asn Asn Tyr Lys
    930                 935                 940

Thr Thr Pro Pro Val Leu Asp Ser Asp Gly Ser Phe Phe Leu Tyr Ser
945                 950                 955                 960

Lys Leu Thr Val Asp Lys Ser Arg Trp Gln Gln Gly Asn Val Phe Ser
                965                 970                 975

Cys Ser Val Met His Glu Ala Leu His Asn His Tyr Thr Gln Lys Ser
            980                 985                 990

Leu Ser Leu Ser Pro Gly Lys
        995

<210>    65
<211>    2997
<212>    DNA
<213>    Artificial Sequence

<220>
<223>    fusion protein

<400>    65
gctgggcctc gggctccttg tgctgccgcc tgcacatgtg caggcgattc cctggactgc      60

ggcggcagag gcctggccgc cctgcctggc gatctgccat cctggacccg gagcctgaac     120

ctgagctaca acaagctgag cgagatcgat cccgccggct ttgaggacct gcctaacctg     180

caggaggtgt atctgaacaa taacgagctg accgcggtac catccctggg cgctgcttca     240

tcacatgtcg tctctctctt tctgcagcac aacaagattc gcagcgtgga ggggagccag     300

ctgaaggcct acctttcctt agaagtgtta gatctgagtt tgaacaacat cacggaagtg     360

cggaacacct gctttccaca cggaccgcct ataaaggagc tcaacctggc aggcaatcgg     420

attggcaccc tggagttggg agcatttgat ggtctgtcac ggtcgctgct aactcttcgc     480

ctgagcaaaa acaggatcac ccagcttcct gtaagagcat tcaagctacc caggctgaca     540

caactggacc tcaatcggaa caggattcgg ctgatagagg gcctcacctt ccaggggctc     600

aacagcttgg aggtgctgaa gcttcagcga aacaacatca gcaaactgac agatggggcc     660

ttctggggac tgtccaagat gcatgtgctg cacctggagt acaacagcct ggtagaagtg     720

aacagcggct cgctctacgg cctcacggcc ctgcatcagc tccacctcag caacaattcc     780

atcgctcgca ttcaccgcaa gggctggagc ttctgccaga agctgcatga gttggtcctg     840

tccttcaaca acctgacacg gctggacgag gagagcctgg ccgagctgag cagcctgagt     900

gtcctgcgtc tcagccacaa ttccatcagc cacattgcgg agggtgcctt caagggactc     960

aggagcctgc gagtcttgga tctggaccat aacgagattt cgggcacaat agaggacacg    1020

agcggcgcct tctcagggct cgacagcctc agcaagctga ctctgtttgg aaacaagatc    1080

aagtctgtgg ctaagagagc attctcgggg ctggaaggcc tggagcacct gaaccttgga    1140

gggaatgcga tcagatctgt ccagtttgat gcctttgtga agatgaagaa tcttaaagag    1200

ctccatatca gcagcgacag cttcctgtgt gactgccagc tgaagtggct gcccccgtgg    1260

ctaattggca ggatgctgca ggcctttgtg acagccacct gtgcccaccc agaatcactg    1320

aagggtcaga gcattttctc tgtgccacca gagagtttcg tgtgcgatga cttcctgaag    1380

ccacagatca tcacccagcc agaaaccacc atggctatgg tgggcaagga catccggttt    1440

acatgctcag cagccagcag cagcagctcc cccatgacct ttgcctggaa gaaagacaat    1500

gaagtcctga ccaatgcaga catggagaac tttgtccacg tccacgcgca ggacggggaa 1560

gtgatggagt acaccaccat cctgcacctc cgtcaggtca ctttcgggca cgagggccgc 1620

taccaatgtg tcatcaccaa ccactttggc tccacctatt cacataaggc caggctcacc 1680

gtgaatgtgt tgccatcatt caccaaaacg ccccacgaca taaccatccg gaccaccacc 1740

gtggcccgcc tcgaatgtgc tgccacaggt cacccaaacc ctcagattgc ctggcagaag 1800

gatggaggca cggatttccc cgctgcccgt gagcgacgca tgcatgtcat gccggatgac 1860

gacgtgtttt tcatcactga tgtgaaaata gatgacgcag gggtttacag ctgtactgct 1920

cagaactcag ccggttctat ttcagctaat gccaccctga ctgtcctaga gaccccatcc 1980

ttggtggtcc ccttggaaga ccgtgtggta tctgtgggag aaacagtggc cctccaatgc 2040

aaagccacgg ggaaccctcc gccccgcatc acctggttca agggggaccg cccgctgagc 2100

ctcactgagc ggcaccacct gacccctgac aaccagctcc tggtggttca gaacgtggtg 2160

gcagaggatg cgggccgata tacctgtgag atgtccaaca ccctgggcac ggagcgagct 2220

cacagccagc tgagcgtcct gcccgcagca ggctgcagga aggatgggac cacggtaggc 2280

atcttcggcg gtggcggatc cgagccaaag tcctctgata agacacacac ctctccacca 2340

tgcccagcac cagagctgct gggaggacca agcgtgttcc tgtttcctcc aaagcccaag 2400

gacacactga tgatctccag gacaccagag gtgacctgcg tggtggtgga cgtgagccac 2460

gaggaccccg aggtgaagtt caactggtac gtggatggcg tggaggtgca caatgccaag 2520

accaagccca gagaggagca gtacaactct acctataggg tggtgagcgt gctgacagtg 2580

ctgcaccagg actggctgaa cggcaaggag tataagtgca aggtgagcaa taaggccctg 2640

cctgccccaa tcgagaagac aatctccaag gccaagggcc agccaagaga gccccaggtg 2700

tacaccctgc cccctagcag ggatgagctg acaaagaacc aggtgtccct gacctgtctg 2760

gtgaagggct tttatccctc cgacatcgcc gtggagtggg agtctaatgg ccagcctgag 2820

aataactaca agacaacccc acccgtgctg gattctgacg gcagcttctt tctgtattct 2880

aagctgaccg tggacaagag caggtggcag cagggcaacg tgttcagctg ctccgtgatg 2940

cacgaagcac tgcacaatca ctacacccag aaatcactgt cactgagccc tggcaaa 2997

<210> 66
<211> 3000
<212> DNA
<213> Artificial Sequence

<220>
<223> fusion protein

<400> 66
gctgggcctc gggctccttg tgctgccgcc tgcacatgtg caggcgattc cctggactgc 60

ggcggcagag gcctggccgc cctgcctggc gatctgccat cctggacccg gagcctgaac 120

ctgagctaca acaagctgag cgagatcgat cccgccggct ttgaggacct gcctaacctg 180

caggaggtgt atctgaacaa taacgagctg accgcggtac catccctggg cgctgcttca 240

tcacatgtcg tctctctctt tctgcagcac aacaagattc gcagcgtgga ggggagccag 300

ctgaaggcct acctttcctt agaagtgtta gatctgagtt tgaacaacat cacggaagtg 360

cggaacacct gctttccaca cggaccgcct ataaaggagc tcaacctggc aggcaatcgg 420

attggcaccc tggagttggg agcatttgat ggtctgtcac ggtcgctgct aactcttcgc 480

ctgagcaaaa acaggatcac ccagcttcct gtaagagcat tcaagctacc caggctgaca 540

caactggacc tcaatcggaa caggattcgg ctgatagagg gcctcacctt ccaggggctc 600

aacagcttgg aggtgctgaa gcttcagcga aacaacatca gcaaactgac agatggggcc 660

ttctggggac tgtccaagat gcatgtgctg cacctggagt acaacagcct ggtagaagtg 720

aacagcggct cgctctacgg cctcacggcc ctgcatcagc tccacctcag caacaattcc 780

atcgctcgca ttcaccgcaa gggctggagc ttctgccaga agctgcatga gttggtcctg 840

tccttcaaca acctgacacg gctggacgag gagagcctgg ccgagctgag cagcctgagt 900

gtcctgcgtc tcagccacaa ttccatcagc cacattgcgg agggtgcctt caagggactc 960

aggagcctgc gagtcttgga tctggaccat aacgagattt cgggcacaat agaggacacg 1020

agcggcgcct tctcagggct cgacagcctc agcaagctga ctctgtttgg aaacaagatc 1080

aagtctgtgg ctaagagagc attctcgggg ctggaaggcc tggagcacct gaaccttgga 1140

gggaatgcga tcagatctgt ccagtttgat gcctttgtga agatgaagaa tcttaaagag 1200

ctccatatca gcagcgacag cttcctgtgt gactgccagc tgaagtggct gcccccgtgg 1260

ctaattggca ggatgctgca ggcctttgtg acagccacct gtgcccaccc agaatcactg 1320

aagggtcaga gcattttctc tgtgccacca gagagtttcg tgtgcgatga cttcctgaag 1380

ccacagatca tcacccagcc agaaaccacc atggctatgg tgggcaagga catccggttt 1440

acatgctcag cagccagcag cagcagctcc cccatgacct ttgcctggaa gaaagacaat 1500

gaagtcctga ccaatgcaga catggagaac tttgtccacg tccacgcgca ggacggggaa 1560

gtgatggagt acaccaccat cctgcacctc cgtcaggtca ctttcggcca cgagggccgc 1620

taccaatgtg tcatcaccaa ccactttggc tccacctatt cacataaggc caggctcacc 1680

gtgaatgtgt tgccatcatt caccaaaacg ccccacgaca taaccatccg gaccaccacc 1740

gtggcccgcc tcgaatgtgc tgccacaggt cacccaaacc ctcagattgc ctggcagaag 1800

gatggaggca cggatttccc cgctgcccgt gagcgacgca tgcatgtcat gccggatgac 1860

gacgtgtttt tcatcactga tgtgaaaata gatgacgcag gggtttacag ctgtactgct 1920

cagaactcag ccggttctat ttcagctaat gccaccctga ctgtcctaga gaccccatcc 1980

ttggtggtcc ccttggaaga ccgtgtggta tctgtgggag aaacagtggc cctccaatgc 2040

aaagccacgg ggaaccctcc gccccgcatc acctggttca agggggaccg cccgctgagc 2100

ctcactgagc ggcaccacct gacccctgac aaccagctcc tggtggttca gaacgtggtg 2160

gcagaggatg cgggccgata tacctgtgag atgtccaaca ccctgggcac ggagcgagct 2220

cacagccagc tgagcgtcct gcccgcagca ggctgcagga aggatgggac cacggtaggc 2280

atcttcggcg gtggcggatc cgagcctcgg ggccctacca tcaagccctg ccccccttgc 2340

aagtgccctg cccctaatct gctgggcgga ccctccgtgt tcctgtttcc tccaaagccc 2400

aaggacacac tgatgatctc caggacacca gaggtgacct gcgtggtggt ggacgtgagc 2460

cacgaggacc ccgaggtgaa gttcaactgg tacgtggatg gcgtggaggt gcacaatgcc 2520

aagaccaagc ccagagagga gcagtacaac tctacctata gggtggtgag cgtgctgaca 2580

gtgctgcacc aggactggct gaacggcaag gagtataagt gcaaggtgag caataaggcc 2640

ctgcctgccc caatcgagaa gacaatctcc aaggccaagg gccagccaag agagccccag 2700

gtgtacaccc tgccccctag cagggatgag ctgacaaaga accaggtgtc cctgacctgt 2760

ctggtgaagg gcttttatcc ctccgacatc gccgtggagt gggagtctaa tggccagcct 2820

gagaataact acaagacaac cccacccgtg ctggattctg acggcagctt ctttctgtat 2880

tctaagctga ccgtggacaa gagcaggtgg cagcagggca acgtgttcag ctgctccgtg 2940

atgcacgaag cactgcacaa tcactacacc cagaaatcac tgtcactgag ccctggcaaa 3000

3000

<210> 67
<211> 3036
<212> DNA
<213> Artificial Sequence

<220>
<223> fusion protein

<400> 67
gctgggcctc gggctccttg tgctgccgcc tgcacatgtg caggcgattc cctggactgc 60

ggcggcagag gcctggccgc cctgcctggc gatctgccat cctggacccg gagcctgaac 120

ctgagctaca acaagctgag cgagatcgat cccgccggct ttgaggacct gcctaacctg 180

caggaggtgt atctgaacaa taacgagctg accgcggtac catccctggg cgctgcttca 240

tcacatgtcg tctctctctt tctgcagcac aacaagattc gcagcgtgga ggggagccag 300

ctgaaggcct acctttcctt agaagtgtta gatctgagtt tgaacaacat cacggaagtg 360

cggaacacct gctttccaca cggaccgcct ataaaggagc tcaacctggc aggcaatcgg 420

attggcaccc tggagttggg agcatttgat ggtctgtcac ggtcgctgct aactcttcgc 480

ctgagcaaaa acaggatcac ccagcttcct gtaagagcat tcaagctacc caggctgaca 540

caactggacc tcaatcggaa caggattcgg ctgatagagg gcctcacctt ccaggggctc 600

aacagcttgg aggtgctgaa gcttcagcga aacaacatca gcaaactgac agatggggcc 660

ttctggggac tgtccaagat gcatgtgctg cacctggagt acaacagcct ggtagaagtg 720

aacagcggct cgctctacgg cctcacggcc ctgcatcagc tccacctcag caacaattcc 780

atcgctcgca ttcaccgcaa gggctggagc ttctgccaga agctgcatga gttggtcctg 840

tccttcaaca acctgacacg gctggacgag gagagcctgg ccgagctgag cagcctgagt 900

gtcctgcgtc tcagccacaa ttccatcagc cacattgcgg agggtgcctt caagggactc 960

aggagcctgc gagtcttgga tctggaccat aacgagattt cgggcacaat agaggacacg 1020

agcggcgcct tctcagggct cgacagcctc agcaagctga ctctgtttgg aaacaagatc 1080

aagtctgtgg ctaagagagc attctcgggg ctggaaggcc tggagcacct gaaccttgga 1140

gggaatgcga tcagatctgt ccagtttgat gcctttgtga agatgaagaa tcttaaagag 1200

ctccatatca gcagcgacag cttcctgtgt gactgccagc tgaagtggct gcccccgtgg 1260

ctaattggca ggatgctgca ggcctttgtg acagccacct gtgcccaccc agaatcactg 1320

aagggtcaga gcattttctc tgtgccacca gagagtttcg tgtgcgatga cttcctgaag 1380

ccacagatca tcacccagcc agaaaccacc atggctatgg tgggcaagga catccggttt 1440

acatgctcag cagccagcag cagcagctcc cccatgacct ttgcctggaa gaaagacaat 1500

gaagtcctga ccaatgcaga catggagaac tttgtccacg tccacgcgca ggacggggaa 1560

gtgatggagt acaccaccat cctgcacctc cgtcaggtca ctttcgggca cgagggccgc 1620

taccaatgtg tcatcaccaa ccactttggc tccacctatt cacataaggc caggctcacc 1680

gtgaatgtgt tgccatcatt caccaaaacg ccccacgaca taaccatccg gaccaccacc 1740

gtggcccgcc tcgaatgtgc tgccacaggt cacccaaacc ctcagattgc ctggcagaag 1800

gatggaggca cggatttccc cgctgcccgt gagcgacgca tgcatgtcat gccggatgac 1860

gacgtgtttt tcatcactga tgtgaaaata gatgacgcag gggtttacag ctgtactgct 1920

cagaactcag ccggttctat ttcagctaat gccaccctga ctgtcctaga gaccccatcc 1980

ttggtggtcc ccttggaaga ccgtgtggta tctgtgggag aaacagtggc cctccaatgc 2040

aaagccacgg ggaaccctcc gccccgcatc acctggttca agggggaccg cccgctgagc 2100

ctcactgagc ggcaccacct gacccctgac aaccagctcc tggtggttca gaacgtggtg 2160

gcagaggatg cgggccgata tacctgtgag atgtccaaca ccctgggcac ggagcgagct 2220

cacagccagc tgagcgtcct gcccgcagca ggctgcagga aggatgggac cacggtaggc 2280

atcttcggcg gtggcggatc ccgcaacacc ggccgcggcg gcgaggagaa gaagaaggag 2340

aaggagaagg aggagcagga ggagcgcgag accaagaccc ccgagtgccc cagccacacc 2400

cagcccctgg gcgtgttcct gtttcctcca aagcccaagg acacactgat gatctccagg 2460

acaccagagg tgacctgcgt ggtggtggac gtgagccacg aggaccccga ggtgaagttc 2520

aactggtacg tggatggcgt ggaggtgcac aatgccaaga ccaagcccag agaggagcag 2580

tacaactcta cctatagggt ggtgagcgtg ctgacagtgc tgcaccagga ctggctgaac 2640

ggcaaggagt ataagtgcaa ggtgagcaat aaggccctgc ctgccccaat cgagaagaca 2700

atctccaagg ccaagggcca gccaagagag ccccaggtgt acaccctgcc ccctagcagg 2760

gatgagctga caaagaacca ggtgtccctg acctgtctgg tgaagggctt ttatccctcc 2820

gacatcgccg tggagtggga gtctaatggc cagcctgaga ataactacaa gacaacccca 2880

cccgtgctgg attctgacgg cagcttcttt ctgtattcta agctgaccgt ggacaagagc 2940

aggtggcagc agggcaacgt gttcagctgc tccgtgatgc acgaagcact gcacaatcac 3000

tacacccaga aatcactgtc actgagccct ggcaaa 3036

<210> 68
<211> 2997
<212> DNA
<213> Artificial Sequence

<220>
<223> fusion protein

<400> 68
gctgggcctc gggctccttg tgctgccgcc tgcacatgtg caggcgattc cctggactgc 60

ggcggcagag gcctggccgc cctgcctggc gatctgccat cctggacccg gagcctgaac 120

ctgagctaca acaagctgag cgagatcgat cccgccggct ttgaggacct gcctaacctg 180

caggaggtgt atctgaacaa taacgagctg accgcggtac catccctggg cgctgcttca 240

tcacatgtcg tctctctctt tctgcagcac aacaagattc gcagcgtgga ggggagccag 300

ctgaaggcct acctttcctt agaagtgtta gatctgagtt tgaacaacat cacggaagtg 360

cggaacacct gctttccaca cggaccgcct ataaaggagc tcaacctggc aggcaatcgg 420

attggcaccc tggagttggg agcatttgat ggtctgtcac ggtcgctgct aactcttcgc 480

ctgagcaaaa acaggatcac ccagcttcct gtaagagcat tcaagctacc caggctgaca 540

caactggacc tcaatcggaa caggattcgg ctgatagagg gcctcacctt ccaggggctc 600

aacagcttgg aggtgctgaa gcttcagcga aacaacatca gcaaactgac agatggggcc 660

ttctggggac tgtccaagat gcatgtgctg cacctggagt acaacagcct ggtagaagtg 720

aacagcggct cgctctacgg cctcacggcc ctgcatcagc tccacctcag caacaattcc 780

atcgctcgca ttcaccgcaa gggctggagc ttctgccaga agctgcatga gttggtcctg 840

tccttcaaca acctgacacg gctggacgag gagagcctgg ccgagctgag cagcctgagt 900

gtcctgcgtc tcagccacaa ttccatcagc cacattgcgg agggtgcctt caagggactc 960

aggagcctgc gagtcttgga tctggaccat aacgagattt cgggcacaat agaggacacg 1020

agcggcgcct tctcagggct cgacagcctc agcaagctga ctctgtttgg aaacaagatc 1080

aagtctgtgg ctaagagagc attctcgggg ctggaaggcc tggagcacct gaaccttgga 1140

gggaatgcga tcagatctgt ccagtttgat gcctttgtga agatgaagaa tcttaaagag 1200

ctccatatca gcagcgacag cttcctgtgt gactgccagc tgaagtggct gcccccgtgg 1260

ctaattggca ggatgctgca ggcctttgtg acagccacct gtgcccaccc agaatcactg 1320

aagggtcaga gcattttctc tgtgccacca gagagtttcg tgtgcgatga cttcctgaag 1380

ccacagatca tcacccagcc agaaaccacc atggctatgg tgggcaagga catccggttt 1440

acatgctcag cagccagcag cagcagctcc cccatgacct ttgcctggaa gaaagacaat 1500

gaagtcctga ccaatgcaga catggagaac tttgtccacg tccacgcgca ggacggggaa 1560

gtgatggagt acaccaccat cctgcacctc cgtcaggtca ctttcgggca cgagggccgc 1620

taccaatgtg tcatcaccaa ccactttggc tccacctatt cacataaggc caggctcacc 1680

gtgaatgtgt tgccatcatt caccaaaacg ccccacgaca taaccatccg gaccaccacc 1740

gtggcccgcc tcgaatgtgc tgccacaggt cacccaaacc ctcagattgc ctggcagaag 1800

gatggaggca cggatttccc cgctgcccgt gagcgacgca tgcatgtcat gccggatgac 1860

gacgtgtttt tcatcactga tgtgaaaata gatgacgcag gggtttacag ctgtactgct 1920

cagaactcag ccggttctat ttcagctaat gccaccctga ctgtcctaga gaccccatcc 1980

ttggtggtcc ccttggaaga ccgtgtggta tctgtgggag aaacagtggc cctccaatgc 2040

aaagccacgg ggaaccctcc gccccgcatc acctggttca agggggaccg cccgctgagc 2100

ctcactgagc ggcaccacct gacccctgac aaccagctcc tggtggttca gaacgtggtg 2160

gcagaggatg cgggccgata tacctgtgag atgtccaaca ccctgggcac ggagcgagct 2220

cacagccagc tgagcgtcct gcccgcagca ggctgcagga aggatgggac cacggtaggc 2280

atcttcggcg gtggcggatc cgaaccgaaa tcttctgaca aaacccacac ctctccgccg 2340

tctccggctc cggaactgct gggtggttct tctgttttcc tgtttcctcc aaagcccaag 2400

gacacactga tgatctccag gacaccagag gtgacctgcg tggtggtgga cgtgagccac 2460

gaggaccccg aggtgaagtt caactggtac gtggatggcg tggaggtgca caatgccaag 2520

accaagccca gagaggagca gtacaactct acctataggg tggtgagcgt gctgacagtg 2580

ctgcaccagg actggctgaa cggcaaggag tataagtgca aggtgagcaa taaggccctg 2640

cctgccccaa tcgagaagac aatctccaag gccaagggcc agccaagaga gccccaggtg 2700

tacaccctgc cccctagcag ggatgagctg acaaagaacc aggtgtccct gacctgtctg 2760

gtgaagggct tttatccctc cgacatcgcc gtggagtggg agtctaatgg ccagcctgag 2820

aataactaca agacaacccc acccgtgctg gattctgacg gcagcttctt tctgtattct 2880

aagctgaccg tggacaagag caggtggcag cagggcaacg tgttcagctg ctccgtgatg 2940

cacgaagcac tgcacaatca ctacacccag aaatcactgt cactgagccc tggcaaa 2997

<210> 69
<211> 997
<212> PRT
<213> Artificial Sequence

<220>
<223> fusion protein

<400> 69
Ala Gln Ala Gly Pro Arg Ala Pro Cys Ala Ala Ala Cys Thr Cys Ala
1               5                   10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Gly Gly Gly Gly Ser Glu Pro Lys
        755                 760                 765

Ser Ser Asp Lys Thr His Thr Ser Pro Pro Cys Pro Ala Pro Glu Leu
    770                 775                 780

Leu Gly Gly Pro Ser Val Phe Leu Phe Pro Pro Lys Pro Lys Asp Thr
785                 790                 795                 800

Leu Met Ile Ser Arg Thr Pro Glu Val Thr Cys Val Val Val Asp Val
                805                 810                 815

Ser His Glu Asp Pro Glu Val Lys Phe Asn Trp Tyr Val Asp Gly Val
            820                 825                 830

Glu Val His Asn Ala Lys Thr Lys Pro Arg Glu Glu Gln Tyr Asn Ser
        835                 840                 845

Thr Tyr Arg Val Val Ser Val Leu Thr Val Leu His Gln Asp Trp Leu
    850                 855                 860

Asn Gly Lys Glu Tyr Lys Cys Lys Val Ser Asn Lys Ala Leu Pro Ala
865                 870                 875                 880

Pro Ile Glu Lys Thr Ile Ser Lys Ala Lys Gly Gln Pro Arg Glu Pro
                885                 890                 895

Gln Val Tyr Thr Leu Pro Pro Ser Arg Asp Glu Leu Thr Lys Asn Gln
            900                 905                 910

Val Ser Leu Thr Cys Leu Val Lys Gly Phe Tyr Pro Ser Asp Ile Ala
        915                 920                 925

Val Glu Trp Glu Ser Asn Gly Gln Pro Glu Asn Asn Tyr Lys Thr Thr
    930                 935                 940

Pro Pro Val Leu Asp Ser Asp Gly Ser Phe Phe Leu Tyr Ser Lys Leu
945                 950                 955                 960

Thr Val Asp Lys Ser Arg Trp Gln Gln Gly Asn Val Phe Ser Cys Ser
                965                 970                 975

Val Met His Glu Ala Leu His Asn His Tyr Thr Gln Lys Ser Leu Ser
            980                 985                 990

Leu Ser Pro Gly Lys
        995


<210>    70
<211>    998
<212>    PRT
<213>    Artificial Sequence

<220>
<223>    fusion protein


<400>    70
Ala Gln Ala Gly Pro Arg Ala Pro Cys Ala Ala Ala Cys Thr Cys Ala
 1               5                  10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
 65                 70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe

165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn

500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Gly Gly Gly Gly Ser Glu Pro Arg
        755                 760                 765

Gly Pro Thr Ile Lys Pro Cys Pro Pro Cys Lys Cys Pro Ala Pro Asn
    770                 775                 780

Leu Leu Gly Gly Pro Ser Val Phe Leu Phe Pro Pro Lys Pro Lys Asp
785                 790                 795                 800

Thr Leu Met Ile Ser Arg Thr Pro Glu Val Thr Cys Val Val Val Asp
                805                 810                 815

Val Ser His Glu Asp Pro Glu Val Lys Phe Asn Trp Tyr Val Asp Gly
            820                 825                 830

Val Glu Val His Asn Ala Lys Thr Lys Pro Arg Glu Glu Gln Tyr Asn

835                 840                 845

Ser Thr Tyr Arg Val Val Ser Val Leu Thr Val Leu His Gln Asp Trp
    850                 855                 860

Leu Asn Gly Lys Glu Tyr Lys Cys Lys Val Ser Asn Lys Ala Leu Pro
865                 870                 875                 880

Ala Pro Ile Glu Lys Thr Ile Ser Lys Ala Lys Gly Gln Pro Arg Glu
                885                 890                 895

Pro Gln Val Tyr Thr Leu Pro Pro Ser Arg Asp Glu Leu Thr Lys Asn
            900                 905                 910

Gln Val Ser Leu Thr Cys Leu Val Lys Gly Phe Tyr Pro Ser Asp Ile
        915                 920                 925

Ala Val Glu Trp Glu Ser Asn Gly Gln Pro Glu Asn Asn Tyr Lys Thr
    930                 935                 940

Thr Pro Pro Val Leu Asp Ser Asp Gly Ser Phe Phe Leu Tyr Ser Lys
945                 950                 955                 960

Leu Thr Val Asp Lys Ser Arg Trp Gln Gln Gly Asn Val Phe Ser Cys
                965                 970                 975

Ser Val Met His Glu Ala Leu His Asn His Tyr Thr Gln Lys Ser Leu
            980                 985                 990

Ser Leu Ser Pro Gly Lys
        995

<210>    71
<211>    1010
<212>    PRT
<213>    Artificial Sequence

<220>
<223>    fusion protein

<400>    71
Ala Gln Ala Gly Pro Arg Ala Pro Cys Ala Ala Ala Cys Thr Cys Ala
1               5                   10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Gly Gly Gly Gly Ser Arg Asn Thr
        755                 760                 765

Gly Arg Gly Gly Glu Glu Lys Lys Lys Glu Lys Glu Lys Glu Glu Gln
    770                 775                 780

Glu Glu Arg Glu Thr Lys Thr Pro Glu Cys Pro Ser His Thr Gln Pro
785                 790                 795                 800

Leu Gly Val Phe Leu Phe Pro Pro Lys Pro Lys Asp Thr Leu Met Ile
                805                 810                 815

Ser Arg Thr Pro Glu Val Thr Cys Val Val Val Asp Val Ser His Glu
            820                 825                 830

Asp Pro Glu Val Lys Phe Asn Trp Tyr Val Asp Gly Val Glu Val His
        835                 840                 845

Asn Ala Lys Thr Lys Pro Arg Glu Glu Gln Tyr Asn Ser Thr Tyr Arg
    850                 855                 860

Val Val Ser Val Leu Thr Val Leu His Gln Asp Trp Leu Asn Gly Lys
865                 870                 875                 880

Glu Tyr Lys Cys Lys Val Ser Asn Lys Ala Leu Pro Ala Pro Ile Glu
                885                 890                 895

Lys Thr Ile Ser Lys Ala Lys Gly Gln Pro Arg Glu Pro Gln Val Tyr
            900                 905                 910

Thr Leu Pro Pro Ser Arg Asp Glu Leu Thr Lys Asn Gln Val Ser Leu
        915                 920                 925

Thr Cys Leu Val Lys Gly Phe Tyr Pro Ser Asp Ile Ala Val Glu Trp
    930                 935                 940

Glu Ser Asn Gly Gln Pro Glu Asn Asn Tyr Lys Thr Thr Pro Pro Val
945                 950                 955                 960

Leu Asp Ser Asp Gly Ser Phe Phe Leu Tyr Ser Lys Leu Thr Val Asp
                965                 970                 975

Lys Ser Arg Trp Gln Gln Gly Asn Val Phe Ser Cys Ser Val Met His
            980                 985                 990

Glu Ala Leu His Asn His Tyr Thr Gln Lys Ser Leu Ser Leu Ser Pro
        995                 1000                1005

Gly Lys
    1010

<210>  72
<211>  997
<212>  PRT
<213>  Artificial Sequence

<220>
<223>  fusion protein

<400>  72
Ala Gln Ala Gly Pro Arg Ala Pro Cys Ala Ala Ala Cys Thr Cys Ala
1               5                   10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Gly Gly Gly Gly Ser Glu Pro Lys
        755                 760                 765

Ser Ser Asp Lys Thr His Thr Ser Pro Pro Ser Pro Ala Pro Glu Leu
    770                 775                 780

Leu Gly Gly Ser Ser Val Phe Leu Phe Pro Pro Lys Pro Lys Asp Thr
785                 790                 795                 800

Leu Met Ile Ser Arg Thr Pro Glu Val Thr Cys Val Val Val Asp Val
                805                 810                 815

Ser His Glu Asp Pro Glu Val Lys Phe Asn Trp Tyr Val Asp Gly Val
            820                 825                 830

Glu Val His Asn Ala Lys Thr Lys Pro Arg Glu Glu Gln Tyr Asn Ser
        835                 840                 845

Thr Tyr Arg Val Val Ser Val Leu Thr Val Leu His Gln Asp Trp Leu
    850                 855                 860

Asn Gly Lys Glu Tyr Lys Cys Lys Val Ser Asn Lys Ala Leu Pro Ala
865                 870                 875                 880

Pro Ile Glu Lys Thr Ile Ser Lys Ala Lys Gly Gln Pro Arg Glu Pro
                885                 890                 895

Gln Val Tyr Thr Leu Pro Pro Ser Arg Asp Glu Leu Thr Lys Asn Gln
            900                 905                 910

Val Ser Leu Thr Cys Leu Val Lys Gly Phe Tyr Pro Ser Asp Ile Ala
        915                 920                 925

Val Glu Trp Glu Ser Asn Gly Gln Pro Glu Asn Asn Tyr Lys Thr Thr
    930                 935                 940

Pro Pro Val Leu Asp Ser Asp Gly Ser Phe Phe Leu Tyr Ser Lys Leu
945                 950                 955                 960

Thr Val Asp Lys Ser Arg Trp Gln Gln Gly Asn Val Phe Ser Cys Ser
                965                 970                 975

Val Met His Glu Ala Leu His Asn His Tyr Thr Gln Lys Ser Leu Ser
            980                 985                 990

Leu Ser Pro Gly Lys
        995

<210>  73
<211>  2991
<212>  DNA
<213>  Artificial Sequence

<220>
<223>    fusion protein


<400>    73
gctcaggctg gacctagggc tccttgcgct gccgcctgca cctgtgcagg cgattctctg      60

gactgcagcg gccggggcct ggccacactg cccagggacc tgccttcctg gaccagatct     120

ctgaacctga gctacaatcg gctgtccgag atcgattctg ccgcctttga ggacctgaca     180

aatctgcagg aggtgtatct gaacagcaat gagctgaccg caatcccctc cctgggagca     240

gcctctatcg gcgtggtgag cctgttcctg cagcacaaca agatcctgag cgtggatggc     300

tcccagctga agagctacct gtctctggag gtgctggacc tgagctccaa caatatcacc     360

gagatcagat ctagctgttt tcctaatggc ctgcggatca gagagctgaa cctggcctct     420

aatcggatca gcatcctgga gtccggcgcc ttcgatggcc tgagcagatc cctgctgaca     480

ctgcgcctgt ccaagaaccg gatcacccag ctgcccgtga aggcctttaa gctgcctagg     540

ctgacacagc tggacctgaa ccggaataga atcaggctga tcgagggcct gaccttccag     600

ggcctggata gcctggaggt gctgcgcctg cagcggaaca atatctcccg cctgacagac     660

ggagcatttt ggggcctgtc taagatgcac gtgctgcacc tggagtacaa tagcctggtg     720

gaggtgaact ctggcagcct gtatggcctg accgccctgc accagctgca cctgtccaac     780

aatagcatca gcagaatcca gagggatggc tggtccttct gccagaagct gcacgagctg     840

atcctgtctt ttaacaatct gaccaggctg gacgaggaga gcctggcaga gctgtcctct     900

ctgtccatcc tgcgcctgtc tcacaatgcc atcagccaca tcgccgaggg cgcctttaag     960

ggcctgaaga gcctgagggt gctggatctg gaccacaacg agatctctgg caccatcgag    1020

gatacaagcg gcgccttcac aggcctggac aatctgtcca agctgaccct gtttggcaac    1080

aagatcaagt ctgtggccaa gcgggccttc tctggcctgg agagcctgga gcacctgaac    1140

ctgggcgaga atgccatcag atccgtgcag ttcgatgcct ttgccaagat gaagaatctg    1200

aaggagctgt acatcagctc cgagagcttc ctgtgcgact gtcagctgaa gtggctgcca    1260

ccttggctga tgggaaggat gctgcaggcc tttgtgaccg ccacatgcgc ccacccagag    1320

agcctgaagg gccagagcat cttctccgtg ctgcccgata gcttcgtgtg cgacgatttt    1380

cctaagccac agatcatcac ccagccagag acaacaatgg ccgtggtggg caaggacatc    1440

cggtttacat gttccgccgc ctctagctcc tctagcccca tgaccttcgc ctggaagaag    1500

gataacgagg tgctggccaa tgccgacatg gagaacttcg cccacgtgag agcccaggat    1560

ggcgaagtga tggagtatac cacaatcctg cacctgcggc acgtgacctt tggccacgag    1620

ggcagatacc agtgcatcat cacaaatcac ttcggctcta cctatagcca caaggccagg    1680

ctgacagtga acgtgctgcc tagctttacc aagatcccac acgacatcgc catcagaaca    1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

ggcggtggcg gatccgagcc aaagtcctct gataagacac acacctctcc accatgccca 2340

gcaccagagc tgctgggagg accaagcgtg ttcctgtttc ctccaaagcc caaggacaca 2400

ctgatgatct ccaggacacc agaggtgacc tgcgtggtgg tggacgtgag ccacgaggac 2460

cccgaggtga agttcaactg gtacgtggat ggcgtggagg tgcacaatgc caagaccaag 2520

cccagagagg agcagtacaa ctctacctat agggtggtga gcgtgctgac agtgctgcac 2580

caggactggc tgaacggcaa ggagtataag tgcaaggtga gcaataaggc cctgcctgcc 2640

ccaatcgaga agacaatctc caaggccaag ggccagccaa gagagcccca ggtgtacacc 2700

ctgcccccta gcagggatga gctgacaaag aaccaggtgt ccctgacctg tctggtgaag 2760

ggcttttatc cctccgacat cgccgtggag tgggagtcta atggccagcc tgagaataac 2820

tacaagacaa ccccacccgt gctggattct gacggcagct tctttctgta ttctaagctg 2880

accgtggaca agagcaggtg gcagcagggc aacgtgttca gctgctccgt gatgcacgaa 2940

gcactgcaca atcactacac ccagaaatca ctgtcactga gccctggcaa a 2991

<210> 74
<211> 2994
<212> DNA
<213> Artificial Sequence

<220>
<223> fusion protein

<400> 74
gctcaggctg gacctagggc tccttgcgct gccgcctgca cctgtgcagg cgattctctg 60

gactgcagcg gccggggcct ggccacactg cccagggacc tgccttcctg gaccagatct 120

ctgaacctga gctacaatcg gctgtccgag atcgattctg ccgcctttga ggacctgaca 180

aatctgcagg aggtgtatct gaacagcaat gagctgaccg caatcccctc cctgggagca 240

gcctctatcg gcgtggtgag cctgttcctg cagcacaaca agatcctgag cgtggatggc 300

tcccagctga agagctacct gtctctggag gtgctggacc tgagctccaa caatatcacc 360

gagatcagat ctagctgttt tcctaatggc ctgcggatca gagagctgaa cctggcctct 420

aatcggatca gcatcctgga gtccggcgcc ttcgatggcc tgagcagatc cctgctgaca 480

ctgcgcctgt ccaagaaccg gatcacccag ctgcccgtga aggcctttaa gctgcctagg 540

ctgacacagc tggacctgaa ccggaataga atcaggctga tcgagggcct gaccttccag 600

ggcctggata gcctggaggt gctgcgcctg cagcggaaca atatctcccg cctgacagac 660

ggagcatttt ggggcctgtc taagatgcac gtgctgcacc tggagtacaa tagcctggtg 720

gaggtgaact ctggcagcct gtatggcctg accgccctgc accagctgca cctgtccaac 780

aatagcatca gcagaatcca gagggatggc tggtccttct gccagaagct gcacgagctg 840

atcctgtctt ttaacaatct gaccaggctg gacgaggaga gcctggcaga gctgtcctct 900

ctgtccatcc tgcgcctgtc tcacaatgcc atcagccaca tcgccgaggg cgcctttaag 960

ggcctgaaga gcctgagggt gctggatctg gaccacaacg agatctctgg caccatcgag 1020

gatacaagcg gcgccttcac aggcctggac aatctgtcca agctgaccct gtttggcaac 1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

ggcggtggcg gatccgagcc tcggggccct accatcaagc cctgcccccc ttgcaagtgc 2340

cctgccccta atctgctggg cggaccctcc gtgttcctgt ttcctccaaa gcccaaggac 2400

acactgatga tctccaggac accagaggtg acctgcgtgg tggtggacgt gagccacgag 2460

gaccccgagg tgaagttcaa ctggtacgtg gatggcgtgg aggtgcacaa tgccaagacc 2520

aagcccagag aggagcagta caactctacc tatagggtgg tgagcgtgct gacagtgctg 2580

caccaggact ggctgaacgg caaggagtat aagtgcaagg tgagcaataa ggccctgcct 2640

gccccaatcg agaagacaat ctccaaggcc aagggccagc caagagagcc ccaggtgtac 2700

accctgcccc ctagcaggga tgagctgaca aagaaccagg tgtccctgac ctgtctggtg 2760

aagggctttt atccctccga catcgccgtg gagtgggagt ctaatggcca gcctgagaat 2820

aactacaaga caaccccacc cgtgctggat tctgacggca gcttctttct gtattctaag 2880

ctgaccgtgg acaagagcag gtggcagcag ggcaacgtgt tcagctgctc cgtgatgcac 2940

gaagcactgc acaatcacta cacccagaaa tcactgtcac tgagccctgg caaa 2994

<210> 75
<211> 3030
<212> DNA
<213> Artificial Sequence

<220>
<223> fusion protein

<400> 75
gctcaggctg gacctagggc tccttgcgct gccgcctgca cctgtgcagg cgattctctg 60

gactgcagcg gccggggcct ggccacactg cccagggacc tgccttcctg gaccagatct 120

ctgaacctga gctacaatcg gctgtccgag atcgattctg ccgcctttga ggacctgaca 180

aatctgcagg aggtgtatct gaacagcaat gagctgaccg caatcccctc cctgggagca 240

gcctctatcg gcgtggtgag cctgttcctg cagcacaaca agatcctgag cgtggatggc 300

tcccagctga agagctacct gtctctggag gtgctggacc tgagctccaa caatatcacc 360

gagatcagat ctagctgttt tcctaatggc ctgcggatca gagagctgaa cctggcctct 420

aatcggatca gcatcctgga gtccggcgcc ttcgatggcc tgagcagatc cctgctgaca 480

ctgcgcctgt ccaagaaccg gatcacccag ctgcccgtga aggcctttaa gctgcctagg 540

ctgacacagc tggacctgaa ccggaataga atcaggctga tcgagggcct gaccttccag 600

ggcctggata gcctggaggt gctgcgcctg cagcggaaca atatctcccg cctgacagac 660

ggagcatttt ggggcctgtc taagatgcac gtgctgcacc tggagtacaa tagcctggtg 720

gaggtgaact ctggcagcct gtatggcctg accgccctgc accagctgca cctgtccaac 780

aatagcatca gcagaatcca gagggatggc tggtccttct gccagaagct gcacgagctg 840

atcctgtctt ttaacaatct gaccaggctg gacgaggaga gcctggcaga gctgtcctct 900

ctgtccatcc tgcgcctgtc tcacaatgcc atcagccaca tcgccgaggg cgcctttaag 960

ggcctgaaga gcctgagggt gctggatctg gaccacaacg agatctctgg caccatcgag 1020

gatacaagcg gcgccttcac aggcctggac aatctgtcca agctgaccct gtttggcaac 1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

ggcggtggcg gatcccgcaa caccggccgc ggcggcgagg agaagaagaa ggagaaggag 2340

aaggaggagc aggaggagcg cgagaccaag acccccgagt gccccagcca cacccagccc 2400

ctgggcgtgt tcctgtttcc tccaaagccc aaggacacac tgatgatctc caggacacca 2460

gaggtgacct gcgtggtggt ggacgtgagc cacgaggacc ccgaggtgaa gttcaactgg 2520

tacgtggatg gcgtggaggt gcacaatgcc aagaccaagc ccagagagga gcagtacaac 2580

tctacctata gggtggtgag cgtgctgaca gtgctgcacc aggactggct gaacggcaag 2640

gagtataagt gcaaggtgag caataaggcc ctgcctgccc caatcgagaa gacaatctcc 2700

aaggccaagg gccagccaag agagccccag gtgtacaccc tgccccctag cagggatgag 2760

ctgacaaaga accaggtgtc cctgacctgt ctggtgaagg gcttttatcc ctccgacatc 2820

gccgtggagt gggagtctaa tggccagcct gagaataact acaagacaac cccacccgtg 2880

ctggattctg acggcagctt ctttctgtat tctaagctga ccgtggacaa gagcaggtgg 2940

cagcagggca acgtgttcag ctgctccgtg atgcacgaag cactgcacaa tcactacacc 3000

cagaaatcac tgtcactgag ccctggcaaa 3030

<210> 76
<211> 2991
<212> DNA
<213> Artificial Sequence

<220>
<223> fusion protein

<400> 76
gctcaggctg gacctagggc tccttgcgct gccgcctgca cctgtgcagg cgattctctg 60

gactgcagcg gccggggcct ggccacactg cccagggacc tgccttcctg gaccagatct 120

ctgaacctga gctacaatcg gctgtccgag atcgattctg ccgcctttga ggacctgaca 180

aatctgcagg aggtgtatct gaacagcaat gagctgaccg caatcccctc cctgggagca 240

gcctctatcg gcgtggtgag cctgttcctg cagcacaaca agatcctgag cgtggatggc 300

tcccagctga agagctacct gtctctggag gtgctggacc tgagctccaa caatatcacc 360

gagatcagat ctagctgttt tcctaatggc ctgcggatca gagagctgaa cctggcctct 420

aatcggatca gcatcctgga gtccggcgcc ttcgatggcc tgagcagatc cctgctgaca 480

ctgcgcctgt ccaagaaccg gatcacccag ctgcccgtga aggcctttaa gctgcctagg 540

ctgacacagc tggacctgaa ccggaataga atcaggctga tcgagggcct gaccttccag 600

ggcctggata gcctggaggt gctgcgcctg cagcggaaca atatctcccg cctgacagac 660

ggagcatttt ggggcctgtc taagatgcac gtgctgcacc tggagtacaa tagcctggtg 720

gaggtgaact ctggcagcct gtatggcctg accgccctgc accagctgca cctgtccaac 780

aatagcatca gcagaatcca gagggatggc tggtccttct gccagaagct gcacgagctg 840

atcctgtctt ttaacaatct gaccaggctg gacgaggaga gcctggcaga gctgtcctct 900

ctgtccatcc tgcgcctgtc tcacaatgcc atcagccaca tcgccgaggg cgcctttaag 960

ggcctgaaga gcctgagggt gctggatctg gaccacaacg agatctctgg caccatcgag 1020

gatacaagcg gcgccttcac aggcctggac aatctgtcca agctgaccct gtttggcaac 1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

ggcggtggcg gatccgaacc gaaatcttct gacaaaaccc acacctctcc gccgtctccg 2340

gctccggaac tgctgggtgg ttcttctgtt ttcctgtttc ctccaaagcc caaggacaca 2400

ctgatgatct ccaggacacc agaggtgacc tgcgtggtgg tggacgtgag ccacgaggac 2460

cccgaggtga agttcaactg gtacgtggat ggcgtggagg tgcacaatgc caagaccaag 2520

cccagagagg agcagtacaa ctctacctat agggtggtga gcgtgctgac agtgctgcac 2580

caggactggc tgaacggcaa ggagtataag tgcaaggtga gcaataaggc cctgcctgcc 2640

ccaatcgaga agacaatctc caaggccaag ggccagccaa gagagcccca ggtgtacacc 2700

ctgcccccta gcagggatga gctgacaaag aaccaggtgt ccctgacctg tctggtgaag 2760

ggcttttatc cctccgacat cgccgtggag tgggagtcta atggccagcc tgagaataac 2820

tacaagacaa ccccacccgt gctggattct gacggcagct tctttctgta ttctaagctg 2880

accgtggaca agagcaggtg gcagcagggc aacgtgttca gctgctccgt gatgcacgaa 2940

gcactgcaca atcactacac ccagaaatca ctgtcactga gccctggcaa a 2991

<210>    77
<211>    997
<212>    PRT
<213>    Artificial Sequence

<220>
<223>    fusion protein


<400>    77
   Ala Gln Ala Gly Pro Arg Ala Pro Cys Ala Ala Ala Cys Thr Cys Ala
   1               5                   10                  15
   Gly Asp Ser Leu Asp Cys Ser Gly Arg Gly Leu Ala Thr Leu Pro Arg
               20                  25                  30
   Asp Leu Pro Ser Trp Thr Arg Ser Leu Asn Leu Ser Tyr Asn Arg Leu
           35                  40                  45
   Ser Glu Ile Asp Ser Ala Ala Phe Glu Asp Leu Thr Asn Leu Gln Glu
       50                  55                  60
   Val Tyr Leu Asn Ser Asn Glu Leu Thr Ala Ile Pro Ser Leu Gly Ala
   65                  70                  75                  80
   Ala Ser Ile Gly Val Val Ser Leu Phe Leu Gln His Asn Lys Ile Leu
                   85                  90                  95
   Ser Val Asp Gly Ser Gln Leu Lys Ser Tyr Leu Ser Leu Glu Val Leu
               100                 105                 110
   Asp Leu Ser Ser Asn Asn Ile Thr Glu Ile Arg Ser Ser Cys Phe Pro
           115                 120                 125
   Asn Gly Leu Arg Ile Arg Glu Leu Asn Leu Ala Ser Asn Arg Ile Ser
       130                 135                 140
   Ile Leu Glu Ser Gly Ala Phe Asp Gly Leu Ser Arg Ser Leu Leu Thr
   145                 150                 155                 160
   Leu Arg Leu Ser Lys Asn Arg Ile Thr Gln Leu Pro Val Lys Ala Phe
                   165                 170                 175
   Lys Leu Pro Arg Leu Thr Gln Leu Asp Leu Asn Arg Asn Arg Ile Arg
               180                 185                 190
   Leu Ile Glu Gly Leu Thr Phe Gln Gly Leu Asp Ser Leu Glu Val Leu
           195                 200                 205
   Arg Leu Gln Arg Asn Asn Ile Ser Arg Leu Thr Asp Gly Ala Phe Trp
       210                 215                 220
   Gly Leu Ser Lys Met His Val Leu His Leu Glu Tyr Asn Ser Leu Val
   225                 230                 235                 240
   Glu Val Asn Ser Gly Ser Leu Tyr Gly Leu Thr Ala Leu His Gln Leu
                   245                 250                 255
   His Leu Ser Asn Asn Ser Ile Ser Arg Ile Gln Arg Asp Gly Trp Ser
               260                 265                 270
   Phe Cys Gln Lys Leu His Glu Leu Ile Leu Ser Phe Asn Asn Leu Thr
           275                 280                 285
   Arg Leu Asp Glu Glu Ser Leu Ala Glu Leu Ser Ser Leu Ser Ile Leu
       290                 295                 300
   Arg Leu Ser His Asn Ala Ile Ser His Ile Ala Glu Gly Ala Phe Lys
   305                 310                 315                 320
   Gly Leu Lys Ser Leu Arg Val Leu Asp Leu Asp His Asn Glu Ile Ser
                   325                 330                 335
   Gly Thr Ile Glu Asp Thr Ser Gly Ala Phe Thr Gly Leu Asp Asn Leu
               340                 345                 350
   Ser Lys Leu Thr Leu Phe Gly Asn Lys Ile Lys Ser Val Ala Lys Arg
           355                 360                 365
   Ala Phe Ser Gly Leu Glu Ser Leu Glu His Leu Asn Leu Gly Glu Asn
       370                 375                 380
   Ala Ile Arg Ser Val Gln Phe Asp Ala Phe Ala Lys Met Lys Asn Leu
   385                 390                 395                 400
   Lys Glu Leu Tyr Ile Ser Ser Glu Ser Phe Leu Cys Asp Cys Gln Leu
                   405                 410                 415
   Lys Trp Leu Pro Pro Trp Leu Met Gly Arg Met Leu Gln Ala Phe Val
               420                 425                 430
   Thr Ala Thr Cys Ala His Pro Glu Ser Leu Lys Gly Gln Ser Ile Phe
           435                 440                 445
   Ser Val Leu Pro Asp Ser Phe Val Cys Asp Asp Phe Pro Lys Pro Gln
       450                 455                 460
   Ile Ile Thr Gln Pro Glu Thr Thr Met Ala Val Val Gly Lys Asp Ile
   465                 470                 475                 480
   Arg Phe Thr Cys Ser Ala Ala Ser Ser Ser Ser Ser Pro Met Thr Phe
                   485                 490                 495
   Ala Trp Lys Lys Asp Asn Glu Val Leu Ala Asn Ala Asp Met Glu Asn
               500                 505                 510
   Phe Ala His Val Arg Ala Gln Asp Gly Glu Val Met Glu Tyr Thr Thr
           515                 520                 525
   Ile Leu His Leu Arg His Val Thr Phe Gly His Glu Gly Arg Tyr Gln
       530                 535                 540
   Cys Ile Ile Thr Asn His Phe Gly Ser Thr Tyr Ser His Lys Ala Arg
   545                 550                 555                 560
   Leu Thr Val Asn Val Leu Pro Ser Phe Thr Lys Ile Pro His Asp Ile
                   565                 570                 575
   Ala Ile Arg Thr Gly Thr Thr Ala Arg Leu Glu Cys Ala Ala Thr Gly
               580                 585                 590
   His Pro Asn Pro Gln Ile Ala Trp Gln Lys Asp Gly Gly Thr Asp Phe
           595                 600                 605
   Pro Ala Ala Arg Glu Arg Arg Met His Val Met Pro Asp Asp Asp Val
       610                 615                 620
   Phe Phe Ile Thr Asp Val Lys Ile Asp Asp Met Gly Val Tyr Ser Cys
   625                 630                 635                 640
   Thr Ala Gln Asn Ser Ala Gly Ser Val Ser Ala Asn Ala Thr Leu Thr
                   645                 650                 655
   Val Leu Glu Thr Pro Ser Leu Ala Val Pro Leu Glu Asp Arg Val Val
               660                 665                 670
   Thr Val Gly Glu Thr Val Ala Phe Gln Cys Lys Ala Thr Gly Ser Pro
           675                 680                 685
   Thr Pro Arg Ile Thr Trp Leu Lys Gly Gly Arg Pro Leu Ser Leu Thr
       690                 695                 700
   Glu Arg His His Phe Thr Pro Gly Asn Gln Leu Leu Val Val Gln Asn
   705                 710                 715                 720
   Val Met Ile Asp Asp Ala Gly Arg Tyr Thr Cys Glu Met Ser Asn Pro
                   725                 730                 735
   Leu Gly Thr Glu Arg Ala His Ser Gln Leu Ser Ile Leu Pro Thr Pro
               740                 745                 750
   Gly Cys Arg Lys Asp Gly Thr Thr Gly Gly Gly Gly Ser Glu Pro Lys
           755                 760                 765
   Ser Ser Asp Lys Thr His Thr Ser Pro Pro Cys Pro Ala Pro Glu Leu
       770                 775                 780
   Leu Gly Gly Pro Ser Val Phe Ile Phe Pro Pro Lys Ile Lys Asp Val
   785                 790                 795                 800
   Leu Met Ile Ser Leu Ser Pro Ile Val Thr Cys Val Val Val Asp Val
                   805                 810                 815
   Ser Glu Asp Asp Pro Asp Val Gln Ile Ser Trp Phe Val Asn Asn Val
               820                 825                 830
   Glu Val His Thr Ala Gln Thr Gln Thr His Arg Glu Asp Tyr Asn Ser
           835                 840                 845
   Thr Leu Arg Val Val Ser Ala Leu Pro Ile Gln His Gln Asp Trp Met
       850                 855                 860
   Ser Gly Lys Glu Phe Lys Cys Lys Val Asn Asn Lys Asp Leu Pro Ala
   865                 870                 875                 880
   Pro Ile Glu Arg Thr Ile Ser Lys Pro Lys Gly Ser Val Arg Ala Pro
                   885                 890                 895
   Gln Val Tyr Val Leu Pro Pro Pro Glu Glu Glu Met Thr Lys Lys Gln
               900                 905                 910
   Val Thr Leu Thr Cys Met Val Thr Asp Phe Met Pro Glu Asp Ile Tyr
           915                 920                 925
   Val Glu Trp Thr Asn Asn Gly Lys Thr Glu Leu Asn Tyr Lys Asn Thr
       930                 935                 940
   Glu Pro Val Leu Asp Ser Asp Gly Ser Tyr Phe Met Tyr Ser Lys Leu
   945                 950                 955                 960
   Arg Val Glu Lys Lys Asn Trp Val Glu Arg Asn Ser Tyr Ser Cys Ser
                   965                 970                 975
   Val Val His Glu Gly Leu His Asn His His Thr Thr Lys Ser Phe Ser
               980                 985                 990
   Arg Thr Pro Gly Lys
           995

<210>    78
<211>    998
<212>    PRT
<213>    Artificial Sequence

<220>
<223>    fusion protein

<400>    78
Ala Gln Ala Gly Pro Arg Ala Pro Cys Ala Ala Ala Cys Thr Cys Ala
 1               5                  10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Gly Gly Gly Gly Ser Glu Pro Arg
        755                 760                 765

Gly Pro Thr Ile Lys Pro Cys Pro Pro Cys Lys Cys Pro Ala Pro Asn
    770                 775                 780

Leu Leu Gly Gly Pro Ser Val Phe Ile Phe Pro Pro Lys Ile Lys Asp
785                 790                 795                 800

Val Leu Met Ile Ser Leu Ser Pro Ile Val Thr Cys Val Val Val Asp
                805                 810                 815

Val Ser Glu Asp Asp Pro Asp Val Gln Ile Ser Trp Phe Val Asn Asn
            820                 825                 830

Val Glu Val His Thr Ala Gln Thr Gln Thr His Arg Glu Asp Tyr Asn
        835                 840                 845

Ser Thr Leu Arg Val Val Ser Ala Leu Pro Ile Gln His Gln Asp Trp
    850                 855                 860

Met Ser Gly Lys Glu Phe Lys Cys Lys Val Asn Asn Lys Asp Leu Pro
865                 870                 875                 880

Ala Pro Ile Glu Arg Thr Ile Ser Lys Pro Lys Gly Ser Val Arg Ala
                885                 890                 895

Pro Gln Val Tyr Val Leu Pro Pro Pro Glu Glu Glu Met Thr Lys Lys
            900                 905                 910

Gln Val Thr Leu Thr Cys Met Val Thr Asp Phe Met Pro Glu Asp Ile
        915                 920                 925

Tyr Val Glu Trp Thr Asn Asn Gly Lys Thr Glu Leu Asn Tyr Lys Asn
    930                 935                 940

Thr Glu Pro Val Leu Asp Ser Asp Gly Ser Tyr Phe Met Tyr Ser Lys
945                 950                 955                 960

Leu Arg Val Glu Lys Lys Asn Trp Val Glu Arg Asn Ser Tyr Ser Cys
                965                 970                 975

Ser Val Val His Glu Gly Leu His Asn His His Thr Thr Lys Ser Phe
            980                 985                 990

Ser Arg Thr Pro Gly Lys
        995

<210> 79
<211> 1010
<212> PRT
<213> Artificial Sequence

<220>
<223> fusion protein

<400> 79
Ala Gln Ala Gly Pro Arg Ala Pro Cys Ala Ala Ala Cys Thr Cys Ala
1               5                   10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe

165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn

500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Gly Gly Gly Gly Ser Arg Asn Thr
        755                 760                 765

Gly Arg Gly Gly Glu Glu Lys Lys Lys Glu Lys Glu Lys Glu Glu Gln
    770                 775                 780

Glu Glu Arg Glu Thr Lys Thr Pro Glu Cys Pro Ser His Thr Gln Pro
785                 790                 795                 800

Leu Gly Val Phe Ile Phe Pro Pro Lys Ile Lys Asp Val Leu Met Ile
                805                 810                 815

Ser Leu Ser Pro Ile Val Thr Cys Val Val Val Asp Val Ser Glu Asp
            820                 825                 830

Asp Pro Asp Val Gln Ile Ser Trp Phe Val Asn Asn Val Glu Val His

835                 840                 845

Thr Ala Gln Thr Gln Thr His Arg Glu Asp Tyr Asn Ser Thr Leu Arg
    850                 855                 860

Val Val Ser Ala Leu Pro Ile Gln His Gln Asp Trp Met Ser Gly Lys
865                 870                 875                 880

Glu Phe Lys Cys Lys Val Asn Asn Lys Asp Leu Pro Ala Pro Ile Glu
                885                 890                 895

Arg Thr Ile Ser Lys Pro Lys Gly Ser Val Arg Ala Pro Gln Val Tyr
            900                 905                 910

Val Leu Pro Pro Pro Glu Glu Glu Met Thr Lys Lys Gln Val Thr Leu
        915                 920                 925

Thr Cys Met Val Thr Asp Phe Met Pro Glu Asp Ile Tyr Val Glu Trp
    930                 935                 940

Thr Asn Asn Gly Lys Thr Glu Leu Asn Tyr Lys Asn Thr Glu Pro Val
945                 950                 955                 960

Leu Asp Ser Asp Gly Ser Tyr Phe Met Tyr Ser Lys Leu Arg Val Glu
                965                 970                 975

Lys Lys Asn Trp Val Glu Arg Asn Ser Tyr Ser Cys Ser Val Val His
            980                 985                 990

Glu Gly Leu His Asn His His Thr Thr Lys Ser Phe Ser Arg Thr Pro
        995                 1000                1005

Gly Lys
   1010

<210>   80
<211>   997
<212>   PRT
<213>   Artificial Sequence

<220>
<223>   fusion protein

<400>   80
Ala Gln Ala Gly Pro Arg Ala Pro Cys Ala Ala Ala Cys Thr Cys Ala
1               5                   10                  15

Gly Asp Ser Leu Asp Cys Ser Gly Arg Gly Leu Ala Thr Leu Pro Arg
            20                  25                  30

Asp Leu Pro Ser Trp Thr Arg Ser Leu Asn Leu Ser Tyr Asn Arg Leu
        35                  40                  45

Ser Glu Ile Asp Ser Ala Ala Phe Glu Asp Leu Thr Asn Leu Gln Glu
    50                  55                  60

Val Tyr Leu Asn Ser Asn Glu Leu Thr Ala Ile Pro Ser Leu Gly Ala
65                  70                  75                  80

Ala Ser Ile Gly Val Val Ser Leu Phe Leu Gln His Asn Lys Ile Leu
                85                  90                  95

Ser Val Asp Gly Ser Gln Leu Lys Ser Tyr Leu Ser Leu Glu Val Leu
            100                 105                 110

Asp Leu Ser Ser Asn Asn Ile Thr Glu Ile Arg Ser Ser Cys Phe Pro
        115                 120                 125

Asn Gly Leu Arg Ile Arg Glu Leu Asn Leu Ala Ser Asn Arg Ile Ser
    130                 135                 140

Ile Leu Glu Ser Gly Ala Phe Asp Gly Leu Ser Arg Ser Leu Leu Thr
145                 150                 155                 160

Leu Arg Leu Ser Lys Asn Arg Ile Thr Gln Leu Pro Val Lys Ala Phe
                165                 170                 175

Lys Leu Pro Arg Leu Thr Gln Leu Asp Leu Asn Arg Asn Arg Ile Arg
            180                 185                 190

Leu Ile Glu Gly Leu Thr Phe Gln Gly Leu Asp Ser Leu Glu Val Leu
        195                 200                 205

Arg Leu Gln Arg Asn Asn Ile Ser Arg Leu Thr Asp Gly Ala Phe Trp
    210                 215                 220

Gly Leu Ser Lys Met His Val Leu His Leu Glu Tyr Asn Ser Leu Val
225                 230                 235                 240

Glu Val Asn Ser Gly Ser Leu Tyr Gly Leu Thr Ala Leu His Gln Leu
                245                 250                 255

His Leu Ser Asn Asn Ser Ile Ser Arg Ile Gln Arg Asp Gly Trp Ser
            260                 265                 270

Phe Cys Gln Lys Leu His Glu Leu Ile Leu Ser Phe Asn Asn Leu Thr
        275                 280                 285

Arg Leu Asp Glu Glu Ser Leu Ala Glu Leu Ser Ser Leu Ser Ile Leu
    290                 295                 300

Arg Leu Ser His Asn Ala Ile Ser His Ile Ala Glu Gly Ala Phe Lys
305                 310                 315                 320

Gly Leu Lys Ser Leu Arg Val Leu Asp Leu Asp His Asn Glu Ile Ser
                325                 330                 335

Gly Thr Ile Glu Asp Thr Ser Gly Ala Phe Thr Gly Leu Asp Asn Leu
            340                 345                 350

Ser Lys Leu Thr Leu Phe Gly Asn Lys Ile Lys Ser Val Ala Lys Arg
        355                 360                 365

Ala Phe Ser Gly Leu Glu Ser Leu Glu His Leu Asn Leu Gly Glu Asn
    370                 375                 380

Ala Ile Arg Ser Val Gln Phe Asp Ala Phe Ala Lys Met Lys Asn Leu
385                 390                 395                 400

Lys Glu Leu Tyr Ile Ser Ser Glu Ser Phe Leu Cys Asp Cys Gln Leu
                405                 410                 415

Lys Trp Leu Pro Pro Trp Leu Met Gly Arg Met Leu Gln Ala Phe Val
            420                 425                 430

Thr Ala Thr Cys Ala His Pro Glu Ser Leu Lys Gly Gln Ser Ile Phe
        435                 440                 445

Ser Val Leu Pro Asp Ser Phe Val Cys Asp Asp Phe Pro Lys Pro Gln
    450                 455                 460

Ile Ile Thr Gln Pro Glu Thr Thr Met Ala Val Val Gly Lys Asp Ile
465                 470                 475                 480

Arg Phe Thr Cys Ser Ala Ala Ser Ser Ser Ser Ser Pro Met Thr Phe
                485                 490                 495

Ala Trp Lys Lys Asp Asn Glu Val Leu Ala Asn Ala Asp Met Glu Asn
            500                 505                 510

Phe Ala His Val Arg Ala Gln Asp Gly Glu Val Met Glu Tyr Thr Thr
        515                 520                 525

Ile Leu His Leu Arg His Val Thr Phe Gly His Glu Gly Arg Tyr Gln
    530                 535                 540

Cys Ile Ile Thr Asn His Phe Gly Ser Thr Tyr Ser His Lys Ala Arg
545                 550                 555                 560

Leu Thr Val Asn Val Leu Pro Ser Phe Thr Lys Ile Pro His Asp Ile
                565                 570                 575

Ala Ile Arg Thr Gly Thr Thr Ala Arg Leu Glu Cys Ala Ala Thr Gly
            580                 585                 590

His Pro Asn Pro Gln Ile Ala Trp Gln Lys Asp Gly Gly Thr Asp Phe
        595                 600                 605

Pro Ala Ala Arg Glu Arg Arg Met His Val Met Pro Asp Asp Asp Val
    610                 615                 620

Phe Phe Ile Thr Asp Val Lys Ile Asp Asp Met Gly Val Tyr Ser Cys
625                 630                 635                 640

Thr Ala Gln Asn Ser Ala Gly Ser Val Ser Ala Asn Ala Thr Leu Thr
                645                 650                 655

Val Leu Glu Thr Pro Ser Leu Ala Val Pro Leu Glu Asp Arg Val Val
            660                 665                 670

Thr Val Gly Glu Thr Val Ala Phe Gln Cys Lys Ala Thr Gly Ser Pro
        675                 680                 685

Thr Pro Arg Ile Thr Trp Leu Lys Gly Gly Arg Pro Leu Ser Leu Thr
    690                 695                 700

Glu Arg His His Phe Thr Pro Gly Asn Gln Leu Leu Val Val Gln Asn
705                 710                 715                 720

Val Met Ile Asp Asp Ala Gly Arg Tyr Thr Cys Glu Met Ser Asn Pro
                725                 730                 735

Leu Gly Thr Glu Arg Ala His Ser Gln Leu Ser Ile Leu Pro Thr Pro
            740                 745                 750

Gly Cys Arg Lys Asp Gly Thr Thr Gly Gly Gly Gly Ser Glu Pro Lys
        755                 760                 765

Ser Ser Asp Lys Thr His Thr Ser Pro Pro Ser Pro Ala Pro Glu Leu
    770                 775                 780

Leu Gly Gly Ser Ser Val Phe Ile Phe Pro Pro Lys Ile Lys Asp Val
785                 790                 795                 800

Leu Met Ile Ser Leu Ser Pro Ile Val Thr Cys Val Val Val Asp Val
                805                 810                 815

Ser Glu Asp Asp Pro Asp Val Gln Ile Ser Trp Phe Val Asn Asn Val
            820                 825                 830

Glu Val His Thr Ala Gln Thr Gln Thr His Arg Glu Asp Tyr Asn Ser
        835                 840                 845

Thr Leu Arg Val Val Ser Ala Leu Pro Ile Gln His Gln Asp Trp Met
    850                 855                 860

Ser Gly Lys Glu Phe Lys Cys Lys Val Asn Asn Lys Asp Leu Pro Ala
865                 870                 875                 880

Pro Ile Glu Arg Thr Ile Ser Lys Pro Lys Gly Ser Val Arg Ala Pro
                885                 890                 895

Gln Val Tyr Val Leu Pro Pro Pro Glu Glu Glu Met Thr Lys Lys Gln
            900                 905                 910

Val Thr Leu Thr Cys Met Val Thr Asp Phe Met Pro Glu Asp Ile Tyr
        915                 920                 925

Val Glu Trp Thr Asn Asn Gly Lys Thr Glu Leu Asn Tyr Lys Asn Thr
    930                 935                 940

Glu Pro Val Leu Asp Ser Asp Gly Ser Tyr Phe Met Tyr Ser Lys Leu
945                 950                 955                 960

Arg Val Glu Lys Lys Asn Trp Val Glu Arg Asn Ser Tyr Ser Cys Ser
                965                 970                 975

Val Val His Glu Gly Leu His Asn His His Thr Thr Lys Ser Phe Ser
            980                 985                 990

Arg Thr Pro Gly Lys
        995


<210>    81
<211>    2991
<212>    DNA
<213>    Artificial Sequence

<220>
<223>    fusion protein


<400>    81
gctcaggctg gacctagggc tccttgcgct gccgcctgca cctgtgcagg cgattctctg        60

gactgcagcg gccggggcct ggccacactg cccagggacc tgccttcctg gaccagatct        120

ctgaacctga gctacaatcg gctgtccgag atcgattctg ccgcctttga ggacctgaca        180

aatctgcagg aggtgtatct gaacagcaat gagctgaccg caatcccctc cctgggagca 240

gcctctatcg gcgtggtgag cctgttcctg cagcacaaca agatcctgag cgtggatggc 300

tcccagctga agagctacct gtctctggag gtgctggacc tgagctccaa caatatcacc 360

gagatcagat ctagctgttt tcctaatggc ctgcggatca gagagctgaa cctggcctct 420

aatcggatca gcatcctgga gtccggcgcc ttcgatggcc tgagcagatc cctgctgaca 480

ctgcgcctgt ccaagaaccg gatcacccag ctgcccgtga aggcctttaa gctgcctagg 540

ctgacacagc tggacctgaa ccggaataga atcaggctga tcgagggcct gaccttccag 600

ggcctggata gcctggaggt gctgcgcctg cagcggaaca atatctcccg cctgacagac 660

ggagcatttt ggggcctgtc taagatgcac gtgctgcacc tggagtacaa tagcctggtg 720

gaggtgaact ctggcagcct gtatggcctg accgccctgc accagctgca cctgtccaac 780

aatagcatca gcagaatcca gagggatggc tggtccttct gccagaagct gcacgagctg 840

atcctgtctt ttaacaatct gaccaggctg gacgaggaga gcctggcaga gctgtcctct 900

ctgtccatcc tgcgcctgtc tcacaatgcc atcagccaca tcgccgaggg cgcctttaag 960

ggcctgaaga gcctgagggt gctggatctg gaccacaacg agatctctgg caccatcgag 1020

gatacaagcg gcgccttcac aggcctggac aatctgtcca agctgaccct gtttggcaac 1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

ggcggtggcg gatccgagcc aaagtcctct gataagacac acacctctcc accatgccca 2340

gcaccagagc tgctgggagg accaagcgtg ttcatcttcc cacccaagat caaggacgtg 2400

ctgatgatct ccctgtcccc catcgtgacc tgcgtggtgg tggacgtgtc cgaggacgac 2460

cccgacgtgc agatcagttg gttcgtgaac aacgtggaag tgcacaccgc ccagacccag 2520

acccacagag aggactacaa ctccaccctg cgggtggtgt ccgccctgcc catccagcac 2580

caggactgga tgtccggcaa agaattcaag tgcaaagtga acaacaagga cctgcctgcc 2640

cccatcgagc ggaccatctc caagcccaag ggctccgtgc gggctcccca ggtgtacgtg 2700

ctgccccctc cagaggaaga gatgaccaag aagcaggtca cactgacctg catggtcacc 2760

gacttcatgc ccgaggacat ctacgtggaa tggaccaaca atggcaagac cgagctgaac 2820

tacaagaaca ccgagcctgt gctggactcc gacggctcct acttcatgta ctccaagctg 2880

cgggtggaaa agaagaactg ggtcgagcgg aactcctact cctgctccgt ggtgcacgag 2940

ggcctgcaca accaccacac caccaagtcc ttctcccgga cccccggcaa a 2991

<210> 82
<211> 2994
<212> DNA
<213> Artificial Sequence

<220>
<223> fusion protein

<400> 82
gctcaggctg gacctagggc tccttgcgct gccgcctgca cctgtgcagg cgattctctg 60

gactgcagcg gccggggcct ggccacactg cccagggacc tgccttcctg gaccagatct 120

ctgaacctga gctacaatcg gctgtccgag atcgattctg ccgcctttga ggacctgaca 180

aatctgcagg aggtgtatct gaacagcaat gagctgaccg caatcccctc cctgggagca 240

gcctctatcg gcgtggtgag cctgttcctg cagcacaaca agatcctgag cgtggatggc 300

tcccagctga agagctacct gtctctggag gtgctggacc tgagctccaa caatatcacc 360

gagatcagat ctagctgttt tcctaatggc ctgcggatca gagagctgaa cctggcctct 420

aatcggatca gcatcctgga gtccggcgcc ttcgatggcc tgagcagatc cctgctgaca 480

ctgcgcctgt ccaagaaccg gatcacccag ctgcccgtga aggcctttaa gctgcctagg 540

ctgacacagc tggacctgaa ccggaataga atcaggctga tcgagggcct gaccttccag 600

ggcctggata gcctggaggt gctgcgcctg cagcggaaca atatctcccg cctgacagac 660

ggagcatttt ggggcctgtc taagatgcac gtgctgcacc tggagtacaa tagcctggtg 720

gaggtgaact ctggcagcct gtatggcctg accgccctgc accagctgca cctgtccaac 780

aatagcatca gcagaatcca gagggatggc tggtccttct gccagaagct gcacgagctg 840

atcctgtctt ttaacaatct gaccaggctg gacgaggaga gcctggcaga gctgtcctct 900

ctgtccatcc tgcgcctgtc tcacaatgcc atcagccaca tcgccgaggg cgcctttaag 960

ggcctgaaga gcctgagggt gctggatctg gaccacaacg agatctctgg caccatcgag 1020

gatacaagcg gcgccttcac aggcctggac aatctgtcca agctgaccct gtttggcaac 1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

ggcggtggcg gatccgagcc tcggggccct accatcaagc cctgcccccc ttgcaagtgc 2340

cctgccccta atctgctggg cggaccctcc gtgttcatct tcccacccaa gatcaaggac 2400

gtgctgatga tctccctgtc ccccatcgtg acctgcgtgg tggtggacgt gtccgaggac 2460

gaccccgacg tgcagatcag ttggttcgtg aacaacgtgg aagtgcacac cgcccagacc 2520

cagacccaca gagaggacta caactccacc ctgcgggtgg tgtccgccct gcccatccag 2580

caccaggact ggatgtccgg caaagaattc aagtgcaaag tgaacaacaa ggacctgcct 2640

gcccccatcg agcggaccat ctccaagccc aagggctccg tgcgggctcc ccaggtgtac 2700

gtgctgcccc ctccagagga agagatgacc aagaagcagg tcacactgac ctgcatggtc 2760

accgacttca tgcccgagga catctacgtg gaatggacca acaatggcaa gaccgagctg 2820

aactacaaga acaccgagcc tgtgctggac tccgacggct cctacttcat gtactccaag 2880

ctgcgggtgg aaaagaagaa ctgggtcgag cggaactcct actcctgctc cgtggtgcac 2940

gagggcctgc acaaccacca caccaccaag tccttctccc ggacccccgg caaa 2994

<210> 83
<211> 3030
<212> DNA
<213> Artificial Sequence

<220>
<223> fusion protein

<400> 83
gctcaggctg gacctagggc tccttgcgct gccgcctgca cctgtgcagg cgattctctg 60

gactgcagcg gccggggcct ggccacactg cccagggacc tgccttcctg gaccagatct 120

ctgaacctga gctacaatcg gctgtccgag atcgattctg ccgcctttga ggacctgaca 180

aatctgcagg aggtgtatct gaacagcaat gagctgaccg caatcccctc cctgggagca 240

gcctctatcg gcgtggtgag cctgttcctg cagcacaaca agatcctgag cgtggatggc 300

tcccagctga agagctacct gtctctggag gtgctggacc tgagctccaa caatatcacc 360

gagatcagat ctagctgttt tcctaatggc ctgcggatca gagagctgaa cctggcctct 420

aatcggatca gcatcctgga gtccggcgcc ttcgatggcc tgagcagatc cctgctgaca 480

ctgcgcctgt ccaagaaccg gatcacccag ctgcccgtga aggcctttaa gctgcctagg 540

ctgacacagc tggacctgaa ccggaataga atcaggctga tcgagggcct gaccttccag 600

ggcctggata gcctggaggt gctgcgcctg cagcggaaca atatctcccg cctgacagac 660

ggagcatttt ggggcctgtc taagatgcac gtgctgcacc tggagtacaa tagcctggtg 720

gaggtgaact ctggcagcct gtatggcctg accgccctgc accagctgca cctgtccaac 780

aatagcatca gcagaatcca gagggatggc tggtccttct gccagaagct gcacgagctg 840

atcctgtctt ttaacaatct gaccaggctg gacgaggaga gcctggcaga gctgtcctct 900

ctgtccatcc tgcgcctgtc tcacaatgcc atcagccaca tcgccgaggg cgcctttaag 960

ggcctgaaga gcctgagggt gctggatctg gaccacaacg agatctctgg caccatcgag 1020

gatacaagcg gcgccttcac aggcctggac aatctgtcca agctgaccct gtttggcaac 1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

ggcggtggcg gatcccgcaa caccggccgc ggcggcgagg agaagaagaa ggagaaggag 2340

aaggaggagc aggaggagcg cgagaccaag acccccgagt gccccagcca cacccagccc 2400

ctgggcgtgt tcatcttccc acccaagatc aaggacgtgc tgatgatctc cctgtccccc 2460

atcgtgacct gcgtggtggt ggacgtgtcc gaggacgacc ccgacgtgca gatcagttgg 2520

ttcgtgaaca acgtggaagt gcacaccgcc cagacccaga cccacagaga ggactacaac 2580

tccaccctgc gggtggtgtc cgccctgccc atccagcacc aggactggat gtccggcaaa 2640

gaattcaagt gcaaagtgaa caacaaggac ctgcctgccc ccatcgagcg gaccatctcc 2700

aagcccaagg gctccgtgcg ggctccccag gtgtacgtgc tgccccctcc agaggaagag 2760

atgaccaaga agcaggtcac actgacctgc atggtcaccg acttcatgcc cgaggacatc 2820

tacgtggaat ggaccaacaa tggcaagacc gagctgaact acaagaacac cgagcctgtg 2880

ctggactccg acggctccta cttcatgtac tccaagctgc gggtggaaaa gaagaactgg 2940

gtcgagcgga actcctactc ctgctccgtg gtgcacgagg gcctgcacaa ccaccacacc 3000

accaagtcct tctcccggac ccccggcaaa 3030

<210> 84
<211> 2991
<212> DNA
<213> Artificial Sequence

<220>
<223> fusion protein

<400> 84
gctcaggctg gacctagggc tccttgcgct gccgcctgca cctgtgcagg cgattctctg 60

gactgcagcg gccggggcct ggccacactg cccagggacc tgccttcctg gaccagatct 120

ctgaacctga gctacaatcg gctgtccgag atcgattctg ccgcctttga ggacctgaca 180

aatctgcagg aggtgtatct gaacagcaat gagctgaccg caatcccctc cctgggagca 240

gcctctatcg gcgtggtgag cctgttcctg cagcacaaca agatcctgag cgtggatggc 300

tcccagctga agagctacct gtctctggag gtgctggacc tgagctccaa caatatcacc 360

gagatcagat ctagctgttt tcctaatggc ctgcggatca gagagctgaa cctggcctct 420

aatcggatca gcatcctgga gtccggcgcc ttcgatggcc tgagcagatc cctgctgaca 480

ctgcgcctgt ccaagaaccg gatcacccag ctgcccgtga aggcctttaa gctgcctagg 540

ctgacacagc tggacctgaa ccggaataga atcaggctga tcgagggcct gaccttccag 600

ggcctggata gcctggaggt gctgcgcctg cagcggaaca atatctcccg cctgacagac 660

ggagcatttt ggggcctgtc taagatgcac gtgctgcacc tggagtacaa tagcctggtg 720

gaggtgaact ctggcagcct gtatggcctg accgccctgc accagctgca cctgtccaac 780

aatagcatca gcagaatcca gagggatggc tggtccttct gccagaagct gcacgagctg 840

atcctgtctt ttaacaatct gaccaggctg gacgaggaga gcctggcaga gctgtcctct 900

ctgtccatcc tgcgcctgtc tcacaatgcc atcagccaca tcgccgaggg cgcctttaag 960

ggcctgaaga gcctgagggt gctggatctg gaccacaacg agatctctgg caccatcgag 1020

gatacaagcg gcgccttcac aggcctggac aatctgtcca agctgaccct gtttggcaac 1080

aagatcaagt ctgtggccaa gcgggccttc tctggcctgg agagcctgga gcacctgaac 1140

ctgggcgaga atgccatcag atccgtgcag ttcgatgcct ttgccaagat gaagaatctg 1200

aaggagctgt acatcagctc cgagagcttc ctgtgcgact gtcagctgaa gtggctgcca 1260

ccttggctga tgggaaggat gctgcaggcc tttgtgaccg ccacatgcgc ccacccagag 1320

agcctgaagg gccagagcat cttctccgtg ctgcccgata gcttcgtgtg cgacgatttt 1380

cctaagccac agatcatcac ccagccagag acaacaatgg ccgtggtggg caaggacatc 1440

cggtttacat gttccgccgc ctctagctcc tctagcccca tgaccttcgc ctggaagaag 1500

gataacgagg tgctggccaa tgccgacatg gagaacttcg cccacgtgag agcccaggat 1560

ggcgaagtga tggagtatac cacaatcctg cacctgcggc acgtgacctt tggccacgag 1620

ggcagatacc agtgcatcat cacaaatcac ttcggctcta cctatagcca caaggccagg 1680

ctgacagtga acgtgctgcc tagctttacc aagatcccac acgacatcgc catcagaaca 1740

ggcaccacag caaggctgga gtgtgcagca accggacacc caaaccctca gatcgcatgg 1800

cagaaggatg gaggcacaga cttccctgca gcccgcgaga ggagaatgca cgtgatgcca 1860

gacgatgacg tgttctttat cacagatgtg aagatcgatg acatgggcgt gtactcctgc 1920

accgcacaga acagcgccgg cagcgtgtcc gccaacgcca ccctgaccgt gctggagaca 1980

ccatccctgg ccgtgcccct ggaggacagg gtggtgaccg tgggcgagac agtggccttt 2040

cagtgtaagg ccaccggctc tccaacacca aggatcacct ggctgaaggg cggcaggccc 2100

ctgagcctga cagagcgcca ccacttcacc cctggcaatc agctgctggt ggtgcagaac 2160

gtgatgatcg atgacgccgg caggtataca tgcgagatga gcaatcctct gggcaccgag 2220

agggcacact cccagctgtc tatcctgcct accccaggct gccggaagga tggcaccaca 2280

ggcggtggcg gatccgaacc gaaatcttct gacaaaaccc acacctctcc gccgtctccg 2340

gctccggaac tgctgggtgg ttcttctgtt ttcatcttcc cacccaagat caaggacgtg 2400

ctgatgatct ccctgtcccc catcgtgacc tgcgtggtgg tggacgtgtc cgaggacgac 2460

cccgacgtgc agatcagttg gttcgtgaac aacgtggaag tgcacaccgc ccagacccag 2520

acccacagag aggactacaa ctccaccctg cgggtggtgt ccgccctgcc catccagcac 2580

caggactgga tgtccggcaa agaattcaag tgcaaagtga acaacaagga cctgcctgcc 2640

cccatcgagc ggaccatctc caagcccaag ggctccgtgc gggctcccca ggtgtacgtg 2700

ctgccccctc cagaggaaga gatgaccaag aagcaggtca cactgacctg catggtcacc 2760

gacttcatgc ccgaggacat ctacgtggaa tggaccaaca atggcaagac cgagctgaac 2820

tacaagaaca ccgagcctgt gctggactcc gacggctcct acttcatgta ctccaagctg 2880

cgggtggaaa agaagaactg ggtcgagcgg aactcctact cctgctccgt ggtgcacgag 2940

ggcctgcaca accaccacac caccaagtcc ttctcccgga cccccggcaa a 2991

<210> 85
<211> 311
<212> PRT
<213> Homo sapiens

<400> 85
Met Gly Val Pro Thr Ala Leu Glu Ala Gly Ser Trp Arg Trp Gly Ser
1               5                   10                  15

Leu Leu Phe Ala Leu Phe Leu Ala Ala Ser Leu Gly Pro Val Ala Ala
            20                  25                  30

Phe Lys Val Ala Thr Pro Tyr Ser Leu Tyr Val Cys Pro Glu Gly Gln
        35                  40                  45

Asn Val Thr Leu Thr Cys Arg Leu Leu Gly Pro Val Asp Lys Gly His
    50                  55                  60

Asp Val Thr Phe Tyr Lys Thr Trp Tyr Arg Ser Ser Arg Gly Glu Val
65                  70                  75                  80

Gln Thr Cys Ser Glu Arg Arg Pro Ile Arg Asn Leu Thr Phe Gln Asp
                85                  90                  95

Leu His Leu His His Gly Gly His Gln Ala Ala Asn Thr Ser His Asp
            100                 105                 110

Leu Ala Gln Arg His Gly Leu Glu Ser Ala Ser Asp His His Gly Asn
        115                 120                 125

Phe Ser Ile Thr Met Arg Asn Leu Thr Leu Leu Asp Ser Gly Leu Tyr
    130                 135                 140

Cys Cys Leu Val Val Glu Ile Arg His His His Ser Glu His Arg Val
145                 150                 155                 160

His Gly Ala Met Glu Leu Gln Val Gln Thr Gly Lys Asp Ala Pro Ser
                165                 170                 175

Asn Cys Val Val Tyr Pro Ser Ser Ser Gln Asp Ser Glu Asn Ile Thr
            180                 185                 190

Ala Ala Ala Leu Ala Thr Gly Ala Cys Ile Val Gly Ile Leu Cys Leu
        195                 200                 205

Pro Leu Ile Leu Leu Leu Val Tyr Lys Gln Arg Gln Ala Ala Ser Asn
    210                 215                 220

Arg Arg Ala Gln Glu Leu Val Arg Met Asp Ser Asn Ile Gln Gly Ile
225                 230                 235                 240

Glu Asn Pro Gly Phe Glu Ala Ser Pro Pro Ala Gln Gly Ile Pro Glu
                245                 250                 255

Ala Lys Val Arg His Pro Leu Ser Tyr Val Ala Gln Arg Gln Pro Ser
            260                 265                 270

Glu Ser Gly Arg His Leu Leu Ser Glu Pro Ser Thr Pro Leu Ser Pro
        275                 280                 285

Pro Gly Pro Gly Asp Val Phe Phe Pro Ser Leu Asp Pro Val Pro Asp
    290                 295                 300

Ser Pro Asn Phe Glu Val Ile
305                 310

<210> 86
<211> 309
<212> PRT
<213> Mus musculus

<400> 86
Met Gly Val Pro Ala Val Pro Glu Ala Ser Ser Pro Arg Trp Gly Thr

1               5                   10                  15

Leu Leu Leu Ala Ile Phe Leu Ala Ala Ser Arg Gly Leu Val Ala Ala
            20                  25                  30

Phe Lys Val Thr Thr Pro Tyr Ser Leu Tyr Val Cys Pro Glu Gly Gln
        35                  40                  45

Asn Ala Thr Leu Thr Cys Arg Ile Leu Gly Pro Val Ser Lys Gly His
    50                  55                  60

Asp Val Thr Ile Tyr Lys Thr Trp Tyr Leu Ser Ser Arg Gly Glu Val
65                  70                  75                  80

Gln Met Cys Lys Glu His Arg Pro Ile Arg Asn Phe Thr Leu Gln His
                85                  90                  95

Leu Gln His His Gly Ser His Leu Lys Ala Asn Ala Ser His Asp Gln
            100                 105                 110

Pro Gln Lys His Gly Leu Glu Leu Ala Ser Asp His His Gly Asn Phe
        115                 120                 125

Ser Ile Thr Leu Arg Asn Val Thr Pro Arg Asp Ser Gly Leu Tyr Cys
    130                 135                 140

Cys Leu Val Ile Glu Leu Lys Asn His His Pro Glu Gln Arg Phe Tyr
145                 150                 155                 160

Gly Ser Met Glu Leu Gln Val Gln Ala Gly Lys Gly Ser Gly Ser Thr
                165                 170                 175

Cys Met Ala Ser Asn Glu Gln Asp Ser Asp Ser Ile Thr Ala Ala Ala
            180                 185                 190

Leu Ala Thr Gly Ala Cys Ile Val Gly Ile Leu Cys Leu Pro Leu Ile
        195                 200                 205

Leu Leu Leu Val Tyr Lys Gln Arg Gln Val Ala Ser His Arg Arg Ala
    210                 215                 220

Gln Glu Leu Val Arg Met Asp Ser Ser Asn Thr Gln Gly Ile Glu Asn
225                 230                 235                 240

Pro Gly Phe Glu Thr Thr Pro Pro Phe Gln Gly Met Pro Glu Ala Lys
                245                 250                 255

Thr Arg Pro Pro Leu Ser Tyr Val Ala Gln Arg Gln Pro Ser Glu Ser
            260                 265                 270

Gly Arg Tyr Leu Leu Ser Asp Pro Ser Thr Pro Leu Ser Pro Pro Gly
        275                 280                 285

Pro Gly Asp Val Phe Phe Pro Ser Leu Asp Pro Val Pro Asp Ser Pro
    290                 295                 300

Asn Ser Glu Ala Ile
305

<210>   87
<211>   4714
<212>   DNA

<213>    Homo sapiens

<400>    87
gggggcgggt gcctggagca cggcgctggg gccgcccgca gcgctcactc gctcgcactc    60

agtcgcggga ggcttccccg cgccggccgc gtcccgcccg ctccccggca ccagaagttc    120

ctctgcgcgt ccgacggcga catgggcgtc cccacggccc tggaggccgg cagctggcgc    180

tggggatccc tgctcttcgc tctcttcctg gctgcgtccc taggtccggt ggcagccttc    240

aaggtcgcca cgccgtattc cctgtatgtc tgtcccgagg ggcagaacgt caccctcacc    300

tgcaggctct tgggccctgt ggacaaaggg cacgatgtga ccttctacaa gacgtggtac    360

cgcagctcga ggggcgaggt gcagacctgc tcagagcgcc ggcccatccg caacctcacg    420

ttccaggacc ttcacctgca ccatggaggc caccaggctg ccaacaccag ccacgacctg    480

gctcagcgcc acgggctgga gtcggcctcc gaccaccatg gcaacttctc catcaccatg    540

cgcaacctga ccctgctgga tagcggcctc tactgctgcc tggtggtgga gatcaggcac    600

caccactcgg agcacagggt ccatggtgcc atgagctgtc aggtgcagac aggcaaagat    660

gcaccatcca actgtgtggt gtacccatcc tcctcccagg atagtgaaaa catcacggct    720

gcagccctgg ctacgggtgc ctgcatcgta ggaatcctct gcctccccct catcctgctc    780

ctggtctaca agcaaaggca ggcagcctcc aaccgccgtg cccaggagct ggtgcggatg    840

gacagcaaca ttcaagggat tgaaaacccc ggctttgaag cctcaccacc tgcccagggg    900

atacccgagg ccaaagtcag gcaccccctg tcctatgtgg cccagcggca gccttctgag    960

tctgggcggc atctgctttc ggagcccagc acccccctgt ctcctccagg ccccggagac    1020

gtcttcttcc catccctgga ccctgtccct gactctccaa actttgaggt catctagccc    1080

agctggggga cagtgggctg ttgtggctgg gtctggggca ggtgcatttg agccagggct    1140

ggctctgtga gtggcctcct tggcctcggc cctggttccc tccctcctgc tctgggctca    1200

gatactgtga catcccagaa gcccagcccc tcaacccctc tggatgctac atggggatgc    1260

tggacggctc agcccctgtt ccaaggattt tggggtgctg agattctccc ctagagacct    1320

gaaattcacc agctacagat gccaaatgac ttacatctta agaagtctca gaacgtccag    1380

cccttcagca gctctcgttc tgagacatga gccttgggat gtggcagcat cagtgggaca    1440

agatggacac tgggccaccc tcccaggcac cagacacagg gcacggtgga gagacttctc    1500

ccccgtggcc gccttggctc ccccgttttg cccgaggctg ctcttctgtc agacttcctc    1560

tttgtaccac agtggctctg gggccaggcc tgcctgccca ctggccatcg ccaccttccc    1620

cagctgcctc ctaccagcag tttctctgaa gatctgtcaa caggttaagt caatctgggg    1680

cttccactgc ctgcattcca gtccccagag cttggtggtc ccgaaacggg aagtacatat    1740

tggggcatgg tggcctccgt gagcaaatgg tgtcttgggc aatctgaggc caggacagat    1800

gttgccccac ccactggaga tggtgctgag ggaggtgggt ggggccttct gggaaggtga 1860

gtggagaggg gcacctgccc cccgccctcc ccatccccta ctcccactgc tcagcgcggg 1920

ccattgcaag ggtgccacac aatgtcttgt ccaccctggg acacttctga gtatgaagcg 1980

ggatgctatt aaaaactaca tggggaaaca ggtgcaaacc ctggagatgg attgtaagag 2040

ccagtttaaa tctgcactct gctgctcctc ccccaccccc accttccact ccatacaatc 2100

tgggcctggt ggagtcttcg cttcagagcc attcggccag gtgcgggtga tgttcccatc 2160

tcctgcttgt gggcatgccc tggctttgtt tttatacaca taggcaaggt gagtcctctg 2220

tggaattgtg attgaaggat tttaaagcag gggaggagag tagggggcat ctctgtacac 2280

tctgggggta aaacagggaa ggcagtgcct gagcatgggg acaggtgagg tggggctggg 2340

cagaccccct gtagcgttta gcaggatggg ggccccaggt actgtggaga gcatagtcca 2400

gcctgggcat ttgtctccta gcagcctaca ctggctctgc tgagctgggc ctgggtgctg 2460

aaagccagga tttggggcta ggcgggaaga tgttcgccca attgcttggg gggttggggg 2520

gatggaaaag gggagcacct ctaggctgcc tggcagcagt gagccctggg cctgtggcta 2580

cagccaggga accccacctg gacacatggc cctgcttcta agccccccag ttaggcccaa 2640

aggaatggtc cactgagggc ctcctgctct gcctgggctg ggccaggggc tttgaggaga 2700

gggtaaacat aggcccggag atggggctga cacctcgagt ggccagaata tgcccaaacc 2760

ccggcttctc ccttgtccct aggcagaggg gggtcccttc ttttgttccc tctggtcacc 2820

acaatgcttg atgccagctg ccataggaag agggtgctgg ctggccatgg tggcacacac 2880

ctgtcctccc agcactttgc agggctgagg tggaaggacc gcttaagccc aggtgttcaa 2940

ggctgctgtg agctgtgttc gagccactac actccagcct ggggacggag caaaactttg 3000

cctcaaaaca aattttaaaa agaaagaaag aaggaaagag ggtatgtttt tcacaattca 3060

tgggggcctg catggcagga gtggggacag gacacctgct gttcctggag tcgaaggaca 3120

agcccacagc ccagattccg gttctcccaa ctcaggaaga gcatgccctg ccctctgggg 3180

aggctggcct ggccccagcc ctcagctgct gaccttgagg cagagacaac ttctaagaat 3240

ttggctgcca gaccccaggc ctggctgctg ctgtgtggag agggaggcgg cccgcagcag 3300

aacagccacc gcacttcctc ctcagcttcc tctggtgcgg ccctgccctc tcttctctgg 3360

acccttttac aactgaacgc atctgggctt cgtggtttcc tgttttcagc gaaatttact 3420

ctgagctccc agttccatct tcatccatgg ccacaggccc tgcctacaac gcactaggga 3480

cgtccctccc tgctgctgct ggggaggggc aggctgctgg agccgccctc tgagttgccc 3540

tgggtgcgag aacatggcgc ctccaggggg cgggaggagc actaggggct ggggcaggag 3600

gctcctggag cgctggattc ctggcacagt ctgaggccct gagagggaaa tccatgcttt 3660

taagaactaa ttcattgtta ggagatcaat caggaattag ggccatcttt acctatctcc 3720

tgacattcac agtttaatag agacttcctg cctttattcc ctcccaggga gaggctgaag 3780

gaatggaatt gaaagcacca tttggagggt tttgctgaca cagcggggac tgctcagcac 3840

tccctaaaaa cacaccatgg aggccactgg tgactgctgg tgggcaggct ggccctgcct 3900

gggggagtcc gtggcgatgg gcgctggggt ggaggtgcag gagccccagg acctgctttt 3960

caaaagactt ctgcctgacc agagctccca ctacatgcag tggcccaggg cagaggggct 4020

gatacatggc ctttttcagg gggtgctcct cgcggggtgg acttgggagt gtgcagtggg 4080

acagggggct gcaggggtcc tgccaccacc gagcaccaac ttggcccctg gggtcctgcc 4140

tcatgaatga ggccttcccc agggctggcc tgactgtgct gggggctggg ttaacgtttt 4200

ctcagggaac cacaatgcac gaaagaggaa ctggggttgc taaccaggat gctgggaaca 4260

aaggcctctt gaagcccagc cacagcccag ctgagcatga ggcccagccc atagacggca 4320

caggccacct ggcccattcc ctgggcattc cctgctttgc attgctgctt ctcttcaccc 4380

catggaggct atgtcaccct aactatcctg gaatgtgttg agagggattc tgaatgatca 4440

atatagcttg gtgagacagt gccgagatag atagccatgt ctgccttggg cacgggagag 4500

ggaagtggca gcatgcatgc tgtttcttgg ccttttctgt tagaatactt ggtgctttcc 4560

aacacacttt cacatgtgtt gtaacttgtt tgatccaccc ccttccctga aaatcctggg 4620

aggttttatt gctgccattt aacacagagg gcaatagagg ttctgaaagg tctgtgtctt 4680

gtcaaaacaa gtaaacggtg gaactacgac taaa 4714

<210> 88
<211> 4795
<212> DNA
<213> Mus musculus

<400> 88
gagcattcac tctagcgagc gagcggcgtg tacagccggc tccctgggct cctggagtcc 60

cgcttgctcc aagcgcactc cagcagtctc tttctgctct tgcccggctc gacggcgaca 120

tgggtgtccc cgcggtccca gaggccagca gcccgcgctg gggaaccctg ctccttgcta 180

ttttcctggc tgcatccaga ggtctggtag cagccttcaa ggtcaccact ccatattctc 240

tctatgtgtg tcccgaggga cagaatgcca ccctcacctg caggattctg ggccccgtgt 300

ccaaagggca cgatgtgacc atctacaaga cgtggtacct cagctcacga ggcgaggtcc 360

agatgtgcaa agaacaccgg cccatacgca acttcacatt gcagcacctt cagcaccacg 420

gaagccacct gaaagccaac gccagccatg accagcccca gaagcatggg ctagagctag 480

cttctgacca ccacggtaac ttctctatca ccctgcgcaa tgtgacccca agggacagcg 540

gcctctactg ctgtctagtg atagaattaa aaaaccacca cccagaacaa cggttctacg 600

ggtccatgga gctacaggta caggcaggca aaggctcggg gtccacatgc atggcgtcta 660

atgagcagga cagtgacagc atcacggctg cggccctggc caccggcgcc tgcatcgtgg 720

gaatcctctg cctccccctt atcctgctgc tggtctataa gcagagacag gtggcctctc 780

accgccgtgc ccaggagttg gtgaggatgg acagcagcaa cacccaagga atcgaaaacc 840

caggcttcga gaccactcca cccttccagg ggatgcctga ggccaagacc aggccgccac 900

tgtcctatgt ggcccagcgg caaccttcgg agtcaggacg gtacctgctc tctgacccca 960

gcacacctct gtcgcctcca ggccctgggg acgtcttttt cccatcccta gatccagtcc 1020

ctgactcccc taactctgaa gccatctaaa ccagctgggg aaccatgaac catggtacct 1080

gggtcaggga tatgtgcact tgatctatgg ctggcccttg gacagtcttt taggcactga 1140

ctccagcttc cttgctcctg ctctgagcct agactctgct tttacaagat gcacagaccc 1200

tcccctatct ctttcagacg ctacttgggg ggcagggaga agatgttgga ttgctcatgg 1260

ctgttctcaa gatcttggga tgctgagttc tccctagaga cttgacttcg acagccacag 1320

atgtcagatg acctgcatcc tatgaacgtc cggcttggca agagcctttc ttcatggaaa 1380

ccagtagccc ggaggggatg aggtaggcac cttgccaccc tcccgggaga gagacacaag 1440

atgtgagaga ctcctgctca ctgtgggggt gtggctggcc tgcttgtttg cctgaggatg 1500

ctcctctgtt ggactgactc tatccccctg gattctggag cttggctggc ctatgtccca 1560

ccagaggagc atctcagcag ccttccacca gcaacctgag ggcctgccag cttcgtggct 1620

ctgggctctc attacctgta tggccgtcca cagagctcag tggccagagg ctttgaaaca 1680

ggaagtacat gtcaggttca ggaaccactg tgagctcatt agtgtcttga gcaatgtgag 1740

gcctggacca gtggacacgg agggagggtg gcgagaggat gatggggatg atgaggggaa 1800

cacgctccct tcctgtcctt gtcatccacc actaccacta ttcagtgtgg agcagtggca 1860

aaggtgaccg acctccacaa tgtcctagtg atgctggacc atttctaagt gtgaaagaga 1920

tgctattaaa aacagtatgt ggcaatggct gccaacagct gagtggactg gaggcactgg 1980

ctttaaggcc ctggaggtgc agggcccggt atggggatag ggatgggagt ttcagtgagg 2040

gcctagggat cactccgctt ctgaccactc ttcttctgag cctcacctca gggtgacctt 2100

caggcacaca gaagagcttg cccctggtcc gatactactc ttggctctca tctccagggt 2160

ttggcatgac ctgggcacac agggggagtc ttcagaaagg attttaaagc atgaaaagaa 2220

agggtagttc ttgtgaggta gggatgggca gctgatgttt gagagtgagg agggatacgg 2280

ctgggcagat cactctccag tctctagagg gaaagtagct ctaagtctgg gagagcagca 2340

gcccagtggt accatatgtc ttcttgcagc ttccactggc tgggctgaac tgggcatggg 2400

taggaaagct cctgttctgg gcctgcagcc agggagaacc ccattcattc cctgaggaca 2460

gatgggtggg gagagaagag agagtttcag gccgggaagc agcaataagc tatctgctgg 2520

ggacccagac aagttgtctg atgaggtcca agatgtggga tgccagttat acctggggct 2580

tggggatcct tagaggcttt gtatcatcat cataggagtg tcggggtggc cagggcatca 2640

aagccatgac cctgttttta tcctcagggt ccactcttct gcaccatcca ttgctctaga 2700

tctatgcagt tactatagac agaatgtgtt gttctgtttg gctttgggga taatggcctg 2760

gcgaactgcc agctgttcag tggcagggct gtgaggccag tcaaagacta gaacccacag 2820

accagctgaa cgatgagtat agcctgtccc ctgggggagc ctgacctgtc tccagcccta 2880

agcttcagac ctcaccactc agatgacttc taagaatttg cctgtgggga cccctgcatg 2940

gctgcagctc cgtggaaagg agaggaggcc cccagcagaa gaaccactcg cttcctgccc 3000

agcttcctcc tgtagggctc taagtctctt cttcttggga ccctgcaagc aaaggcatgt 3060

cagcttggtg gtttcctgtt ttgggtgaag ttttgtgtgg tccgggttct gtctacatcc 3120

atgaacttgg ggtgctacca ccttgctgct gctgtagaga cagctgcagg atcttagggt 3180

ggaaaatgga ggtgccctga ggtgctagcc cttggggcaa aagatggggt ggcaatgaga 3240

cacagtgggg aactgagttc cccaagagga gggaggagcc ctgtagcctc aagggccata 3300

ttgggttcct ggtaccagca aaagcctaga gagcgaagtc tgtattttga ggaggtaatt 3360

gatccttacg gaatccatca gaaatttgga gcgggtgctt tatctatctc tggagggtct 3420

ctacctatct ccgatgaagc tctccctggg cctgggatgg gagaaaccag gaggaaaggt 3480

gtctgataaa gcagggcttt cttgacaagc caaaggccca ctggtagctg ttgtggaccg 3540

agctgaccct gctgaagtat tgtagtgtgc cttggaccaa cttctcaaaa gagcaacccc 3600

ggggctaccc tacttctgcc aggaagaggc ggagaagggg ctgagaggcc tggaaggggc 3660

tagctccttc tttgagaact gctccccgga ggacttggag gaggcggcta ggctacgggc 3720

tgctgagggc cctttgtctt tcctaacctg ggcactgtta ggatgctccc tcctggaaaa 3780

ggctttcctg ggtgtgagct agagcagtgt ccatgccagc gctgaacctg ccatggtggg 3840

agctgaacta aaaatttctc agggaactaa aataggcaaa agaggaactg gggaggaggg 3900

gtgccaggca ggatgggggg aagggagggc agtgcaaaag tctcttgaaa cacagacagc 3960

ccagctgagt gccagtccca gatcacagag aatacggctc atctggctca tgttctgcat 4020

gcttgctgct ttaccctggc actttccttc tccaccatga gtgcgagtcc tgggagtcct 4080

gggagggtga ggattaatgc cagcctgggg agcagatagc tgacagagtc cttgggtaac 4140

tggcttgaac caggacctca ggattccact ctggggatct agctttgtct gggccagtga 4200

agatctctat aatggcatta ttgccagggg ataaacattt cactgggttc tgatctgttg 4260

ggtgtggctt cctggaaaat atggtgagag gaattctgct aaggatacag ttgataagaa 4320

agttctgaga ttgattagta atgcctgcct tggactcagg aagggaagtg gcagtatgaa 4380

tgccatgtct taatcatttt ggttaaaata tgcttcccaa aagatttcca cgtgtgttct 4440

tgtttatttg acatctgtct ccatatcagt cttgaaagcc tttctgtgtg tatatatatg 4500

atgtttgcgt gtatatatgt ttttgtgtgt gcatatggaa gtcagaaatc actgggtgtc 4560

ttcctccatt cctttgcaat gtatgttttt ttttttttta cgatttattt actatatgaa 4620

tgttttgcct gaatacatgc ataggtgtca cgtacatgcc tgctggaacg cttggaactg 4680

gagttacagg tggctatgag ctacagtgtg agcactggga atcaaacctg ggtcttctgc 4740

aagagcaaca aattaaaagt cagctcttaa ctacttgagc tatttttcca actcc 4795

<210> 89
<211> 162
<212> PRT
<213> Homo sapiens

<400> 89
Phe Lys Val Ala Thr Pro Tyr Ser Leu Tyr Val Cys Pro Glu Gly Gln
1               5                   10                  15

Asn Val Thr Leu Thr Cys Arg Leu Leu Gly Pro Val Asp Lys Gly His
            20                  25                  30

Asp Val Thr Phe Tyr Lys Thr Trp Tyr Arg Ser Ser Arg Gly Glu Val
        35                  40                  45

Gln Thr Cys Ser Glu Arg Arg Pro Ile Arg Asn Leu Thr Phe Gln Asp
    50                  55                  60

Leu His Leu His His Gly Gly His Gln Ala Ala Asn Thr Ser His Asp
65                  70                  75                  80

Leu Ala Gln Arg His Gly Leu Glu Ser Ala Ser Asp His His Gly Asn
                85                  90                  95

Phe Ser Ile Thr Met Arg Asn Leu Thr Leu Leu Asp Ser Gly Leu Tyr
            100                 105                 110

Cys Cys Leu Val Val Glu Ile Arg His His His Ser Glu His Arg Val
        115                 120                 125

His Gly Ala Met Glu Leu Gln Val Gln Thr Gly Lys Asp Ala Pro Ser
    130                 135                 140

Asn Cys Val Val Tyr Pro Ser Ser Ser Gln Asp Ser Glu Asn Ile Thr
145                 150                 155                 160

Ala Ala

<210> 90
<211> 159
<212> PRT
<213> Mus musculus

<400> 90
Phe Lys Val Thr Thr Pro Tyr Ser Leu Tyr Val Cys Pro Glu Gly Gln
1               5                   10                  15

Asn Ala Thr Leu Thr Cys Arg Ile Leu Gly Pro Val Ser Lys Gly His
            20                  25                  30

Asp Val Thr Ile Tyr Lys Thr Trp Tyr Leu Ser Ser Arg Gly Glu Val
        35                  40                  45

Gln Met Cys Lys Glu His Arg Pro Ile Arg Asn Phe Thr Leu Gln His
    50                  55                  60

Leu Gln His His Gly Ser His Leu Lys Ala Asn Ala Ser His Asp Gln
65                  70                  75                  80

Pro Gln Lys His Gly Leu Glu Leu Ala Ser Asp His His Gly Asn Phe
                85                  90                  95

Ser Ile Thr Leu Arg Asn Val Thr Pro Arg Asp Ser Gly Leu Tyr Cys
            100                 105                 110

Cys Leu Val Ile Glu Leu Lys Asn His His Pro Glu Gln Arg Phe Tyr
        115                 120                 125

Gly Ser Met Glu Leu Gln Val Gln Ala Gly Lys Gly Ser Gly Ser Thr
    130                 135                 140

Cys Met Ala Ser Asn Glu Gln Asp Ser Asp Ser Ile Thr Ala Ala
145                 150                 155


<210>    91
<211>    95
<212>    PRT
<213>    Homo sapiens

<400>    91
Phe Lys Val Ala Thr Pro Tyr Ser Leu Tyr Val Cys Pro Glu Gly Gln
1               5                   10                  15

Asn Val Thr Leu Thr Cys Arg Leu Leu Gly Pro Val Asp Lys Gly His
            20                  25                  30

Asp Val Thr Phe Tyr Lys Thr Trp Tyr Arg Ser Ser Arg Gly Glu Val
        35                  40                  45

Gln Thr Cys Ser Glu Arg Arg Pro Ile Arg Asn Leu Thr Phe Gln Asp
    50                  55                  60

Leu His Leu His His Gly Gly His Gln Ala Ala Asn Thr Ser His Asp
65                  70                  75                  80

Leu Ala Gln Arg His Gly Leu Glu Ser Ala Ser Asp His His Gly
                85                  90                  95


<210>    92
<211>    158
<212>    PRT
<213>    Homo sapiens

<400>    92
Phe Lys Val Ala Thr Pro Tyr Ser Leu Tyr Val Cys Pro Glu Gly Gln
1               5                   10                  15

Asn Val Thr Leu Thr Cys Arg Leu Leu Gly Pro Val Asp Lys Gly His

20                  25                  30

Asp Val Thr Phe Tyr Lys Thr Trp Tyr Arg Ser Ser Arg Gly Glu Val
            35                  40                  45

Gln Thr Cys Ser Glu Arg Arg Pro Ile Arg Asn Leu Thr Phe Gln Asp
    50                  55                  60

Leu His Leu His His Gly Gly His Gln Ala Ala Asn Thr Ser His Asp
65                  70                  75                  80

Leu Ala Gln Arg His Gly Leu Glu Ser Ala Ser Asp His His Gly Asn
                85                  90                  95

Phe Ser Ile Thr Met Arg Asn Leu Thr Leu Leu Asp Ser Gly Leu Tyr
            100                 105                 110

Cys Cys Leu Val Val Glu Ile Arg His His His Ser Glu His Arg Val
        115                 120                 125

His Gly Ala Met Glu Leu Gln Val Gln Thr Gly Lys Asp Ala Pro Ser
    130                 135                 140

Asn Cys Val Val Tyr Pro Ser Ser Ser Gln Asp Ser Glu Asn
145                 150                 155

<210>    93
<211>    63
<212>    PRT
<213>    Homo sapiens

<400>    93
Asn Phe Ser Ile Thr Met Arg Asn Leu Thr Leu Leu Asp Ser Gly Leu
1               5                   10                  15

Tyr Cys Cys Leu Val Val Glu Ile Arg His His His Ser Glu His Arg
            20                  25                  30

Val His Gly Ala Met Glu Leu Gln Val Gln Thr Gly Lys Asp Ala Pro
        35                  40                  45

Ser Asn Cys Val Val Tyr Pro Ser Ser Ser Gln Asp Ser Glu Asn
    50                  55                  60

<210>    94
<211>    94
<212>    PRT
<213>    Mus musculus

<400>    94
Phe Lys Val Thr Thr Pro Tyr Ser Leu Tyr Val Cys Pro Glu Gly Gln
1               5                   10                  15

Asn Ala Thr Leu Thr Cys Arg Ile Leu Gly Pro Val Ser Lys Gly His
            20                  25                  30

Asp Val Thr Ile Tyr Lys Thr Trp Tyr Leu Ser Ser Arg Gly Glu Val
        35                  40                  45

Gln Met Cys Lys Glu His Arg Pro Ile Arg Asn Phe Thr Leu Gln His
    50                  55                  60

Leu Gln His His Gly Ser His Leu Lys Ala Asn Ala Ser His Asp Gln
65                  70                  75                  80

Pro Gln Lys His Gly Leu Glu Leu Ala Ser Asp His His Gly
                85                  90

<210>   95
<211>   65
<212>   PRT
<213>   Mus musculus

<400>   95
Asn Phe Ser Ile Thr Leu Arg Asn Val Thr Pro Arg Asp Ser Gly Leu
1               5                   10                  15

Tyr Cys Cys Leu Val Ile Glu Leu Lys Asn His His Pro Glu Gln Arg
            20                  25                  30

Phe Tyr Gly Ser Met Glu Leu Gln Val Gln Ala Gly Lys Gly Ser Gly
        35                  40                  45

Ser Thr Cys Met Ala Ser Asn Glu Gln Asp Ser Asp Ser Ile Thr Ala
    50                  55                  60

Ala
65

<210>   96
<211>   46
<212>   PRT
<213>   Artificial Sequence

<220>
<223>   peptide linker

<400>   96
Ala Glu Ala Ala Ala Lys Glu Ala Ala Ala Lys Glu Ala Ala Ala Lys
1               5                   10                  15

Glu Ala Ala Ala Lys Ala Leu Glu Ala Glu Ala Ala Ala Lys Glu Ala
            20                  25                  30

Ala Ala Lys Glu Ala Ala Ala Lys Glu Ala Ala Ala Lys Ala
        35                  40                  45

<210>   97
<211>   5
<212>   PRT
<213>   Artificial Sequence

<220>
<223>   peptide linker

<400>   97

Pro Ala Pro Ala Pro
1               5

<210>   98
<211>   12
<212>   PRT
<213>   Artificial Sequence

<220>
<223>   peptide linker

<400>   98
Ala Glu Ala Ala Ala Lys Glu Ala Ala Ala Lys Ala
1               5                   10

<210>   99
<211>   6
<212>   PRT
<213>   Artificial Sequence

<220>
<223>   peptide linker

<400>   99
Ile Glu Gly Arg Met Asp
1               5

<210>   100
<211>   7
<212>   PRT
<213>   Artificial Sequence

<220>
<223>   peptide linker

<400>   100
Asp Ile Glu Gly Arg Met Asp
1               5